# EUROPEAN PATENT APPLICATION

(11) **EP 1 354 603 A1**
(43) Date of publication of application: **22.10.2003**
(21) Application number: 01271876.3
(22) Date of filing: 25.12.2001
(51) Int. Cl.: A61K 45/06

(54) **CONCOMITANT DRUGS**

(30) Priority: 26.12.2000 JP 2000396220; 02.02.2001 JP 2001027572
(71) Applicant: Takeda Chemical Industries, Ltd., Osaka-shi, Osaka 541-0045 (JP)
(72) Inventor: OHKAWA, Shigenori, Takatsuki-shi, Osaka 569-1121 (JP); NARUO, Kenichi, Sanda-shi, Hyogo 669-1535 (JP); MIWATASHI, Seiji, Ikeda-shi, Osaka 563-0056 (JP)
(74) Representative: Rickard, Timothy Mark Adrian
(86) International application number: JP0111353
(87) International publication number: WO02051442

(57) **Abstract**

The present invention relates to a pharmaceutical agent containing one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor in combination. This combination agent is useful as a prophylactic or therapeutic agent of the diseases such as rheumatism, arthritis and the like, and other diseases.

## Description

### Technical Field

The present invention relates to a combination agent of a p38 MAP kinase inhibitor or a TNF-α production inhibitor.

### Background Art

Cytokines such as TNF-α (tumor necrosis factor-α), IL-1 (interleukin-1) and the like are biological substances, which are produced by a variety of cells such as monocyte or macrophage in response to infection and other cellular stress (Koj, A., Biochim. Biophys. Acta, 1317, 84-94 (1996)). Although these cytokines play important roles in the immune response when they are present at an appropriate amount, it is thought that the overproduction is associated with a variety of inflammatory diseases (Dinarello, C.A., Curr. Opin. Immunol., 3, 941-948 (1991)). p38 MAP kinase which was cloned as a homologue of MAP kinase is involved in the control of production of these cytokines and signal transduction system coupled with receptors, and there is a possibility that the inhibition of p38 MAP kinase provides a drug for treating inflammatory diseases (Stein, B., Anderson, D., Annual Report in Medicinal Chemistry, edited by Bristol, J.A., Academic Press, vol.31, pages 289-298, 1996).

As compounds having a p38 MAP kinase inhibitory activity, imidazole derivatives are described in JP-T 7-50317 (WO 93/14081) and oxazole derivatives are described in JP-T 9-505055 (WO 95/13067), respectively.

On the other hand, as thiazole compounds, the following compounds are known:
1) 1,3-thiazole derivatives represented by the formula: 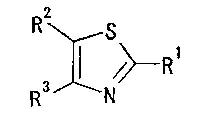 wherein R¹ represents a cycloalkyl group, a cyclic amino group, an amino group optionally having, as substituent(s), 1 or 2 lower alkyl, phenyl, acetyl or lower alkoxycarbonylacetyl, an alkyl group optionally having, as substituent(s), hydroxyl, carboxyl or lower alkoxycarbonyl, or a phenyl group optionally having, as substituent(s), carboxyl, 2-carboxyethenyl or 2-carboxy-1-propenyl, R² represents a pyridyl group optionally having, as substituent(s), lower alkyl, R³ represents a phenyl group optionally having, as substituent(s), lower alkoxy, lower alkyl, hydroxyl, halogen or methylenedioxy, or salts thereof, which have analgesic, antipyretic, anti-inflammatory, anti-ulcerative, thromboxane A₂ (TXA₂) synthase-inhibitory, and platelet coagulation-inhibitory activities (JP-A 60-58981),
2) 1,3-thiazole derivatives represented by the formula: 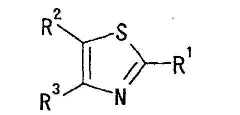 wherein R¹ represents an alkyl group, an alkenyl group, an aryl group, an aralkyl group, a cycloalkyl group, a heterocyclic group employing carbon as an attachment point or an amino group optionally having substituent(s), R² represents a pyridyl group optionally substituted with alkyl group(s), R³ represents a phenyl group optionally having substituent(s), or salts thereof, which have analgesic, antipyretic, anti-inflammatory, anti-ulcerative, TXA₂ synthase-inhibitory, and platelet coagulation-inhibitory activities (JP-A 61-10580),
3) 1,3-thiazole derivatives represented by the formula: 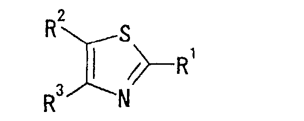 wherein R¹ represents an alkyl group, an alkenyl group, an aryl group, an aralkyl group, a cycloalkyl group, a heterocyclic group employing carbon as an attachment point or an amino group optionally having substituent(s), R² represents a pyridyl group optionally substituted with alkyl group(s), R³ represents an aryl group optionally having substituent(s), or salts thereof, which have analgesic, antipyretic, anti-inflammatory, anti-ulcerative, TXA₂ synthase-inhibitory, and platelet coagulation-inhibitory activities (USP 4,612,321),
4) a compound of the formula 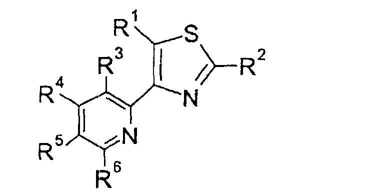 wherein R¹ represents an optionally substituted phenyl, R² represents C₁₋₆ alkyl or (CH₂)ₙAr, n represents 0-2, Ar represents an optionally substituted phenyl, R³ represents a hydrogen or C₁₋₄ alkyl, R⁴ represents a hydrogen, C₁₋₄ alkyl and the like, R⁵ represents a hydrogen or C₁₋₄ alkyl, R⁶ represents a hydrogen, C₁₋₄ alkyl and the like, or a salt thereof, having an inhibitory activity of gastric acid secretion (JP-T 7-503023, WO93/15071),
5) a compound of the formula 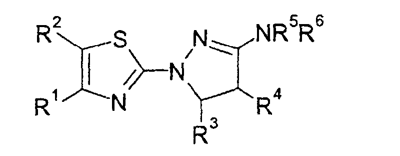 wherein R¹ represents pyridyl and the like, R² represents phenyl and the like, R³ and R⁴ represent a hydrogen or methyl, R⁵ represents methyl and the like, and R⁶ represents a hydrogen, methyl and the like, or a salt thereof, which is an antiinflammatory agent and antiallergic agent (DE-A-3601411),
6) a compound of the formula 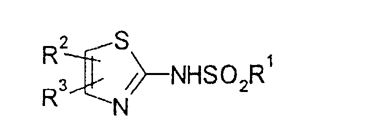 wherein R¹ represents a lower alkyl substituted by halogen, R² represents pyridyl and the like, and R³ represents phenyl and the like, or a salt thereof, having an antiinflammatory, antipyretic, analgesic and antiallergic activity (JP-A-5-70446), and
7) a thiazole compound of the formula 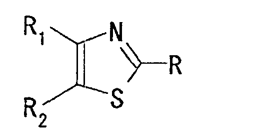
wherein R represents a lower alkyl group; a lower haloalkyl group; a lower hydroxyalkyl group; a lower alkoxy(lower)alkyl group; an aralkyloxy(lower)alkyl group and the like, R¹ represents a cycloalkyl group optionally substituted by lower alkyl group(s) and the like, and R² represents an optionally substituted aryl group and the like, or a pharmaceutically acceptable salt thereof, having a selective inhibitory activity of TNF-α production and/or IFN-γ production (JP-A-11-49762).

WO00/64894 describes that an optionally N-oxidized compound represented by the formula: 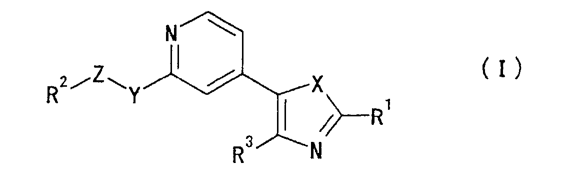 wherein R¹ represents a hydrogen atom, a hydrocarbon group optionally having substituents, a heterocyclic group optionally having substituents, an amino group optionally having substituents or an acyl group,
R² represents an aromatic group optionally having substituents,
R³ represents a hydrogen atom, a pyridyl group optionally having substituents or an aromatic hydrocarbon group optionally having substituents,
X represents an oxygen atom or an optionally oxidized sulfur atom,
Y represents a bond, an oxygen atom, an optionally oxidized sulfur atom or a group represented by the formula: NR⁴ (wherein R⁴ represents a hydrogen atom, a hydrocarbon group optionally having substituents or an acyl group) and
Z represents a bond or a divalent acyclic hydrocarbon group optionally having substituents, or a salt thereof,
has a superior p38 MAP kinase inhibitory activity and TNF-α inhibitory activity and is useful as a prophylactic or therapeutic agent for p38 MAP kinase related diseases and TNF-α related diseases.

Moreover, WO00/63204 describes that a compound of the formula 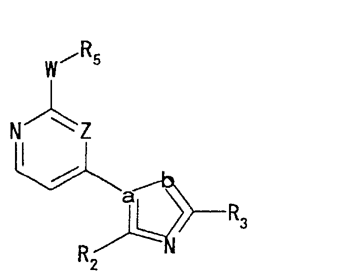 wherein
- a: is N or C;
- b: is CH when a is N, or O when a is C;
- =: denotes a single or a double bond dependent upon whether the azole ring is an imidazole or an oxazole ring;
- Z: is N or CH;
- W: is -NR₆-Y-, -O- or -S-,
where R₆ is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y- is C₁₋₄ alkylene group or a bond;
- R₂: is phenyl group, optionally substituted by one or more substituents selected from the group consisting of a halogen atom, trifluoromethyl, cyano, amido, thioamido, carboxylate, thiocarboxylate, C₁₋₄ alkoxy, C₁₋₄ alkyl, amino, and mono- or di-C₁₋₄ alkylamino;
- R₃: is a hydrogen atom, a halogen atom, C₁₋₁₀ alkyl group, C₁₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂, (each of which is optionally substituted by 1 to 4 substituents selected from C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5 to 7 membered N-heterocyclic group optionally further containing heteroatom(s));
- R₅: is C₆₋₁₈ aryl group, C₃₋₁₈ heteroaryl group or C₃₋₁₂ cycloalkyl group each of which is optionally substituted by 1 to 4 substituents selected from C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5 to 7 membered N-heterocyclic group optionally further containing heteroatom(s), or a salt thereof has a p38 MAP kinase inhibitory activity and is useful as a prophylactic or therapeutic agent of rheumatoid arthritis and the like.

### Disclosure of the Invention

The present invention aims at provision of a combination agent of a p38 MAP kinase inhibitor or a TNF-α production inhibitor.

In view of the above-mentioned problems, the present inventors have conducted intensive studies and found that use of a p38 MAP kinase inhibitor or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor affords effective treatment of diseases such as rheumatism, arthritis and the like. Based on this finding, the present inventors have conducted further studies and completed the present invention.

Accordingly, the present invention relates to
[1] a pharmaceutical agent comprising one or more kinds of a p38 MAP kinase inhibitor and/or a TNF-α production inhibitor and one or more kinds of drugs selected from the group consisting of (1) a non-steroidal antiinflammatory drug (NSAIDs), (2) a disease-modifying anti-rheumatic drug (DMARDs), (3) an anti-cytokine drug, (4) an immunomodulator, (5) a steroid and (6) a c-Jun N-terminal kinase inhibitor in combination,
[2] the pharmaceutical agent of [1], wherein the p38 MAP kinase inhibitor and/or the TNF-α production inhibitor are/is a 1,3--thiazole compound substituted at the 5-position by a pyridyl group optionally having substituents, or a salt thereof or a prodrug thereof,
[3] the pharmaceutical agent of [1], wherein the p38 MAP kinase inhibitor and/or the TNF-α production inhibitor are/is a compound represented by the formula 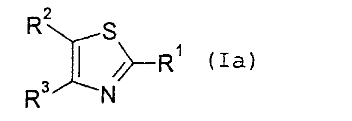 wherein
   - R¹: represents a hydrogen atom, a hydrocarbon group optionally having substituent(s), a heterocyclic group optionally having substituent(s), an amino group optionally having substituent(s) or an acyl group;
   - R²: represents a pyridyl group optionally having substituent(s); and
   - R³: represents an aromatic group optionally having substituent(s), a salt thereof or a prodrug thereof,
[4] the pharmaceutical agent of [1], wherein the p38 MAP kinase inhibitor and/or the TNF-α production inhibitor are/is an optionally N-oxidized compound represented by the formula: 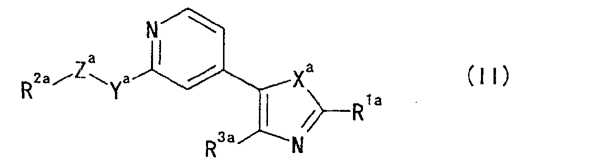
   wherein R^{1a} represents a hydrogen atom, a hydrocarbon group optionally having substituents, a heterocyclic group optionally having substituents, an amino group optionally having substituents or an acyl group,
   R^{2a} represents an aromatic group optionally having substituents,
   R^{3a} represents a hydrogen atom, a pyridyl group optionally having substituents or an aromatic hydrocarbon group optionally having substituents,
   X^{a} represents an oxygen atom or an optionally oxidized sulfur atom,
   Y^{a} represents a bond, an oxygen atom, an optionally oxidized sulfur atom or a group represented by the formula: NR^{4a} (wherein R^{4a} represents a hydrogen atom, a hydrocarbon group optionally having substituents or an acyl group) and
   Z^{a} represents a bond or a divalent acyclic hydrocarbon group optionally having substituents, or a salt thereof, or a prodrug thereof,
[5] the pharmaceutical agent of [1], wherein the p38 MAP kinase inhibitor and/or the TNF-α production inhibitor are/is
   N-[5-(2-benzoylamino-4-pyridyl)-4-(3,5-dimethylphenyl)-1,3-thiazol-2-yl]acetamide,
   N-[5-(2-benzylamino-4-pyridyl)-4-(3,5-dimethylphenyl)-1,3-thiazol-2-yl]acetamide,
   N-[4-[4-(4-methoxyphenyl)-2-methyl-1,3-thiazol-5-yl]-2-pyridyl]benzamide,
   N-[4-[2-(4-fluorophenyl)-4-(3-methylphenyl)-1,3-thiazol-5-yl]-2-pyridyl]phenylacetamide,
   N-[4-[2-ethyl-4-(3-methylphenyl)-1,3-thiazol-5-yl]-2-pyridyl]phenylacetamide,
   N-[4-[4-(3-methylphenyl)-2-propyl-1,3-thiazol-5-yl]-2-pyridyl]phenylacetamide,
   N-[4-[2-butyl-4-(3-methylphenyl)-1,3-thiazol-5-yl]-2-pyridyl]phenylacetamide,
   N-[4-[4-(3-methylphenyl)-2-(4-methylthiophenyl)-1,3-thiazol-5-yl]-2-pyridyl]phenylacetamide,
   N-[4-[2-ethyl-4-(3-methylphenyl)-1,3-thiazol-5-yl]-2-pyridyl]benzamide,
   N-[4-[2-ethyl-4-(3-methylphenyl)-1,3-thiazol-5-yl]-2-pyridyl]-3-phenylpropionamide,
   N-[4-[2-ethyl-4-(3-methylphenyl)-1,3-thiazol-5-yl]-2-pyridyl]-3-(4-methoxyphenyl)propionamide,
   N-[4-[2-ethyl-4-(3-methylphenyl)-1,3-thiazol-5-yl]-2-pyridyl]-4-phenylbutyramide,
   N-[4-[4-(3-methylphenyl)-2-propyl-1,3-thiazol-5-yl]-2-pyridyl]benzamide,
   N-[4-[4-(3-methylphenyl)-2-propyl-1,3-thiazol-5-yl]-2-pyridyl]-3-phenylpropionamide,
   N-[4-[2-butyl-4-(3-methylphenyl)-1,3-thiazol-5-yl]-2-pyridyl]benzamide,
   N-[4-[2-butyl-4-(3-methylphenyl)-1,3-thiazol-5-yl]-2-pyridyl]-3-phenylpropionamide,
   N-[4-[2-(4-fluorophenyl)-4-(3-methylphenyl)-1,3-thiazol-5-yl]-2-pyridyl]benzamide,
   N-[4-[2-(4-fluorophenyl)-4-(3-methylphenyl)-1,3-thiazol-5-yl]-2-pyridyl]-3-phenylpropionamide,
   N-[4-[4-(3-methylphenyl)-2-(4-methylthiophenyl)-1,3-thiazol-5-yl]-2-pyridyl]benzamide,
   N-[4-[4-(3-methylphenyl)-2-(4-methylthiophenyl)-1,3-thiazol-5-yl]-2-pyridyl]-3-phenylpropionamide,
   N-benzyl-N-[4-[2-ethyl-4-(3-methylphenyl)-1,3-thiazol-5-yl]-2-pyridyl]amine,
   N-[4-[2-ethyl-4-(3-methylphenyl)-1,3-thiazol-5-yl]-2-pyridyl]-N-(2-phenylethyl)amine,
   N-[4-[2-ethyl-4-(3-methylphenyl)-1,3-thiazol-5-yl]-2-pyridyl]-N-(3-phenylpropyl)amine,
   N-benzyl-N-[4-[4-(3-methylphenyl)-2-propyl-1,3-thiazol-5-yl]-2-pyridyl]amine,
   N-[4-[4-(3-methylphenyl)-2-propyl-1,3-thiazol-5-yl]-2-pyridyl)-N-(2-phenylethyl)amine,
   N-[4-[4-(3-methylphenyl)-2-propyl-1,3-thiazol-5-yl]-2-pyridyl]-N-(3-phenylpropyl)amine,
   N-benzyl-N-[4-[2-butyl-4-(3-methylphenyl)-1,3-thiazol-5-yl]-2-pyridyl]amine,
   N-[4-[2-butyl-4-(3-methylphenyl)-1,3-thiazol-5-yl]-2-pyridyl]-N-(2-phenylethyl)amine,
   N-[4-[2-butyl-4-(3-methylphenyl)-1,3-thiazol-5-yl]-2-pyridyl]-N-(3-phenylpropyl)amine,
   N-benzyl-N-[4-[4-(3-methylphenyl)-2-(4-methylthiophenyl)-1,3-thiazol-5-yl]-2-pyridyl]amine,
   N-[4-[4-(3-methylphenyl)-2-(4-methylthiophenyl)-1,3-thiazol-5-yl]-2-pyridyl]-N-(2-phenylethyl)amine,
   N-[4-[4-(3-methylphenyl)-2-(4-methylthiophenyl)-1,3-thiazol-5-yl]-2-pyridyl]-N-(3-phenylpropyl)amine,
   N-[4-[4-(3-methylphenyl)-2-(4-methylsulfonylphenyl)-1,3-thiazol-5-yl]-2-pyridyl]benzamide,
   N-[4-[4-(3-methylphenyl)-2-(4-methylsulfonylphenyl)-1,3-thiazol-5-yl]-2-pyridyl]phenylacetamide,
   N-[4-[4-(3-methylphenyl)-2-(4-methylsulfonylphenyl)-1,3-thiazol-5-yl]-2-pyridyl]-3-phenylpropionamide,
   N-benzyl-N-[4-[4-(3-methylphenyl)-2-(4-methylsulfonylphenyl)-1,3-thiazol-5-yl]-2-pyridyl]amine,
   N-[4-[4-(3-methylphenyl)-2-(4-methylsulfonylphenyl)-1,3-thiazol-5-yl]-2-pyridyl]-N-(3-phenylpropyl)amine,
   N-[4-[4-(3-methylphenyl)-2-(4-methylsulfonylphenyl)-1,3-thiazol-5-yl]-2-pyridyl]-N-(2-phenylethyl)amine,
   N-(4-fluorobenzyl)-N-[4-[4-(3-methylphenyl)-2-(4-methylsulfonylphenyl)-1,3-thiazol-5-yl]-2-pyridyl]amine,
   (S)-N-[4-(3-methylphenyl)-5-(2-(1-phenylethylamino)-4-pyridyl)-1,3-thiazol-2-yl]nicotinamide,
   (R)-N-[4-(3-methylphenyl)-5-(2-(1-phenylethylamino)-4-pyridyl)-1,3-thiazol-2-yl]nicotinamide,
   (S)-N-[4-(3-methylphenyl)-5-(2-(1-phenylethylamino)-4-pyridyl)-1,3-thiazol-2-yl]-2-methylnicotinamide,
   (R)-N-[4-(3-methylphenyl)-5-(2-(1-phenylethylamino)-4-pyridyl)-1,3-thiazol-2-yl]-2-methylnicotinamide,
   (S)-N-[4-(3-methylphenyl)-5-(2-(1-phenylethylamino)-4-pyridyl)-1,3-thiazol-2-yl]-2-chloronicotinamide,
   (R)-N-[4-(3-methylphenyl)-5-(2-(1-phenylethylamino)-4-pyridyl)-1,3-thiazol-2-yl]-2-chloronicotinamide,
   (S)-N-[4-(3-methylphenyl)-5-(2-(1-phenylethylamino)-4-pyridyl)-1,3-thiazol-2-yl]-2-methoxynicotinamide,
   (R)-N-[4-(3-methylphenyl)-5-(2-(1-phenylethylamino)-4-pyridyl)-1,3-thiazol-2-yl]-2-methoxynicotinamide,
   N-[5-(2-benzylamino-4-pyridyl)-4-(3-methylphenyl)-1,3-thiazol-2-yl]nicotinamide,
   N-[5-(2-benzylamino-4-pyridyl)-4-(3-methylphenyl)-1,3-thiazol-2-yl]-2-methoxynicotinamide,
   N-[5-(2-benzylamino-4-pyridyl)-4-(3-methylphenyl)-1,3-thiazol-2-yl]-2-chloronicotinamide,
   N-[5-(2-benzylamino-4-pyridyl)-4-(3-methylphenyl)-1,3-thiazol-2-yl]-2-methylnicotinamide,
   N-[5-(2-benzoylamino-4-pyridyl)-4-(3-methylphenyl)-1,3-thiazol-2-yl]nicotinamide,
   N-[5-(2-benzoylamino-4-pyridyl)-4-(3-methylphenyl)-1,3-thiazol-2-yl]-2-methylnicotinamide,
   N-[5-(2-benzoylamino-4-pyridyl)-4-(3-methylphenyl)-1,3-thiazol-2-yl]-2-chloronicotinamide,
   N-[5-(2-benzoylamino-4-pyridyl)-4-(3-methylphenyl)-1,3-thiazol-2-yl]-2-methoxynicotinamide,
   (S)-N-(1-phenylethyl)-4-[2-ethyl-4-(3-methylphenyl)-1,3-thiazol-5-yl]-2-pyridylamine,
   (R)-N-(1-phenylethyl)-4-[2-ethyl-4-(3-methylphenyl)-1,3-thiazol-5-yl]-2-pyridylamine,
   (S)-N-(1-phenylethyl)-4-[4-(3-methylphenyl)-2-propyl-1,3-thiazol-5-yl]-2-pyridylamine,
   (R)-N-(1-phenylethyl)-4-[4-(3-methylphenyl)-2-propyl-1,3-thiazol-5-yl]-2-pyridylamine,
   (S)-N-(1-phenylethyl)-4-[2-butyl-4-(3-methylphenyl)-1,3-thiazol-5-yl]-2-pyridylamine,
   (R)-N-(1-phenylethyl)-4-[2-butyl-4-(3-methylphenyl)-1,3-thiazol-5-yl]-2-pyridylamine,
   (S)-N-(1-phenylethyl)-4-[4-(3-methylphenyl)-2-(4-methylthiophenyl)-1,3-thiazol-5-yl]-2-pyridylamine,
   (R)-N-(1-phenylethyl)-4-[4-(3-methylphenyl)-2-(4-methylthiophenyl)-1,3-thiazol-5-yl]-2-pyridylamine,
   (S)-N-(1-phenylethyl)-4-[4-(3-methylphenyl)-2-(4-methylsulfonylphenyl)-1,3-thiazol-5-yl]-2-pyridylamine,
   (R)-N-(1-phenylethyl)-4-[4-(3-methylphenyl)-2-(4-methylsulfonylphenyl)-1,3-thiazol-5-yl]-2-pyridylamine,
   (S)-N-(1-phenylethyl)-4-[2-(4-fluorophenyl)-4-(3-methylphenyl)-1,3-thiazol-5-yl]-2-pyridylamine,
   (R)-N-(1-phenylethyl)-4-[2-(4-fluorophenyl)-4-(3-methylphenyl)-1,3-thiazol-5-yl]-2-pyridylamine, or a salt thereof,
[6] the pharmaceutical agent of [1], wherein the p38 MAP kinase inhibitor and/or the TNF-α production inhibitor are/is a compound represented by the formula 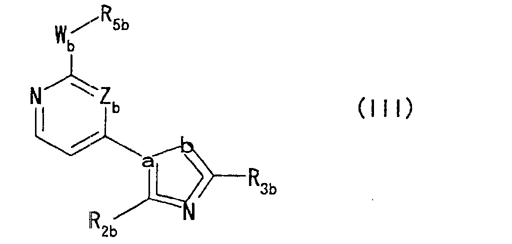 wherein
   - a: is N or C;
   - b: is CH when a is N, or O when a is C;
   - =: denotes a single or a double bond dependent upon whether the azole ring is an imidazole or an oxazole ring;
   - Z_{b}: is N or CH;
   - W_{b}: is -NR_{6b}-Y_{b}-, -O- or -S-,
   where R_{6b} is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y_{b}- is C₁₋₄ alkylene group or a bond;
   - R_{2b}: is phenyl group, optionally substituted by one or more substituents selected from a halogen atom, trifluoromethyl, cyano, amido, thioamido, carboxylate, thiocarboxylate, C₁₋₄ alkoxy, C₁₋₄ alkyl, amino, and mono- or di-C₁₋₄ alkylamino;
   - R_{3b}: is a hydrogen atom, a halogen atom, C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂ (wherein C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group and -CH=N-NH-C(NH)NH₂ are each optionally substituted by 1 to 4 substituents selected from C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5 to 7 membered N-heterocyclic group optionally further containing heteroatom(s)) ;
   - R_{5b}: is C₆₋₁₈ aryl group, C₃₋₁₈ heteroaryl group or C₃₋₁₂ cycloalkyl group each of which is optionally substituted by 1 to 4 substituents selected from C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5 to 7 membered N-heterocyclic group optionally further containing heteroatom(s), or a salt thereof or a prodrug thereof,
[7] the pharmaceutical agent of [1], which is a prophylactic or therapeutic agent of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation,
[8] the pharmaceutical agent of [1], which is a prophylactic or therapeutic agent of chronic rheumatoid arthritis or osteoarthritis,
[9] a method for the prophylaxis or treatment of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation, which comprises administration of an effective amount of one or more kinds of a p38 MAP kinase inhibitor and/or a TNF-α production inhibitor and an effective amount of one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor in combination to a mammal,
[10] a method for the prophylaxis or treatment of chronic rheumatoid arthritis or osteoarthritis, which comprises administration of an effective amount of one or more kinds of a p38 MAP kinase inhibitor and/or a TNF-α production inhibitor and an effective amount of one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor in combination to a mammal,
[11] use of one or more kinds of a p38 MAP kinase inhibitor and/or a TNF-α production inhibitor and one or more kinds of drugs selected from the group consisting of (1) a non-stercidal antiinflammatory drug, (2) a disease-modifying anti-rheumatic drug, (3) an anti-cytokine drug, (4) an immunomodulator, (5) a steroid and (6) a c-Jun N-terminal kinase inhibitor for the production of a prophylactic or therapeutic agent of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation, and
[12] use of one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor for the production of a prophylactic or therapeutic agent of chronic rheumatoid arthritis or osteoarthritis.

The present invention also relates to
[13] a pharmaceutical agent comprising one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid, (6) a c-Jun N-terminal kinase inhibitor, (7) an angiotensin converting enzyme inhibitor, (8) an angiotensin II receptor antagonist, (9) a diuretic drug, (10) a cardiotonic drug, (11) a β receptor antagonist, (12) a Ca sensitizer, (13) a Ca channel antagonist, (14) an anti-platelet drug or anticoagulator and (15) an HMG-CoA reductase inhibitor,
[14] the pharmaceutical agent of [1] or [13], which is a prophylactic or therapeutic agent of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation,
[15] a method for the prophylaxis or treatment of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation, which comprises administration of an effective amount of one or more kinds of a p38 MAP kinase inhibitor and/or a TNF-α production inhibitor and an effective amount of one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid, (6) a c-Jun N-terminal kinase inhibitor, (7) an angiotensin converting enzyme inhibitor, (8) an angiotensin II receptor antagonist, (9) a diuretic drug, (10) a cardiotonic drug, (11) a β receptor antagonist, (12) a Ca sensitizer, (13) a Ca channel antagonist, (14) an anti-platelet drug or anticoagulator and (15) an HMG-CoA reductase inhibitor in combination to a mammal,
[16] use of one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid, (6) a c-Jun N-terminal kinase inhibitor, (7) an angiotensin converting enzyme inhibitor, (8) an angiotensin II receptor antagonist, (9) a diuretic drug, (10) a cardiotonic drug, (11) a β receptor antagonist, (12) a Ca sensitizer, (13) a Ca channel antagonist, (14) an anti-platelet drug or anticoagulator and (15) an HMG-CoA reductase inhibitor for the production of a prophylactic or therapeutic agent of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation, and
[17] a pharmaceutical composition comprising one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid, (6) a c-Jun N-terminal kinase inhibitor, (7) an angiotensin converting enzyme inhibitor, (8) an angiotensin II receptor antagonist, (9) a diuretic drug, (10) a cardiotonic drug, (11) a β receptor antagonist, (12) a Ca sensitizer, (13) a Ca channel antagonist drug, (14) an anti-platelet drug or anticoagulator and (15) an HMG-CoA reductase inhibitor.

While the p38 MAP kinase inhibitor and/or the TNF-α production inhibitor to be used in the present invention are/is not particularly limited as long as the inhibitor(s) has(ve) a p38 MAP kinase inhibitory activity and/or a TNF-α production inhibitory activity, and exemplified by, for example, the following compounds (I)-(III) and the like.

### [compound (I)]

(1) a 1,3-thiazole compound substituted at the 5-position by a pyridyl group optionally having substituent(s) or a salt thereof,
(2) a 1,3-thiazole compound substituted at the 5-position by a pyridyl group optionally having substituent(s) or a salt thereof, excluding a compound of the formula 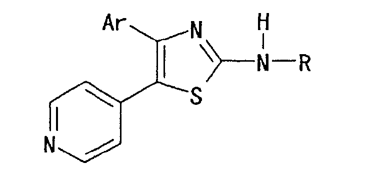 wherein Ar is an unsubstituted or substituted aryl group bonded to a thiazole ring by a carbon atom of an aromatic ring, and R is a hydrogen atom, an acyl group, or a monovalent aromatic group having not more than 10 carbon atoms, which is bonded to a nitrogen atom by a carbon atom of the aromatic ring, and a salt thereof,
(3) the compound of (1) or (2), wherein the 1,3-thiazole compound is a 1,3-thiazole compound substituted at the 4-position by an aromatic group optionally having substituent(s),
(4) the compound of (1) or (2), wherein the 1,3-thiazole compound is a 1,3-thiazole compound substituted at the 2-position by an aryl group optionally having substituent(s) or an amino group optionally having substituent(s),
(5) the compound of (1) or (2), wherein the 1,3-thiazole compound is a compound of the formula 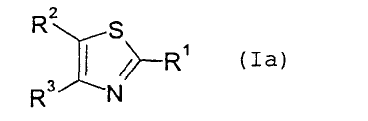
   wherein R¹ represents a hydrogen atom, a hydrocarbon group optionally having substituent(s), a heterocyclic group optionally having substituent(s), an amino group optionally having substituent(s) or an acyl group;
   R² represents a pyridyl group optionally having substituent(s); and
   R³ represents an aromatic group optionally having substituent(s), or a salt thereof,
(6) the compound of (5), wherein R¹ is
   (i) a hydrogen atom,
   (ii) a C₁₋₁₀ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group [these groups may have substituent(s) selected from the group (substituent group A) consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋ ₁₆ aralkyloxy-carbonyl, 5 or 6 membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5 or 6 membered heterocyclic carbamoyl, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5 to 7 membered saturated cyclic amino optionally having 1 to 4 of one or two kinds of heteroatom(s) selected from a nitrogen atom, a sulfur atom and an oxygen atom in addition to one nitrogen atom and carbon atoms (this cyclic amino may have substituents selected from the group consisting of C₁₋₆ alkyl, C₆₋₁₄ aryl, C₁₋₆ alkyl-carbonyl, 5 to 10 membered aromatic heterocyclic group and oxo), 5 to 10 membered aromatic heterocyclic group containing 1 to 4 of one or two kinds of heteroatom(s) selected from a nitrogen atom, a sulfur atom and an oxygen atom, in addition to carbon atoms, sulfo, sulfamoyl, sulfinamoyl and sulfenamoyl],
   (iii) a monovalent heterocyclic group obtained by removing one arbitrary hydrogen atom from a 5 to 14 membered heterocycle containing 1 to 4 of one or two kinds of heteroatom(s) selected from a nitrogen atom, a sulfur atom and an oxygen atom in addition to carbon atoms optionally having substituents selected from the above-mentioned substituent group A,
   (iv) an acyl group represented by the formula:

      -(C=O)-R⁵, -(C=O)-OR⁵, -(C=O)-NR⁵R⁶, -(C=S)-NHR⁵ or -SO₂-R⁷

      wherein R⁵ represents (a) a hydrogen atom, (b) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group as defined in the above (ii) or (c) a heterocyclic group as defined in the above (iii), R⁶ represents a hydrogen atom or a C₁₋₆ alkyl group, R⁷ represents (a) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group as defined in the above (ii), or (b) a heterocyclic group as defined in the above (iii),
   (v) an amino group (this amino group may have substituent(s) selected from the group consisting of (a) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group as defined in the above (ii), (b) a heterocyclic group as defined in the above (iii), (c) an acyl group as defined in the above (iv), and (d) a C₁₋₆ alkylidene group optionally having substituent(s) selected from the above substituent group A), or
   (vi) a 5 to 7 membered non-aromatic cyclic amino group optionally containing 1 to 4 of one or two kinds of heteroatom(s) selected from a nitrogen atom, a sulfur atom and an oxygen atom in addition to one nitrogen atom and carbon atoms (this cyclic amino group may have substituent(s) selected from the group consisting of C₁₋₆ alkyl, C₆₋₁₄ aryl, C₁₋₆ alkyl-carbonyl, 5 to 10 membered aromatic heterocyclic group and oxo);
   R² represents a pyridyl group optionally having substituent(s) selected from the above substituent group A; and
   R³ represents (a) a C₆₋₁₄ monocyclic or fused polycyclic aromatic hydrocarbon group optionally having substituents selected from the substituent group A or (b) a monovalent aromatic heterocyclic group obtained by removing one arbitrary hydrogen atom from a 5 to 14 membered aromatic heterocycle containing 1 to 4 of one or two kinds of heteroatom(s) selected from a nitrogen atom, a sulfur atom and an oxygen atom in addition to carbon atoms, said 5 to 14 membered aromatic heterocycle optionally having substituent(s) selected from the substituent group A,
(7) the compound of (5), wherein
   R¹ is (a) a C₆₋₁₄ aryl group (preferably C₆₋₁₀ aryl) optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl, C₆₋₁₄ aryl-carbonylamino, C₁₋₃ alkylenedioxy, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and nitro,
      (b) a C₁₋₈ alkyl group optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
      (c) a C₃₋₆ cycloalkyl group (e.g., cyclohexyl) optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
      (d) a C₇₋₁₆ aralkyl group (e.g., phenyl-C₁₋₆ alkyl group),
      (e) a 5 to 10 membered aromatic heterocyclic group containing 1 to 4 of one or two kinds of heteroatom(s) selected from a nitrogen atom, a sulfur atom and an oxygen atom in addition to carbon atoms (e.g., 5 or 6 membered aromatic heterocyclic group such as pyridyl, thienyl and the like),
      (f) a 5 to 10 membered non-aromatic heterocyclic group containing 1 or 2 of one or two kinds of heteroatom(s) selected from a nitrogen atom, a sulfur atom and an oxygen atom in addition to carbon atoms, said 5 to 10 membered non-aromatic heterocyclic group may have C₆₋₁₄ aryl (e.g., phenyl), C₁₋₆ alkyl-carbonyl or oxo (e.g., 5 or 6 membered non-aromatic cyclic amino group such as piperidino, piperazino and the like),
      (g) an amino group optionally having 1 or 2 substituent(s) selected from the group consisting of the following (1) to (7) [(1) C₁₋₆ alkyl, (2) C₆₋₁₄ aryl, (3) C₇₋₁₆ aralkyl, (4) 5 or 6 membered heterocyclic group containing 1 or 2 heteroatom(s) selected from a nitrogen atom, a sulfur atom and an oxygen atom in addition to carbon atoms (e.g., pyridyl), (5) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl group, each optionally having 1 to 3 substituent(s) selected from halogen atom, C₁₋₆ alkyl, C₁₋₆ alkoxy, carboxy, C₁₋₆ alkoxy-carbonyl, cyano, tetrazine and the like, (6) C₆₋₁₄ aryl-carbamoyl group optionally having 1 to 3 substituent(s) selected from halogen atom, C₁₋₆ alkyl, C₁₋₆ alkoxy, carboxy, C₁₋₆ alkoxy-carbonyl, cyano, nitro, mono- or di-C₁₋₆ alkylamino and the like and (7) di-C₁₋₆ alkylamino-C₁₋₆ alkylidene], or
      (h) a carboxy group,
(8) the compound of (5), wherein R¹ is a C₆₋₁₄ aryl group optionally having C₁₋₆ alkylsulfonyl,
(9) the compound of (5), wherein R² is a 4-pyridyl group optionally having substituent(s),
(10) the compound of (5), wherein R³ is a C₆₋₁₀ aryl group optionally having substituent(s),
(11) the compound of (5), wherein R³ is a phenyl group optionally having substituent(s),
(12) the compound of (5), wherein R³ is a C₆₋₁₄ aryl group optionally having substituent(s) selected from the group consisting of halogen atom, C₁₋₃ alkylenedioxy, optionally halogenated C₁₋₆ alkyl, carboxy C₂₋₆ alkenyl, optionally halogenated C₁₋₈ alkoxy, carboxy C₁₋₈ alkoxy, hydroxy, C₆₋₁₄ aryloxy, C₁₋₆ alkoxy-carbonyl, C₁₋₆ alkyl-carbonyloxy, mono- or di-C₁₋₆ alkylamino and C₁₋₆ alkoxy-carbonyl-C₁₋₆ alkoxy,
(13) the compound of (5), wherein R³ is a phenyl group optionally having substituent(s) selected from the group consisting of halogen atom and C₁₋₆ alkyl group,
(14) the compound of (5), wherein R¹ is (a) an amino group optionally having 1 or 2 acyl groups represented by the formula: -(C=O)-R⁵ or -(C=O)-NR⁵R⁶ wherein each symbol is as defined above, (b) C₆₋₁₄ aryl group optionally having 1 to 5 substituent(s) selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy or (c) C₁₋₆ alkyl group optionally substituted by halogen atom,
   R² is a pyridyl group, and
   R³ is a C₆₋₁₄ aryl group optionally having 1 to 5 substituent(s) selected from halogen atom, optionally halogenated C₁₋₆ alkyl, optionally halogenated C₁₋₆ alkoxy and carboxy,
(15) the compound of (5), wherein R¹ is
   (i) C₁₋₈ alkyl, C₃₋₆ cycloalkyl or C₆₋₁₄ aryl, each optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
   (ii) a 5 membered heterocyclic group,
   (iii) an amino group optionally having 1 or 2 substituent(s) selected from (a) C₁₋₆ alkyl, (b) C₆₋₁₄ aryl, (c) C₇₋₁₆ aralkyl, (d) 6 membered heterocyclic group and (e) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl, each optionally having 1 to 3 substituent(s) selected from halogen atom, C₁₋₆ alkyl, C₁₋₆ alkoxy, carboxy and C₁₋₆ alkoxy-carbonyl, or an amino group optionally having di-C₁₋₆ alkylamino-C₁₋₆ alkylidene,
   (iv) a 5 or 6 membered non-aromatic cyclic amino group optionally substituted by C₁₋₆ alkyl-carbonyl or oxo, or
   (v) a carboxy group;
   R² is a pyridyl group; and
   R³ is a C₆₋₁₀ aryl group optionally having 1 to 3 substituent(s) selected from halogen atom, C₁₋₃ alkylenedioxy, optionally halogenated C₁₋₆ alkyl, carboxy C₂₋₆ alkenyl, optionally halogenated C₁₋₈ alkoxy, hydroxy, C₇₋₁₆ aralkyloxy and C₁₋₆ alkyl-carbonyloxy (two adjacent alkyl groups as substituents may be bonded to form a 5 membered non-aromatic carbon ring),
(16) the compound of (5), wherein R¹ is a C₆₋₁₄ aryl group optionally having C₁₋₆ alkylsulfonyl, R² is a pyridyl group, and R³ is a C₆₋₁₄ aryl group optionally having halogen atom(s),
(17) N-ethyl-[4-(4-methoxyphenyl)-5-(4-pyridyl)-1,3-thiazol-2-yl]amine (Reference Example A 23-269),
   N-propyl-[4-(3,5-dimethylphenyl)-5-(4-pyridyl)-1,3-thiazol-2-yl]amine (Reference Example A 23-276),
   N-butyl-[4-(3,5-dimethylphenyl)-5-(4-pyridyl)-1,3-thiazol-2-yl]amine (Reference Example A 23-280),
   N-benzyl-[4-(3,5-dimethylphenyl)-5-(4-pyridyl)-1,3-thiazol-2-yl]amine (Reference Example A 23-281),
   N-propyl-[4-(4-methoxyphenyl)-5-(4-pyridyl)-1,3-thiazol-2-yl]amine (Reference Example A 23-290),
   N-isopropyl-[4-(4-methoxyphenyl)-5-(4-pyridyl)-1,3-thiazol-2-yl]amine (Reference Example A 23-291),
   N-[4-(4-methoxyphenyl)-5-(4-pyridyl)-1,3-thiazol-2-yl]-N'-phenylurea (Reference Example A 23-296),
   4-[[[4-(4-methoxyphenyl)-5-(4-pyridyl)-1,3-thiazol-2-yl]amino]carbonyl]benzoic acid (Reference Example A 23-299),
   methyl 4-[2-[4-(methylthio)phenyl]-5-(4-pyridyl)-1,3-thiazol-4-yl]phenyl ether (Reference Example A 23-300),
   4-[4-(3,5-dimethylphenyl)-5-(4-pyridyl)-1,3-thiazol-2-yl]phenyl methyl sulfide (Reference Example A 23-302),
   4-[4-(4-methoxyphenyl)-5-(4-pyridyl)-1,3-thiazol-2-yl]phenyl methyl sulfoxide (Reference Example A 23-303),
   4-[4-(3,5-dimethylphenyl)-5-(4-pyridyl)-1,3-thiazol-2-yl]phenyl methyl sulfoxide (Reference Example A 23-305),
   4-[4-(4-methoxyphenyl)-5-(4-pyridyl)-1,3-thiazol-2-yl]phenyl methyl sulfone (Reference Example A 23-306),
   4-[4-(3,5-dimethylphenyl)-5-(4-pyridyl)-1,3-thiazol-2-yl]phenyl methyl sulfone (Reference Example A 23-308),
   4-[4-(4-fluorophenyl)-5-(4-pyridyl)-1,3-thiazol-2-yl]phenyl methyl sulfide (Reference Example A 23-309),
   4-[4-(4-chlorophenyl)-5-(4-pyridyl)-1,3-thiazol-2-yl]phenyl methyl sulfide (Reference Example A 23-310),
   4-[4-(4-fluorophenyl)-5-(4-pyridyl)-1,3-thiazol-2-yl]phenyl methyl sulfoxide (Reference Example A 23-311),
   4-[4-(4-chlorophenyl)-5-(4-pyridyl)-1,3-thiazol-2-yl]phenyl methyl sulfoxide (Reference Example A 23-312),
   4-[4-(4-fluorophenyl)-5-(4-pyridyl)-1,3-thiazol-2-yl]phenyl methyl sulfone (Reference Example A 23-313),
   4-[4-(4-chlorophenyl)-5-(4-pyridyl)-1,3-thiazol-2-yl]phenyl methyl sulfone (Reference Example A 23-314),
   N-[4-(3,5-dimethylphenyl)-5-(4-pyridyl)-1,3-thiazol-2-yl]-N'-phenylurea (Reference Example A 23-315),
   2-hydroxy-N-[4-(4-methoxyphenyl)-5-(4-pyridyl)-1,3-thiazol-2-yl]propionamide (Reference Example A 23-325),
   4-[4-(3,4-dimethylphenyl)-5-(4-pyridyl)-1,3-thiazol-2-yl]phenyl methyl sulfide (Reference Example A 23-326),
   4-[4-(3,4-dimethylphenyl)-5-(4-pyridyl)-1,3-thiazol-2-yl]phenyl methyl sulfoxide (Reference Example A 23-327),
   4-[4-(3,4-dimethylphenyl)-5-(4-pyridyl)-1,3-thiazol-2-yl]phenyl methyl sulfone (Reference Example A 23-328),
   2-hydroxy-N-[4-(4-methoxyphenyl)-5-(4-pyridyl)-1,3-thiazol-2-yl]acetamide (Reference Example A 23-329),
   4-[[[4-(3,5-dimethylphenyl)-5-(4-pyridyl)-1,3-thiazol-2-yl]amino]carbonyl]benzoic acid (Reference Example A 23-337),
   3-[[[4-(3,5-dimethylphenyl)-5-(4-pyridyl)-1,3-thiazol-2-yl]amino]carbonyl]benzoic acid (Reference Example A 23-342),
   4-(4-fluorophenyl)-2-phenyl-5-(4-pyridyl)-1,3-thiazole (Reference Example A 44-1),
   methyl 4-[4-(3-methylphenyl)-5-(4-pyridyl)-1,3-thiazol-2-yl]phenyl sulfide (Reference Example A 44-7),
   methyl 4-[4-(3-methylphenyl)-5-(4-pyridyl)-1,3-thiazol-2-yl]phenyl sulfoxide (Reference Example A 44-8),
   methyl 4-[4-(3-methylphenyl)-5-(4-pyridyl)-1,3-thiazol-2-yl]phenyl sulfone (Reference Example A 44-26), or a salt thereof,

As "acyl group", for example, there are an acyl group represented by the formula:

-(C=O)-R⁵, -(C=O)-OR⁵, -(C=O)-NR⁵R⁶, -(C=S)-NHR⁵ or -SO₂-R⁷

(wherein R⁵ represents a hydrogen atom, a hydrocarbon group optionally having substituent(s) or a heterocyclic group optionally having substituent(s), R⁶ represents a hydrogen atom or a C₁₋₆ alkyl, R⁷ represents a hydrocarbon group optionally having substituent(s) or a heterocyclic group optionally having substituent(s)) and the like.

In the aforementioned formula, as "hydrocarbon group" of "hydrocarbon group optionally having substituent(s)" represented by R⁵, for example, there are an acyclic or cyclic hydrocarbon group (for example, alkyl, alkenyl, alkynyl, cycloalkyl, aryl, aralkyl and the like) and the like. Among them, acyclic or cyclic hydrocarbon groups having 1 to 16 carbon atom(s) are preferable.

As "alkyl", for example, C₁₋₆ alkyl (for example, methyl, ethyl, propyl, isopropyl, butyl, isobutyl, sec-butyl, tert-butyl, pentyl, hexyl and the like) and the like are preferable.

As "alkenyl", for example, C₂₋₆ alkenyl (for example, vinyl., allyl, isopropenyl, 1-butenyl, 2-butenyl, 3-butenyl, 2-methyl-2-propenyl, 1-methyl-2-propenyl, 2-methyl-1-propenyl and the like) and the like are preferable.

As "alkynyl", for example, C₂₋₆ alkynyl (for example, ethynyl, propargyl, 1-butynyl, 2-butynyl, 3-butynyl, 1-hexynyl and the like) and the like are preferable.

As "cycloalkyl", for example, C₃₋₆ cycloalkyl (for example, cyclopropyl, cyclobutyl, cyclopentyl, cyclohexyl and the like) and the like are preferable.

As "aryl", for example, C₆₋₁₄ aryl (for example, phenyl, 1-naphthyl, 2-naphthyl, 2-biphenylyl, 3-biphenylyl, 4-biphenylyl, 2-anthryl and the like) and the like are preferable.

As "aralkyl", for example, C₇₋₁₆ aralkyl (for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl and the like) and the like are preferable.

As "substituent(s)" of "hydrocarbon group optionally having substituent(s)" represented by R⁵, for example, there are oxo, halogen atom (for example, fluorine, chlorine, bromine, iodine and the like), C₁₋₃ alkylenedioxy (for example, methylenedioxy, ethylenedioxy and the like), nitro, cyano, optionally halogenated C₁₋₆ alkyl, optionally halogenated C₂₋₆ alkenyl, carboxy C₂₋₆ alkenyl (for example, 2-carboxyethenyl, 2-carboxy-2-methylethenyl and the like), optionally halogenated C₂₋₆ alkynyl, optionally halogenated C₃₋₆ cycloalkyl, C₆₋₁₄ aryl (for example, phenyl, 1-naphthyl, 2-naphthyl, 2-biphenylyl, 3-biphenylyl, 4-biphenylyl, 2-anthryl and the like), optionally halogenated C₁₋₈ alkoxy, C₁₋₆ alkoxy-carbonyl-C₁₋₆ alkoxy (for example, ethoxycarbonylmethyloxy and the like), hydroxy, C₆₋₁₄ aryloxy (for example, phenyloxy, 1-naphthyloxy, 2-naphthyloxy and the like), C₇₋₁₆ aralkyloxy (for example, benzyloxy, phenethyloxy and the like), mercapto, optionally halogenated C₁₋₆ alkylthio, C₆₋₁₄ arylthio (for example, phenylthio, 1-naphthylthio, 2-naphthylthio and the like), C₇₋₁₆ aralkylthio (for example, benzylthio, phenethylthio and the like), amino, mono-C₁₋₆ alkylamino (for example, methylamino, ethylamino and the like), mono-C₆₋₁₄ arylamino (for example, phenylamino, 1-naphthylamino, 2-naphthylamino and the like), di-C₁₋₆ alkylamino (for example, dimethylamino, diethylamino, ethylmethylamino and the like), di-C₆₋₁₄ arylamino (for example, diphenylamino and the like), formyl, carboxy, carboxy-C₂₋₆ alkenyl, carboxy-C₁₋₆ alkyl, C₁₋₆ alkyl-carbonyl (for example, acetyl, propionyl and the like), C₃₋₆ cycloalkyl-carbonyl (for example, cyclopropylcarbonyl, cyclopentylcarbonyl, cyclohexylcarbonyl and the like), C₁₋₆ alkoxy-carbonyl (for example, methoxycarbonyl, ethoxycarbonyl, propoxycarbonyl, tert-butoxycarbonyl and the like), C₆₋₁₄ aryl-carbonyl (for example, benzoyl, 1-naphthoyl, 2-naphthoyl and the like), C₇₋₁₆ aralkyl-carbonyl (for example, phenylacetyl, 3-phenylpropionyl and the like), C₆₋₁₄ aryloxy-carbonyl (for example, phenoxycarbonyl and the like), C₇₋₁₆ aralkyloxy-carbonyl (for example, benzyloxycarbonyl, phenethyloxycarbonyl and the like), 5 or 6 membered heterocyclic carbonyl (for example, nicotinoyl, isonicotinoyl, thenoyl, furoyl, morpholinocarbonyl, thiomorpholinocarbonyl, piperazin-1-ylcarbonyl, pyrrolidin-1-ylcarbonyl and the like), carbamoyl, thiocarbamoyl, mono-C₁₋₆ alkyl-carbamoyl (for example, methylcarbamoyl, ethylcarbamoyl and the like), di-C₁₋₆ alkyl-carbamoyl (for example, dimethylcarbamoyl, diethylcarbamoyl, ethylmethylcarbamoyl and the like), mono- or di-C₆₋₁₄ aryl-carbamoyl (for example, phenylcarbamoyl, 1-naphthylcarbamoyl, 2-naphthylcarbamoyl and the like), mono- or di-5 or 6 membered heterocyclic carbamoyl (for example, 2-pyridylcarbamoyl, 3-pyridylcarbamoyl, 4-pyridylcarbamoyl, 2-thienylcarbamoyl, 3-thienylcarbamoyl and the like), C₁₋₆ alkylsulfonyl (for example, methylsulfonyl, ethylsulfonyl and the like), C₁₋₆ alkylsulfinyl (for example, methyl.sulfinyl, ethylsulfinyl and the like), C₆₋₁₄ arylsulfonyl (for example, phenylsulfonyl, 1-naphthylsulfonyl, 2-naphthylsulfonyl and the like), C₆₋₁₄ arylsulfinyl (for example, phenylsulfinyl, 1-naphthylsulfinyl, 2-naphthylsulfinyl and the like), formylamino, C₁₋₆ alkyl-carbonylamino (for example, acetylamino and the like), C₆₋₁₄ aryl-carbonylamino (for example, benzoylamino, naphthoylamino and the like), C₁₋₆ alkoxy-carbonylamino (for example, methoxycarbonylamino, ethoxycarbonylamino, propoxycarbonylamino, butoxycarbonylamino and the like), C₁₋₆ alkylsulfonylamino (for example, methylsulfonylamino, ethylsulfonylamino and the like), C₆₋₁₄ arylsulfonylamino (for example, phenylsulfonylamino, 2-naphthylsulfonylamino, 1-naphthylsulfonylamino and the like), C₁₋₆ alkyl-carbonyloxy (for example, acetoxy, propionyloxy and the like), C₆₋₁₄ aryl-carbonyloxy (for example, benzoyloxy, naphthylcarbonyloxy and the like), C₁₋₆ alkoxy-carbonyloxy (for example, methoxycarbonyloxy, ethoxycarbonyloxy, propoxycarbonyloxy, butoxycarbonyloxy and the like), mono-C₁₋₆ alkyl-carbamoyloxy (for example, methylcarbamoyloxy, ethylcarbamoyloxy and the like), di-C₁₋₆ alkyl-carbamoyloxy (for example, dimethylcarbamoyloxy, diethylcarbamoyloxy and the like), C₆₋₁₄ aryl-carbamoyloxy (for example, phenylcarbamoyloxy, naphthylcarbamoyloxy and the like), nicotinoyloxy, 5 to 7 membered saturated cyclic amino optionally having substituent(s), 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), sulfo and the like.

The "hydrocarbon group" may have 1 to 5, preferably 1 to 3 aforementioned substituent(s) at a substitutable position and, when the number of substituents is 2 or more, respective substituents may be the same or different.

As aforementioned "optionally halogenated C₁₋₆ alkyl", for example, there are C₁₋₆ alkyl (for example, methyl, ethyl, propyl, isopropyl, butyl, isobutyl, sec-butyl, tert-butyl, pentyl, hexyl and the like) and the like optionally having 1 to 5, preferably 1 to 3 halogen atom(s) (for example, fluorine, chlorine, bromine, iodine and the like). Examples thereof are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the aforementioned "optionally halogenated C₂₋₆ alkenyl", for example, there are C₂₋₆ alkenyl (for example, vinyl, propenyl, isopropenyl, 2-buten-1-yl, 4-penten-1-yl, 5-hexen-1-yl) and the like optionally having 1 to 5, preferably 1 to 3 halogen atom(s) (for example, fluorine, chlorine, bromine, iodine and the like).

As the aforementioned "optionally halogenated C₂₋₆ alkynyl", there are C₂₋₆ alkynyl (for example, 2-butyn-1-yl, 4-pentyn-1-yl, 5-hexyn-1-yl and the like) and the like optionally having 1 to 5, preferably 1 to 3 halogen atom(s) (for example, fluorine, chlorine, bromine, iodine and the like).

As the aforementioned "optionally halogenated C₃₋₆ cycloalkyl", for example, there are C₃₋₆ cycloalkyl (for example, cyclopropyl, cyclobutyl, cyclopentyl, cyclohexyl and the like) and the like optionally having 1 to 5, preferably 1 to 3 halogen atom(s) (for example, fluorine, chlorine, bromine, iodine and the like). Examples thereof are cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

As the aforementioned "optionally halogenated C₁₋₈ alkoxy", for example, there are C₁₋₈ alkoxy (for example, methoxy, ethoxy, propoxy, isopropoxy, butoxy, isobutoxy, sec-butoxy, pentyloxy, hexyloxy and the like) and the like optionally having 1 to 5, preferably 1 to 3 halogen atom(s) (for example, fluorine, chlorine, bromine, iodine and the like). Examples thereof are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

As the aforementioned "optionally halogenated C₁₋₆ alkylthio", for example, there are C₁₋₆ alkylthio (for example, methylthio, ethylthio, propylthio, isopropylthio, butylthio, sec-butylthio, tert-butylthio and the like) and the like optionally having 1 to 5, preferably 1 to 3 halogen atom(s) (for example, fluorine, chlorine, bromine, iodine and the like). Examples thereof are methylthio, difluoromethylthio, trifluoromethylthio, ethylthio, propylthio, isopropylthio, butylthio, 4,4,4-trifluorobutylthio, pentylthio, hexylthio and the like.

As "5 to 7 membered saturated cyclic amino" of the aforementioned "5 to 7 membered saturated cyclic amino optionally having substituent(s)", there are 5 to 7 membered saturated cyclic amino optionally containing 1 to 4 of one or two kinds of heteroatom(s)selected from a nitrogen atom, a sulfur atom and an oxygen atom in addition to one nitrogen atom and carbon atoms and examples thereof are pyrolidin-1-yl, piperidino, piperazin-1-yl, morpholino, thiomorpholino, hexahydroazepin-1-yl and the like.

As "substituents" of the "5 to 7 membered saturated cyclic amino optionally having substituent(s)", for example, there are 1 to 3 C₁₋₆ alkyl (for example, methyl, ethyl, propyl, isopropyl, butyl, isobutyl, sec-butyl, tert-butyl, pentyl, hexyl and the like), C₆₋₁₄ aryl (for example, phenyl, 1-naphthyl, 2-naphthyl, 2-biphenylyl, 3-biphenylyl, 4-biphenylyl, 2-anthryl and the like), C₁₋₆ alkyl-carbonyl (for example, acetyl, propionyl and the like), 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), oxo and the like.

As "heterocyclic group" of "heterocyclic group optionally having substituent(s)" represented by R⁵, for example, there is a monovalent group obtained by removing one arbitrary hydrogen atom from a 5 to 14 membered (monocyclic, bicyclic or tricyclic) heterocycle containing 1 to 4 of one or two kinds of heteroatom(s) selected from a nitrogen atom, a sulfur atom and an oxygen atom in addition to carbon atoms, preferably (i) a 5 to 14 membered (preferably 5 to 10 membered) aromatic heterocycle, (ii) a 5 to 10 membered non-aromatic heterocycle or (iii) a 7 to 10 membered bridged heterocycle.

As the aforementioned "5 to 14 membered (preferably 5 to 10 membered) aromatic heterocycle", there are an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, and a ring formed by fusing these rings (preferably monocyclic) with one or more (preferably 1 to 2) aromatic ring(s) (for example, benzene ring and the like).

As the aforementioned "5 to 10 membered non-aromatic heterocycle", for example, there are pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole and the like.

As the aforementioned "7 to 10 membered bridged heterocycle", for example, there are quinuclidine, 7-azabicyclo[2.2.1]heptane and the like.

The "heterocyclic group" is preferably a 5 to 14 membered (preferably 5 to 10 membered) (monocyclic or bicyclic) heterocyclic group containing preferably 1 to 4 of one or two kinds of heteroatom(s) selected from a nitrogen atom, a sulfur atom and an oxygen atom in addition to carbon atoms. More particularly, examples thereof are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, and a non-aromatic heterocyclic group such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

Among them, for example, a 5 or 6 membered heterocyclic group containing 1 to 3 heteroatom(s) selected from a nitrogen atom, a sulfur atom and an oxygen atom in addition to carbon atoms is further preferable. More particularly, examples thereof are 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

As "substituent(s)" of "heterocyclic group optionally having substituent(s)", for example, there are the same "substituent(s)" as substituent(s) of "hydrocarbon group optionally having substituent(s)" represented by R⁵.

The "heterocyclic group" may have 1 to 5, preferably 1 to 3 aforementioned substituent(s) at a substitutable position and, when the number of substituents is 2 or more, respective substituents may be the same or different.

As "C₁₋₆ alkyl" represented by R⁶, for example, there are methyl, ethyl, propyl, isopropyl, butyl, isobutyl, sec-butyl, tert-butyl, pentyl, hexyl and the like.

As "hydrocarbon group optionally having substituent(s)" and "heterocyclic group optionally having substituent(s)" represented by R⁷, for example, there are the aforementioned "hydrocarbon group optionally having substituent(s)" and "heterocyclic group optionally having substituent(s)" represented by R⁵, respectively.

As "hydrocarbon group optionally having substituent(s)" represented by R¹, for example, "hydrocarbon group optionally having substituent(s)" represented by R⁵ can be mentioned.

As "heterocyclic group optionally having substituent(s)" represented by R¹, for example, "heterocyclic group optionally having substituent(s)" represented by R⁵ can be mentioned.

As "amino group optionally having substituent(s)" represented by R¹, for example, there are (1) an amino group optionally having 1 or 2 substituent(s) and (2) a cyclic amino group optionally having substituent(s), and the like.

As "substituent(s)" of "amino group optionally having 1 or 2 substituent(s)" of the aforementioned (1), for example, there are a hydrocarbon group optionally having substituent(s), a heterocyclic group optionally having substituent(s), an acyl group, an alkylidene group optionally having substituent(s), and the like. As these "hydrocarbon group optionally having substituent(s)" and "heterocyclic group optionally having substituent(s)", there are the same "hydrocarbon group optionally having substituent(s)" and "heterocyclic group optionally having substituent(s)" as those represented by R⁵ described above, respectively.

As "alkylidene group" of "alkylidene group optionally having substituent(s)", for example, there are a C₁₋₆ alkylidene group (for example, methylidene, ethylidene, propylidene and the like) and the like. As "substituent(s)" of "alkylidene group optionally having substituent(s)", there are 1 to 5, preferably 1 to 3 same substituent(s) as "substituent(s)" of "hydrocarbon group optionally having substituent(s)" represented by R⁵.

When the number of the aforementioned "substituent(s)" of "amino group optionally having 1 or 2 substituent(s)" is 2, respective substituent(s) may be the same or different.

As "cyclic amino group" of "cyclic amino group optionally having substituent(s)" of the aforementioned (2), there are a 5 to 7 membered non-aromatic cyclic amino group optionally containing 1 to 4 of one or two kinds of heteroatom(s) selected from a nitrogen atom, a sulfur atom and an oxygen atom in addition to one nitrogen atom and carbon atoms. More particularly, examples thereof are pyrrolidin-1-yl, piperidino, piperazin-1-yl, morpholino, thiomorpholino, hexahydroazepin-1-yl, imidazolidin-1-yl, 2,3-dihydro-1H-imidazol-1-yl, tetrahydro-1(2H)-pyrimidinyl, 3,6-dihydro-1(2H)-pyrimidinyl, 3,4-dihydro-1(2H)-pyrimidinyl and the like. As "substituent(s)" of "cyclic amino optionally having substituent(s)", there are 1 to 3 of the same ones as "substituent(s)" of "5 to 7 membered saturated cyclic amino group optionally having substituent(s)" which were described in detail as "substituent(s)" of "hydrocarbon group optionally having substituent(s)" represented by R⁵.

Examples of the 5 to 7 membered non-aromatic cyclic amino group having one oxo, there are 2-oxoimidazolidin-1-yl, 2-oxo-2,3-dihydro-1H-imidazol-1-yl, 2-oxotetrahydro-1(2H)-pyrimidinyl, 2-oxo-3,6-dihydro-1(2H) -pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl and the like.

As R¹, an amino group optionally having substituent(s) and an aryl group optionally having substituent(s) are preferable. As further preferable example of the "amino group optionally having substituent(s)" is an amino group optionally having 1 or 2 acyl groups represented by the formula:

-(C=O)-R⁵, -(C=O)-OR⁵, -(C=O)-NR⁵R⁶, -(C=S) -NHR⁵ or -SO₂-R⁷

[wherein respective symbols represent the same meanings as described above].

More preferable example is an amino group optionally having 1 or 2 acyl groups represented by the formula:

-C(C=O)-R⁵ or -(C=C)-NR⁵R⁶

[wherein respective symbols represent the same meanings as described above].

As the "aryl group optionally having substituent(s)", for example, there is preferably a C₆₋₁₄ aryl group (preferably a phenyl group and the like) optionally having 1 to 5 substituent(s) selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy.

Particularly, as R¹, there are mentioned
(1) C₆₋₁₄ aryl group (preferably C₆₋₁₀ aryl) optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl, C₆₋₁₄ aryl-carbonylamino, C₁₋₃ alkylenedioxy, C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl, nitro and the like,
(2) C₁₋₈ alkyl group optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
(3) C₃₋₆ cycloalkyl group (e.g., cyclohexyl) optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
(4) C₇₋₁₆ aralkyl group (e.g., phenyl-C₁₋₆ alkyl group),
(5) 5 to 10 membered aromatic heterocyclic group containing 1 to 4 of one or two kinds of heteroatom(s) selected from a nitrogen atom, a sulfur atom and an oxygen atom in addition to carbon atoms (e.g., 5 or 6 membered aromatic heterocyclic group such as pyridyl, thienyl and the like),
(6) 5 to 10 membered non-aromatic heterocyclic group containing 1 or 2 of one or two kinds of heteroatom(s) selected from a nitrogen atom, a sulfur atom and an oxygen atom in addition to carbon atoms, which may have C₆₋₁₄ aryl (e.g., phenyl), C₁₋₆ alkyl-carbonyl or oxo, such as 5 or 6 membered non-aromatic cyclic amino group (e.g., piperidino, piperazino and the like),
(7) amino group optionally having 1 or 2 substituent(s) selected from the group consisting of the following (1) to (7) [(1) C₁₋₆ alkyl, (2) C₆₋₁₄ aryl, (3) C₇₋₁₆ aralkyl, (4) a 5 or 6 membered heterocyclic group (e.g., pyridyl) containing 1 or 2 heteroatom(s) selected from a nitrogen atom, a sulfur atom and an oxygen atom in addition to carbon atoms, (5) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl group, each optionally having 1 to 3 substituent(s) selected from halogen atom, C₁₋₆ alkyl, C₁₋₆ alkoxy, carboxy, C₁₋₆ alkoxy-carbonyl, cyano, tetrazine and the like, (6) C₆₋₁₄ aryl-carbamoyl group optionally having 1 to 3 substituent(s) selected from halogen atom, C₁₋₆ alkyl, C₁₋₆ alkoxy, carboxy, C₁₋₆ alkoxy-carbonyl, cyano, nitro, mono- or di-C₁₋₆ alkylamino and the like, (7) di-C₁₋₆ alkylamino-C₁₋₆ alkylidene], or (8) carboxy group and the like are preferable.

As the "pyridyl group" of the "pyridyl group optionally having substituent(s)" represented by R², 1-, 2-, 3- or 4-pyridyl group is used.

As the "substituent(s)" of the "pyridyl group optionally having substituent(s)" represented by R², for example, those similar to the "substituent(s)" of the "hydrocarbon group optionally having substituent(s)" represented by the aforementioned R⁵ are used.

The "pyridyl group" may have 1 to 5, preferably 1 to 3, substituent(s) such as those mentioned above at substitutable position(s). When the number of substituent is 2 or more, the respective substituent(s) may be the same or different. In addition, the nitrogen atom in the ring of the "pyridyl group" may be N-oxidized.

R² is preferably a pyridyl group optionally having substituent(s) (e.g., 3-pyridyl group, 4-pyridyl group and the like, preferably 4-pyridyl group).

As R², pyridyl group optionally having 1 or 2 substituent(s) selected from the group consisting of C₁₋₆ alkyl (e.g., methyl), hydroxy and C₁₋₆ alkyl-carbonyloxy (e.g., acetyloxy) and the like are preferable.

As the "aromatic group" of "aromatic group optionally having substituent(s)" represented by R³, for example, there are an aromatic hydrocarbon group and an aromatic heterocyclic group.

As the "aromatic hydrocarbon group", examples thereof include a C₆₋₁₄ monocyclic or fused polycyclic (bicyclic or tricyclic) aromatic hydrocarbon group. As examples, there are a C₆₋₁₄ aryl group and the like such as phenyl, 1-naphthyl, 2-naphthyl, 2-biphenylyl, 3-biphenylyl, 4-biphenylyl, 2-anthryl and the like.

As the "aromatic heterocyclic group", there are 5 to 14 membered (preferably 5 to 10 membered)(monocyclic or bicyclic) aromatic heterocyclic groups containing preferably 1 to 4 of one or two kinds of heteroatom(s) selected from a nitrogen atom, a sulfur atom and an oxygen atom in addition to carbon atoms and the like and, more particularly, an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like.

As the "substituent(s)" of the "aromatic group optionally having substituent(s)", there are 1 to 5, preferably 1 to 3 same substituent(s) as "substituent(s)" of "hydrocarbon group optionally having substituent(s)" represented by the aforementioned R⁵. When the number of substituents is 2 or more, respective substituents may be the same or different. The adjacent two substituents may form a 4 to 7 membered non-aromatic carbon ring. Preferably, it is a 5 membered non-aromatic carbon ring.

R³ is preferably a C₆₋₁₀ aryl group optionally having substituent(s). More preferably, it is a phenyl group optionally having substituent(s). The substituent of the C₆₋₁₀ aryl group and phenyl group is preferably 1 to 3 substituent(s) selected from halogen atom, C₁₋₃ alkylenedioxy, optionally halogenated C₁₋₆ alkyl, carboxy C₂₋₆ alkenyl, C₃₋₆ cycloalkyl, optionally halogenated C₁₋₈ alkoxy, hydroxy, C₇₋₁₆ aralkyloxy, C₁₋₆ alkyl-carbonyloxy and carboxy, particularly preferably, is optionally halogenated C₁₋₆ alkyl (e.g., C₁₋₃ alkyl such as methyl, ethyl and the like), optionally halogenated C₁₋₈ alkoxy (e.g., C₁₋₃ alkoxy such as methoxy, ethoxy and the like). The two adjacent alkyl groups as substituents may be bonded to form a 5 membered non-aromatic carbon ring.

The compound (I) preferably does not include a compound of the formula 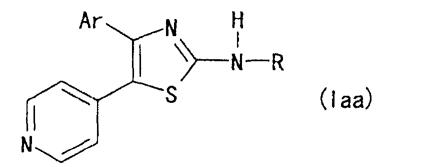 wherein Ar is an unsubstituted or substituted aryl group bonded to a thiazole ring by a carbon atom of the aromatic ring, and R is a hydrogen atom, acyl group, or a monovalent aromatic group having not more than 10 carbon atoms, which is bonded to a nitrogen atom by a carbon atom of the aromatic ring.

As the compound (I), for example, compound (Ia) is preferable.

As compound (Ia), the following compounds of (A)-(B) and the like are preferable.
(A) A compound (Ia) wherein R¹ is (a) an amino group which may have 1 or 2 acyl groups of the formula: -(C=O)-R⁵ or -(C=O)-NR⁵R⁶ wherein each symbol is as defined above or (b) a C₆₋₁₄ aryl group optionally having 1 to 5 substituent(s) selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy and the like;
   R² is pyridyl group optionally having 1 to 5 substituent(s) selected from C₁₋₆ alkyl, hydroxy and C₁₋₆ alkyl-carbonyloxy; and
   R³ is a C₆₋₁₄ aryl group optionally having 1 to 5 substituent(s) selected from halogen atom, optionally halogenated C₁₋₆ alkyl, optionally halogenated C₁₋₆ alkoxy and carboxy.
(B) A compound (Ia) wherein R¹ is (i) C₁₋₈ alkyl, C₃₋₆ cycloalkyl or C₆₋₁₄ aryl (preferably C₆₋₁₀ aryl) , each optionally having 1 to 5 substituent(s) selected from halogen atom, optionally halogenated C₁₋₆ alkyl, carboxy C₂₋₆ alkenyl, optionally halogenated C₁₋₆ alkoxy, C₁₋₆ alkoxy-carbonyl-C₁₋₆ alkoxy, hydroxy, amino, mono- or di-C₁₋₆ alkylamino, carboxy, C₁₋₆ alkoxy-carbonyl, mono- or di-C₁₋₆ alkyl-carbamoyl and C₆₋₁₄ aryl-carbonylamino,
   (ii) a 5 membered heterocyclic group,
   (iii) an amino group optionally having 1 or 2 substituent(s) selected from (1) C₁₋₆ alkyl, (2) C₆₋₁₄ aryl, (3) C₇₋₁₆ aralkyl, (4) 6 membered heterocyclic group and (5) C₁₋₆ alkyl-carbonyl, C₃₋₆ cycloalkyl-carbonyl, C₆₋₁₄ aryl-carbonyl, C₇₋₁₆ aralkyl-carbonyl, C₁₋₆ alkyl-carbamoyl or 5 or 6 membered heterocyclic carbonyl, each optionally having 1 to 3 substituent(s) selected from halogen atom, C₁₋₆ alkyl, C₁₋₆ alkoxy, carboxy and C₁₋₆ alkoxy-carbonyl, or an amino group optionally having di-C₁₋₆ alkylamino-C₁₋₆ alkylidene,
   (iv) a 5 or 6 membered non-aromatic cyclic amino group optionally substituted by C₁₋₆ alkyl-carbonyl or oxo, or
   (v) a carboxy group;
   R² is a pyridyl group optionally having 1 to 3 substituent(s) selected from C₁₋₆ alkyl, hydroxy and C₁₋₆ alkyl-carbonyloxy;
   R³ is a C₆₋₁₀ aryl group optionally having 1 to 3 substituent(s) selected from halogen atom, C₁₋₃ alkylenedioxy, optionally halogenated C₁₋₆ alkyl, carboxy C₂₋₆ alkenyl, optionally halogenated C₁₋₈ alkoxy, hydroxy, C₇₋₁₆ aralkyloxy and C₁₋₆ alkyl-carbonyloxy (two adjacent alkyl groups as substituents may be bonded to form a 5 membered non-aromatic carbon ring).

Moreover, preferable examples of compound (I) and compound (Ia) include:
[4-(4-methoxyphenyl)-5-(4-pyridyl)-1,3-thiazol-2-yl]amine (Reference Example A 13-14),
[4-phenyl-5-(4-pyridyl)-1,3-thiazol-2-yl]amine (Reference Example A 13-15),
N-methyl [4-(4-methoxyphenyl)-5-(4-pyridyl)-1,3-thiazol-2-yl]amine (Reference Example A 13-16),
N-methyl [4-phenyl-5-(4-pyridyl)-1,3-thiazol-2-yl]amine (Reference Example A 13-47),
N-methyl [4-(4-fluorophenyl)-5-(4-pyridyl)-1,3-thiazol-2-yl]amine (Reference Example A 13-69),
N-methyl [4-(4-chlorophenyl)-5-(4-pyridyl)-1,3-thiazol-2-yl]amine (Reference Example A 13-70),
N-methyl [4-(4-bromophenyl)-5-(4-pyridyl)-1,3-thiazol-2-yl]amine (Reference Example A 13-71),
2-phenyl-N-[4-phenyl-5-(4-pyridyl)-1,3-thiazol-2-yl]acetamide (Reference Example A 23-29),
3-phenyl-N-[4-phenyl-5-(4-pyridyl)-1,3-thiazol-2-yl]propionamide (Reference Example A 23-30),
N-[4-(3-chlorophenyl)-5-(4-pyridyl)-1,3-thiazol-2-yl]acetamide (Reference Example A 23-49),
N-[4-(3-chlorophenyl)-5-(4-pyridyl)-1,3-thiazol-2-yl]propionamide (Reference Example A 23-50),
N-[4-(3-methylphenyl)-5-(4-pyridyl)-1,3-thiazol-2-yl]acetamide (Reference Example A 23-51),
N-[4-(3-methylphenyl)-5-(4-pyridyl)-1,3-thiazol-2-yl]propionamide (Reference Example A 23-52),
[4-(3-chlorophenyl)-5-(4-pyridyl)-1,3-thiazol-2-yl]amine (Reference Example A 23-59),
[4-(3-methylphenyl)-5-(4-pyridyl)-1,3-thiazol-2-yl]amine (Reference Example A 23-60),
[4-(4-chlorophenyl)-5-(4-pyridyl)-1,3-thiazol-2-yl]amine (Reference Example A 23-61),
[4-(4-methylphenyl)-5-(4-pyridyl)-1,3-thiazol-2-yl]amine (Reference Example A 23-62),
N-[4-phenyl-5-(4-pyridyl)-1,3-thiazol-2-yl]acetamide (Reference Example A 23-71),
N-phenyl-[4-(4-methoxyphenyl)-5-(4-pyridyl)-1,3-thiazol-2-yl]amine (Reference Example A 23-80),
N-[4-(4-methoxyphenyl)-5-(4-pyridyl)-1,3-thiazol-2-yl]nicotinamide (Reference Example A 23-101),
N-[4-(4-methoxyphenyl)-5-(4-pyridyl)-1,3-thiazol-2-yl]isonicotinamide (Reference Example A 23-102),
[4-(3,4-dimethylphenyl)-5-(4-pyridyl)-1,3-thiazol-2-yl]amine (Reference Example A 23-125),
N-[4-(3,5-dimethylphenyl)-5-(4-pyridyl)-1,3-thiazol-2-yl]acetamide (Reference Example A 23-128),
[4-(2-naphthyl)-5-(4-pyridyl)-1,3-thiazol-2-yl]amine (Reference Example A 23-144),
N-ethyl-N'-[4-(4-methoxyphenyl)-5-(4-pyridyl)-1,3-thiazol-2-yl]urea (Reference Example A 23-156),
N-[4-(3,5-dimethylphenyl)-5-(4-pyridyl)-1,3-thiazol-2-yl]isonicotinamide (Reference Example A 23-200),
N-ethyl-[4-(4-methoxyphenyl)-5-(4-pyridyl)-1,3-thiazol-2-yl]amine (Reference Example A 23-269),
N-propyl-[4-(3,5-dimethylphenyl)-5-(4-pyridyl)-1,3-thiazol-2-yl]amine (Reference Example A 23-276),
N-butyl-[4-(3,5-dimethylphenyl)-5-(4-pyridyl)-1,3-thiazol-2-yl]amine (Reference Example A 23-280),
N-benzyl-[4-(3,5-dimethylphenyl)-5-(4-pyridyl)-1,3-thiazol-2-yl]amine (Reference Example A 23-281),
N-propyl-[4-(4-methoxyphenyl)-5-(4-pyridyl)-1,3-thiazol-2-yl]amine (Reference Example A 23-290),
N-isopropyl-[4-(4-methoxyphenyl)-5-(4-pyridyl)-1,3-thiazol-2-yl]amine (Reference Example A 23-291),
N-[4-(4-methoxyphenyl)-5-(4-pyridyl)-1,3-thiazol-2-yl]-N'-phenylurea (Reference Example A 23-296),
4-[[[4-(4-methoxyphenyl)-5-(4-pyridyl)-1,3-thiazol-2-yl]amino]carbonyl]benzoic acid (Reference Example A 23-299),
methyl 4-[2-[4-(methylthio)phenyl]-5-(4-pyridyl)-1,3-thiazol-4-yl]phenyl ether (Reference Example A 23-300),
4-[4-(3,5-dimethylphenyl)-5-(4-pyridyl)-1,3-thiazol-2-yl]phenyl methyl sulfide (Reference Example A 23-302),
4-[4-(4-methoxyphenyl)-5-(4-pyridyl)-1,3-thiazol-2-yl]phenyl methyl sulfoxide (Reference Example A 23-303),
4-[4-(3,5-dimethylphenyl)-5-(4-pyridyl)-1,3-thiazol-2-yl]phenyl methyl sulfoxide (Reference Example A 23-305),
4-[4-(4-methoxyphenyl)-5-(4-pyridyl)-1,3-thiazol-2-yl]phenyl methyl sulfone (Reference Example A 23-306),
4-[4-(3,5-dimethylphenyl)-5-(4-pyridyl)-1,3-thiazol-2-yl]phenyl methyl sulfone (Reference Example A 23-308),
4-[4-(4-fluorophenyl)-5-(4-pyridyl)-1,3-thiazol-2-yl]phenyl methyl sulfide (Reference Example A 23-309),
4-[4-(4-chlorophenyl)-5-(4-pyridyl)-1,3-thiazol-2-yl]phenyl methyl sulfide (Reference Example A 23-310),
4-[4-(4-fluorophenyl)-5-(4-pyridyl)-1,3-thiazol-2-yl]phenyl methyl sulfoxide (Reference Example A 23-311),
4-[4-(4-chlorophenyl)-5-(4-pyridyl)-1,3-thiazol-2-yl]phenyl methyl sulfoxide (Reference Example A 23-312),
4-[4-(4-fluorophenyl)-5-(4-pyridyl)-1,3-thiazol-2-yl]phenyl methyl sulfone (Reference Example A 23-313),
4-[4-(4-chlorophenyl)-5-(4-pyridyl)-1,3-thiazol-2-yl]phenyl methyl sulfone (Reference Example A 23-314),
N-[4-(3,5-dimethylphenyl)-5-(4-pyridyl)-1,3-thiazol-2-yl]-N'-phenylurea (Reference Example A 23-315),
2-hydroxy-N-[4-(4-methoxyphenyl)-5-(4-pyridyl)-1,3-thiazol-2-yl]propionamide (Reference Example A 23-325),
4-[4-(3,4-dimethylphenyl)-5-(4-pyridyl)-1,3-thiazol-2-yl]phenyl methyl sulfide (Reference Example A 23-326),
4-[4-(3,4-dimethylphenyl)-5-(4-pyridyl)-1,3-thiazol-2-yl]phenyl methyl sulfoxide (Reference Example A 23-327),
4-[4-(3,4-dimethylphenyl)-5-(4-pyridyl)-1,3-thiazol-2-yl]phenyl methyl sulfone (Reference Example A 23-328),
2-hydroxy-N-[4-(4-methoxyphenyl)-5-(4-pyridyl)-1,3-thiazol-2-yl]acetamide (Reference Example A 23-329),
4-[[[4-(3,5-dimethylphenyl)-5-(4-pyridyl)-1,3-thiazol-2-yl]amino]carbonyl]benzoic acid (Reference Example A 23-337),
3-[[[4-(3,5-dimethylphenyl)-5-(4-pyridyl)-1,3-thiazol-2-yl]amino]carbonyl]benzoic acid (Reference Example A 23-342), salts thereof and the like.

Preferable examples of compound (I) and compound (Ia) further include 4-(4-fluorophenyl)-2-phenyl-5-(4-pyridyl)-1,3-thiazole (Reference Example A 44-1), methyl 4-[4-(3-methylphenyl)-5-(4-pyridyl)-1,3-thiazol-2-yl]phenyl sulfide (Reference Example A 44-7), methyl 4-[4-(3-methylphenyl)-5-(4-pyridyl)-1,3-thiazol-2-yl]phenyl sulfoxide (Reference Example A 44-8), methyl 4-[4-(3-methylphenyl)-5-(4-pyridyl)-1,3-thiazol-2-yl]phenyl sulfone (Reference Example A 44-26) and the like.

Furthermore, as compound (I) and (Ia),
(S)-N-[4-(3-methylphenyl)-5-(2-(1-phenylethylamino)-4-pyridyl)-1,3-thiazol-2-yl]nicotinamide,
(R)-N-[4-(3-methylphenyl)-5-(2-(1-phenylethylamino)-4-pyridyl)-1,3-thiazol-2-yl]nicotinamide,
(S)-N-[4-(3-methylphenyl)-5-(2-(1-phenylethylamino)-4-pyridyl)-1,3-thiazol-2-yl]-2-methylnicotinamide,
(R)-N-[4-(3-methylphenyl)-5-(2-(1-phenylethylamino)-4-pyridyl)-1,3-thiazol-2-yl]-2-methylnicotinamide,
(S)-N-[4-(3-methylphenyl)-5-(2-(1-phenylethylamino)-4-pyridyl)-1,3-thiazol-2-yl]-2-chloronicotinamide,
(R)-N-[4-(3-methylphenyl)-5-(2-(1-phenylethylamino)-4-pyridyl)-1,3-thiazol-2-yl]-2-chloronicotinamide,
(S)-N-[4-(3-methylphenyl)-5-(2-(1-phenylethylamino)-4-pyridyl)-1,3-thiazol-2-yl]-2-methoxynicotinamide,
(R)-N-[4-(3-methylphenyl)-5-(2-(1-phenylethylamino)-4-pyridyl)-1,3-thiazol-2-yl]-2-methoxynicotinamide,
N-[5-(2-benzylamino-4-pyridyl)-4-(3-methylphenyl)-1,3-thiazol-2-yl] nicotinamide,
N-[5-(2-benzylamino-4-pyridyl)-4-(3-methylphenyl)-1,3-thiazol-2-yl]-2-methoxynicotinamide,
N-[5-(2-benzylamino-4-pyridyl)-4-(3-methylphenyl)-1,3-thiazol-2-yl]-2-chloronicotinamide,
N-[5-(2-benzylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]nicotinamide,
N-[5-(2-benzoylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]-2-chloronicotinamide,
N-[5-(2-benzoylamino-4-pyridyl)-4-(3-methylphenyl)-1,3-thiazol-2-yl]-2-methoxynicotinamide,
(S)-N-(1-phenylethyl)-4-[2-ethyl-4-(3-methylphenyl)-1,3-thiazol-5-yl]-2-pyridylamine, (R)-N-(1-phenylethyl)-4-[2-ethyl-4-(3-methylphenyl)-1,3-thiazol-5-yl]-2-pyridylamine,
(S)-N-(1-phenylethyl)-4-[4-(3-methylphenyl)-2-propyl-1,3-thiazol-5-yl]-2-pyridylamine,
(R)-N-(1-phenylethyl)-4-[4-(3-methylphenyl)-2-propyl-1,3-thiazol-5-yl]-2-pyridylamine,
(S)-N-(1-phenylethyl)-4-[2-butyl-4-(3-methylphenyl)-1,3-thiazol-5-yl]-2-pyridylamine,
(R)-N-(1-phenylethyl)-4-[2-butyl-4-(3-methylphenyl)-1,3-thiazol-5-yl]-2-pyridylamine,
(S)-N-(1-phenylethyl)-4-[4-(3-methylphenyl)-2-(4-methylthiophenyl)-1,3-thiazol-5-yl]-2-pyridylamine,
(R)-N-(1-phenylethyl)-4-[4-(3-methylphenyl)-2-(4-methylthiophenyl)-1,3-thiazol-5-yl]-2-pyridylamine,
(S)-N-(1-phenylethyl)-4-[4-(3-methylphenyl)-2-(4-methylsulfonylphenyl)-1,3-thiazol-5-yl]-2-pyridylamine,
(R)-N-(1-phenylethyl)-4-[4-(3-methylphenyl)-2-(4-methylsulfonylphenyl)-1,3-thiazol-5-yl]-2-pyridylamine,
(S)-N-(1-phenylethyl)-4-[2-(4-fluorophenyl)-4-(3-methylphenyl)-1,3-thiazol-5-yl]-2-pyridylamine,
(R)-N-(1-phenylethyl)-4-[2-(4-fluorophenyl)-4-(3-methylphenyl)-1,3-thiazol-5-yl]-2-pyridylamine, salts thereof and the like are preferable.

As the salt of Compounds (I) and (Ia), for example, there are a metal salt, ammonium salt, a salt with an organic base, a salt with an inorganic acid, a salt with an organic acid, a salt with basic or acidic amino acid and the like. As a suitable metal salt, there are alkali metal salt such as sodium salt, potassium salt and the like; alkaline earth metal salt such as calcium salt, magnesium salt, barium salt and the like; aluminum salt and the like. As a suitable example of a salt with an organic base, for example, there are salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. As a suitable example of a salt with an inorganic acid, for example, there are salts with hydrochloric acid, hydrobromic acid, nitric acid, sulfuric acid, phosphoric acid and the like. As a suitable example of a salt with an organic acid, for example, there are salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. As a suitable example of a salt with a basic amino acid, for example, there are salts with arginine, lysine, ornithine and the like. As a suitable example of a salt with an acidic amino acid, for example, there are salts with aspartic acid, glutamic acid and the like.

Among them, pharmaceutically acceptable salts are preferable. For example, when a compound has an acidic functional group therein, there are inorganic salts such as alkali metal salts (for example, sodium salt, potassium salt and the like), alkaline earth metal salts (for example, calcium salt, magnesium salt, barium salt and the like), ammonium salts and the like and, when a compound has a basic functional group therein, there are salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like.

A process for producing Compound (I) including Compound (Ia) will be described below.

Compound (I) can be obtained by a method shown by the following reaction formulas 1 and 2 or a similar method to that, and additionally, for example, it can be obtained according to the methods described in JP-A-60-58981, JP-A-61-10580, JP-T 7-503023, WO 93/15071, DE-A-3601411, JP-A-5-70446 and the like, a method similar to these methods and the like.

Respective symbols in the compounds in the following reaction formulas 1 and 2 have the same meanings as those described above. Compounds in the reaction formulas include salts thereof and, as the salts, for example, those similar to the salts of Compound (I) can be mentioned.

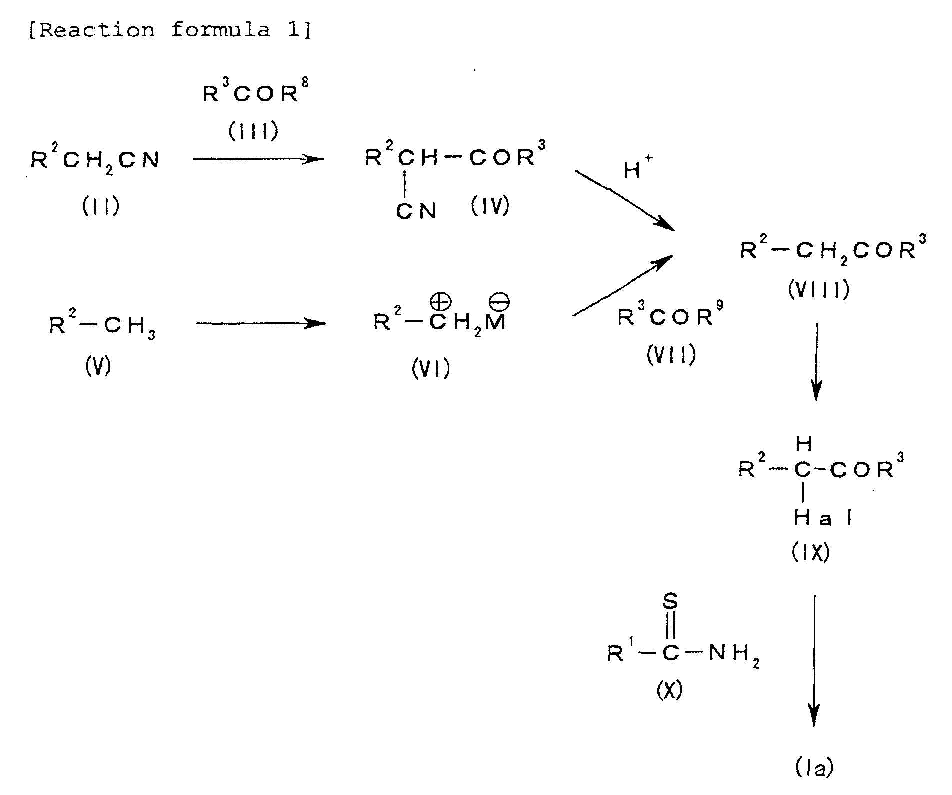

Compounds (II), (III), (V), (VII), (XI), (XIII) and (XIV) can be used as they are when they are commercially available or can be prepared by a method known per se or according to the similar method to this.

Compound (IV) can be obtained by condensing Compound (II) and Compound (III) in the presence of a base.

In the compound (III), R⁸ is, for example, (1) C₁₋₆ alkoxy (e.g., methoxy, ethoxy and the like), (2) di-C₁₋₆ alkylamino (e.g., dimethylamino, diethylamino and the like), (3) N-C₆₋₁₀ aryl-N-C₁₋₆ alkylamino (e.g., N-phenyl-N-methylamino and the like), (4) a 3 to 7 membered cyclic amino optionally substituted by C₆₋₁₀ aryl and(or) C₁₋₆ alkyl (e.g., pyrrolidino, morpholino, methylaziridin-1-yl and the like) and the like.

The amount of Compound (III) to be used is about 0.5 to about 3.0 moles, preferably about 0.8 to about 2.0 moles relative to 1 mole of Compound (II).

The amount of base to be used is about 1.0 to about 30 moles, preferably about 1.0 to about 10 moles relative to 1 mole of Compound (II).

As the "base", for example, there are a basic salt such as sodium carbonate, potassium carbonate, cesium carbonate and the like, an inorganic base such as sodium hydroxide, potassium hydroxide and the like, an aromatic amine such as pyridine, lutidine and the like, a tertiary amine such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, an alkali metal hydride such as sodium hydride, potassium hydride and the like, a metal amide such as sodium amide, lithium diisopropylamide, lithium hexamethyldisilazide and the like, a metal alkoxide such as sodium methoxide, sodium ethoxide, potassium tert-butoxide and the like.

It is advantageous that this reaction is conducted without a solvent or in the presence of an inert solvent. Although the solvent is not particularly limited as long as the reaction proceeds, for example, halogenated hydrocarbons, aliphatic hydrocarbons, aromatic hydrocarbons, ethers, amides, alcohols, water or a mixture of two or more of them are used.

The reaction temperature is usually about -5 to about 200°C, preferably about 5 to about 150°C. The reaction time is usually about 5 minutes to about 72 hours, preferably about 0.5 to about 30 hours.

Although the reaction product can be used as the reaction solution itself or as a crude product in the next step, it can be isolated from the reaction mixture according to conventional methods and can be easily purified by a separating means such as recrystallization, distillation, chromatography and the like.

Compound (VIII) can be obtained by treating compound (IV) with an acid.

The amount of acid to be used is about 1.0 to about 100 moles, preferably about 1.0 to about 30 moles, relative to 1 mole of Compound (IV).

As the "acid", for example, mineral acids such as hydrochloric acid, hydrobromic acid, sulfuric acid and the like are used.

This reaction is conducted in the presence of an inert solvent for a reaction. The solvent is not particularly limited as long as a reaction proceeds but, for example, water, a mixture of water and amides, a mixture of water and alcohols and the like are used.

The reaction temperature is usually about 20 to about 200°C, preferably about 60 to about 150°C. The reaction time is generally about 30 minutes to about 72 hours, preferably about 1 to about 30 hours.

Although the reaction product can be used as the reaction solution itself or as a crude product in the next step, it can be isolated from the reaction mixture according to conventional methods and can be easily purified by a separating means such as recrystallization, distillation, chromatography and the like.

The compound (VIII) can be also obtained by condensing compound (VI) obtained by treating compound (V) with a base, and compound (VII).

In the compound (VI), M represents, for example, an alkali metal such as lithium, sodium, potassium and the like.

In the compound (VII), R⁹ represents, for example, those similar to the aforementioned R⁸.

The amount of base to be used is about 1.0 to about 30 moles, preferably about 1.0 to about 10 moles relative to 1 mole of Compound (V).

As the "base", for example, metal amides such as sodium amide, lithium diisopropylamide, lithium hexamethyldisilazide and the like are used.

It is advantageous that this reaction is conducted without a solvent or in the presence of an inert solvent for a reaction. The solvent is not particularly limited as long as a reaction proceeds but, for example, aliphatic hydrocarbons, aromatic hydrocarbons, ethers or a mixture of two or more of them and the like are used.

The reaction temperature is usually about -78 to about 60°C, preferably about -78 to about 20°C. The reaction time is usually about 5 minutes to about 24 hours, preferably about 0.5 to about 3 hours.

Although a product can be used as the reaction solution itself or as a crude product in the next reaction, it can be isolated from the reaction mixture by the conventional methods, and can be easily purified by a separating means such as recrystallization, distillation, chromatography and the like.

Compound (IX) can be obtained by treating Compound (VIII) with halogens. This reaction is performed in the presence of a base or a basic salt if desired.

The amount of halogens to be used is about 1.0 to about 5.0 moles, preferably about 1.0 to about 2.0 moles relative to 1 mole of Compound (VIII).

As the "halogens", there are bromine, chlorine, iodine and the like.

The amount of base to be used is about 1.0 to about 10.0 moles, preferably about 1.0 to about 3.0 moles relative to 1 mole of Compound (VIII).

As the "base", for example, there are aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like.

The amount of basic salt to be used is about 1.0 to about 10.0 moles, preferably about 1.0 to about 3.0 moles, relative to 1 mole of Compound (VIII).

As the "basic salt", for example, sodium carbonate, potassium carbonate, cesium carbonate, sodium bicarbonate, sodium acetate, potassium acetate and the like can be used.

It is advantageous that this reaction is performed without a solvent or in the presence of an inert solvent for a reaction. The solvent is not particularly limited as long as a reaction proceeds but, for example, ethers, aromatic hydrocarbons, aliphatic hydrocarbons, amides, halogenated hydrocarbons, nitriles, sulfoxides, organic acids, aromatic amines or a mixture of two or more of them and the like are used.

The reaction temperature is about -20 to about 150°C, preferably about 0 to about 100°C. The reaction time is usually about 5 minutes to about 24 hours, preferably about 10 minutes to about 5 hours.

Although a product can be used as the reaction solution itself or as a crude product in the next reaction, it can be isolated from the reaction mixture by the conventional methods, and can be easily purified by a separating means such as recrystallization, distillation, chromatography and the like.

Compound (Ia) can be obtained by condensing Compound (IX) with Compound (X). This reaction is performed in the presence of a base if desired.

In Compound (IX), Hal represents a halogen atom.

When Compound (X) is commercially available, it can be used as it is, or can be obtained by the method known per se or a method according to the known method or further a method shown in the reaction formula 2.

The amount of Compound (X) to be used is about 0.5 to about 3.0 moles, preferably about 0.8 to about 2.0 moles relative to 1 mole of Compound (IX).

The amount of base to be used is about 1.0 to about 30 moles, preferably about 1.0 to about 10 moles relative to 1 mole of Compound (IX).

As the "base", for example, there are basic salts such as sodium carbonate, potassium carbonate, cesium carbonate, sodium bicarbonate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, ethers, amides, alcohols, nitriles or a mixture of two or more of them and the like are used.

The reaction temperature is about -5 to about 200°C, preferably about 5 to about 150°C. The reaction time is usually about 5 minutes to about 72 hours, preferably about 0.5 to about 30 hours.

Although a product can be used as the reaction solution itself or as a crude product in the next reaction, it can be isolated from the reaction mixture by the conventional methods, and can be easily purified by a separating means such as recrystallization, distillation, chromatography and the like.

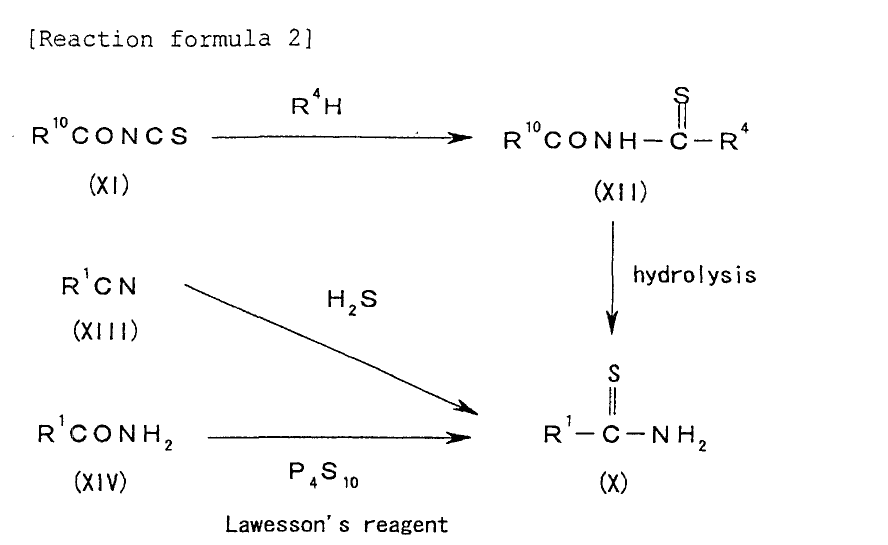

Compound (XII) is obtained by condensing Compound (XI) and amines represented by the formula R⁴H.

R⁴ represents "amino group optionally having substituent(s)" represented by the above-mentioned R¹.

In Compound (XI), R¹⁰ represents an alkoxy group. As the "alkoxy group", for example, there are C₁₋₆ alkoxy groups such as methoxy, ethoxy, propoxy, isopropoxy, butoxy and the like.

The amount of the "amines" to be used is about 1.0 to about 30 moles, preferably about 1.0 to about 10 moles relative to 1 mole of Compound (XI).

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, ethers, amides, alcohols, nitriles, ketones or a mixture of two or more of them and the like are used.

The reaction temperature is about -5 to about 200°C, preferably about 5 to about 120°C. The reaction time is usually about 5 minutes to about 72 hours, preferably about 0.5 to about 30 hours.

Although a product can be used as the reaction solution itself or as a crude product in the next reaction, it can be isolated from the reaction mixture by the conventional methods, and can be easily purified by a separating means such as recrystallization, distillation, chromatography and the like.

Compound (X) is obtained by hydrolysing Compound (XII) using an acid or a base.

The amount of acid or base to be used is about 0.1 to about 50 moles, preferably about 1 to about 20 moles relative to 1 mole of Compound (XII), respectively.

As the "acid", for example, mineral acids such as hydrochloric acid, hydrobromic acid, sulfuric acid and the like, Lewis acids such as boron trichloride, boron tribromide and the like, the use of Lewis acid together with thiols or sulfides, organic acids such as trifluoroacetic acid, p-toluenesulfonic acid and the like are used.

As the "base", for example, metal hydroxides such as sodium hydroxide, potassium hydroxide, barium hydroxide and the like, basic salts such as sodium carbonate, potassium carbonate and the like, metal alkoxides such as sodium methoxide, sodium ethoxide, potassium tert-butoxide and the like, organic bases such as triethylamine, imidazole, formamidine and the like are used.

It is advantageous that this reaction is performed without a solvent or in the presence of an inert solvent for a reaction. The solvent is not particularly limited as long as a reaction proceeds but, for example, alcohols, ethers, aromatic hydrocarbons, aliphatic hydrocarbons, halogenated hydrocarbons, sulfoxides, water or a mixture of two or more of them and the like are used.

The reaction time is usually about 10 minutes to about 50 hours, preferably about 30 minutes to about 12 hours. The reaction temperature is about 0 to about 200°C, preferably about 20 to about 120°C.

Compound (X) can be also obtained by treating Compound (XIII) with hydrogen sulfide in the presence of a base.

The amount of hydrogen sulfide is about 1 mole to about 30 moles relative to 1 mole of Compound (XIII).

The amount of base to be used is about 1.0 to about 30 moles, preferably about 1.0 to about 10 moles relative to 1 mole of Compound (XIII).

As the "base", for example, there are aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, ethers, aromatic amines or a mixture of two or more of them and the like are used.

This reaction is performed under atmospheric pressure or under a pressurized condition. The reaction temperature is usually about -20 to about 80°C, preferably about -10 to about 30°C. The reaction time is usually about 5 minutes to about 72 hours, preferably about 0.5 to about 30 hours.

Although a product can be used as the reaction solution itself or as a crude product in the next reaction, it can be isolated from the reaction mixture by the conventional methods, and can be easily purified by a separating means such as recrystallization, distillation, chromatography and the like.

Compound (X) can be also obtained by treating compound (XIII) with O,O-diethyl dithiophosphate in the presence of an acid.

The amount of O,O-diethyl dithiophosphate to be used is about 1 to about 3 moles, preferably about 1 to about 2 moles, relative to 1 mole of Compound (XIII).

The amount of acid to be used is about 3 to about 10 moles, relative to 1 mole of Compound (XIII).

As the "acid", for example, mineral acids such as hydrogen chloride, hydrogen bromide and the like, and the like are used.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, ethers, esters, alcohols, water or a mixture of two or more of them and the like are used.

The reaction temperature is generally about -20 to about 80°C, preferably about -10 to about 30°C. The reaction time is generally about 5 minutes to about 72 hours, preferably about 0.5 to about 30 hours.

Although a product can be used as the reaction solution itself or as a crude product in the next reaction, it can be isolated from the reaction mixture by the conventional methods, and can be easily purified by a separating means such as recrystallization, distillation, chromatography and the like.

Compound (X) can also be obtained by treating Compound (XIV) with phosphorus pentasulfide or Lawesson's reagent.

The amount of phosphorus pentasulfide or Lawesson's reagent to be used is about 0.5 to about 10 moles, preferably about 0.5 to about 3 moles relative to 1 mole of Compound (XIV).

It is advantageous that this reaction is performed without a solvent or in the presence of an inert solvent for a reaction. The solvent is not particularly limited as long as a reaction proceeds but, for example, ethers, aromatic hydrocarbons, aliphatic hydrocarbons, halogenated hydrocarbons or a mixture of two or more of them and the like are used.

The reaction time is usually 10 minutes to about 50 hours, preferably about 30 minutes to about 12 hours. The reaction temperature is usually about 0 to about 150°C, preferably about 20 to about 120°C.

Although a product (X) can be used as the reaction solution itself or as a crude product in the next reaction, it can be isolated from the reaction mixture by the conventional methods, and can be easily purified by a separating means such as recrystallization, distillation, chromatography and the like.

When Compound (Ia) is acylamino compound, an objective compound can be also obtained by subjecting the corresponding amine compound to an acylating reaction known per se.

For example, among Compound (Ia), a compound wherein R¹ is acylamino group optionally having substituent(s) is obtained by reacting the corresponding 2-thiazolamine and an acylating agent optionally in the presence of a base or an acid.

The amount of acylating agent to be used is about 1.0 to about 5.0 moles, preferably about 1.0 to about 2.0 moles relative to 1 mole of the corresponding 2-thiazolamine.

As the "acylating agent", for example, there are carboxylic acids corresponding to an objective acyl group or a reactive derivative thereof (for example, acid halide, acid anhydride, ester and the like) and the like.

The amount of base or acid to be used is about 0.8 to about 5.0 moles, preferable about 1.0 to about 2.0 moles relative to 1 mole of the corresponding 2-thiazolamine.

As the "base", for example, there are triethylamine, pyridine, 4-dimethylaminopyridine and the like.

As the "acid", for example, there are methanesulfonic acid, p-toluenesulfonic acid, camphorsulfonic acid and the like.

It is advantageous that this reaction is performed without a solvent or in the presence of an inert solvent for a reaction. The solvent is not particularly limited as long as a reaction proceeds but, for example, ethers, aromatic hydrocarbons, aliphatic hydrocarbons, amides, halogenated hydrocarbons, nitriles, sulfoxides, aromatic amines or a mixture of two or more of them and the like are used.

The reaction temperature is about -20 to about 150°C, preferably about 0 to about 100°C. The reaction time is usually 5 minutes to about 24 hours, preferably about 10 minutes to about 5 hours.

Although a product can be used as the reaction solution itself or as a crude product in the next reaction, it can be isolated from the reaction mixture by the conventional methods, and can be easily purified by a separating means such as recrystallization, distillation, chromatography and the like.

When Compound (Ia) is an N-oxide compound, it is obtained by treating the corresponding pyridyl compound with an organic peroxy acid.

The amount of organic peroxy acid to be used is about 0.8 to about 10 moles, preferable about 1.0 to about 3.0 moles relative to 1 mole of the corresponding pyridyl compound.

As the "organic peroxy acid", for example, there are peracetic acid, trifluoroperacetic acid, m-chloroperbenzoic acid and the like.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is about -20 to about 130°C, preferably about 0 to about 100°C. The reaction time is usually 5 minutes to about 72 hours, preferably about 0.5 to about 12 hours.

Alternatively, the N-oxide compound is also obtained by treating the corresponding pyridyl compound with hydrogen peroxide or alkyl hydroperoxide optionally in the presence of a base, an acid or a metal oxide.

The amount of hydrogen peroxide or alkyl hydroperoxide to be used is about 0.8 to about 10 moles, preferably about 1.0 to 3.0 moles relative to 1 mole of the corresponding pyridyl compound.

As the "alkyl hydroperoxide", for example, there are tert-butyl hydroperoxide, cumene hydroperoxide and the like.

The amount of base, acid or metal oxide to be used is about 0.1 to about 30 moles, preferably 0.8 to about 5 moles relative to 1 mole of the corresponding pyridyl compound.

As the "base", for example, there are inorganic bases such as sodium hydroxide, potassium hydroxide and the like, basic salts such as sodium carbonate, potassium carbonate and the like.

As the "acid", for example, there are mineral acids such as hydrochloric acid, sulfuric acid, perchloric acid and the like, Lewis acids such as boron trifluoride, aluminum chloride, titanium tetrachloride and the like, organic acids such as formic acid, acetic acid and the like.

As the "metal oxide", for example, there are vanadium oxide (V₂O₅), osmium tetroxide (OsO₄), tungsten oxide (WO₃), molybdenum oxide (MoO₃), selenium dioxide (SeO₂), chromium oxide (CrO₃) and the like.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is about -20 to about 130°C, preferably about 0 to about 100°C. The reaction time is usually 5 minutes to about 72 hours, preferably about 0.5 to about 12 hours.

Although a product can be used as the reaction solution itself or as a crude product in the next reaction, it can be isolated from the reaction mixture by the conventional methods, and can be easily purified by a separating means such as recrystallization, distillation, chromatography and the like.

When compound (Ia) is an S-oxide compound, it can be obtained by treating the corresponding sulfide compound with peroxide.

The amount of peroxide to be used is about 0.8 to about 10 moles, preferably about 1.0 to about 3.0 moles,relative to 1 mole of the corresponding sulfide compound.

As the "peroxide", for example, peracetic acid, trifluoroperacetic acid, m-chloroperbenzoic acid, potassium persulfate, metaperiodic acid and the like can be mentioned.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is usually about -20 to about 130°C, preferably about 0 to about 100°C. The reaction time is usually about 5 minutes to about 72 hours, preferably about 0.5 to about 12 hours.

In addition, S-oxide compound can be obtained by treating the corresponding sulfide compound with hydrogen peroxide or alkyl hydroperoxide in the presence of a base, acid or metal oxide, if desired.

The amount of hydrogen peroxide or alkyl hydroperoxide to be used is about 0.8 to about 10 moles, preferably about 1.0 to about 3.0 moles, relative to 1 mole of the corresponding sulfide compound.

As the "alkyl hydroperoxide", for example, tert-butyl hydroperoxide, cumene hydroperoxide and the like can be mentioned.

The amount of "base, acid or metal oxide" to be used is about 0.1 to about 30 moles, preferably about 0.8 to about 5 moles, relative to 1 mole of the corresponding sulfide compound.

As the "base", for example, there are inorganic bases such as sodium hydroxide, potassium hydroxide and the like, basic salts such as sodium carbonate, potassium carbonate and the like, and the like.

As the "acid", for example, there are mineral acids such as hydrochloric acid, sulfuric acid, perchloric acid and the like, Lewis acids such as boron trifluoride, aluminum chloride, titanium tetrachloride and the like, organic acids such as formic acid, acetic acid and the like, and the like.

As the "metal oxide", for example, there are vanadium oxide (V₂O₅), osmium tetroxide (OsO₄), tungsten oxide (WO₃), molybdenum oxide (MoO₃), selenium dioxide (SeO₂), chromium oxide (CrO₃) and the like.

It is advantageous that this reaction is performed without a solvent or in the presence of an inert solvent for a reaction. The solvent is not particularly limited as long as a reaction proceeds but, for example, halogenated hydrocarbons, aliphatic hydrocarbons, aromatic hydrocarbons, organic acids, ethers, amides, sulfoxides, alcohols, nitriles, ketones or a mixture of two or more of them and the like are used.

The reaction temperature is usually about -20 to about 130°C, preferably about 0 to about 100°C. The reaction time is usually about 5 minutes to about 72 hours, preferably about 0.5 to about 12 hours.

Although a product can be used as the reaction solution itself or as a crude product in the next reaction, it can be isolated from the reaction mixture by the conventional methods, and can be easily purified by a separating means such as recrystallization, distillation, chromatography and the like.

In the above respective reactions, when starting compounds have amino, carboxy, hydroxy as substituents, a protecting groups which are generally used in the peptide chemistry or the like may be introduced into these groups and, after reaction, a desired compound can be obtained by removing protecting groups if needed.

As a protecting group for amino, for example, formyl or C₁₋₆ alkyl-carbonyl (for example, acetyl, propionyl and the like), phenylcarbonyl, C₁₋₆ alkoxy-carbonyl (for example, methoxycarbonyl, ethoxycarbonyl and the like), phenyloxycarbonyl, C₇₋₁₀ aralkyloxy-carbonyl (for example, benzyloxycarbonyl and the like), trityl, phthaloyl and the like which may have substituent(s), respectively, are used. As these substituent(s), halogen atom(s) (for example, fluorine, chlorine, bromine, iodine and the like), C₁₋₆ alkyl-carbonyl (for example, acetyl, propionyl, valeryl and the like), nitro and the like are used and the number of substituents is 1 to 3.

As a protecting group for carboxy, for example, C₁₋₆ alkyl (for example, methyl, ethyl, propyl, isopropyl, butyl, tert-butyl and the like), phenyl, trityl, silyl and the like which may have substituent(s), respectively, are used. As these substituent(s), halogen atom(s) (for example, fluorine, chlorine, bromine, iodine and the like), formyl, C₁₋₆ alkyl-carbonyl (for example, acetyl, propionyl, butylcarbonyl and the like), nitro, C₁₋₆ alkyl (for example, methyl, ethyl, tert-butyl and the like), C₆₋₁₀ aryl (for example, phenyl, naphthyl and the like) and the like are used and the number of substituents is 1 to 3.

As a protecting group for hydroxy, for example, C₁₋₆ alkyl (for example, methyl, ethyl, propyl, isopropyl, butyl, tert-butyl and the like), phenyl, C₇₋₁₁ aralkyl (for example, benzyl and the like), formyl, C₁₋₆ alkyl-carbonyl (for example, acetyl, propionyl and the like), phenyloxycarbonyl, C₇₋₁₁ aralkyloxy-carbonyl (for example, benzyloxycarbonyl and the like), tetrahydropyranyl, tetrahydrofuranyl, silyl and the like which may have substituent(s), respectively, are used. As these substituent(s), halogen atom(s) (for example, fluorine, chlorine, bromine, iodine and the like), C₁₋₆ alkyl (for example, methyl, ethyl, tert-butyl and the like), C₇₋₁₁ aralkyl (for example, benzyl and the like), C₆₋₁₀ aryl (for example, phenyl, naphthyl and the like), nitro and the like are used and the number of substituents is 1 to 4.

In addition, as a method of removing a protecting group, the method known per se or a method according to this method is used and, for example, method by treating with an acid, a base, the ultraviolet ray, hydrazine, phenylhydrazine, sodium N-methyldithiocarbamate, tetrabutylammonium fluoride, palladium acetate and the like or a method of reduction is used.

In any cases, Compound (I) can be synthesized by further, optionally, performing known deprotection, acylation, alkylation, hydrogenation, oxidation, reduction, carbon chain extension and substituent exchange reactions alone or in a combination of two or more of them. As these reactions, the reactions described in Shinjikkenkagakukoza 14, vol.15, 1977 (Maruzen Press) are adopted.

As the above "alcohols", for example, there are methanol, ethanol, propanol, isopropanol, tert-butanol and the like.

As the above "ethers", for example, there are diethyl ether, diisopropyl ether, diphenyl ether, tetrahydrofuran, dioxane, 1,2-dimethoxyethane and the like.

As the above "halogenated hydrocarbons", for example, there are dichloromethane, chloroform, 1,2-dichloroethane, carbon tetrachloride and the like.

As the above "aliphatic hydrocarbons", for example, there are hexane, pentane, cyclohexane and the like.

As the above "aromatic hydrocarbons", for example, there are benzene, toluene, xylene, chlorobenzene and the like.

As the above "aromatic amines", for example, there are pyridine, lutidine, quinoline and the like.

As the above "amides", for example, there are N,N-dimethylformamide, N,N-dimethylacetamide, hexamethylphosphoric triamide and the like.

As the above "ketones", for example, there are acetone, methyl ethyl ketone and the like.

As the above "sulfoxides", for example, there are dimethyl sulfoxide and the like.

As the above "nitriles", for example, there are acetonitrile, propionitrile and the like.

As the above "organic acids", for example, there are acetic acid, propionic acid, trifluoroacetic acid and the like.

As the aforementioned "esters", for example, methyl acetate, ethyl acetate, methyl propionate and the like can be mentioned.

When a desired product is obtained in a free form by the above reaction, it may be converted into a salt according to conventional methods or, when a desired product is obtained as a salt, it can be converted into a free form or another salt according to conventional methods. Compound (I) thus obtained can be isolated and purified from the reaction solution by the known means, for example, trans-solvation, concentration, solvent extraction, fractional distillation, crystallization, recrystallization, chromatography and the like.

When Compound (I) is present as a configurational isomer, diastereomer, conformer or the like, each can be optionally isolated by the above separation and purification means. In addition, Compound (I) is in the form of its racemate, they can be separated into S- and R-forms by any conventional optical resolution.

When Compound (I) includes stereoisomers, both the isomers alone and mixtures of each isomers are included in the scope of the present invention.

In addition, Compound (I) may be hydrated or anhydrous.

Compound (I) may be labeled with an isotope (for example, ³H, ¹⁴C, ³⁵S) or the like.

### [compound (II)]

(1) an optionally N-oxidized compound represented by the formula: 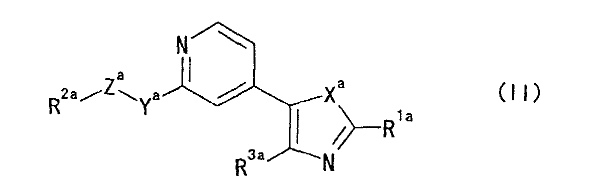 wherein R^{1a} represents a hydrogen atom, a hydrocarbon group optionally having substituents, a heterocyclic group optionally having substituents, an amino group optionally having substituents or an acyl group,
   R^{2a} represents an aromatic group optionally having substituents,
   R^{3a} represents a hydrogen atom, a pyridyl group optionally having substituents or an aromatic hydrocarbon group optionally having substituents,
   X^{a} represents an oxygen atom or an optionally oxidized sulfur atom,
   Y^{a} represents a bond, an oxygen atom, an optionally oxidized sulfur atom or a group represented by the formula: NR^{4a} (wherein R^{4a} represents a hydrogen atom, a hydrocarbon group optionally having substituents or an acyl group) and
   Z^{a} represents a bond or a divalent acyclic hydrocarbon group optionally having substituents, or a salt thereof,
(2) the compound according to (1), wherein Z^{a} is a divalent acyclic hydrocarbon group optionally having substituents,
(3) the compound according to (1), which is a compound represented by the formula: 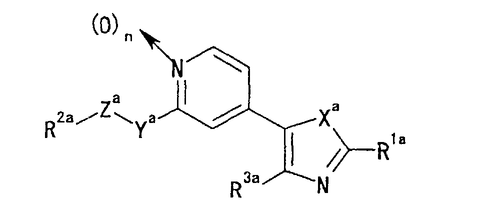 wherein n represents 0 or 1, and other symbols are as defined in (1), or a salt thereof,
(4) the compound according to (1) or (3), wherein R^{1a} represents
   (i) a hydrogen atom,
   (ii) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group [these groups may have substituents selected from the group (substituent group A) consisting of oxo, halogen atom, C₁₋₃ alkylenedioxy, nitro, cyano, optionally halogenated C₁₋₆ alkyl, optionally halogenated C₂₋₆ alkenyl, carboxy C₂₋₆ alkenyl, optionally halogenated C₂₋₆ alkynyl, optionally halogenated C₃₋₆ cycloalkyl, C₆₋₁₄ aryl, optionally halogenated C₁₋₈ alkoxy, C₁₋₆ alkoxy-carbonyl-C₁₋₆ alkoxy, hydroxy, C₆₋₁₄ aryloxy, C₇₋₁₆ aralkyloxy, mercapto, optionally halogenated C₁₋₆ alkylthio, C₆₋₁₄ arylthio, C₇₋₁₆ aralkylthio, amino, mono-C₁₋₆ alkylamino, mono-C₆₋₁₄ arylamino, di-C₁₋₆ alkylamino, di-C₆₋₁₄ arylamino, formyl, carboxy, C₁₋₆ alkyl-carbonyl, C₃₋₆ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5 or 6 membered heterocyclic carbonyl, carbamoyl, thiocarbamoyl, mono-C₁₋₆ alkyl-carbamoyl, di-C₁₋₆ alkyl-carbamoyl, C₆₋₁₄ aryl-carbamoyl, 5 or 6 membered heterocyclic carbamoyl, C₁₋₆ alkylsulfonyl, C₆₋₁₄ arylsulfonyl, C₁₋₆ alkylsulfinyl, C₆₋₁₄ arylsulfinyl, formylamino, C₁₋₆ alkyl-carbonylamino, C₆₋₁₄ aryl-carbonylamino, C₁₋₆ alkoxy-carbonylamino, C₁₋₆ alkylsulfonylamino, C₆₋₁₄ arylsulfonylamino, C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, C₆₋₁₄ aryl-carbamoyloxy, nicotinoyloxy, 5 to 7 membered saturated cyclic amino optionally having 1 to 4 heteroatoms of one or two kinds selected from a nitrogen atom, a sulfur atom and an oxygen atom in addition to one nitrogen atom and carbon atoms (this cyclic amino may have substituents selected from the group consisting of C₁₋₆ alkyl, C₆₋₁₄ aryl, C₁₋₆ alkyl-carbonyl, 5 to 10 membered aromatic heterocyclic group and oxo), 5 to 10 membered aromatic heterocyclic group containing 1 to 4 heteroatoms of one or two kinds selected from a nitrogen atom, a sulfur atom and an oxygen atom in addition to carbon atoms, sulfo, sulfamoyl, sulfinamoyl and sulfenamoyl]
   (iii) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A,
   (iv) an acyl group represented by the formula:

      -(C=O)-R^{5a}, -(C=O)-OR^{5a}, -(C=O)-NR^{5a}R^{6a}, -(C=S)-NHR^{5a} or -SO₂-R^{7a}

      (wherein R^{5a} represents (1) a hydrogen atom, (2) a C₁₋₆ alkyl group, an C₂₋₆ alkenyl group, an C₂₋₆ alkynyl group, a C₃₋₆ cycloalkyl group, a C₆₋₁₄ aryl group or a C₇₋₁₆ aralkyl group optionally having substituents selected from the substituent group A or (3) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A, R^{6a} represents a hydrogen atom or a C₁₋₆ alkyl group, R^{7a} represents (1) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group optionally having substituents selected from the substituent group A or (2) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A),
   (v) an amino group (this amino group may have substituents selected from the group consisting of (1) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group optionally having substituents selected from the substituent group A, (2) a 5 to 14 membered heterocyclic group containing 1 to 4 heteroatoms of one or two kinds selected from a nitrogen atom, a sulfur atom and an oxygen atom in addition to carbon atoms optionally having substituents selected from the substituent group A, (3) an acyl group as defined in the (iv), and (4) a C₁₋₆ alkylidene group optionally having substituents selected from the substituent group A), or
   (vi) a 5 to 7 membered non-aromatic cyclic amino group optionally containing 1 to 4 heteroatoms of one or two kinds selected from a nitrogen atom, a sulfur atom and an oxygen atom in addition to one nitrogen atom and carbon atoms (this cyclic amino may have substituents selected from the group consisting of C₁₋₆ alkyl, C₆₋₁₄ aryl, C₁₋₆ alkyl-carbonyl, 5 to 10 membered aromatic heterocyclic group and oxo) ;
      R^{2a} represents (1) a C₆₋₁₄ monocyclic or fused polycyclic aromatic hydrocarbon group optionally having substituents selected from the substituent group A or (2) a 5 to 14 membered aromatic heterocyclic group containing 1 to 4 heteroatoms of one or two kinds selected from a nitrogen atom, a sulfur atom and an oxygen atom in addition to carbon atoms, optionally having substituents selected from the substituent group A;
      R^{3a} represents (1) a hydrogen atom, (2) a pyridyl group optionally having substituents selected from the substituent group A, or (3) a C₆₋₁₄ monocyclic or fused polycyclic aromatic hydrocarbon group optionally having substituents selected from the substituent group A;
      X^{a} represents O, S, SO or SO₂;
      Y^{a} represents a bond, O, S, SO, SO₂ or a group represented by the formula: NR^{4a} (wherein R^{4a} represents (1) a hydrogen atom, (2) a C₁₋₆ alkyl group, a C₂₋₆ alkenyl group, a C₂₋₆ alkynyl group, a C₃₋₆ cycloalkyl group, a C₆₋₁₄ aryl group or a C₇₋₁₆ aralkyl group optionally having substituents selected from the substituent group A or (3) an acyl group as defined in the (iv)),
      Z^{a} represents a bond, a C₁₋₁₅ alkylene group, a C₂₋₁₆ alkenylene group or a C₂₋₁₆ alkynylene group optionally having substituents selected from the substituent group A,
(5) the compound according to (1), wherein R^{1a} is an amino group optionally having substituents,
(6) the compound according to (1), wherein R^{1a} is (i) a C₁₋₆ alkyl group, (ii) a C₆₋₁₄ aryl group optionally substituted with substituents selected from C₁₋₆ alkylthio, C₁₋₆ alkylsulfonyl and halogen atom, or (iii) an amino group optionally having 1 or 2 acyl groups represented by the formula: -(C=O)-R^{5a}' (wherein R^{5a}' represents (1) a C₁₋₆ alkyl group, (2) a C₆₋₁₄ aryl group or (3) a 5 to 14 membered heterocyclic group containing 1 to 4 heteroatoms of one or two kinds selected from a nitrogen atom, a sulfur atom and an oxygen atom in addition to carbon atoms),
(7) the compound according to (1), wherein R^{1a} is an amino group optionally having 1 or 2 acyl groups represented by -(C=O)-R^{5a}" (wherein R^{5a}" represents (1) a C₆₋₁₄ aryl group or (2) a 5 to 14 membered heterocyclic group containing 1 to 4 heteroatoms of one or two kinds selected from a nitrogen atom, a sulfur atom and an oxygen atom in addition to carbon atoms),
(8) the compound according to (1), wherein R^{2a} is a C₆₋₁₄ aryl group optionally having substituents,
(9) the compound according to (1), wherein R^{2a} is a C₆₋₁₄ aryl group optionally substituted with halogen atom or C₁₋₆ alkoxy, or a 5 to 14 membered aromatic heterocyclic group containing 1 to 4 heteroatoms of one or two kinds selected from a nitrogen atom, a sulfur atom and an oxygen atom in addition to carbon atoms,
(10) the compound according to (1), wherein R^{2a} is a C₆₋₁₄ aryl group, or a 5 to 14 membered heterocyclic group containing 1 to 4 heteroatoms of one or two kinds selected from nitrogen atom, a sulfur atom and an oxygen atom in addition to carbon atoms,
(11) the compound according to (1), wherein R^{3a} is a C₆₋₁₄ aryl group optionally having substituents,
(12) the compound according to (1), wherein R^{3a} is a C₆₋₁₄ aryl group optionally substituted with one or two C₁₋₆ alkyl or C₁₋₆ alkoxy groups,
(13) the compound according to (1), wherein X^{a} is an optionally oxidized sulfur atom,
(14) the compound according to (1), wherein X^{a} is a sulfur atom,
(15) the compound according to (1), wherein Y^{a} is an oxygen atom or a group represented by the formula: NR^{4a} (wherein R^{4a} is as defined in (1)),
(16) the compound according to (1), wherein Y^{a} is an oxygen atom, an optionally oxidized sulfur atom or a group represented by the formula: NR^{4a'} (wherein R^{4a'} represents a C₁₋₆ alkyl group),
(17) the compound according to (1), wherein Y^{a} is O, NH or S,
(18) the compound according to (1), wherein Z^{a} is a lower alkylene group optionally having substituents,
(19) the compound according to (1), wherein Z^{a} is a bond or a C₁₋₆ alkylene group optionally having oxo,
(20) the compound according to (1), wherein R^{1a} is (i) a C₁₋₆ alkyl group, (ii) a C₆₋₁₄ aryl group optionally substituted with C₁₋₆ alkylthio, C₁₋₆ sulfonyl and halogen atom, or (iii) an amino group optionally having 1 or 2 acyl groups represented by the formula: -(C=O)-R^{5a}' (wherein R^{5a}' represents (1) a C₁₋₆ alkyl group, (2) a C₆₋₁₄ aryl group or (3) a 5 to 14 membered heterocyclic group containing 1 to 4 heteroatoms of one or two kinds selected from a nitrogen atom, a sulfur atom and an oxygen atom in addition to carbon atoms;
   R^{2a} is a C₆₋₁₄ aryl group optionally substituted with halogen atom or C₁₋₆ alkoxy, or a 5 to 14 membered aromatic heterocyclic group containing 1 to 4 heteroatoms of one or two kinds selected from a nitrogen atom, a sulfur atom and an oxygen atom in addition to carbon atoms;
   R^{3a} is a C₆₋₁₄ aryl group optionally substituted with 1 or 2 C₁₋₆ alkyl or C₁₋₆ alkoxy groups;
   X^{a} is a sulfur atom;
   Y^{a} is an oxygen atom, an optionally oxidized sulfur atom or a group represented by the formula: NR^{4a}' (wherein R^{4a}' represents a C₁₋₆ alkyl group) ;
   Z^{a} is a C₁₋₆ alkylene group optionally having oxo or C₁₋₆ alkyl or a bond,
(21) the compound according to (1), wherein R^{1a} is an amino group optionally having 1 or 2 acyl groups represented by -(C=O)-R^{5a}" (wherein R^{5a}" represents (1) a C₆₋₁₄ aryl group or (2) a 5 to 14 membered heterocyclic group containing 1 to 4 heteroatoms of one or two kinds selected from a nitrogen atom, a sulfur atom and an oxygen atom in addition to carbon atoms) ;
   R^{2a} is a C₆₋₁₄ aryl group or a 5 to 14 membered aromatic heterocyclic group containing 1 to 4 heteroatoms of one or two kinds selected from a nitrogen atom, a sulfur atom and an oxygen atom in addition to carbon atoms;
   R^{3a} is a C₆₋₁₄ aryl group optionally substituted with 1 or 2 C₁₋₆ alkyl or C₁₋₆ alkoxy groups;
   X^{a} is a sulfur atom; Y^{a} is O, NH or S; Z^{a} is a bond or a C₁₋₆ alkylene group optionally having oxo,
(22) N-[5-(2-benzoylamino-4-pyridyl)-4-(3,5-dimethylphenyl)-1,3-thiazol-2-yl]acetamide (Reference Example D Compound No.9),
   N-[5-(2-benzylamino-4-pyridyl)-4-(3,5-dimethylphenyl)-1,3-thiazol-2-yl]acetamide (Reference Example D Compound No.10),
   N-[4-[4-(4-methoxyphenyl)-2-methyl-1,3-thiazol-5-yl]-2-pyridyl]benzamide (Reference Example D Compound No.13),
   N-[4-[2-(4-fluorophenyl)-4-(3-methylphenyl)-1,3-thiazol-5-yl]-2-pyridyl]phenylacetamide (Reference Example D Compound No.1.4),
   N-[4-[2-ethyl-4-(3-methylphenyl)-1,3-thiazol-5-yl]-2-pyridyl]phenylacetamide (Reference Example D Compound No.15-2),
   N-[4-[4-(3-methylphenyl)-2-propyl-1,3-thiazol-5-yl]-2-pyridyl]phenylacetamide (Reference Example D Compound No.15-3),
   N-[4-[2-butyl-4-(3-methylphenyl)-1,3-thiazol-5-yl]-2-pyridyl]phenylacetamide (Reference Example D Compound No.15-4),
   N-[4-[4-(3-methylphenyl)-2-(4-methylthiophenyl)-1,3-thiazol-5-yl]-2-pyridyl]phenylacetamide (Reference Example D Compound No.15-6),
   N-[4-[2-ethyl-4-(3-methylphenyl)-1,3-thiazol-5-yl]-2-pyridyl]benzamide (Reference Example D Compound No.16-1),
   N-[4-[2-ethyl-4-(3-methylphenyl)-1,3-thiazol-5-yl]-2-pyridyl]-3-phenylpropionamide (Reference Example D Compound No.16-2),
   N-[4-[2-ethyl-4-(3-methylphenyl)-1,3-thiazol-5-yl]-2-pyridyl]-3-(4-methoxyphenyl)propionamide (Reference Example D Compound No.16-3),
   N-[4-[2-ethyl-4-(3-methylphenyl)-1,3-thiazol-5-yl]-2-pyridyl]-4-phenylbutyramide (Reference Example D Compound No.16-5),
   N-[4-[4-(3-methylphenyl)-2-propyl-1,3-thiazol-5-yl]-2-pyridyl]benzamide (Reference Example D Compound No.16-7),
   N-[4-[4-(3-methylphenyl)-2-propyl-1,3-thiazol-5-yl]-2-pyridyl]-3-phenylpropionamide (Reference Example D Compound No.16-8),
   N-[4-[2-butyl-4-(3-methylphenyl)-1,3-thiazol-5-yl]-2-pyridyl]benzamide (Reference Example D Compound No.16-9),
   N-[4-[2-butyl-4-(3-methylphenyl)-1,3-thiazol-5-yl]-2-pyridyl]-3-phenylpropionamide (Reference Example D Compound No.16-10),
   N-[4-[2-(4-fluorophenyl)-4-(3-methylphenyl)-1,3-thiazol-5-yl]-2-pyridyl]benzamide (Reference Example D Compound No.16-11),
   N-[4-[2-(4-fluorophenyl)-4-(3-methylphenyl)-1,3-thiazol-5-yl]-2-pyridyl]-3-phenylpropionamide (Reference Example D Compound No.16-12),
   N-[4-[4-(3-methylphenyl)-2-(4-methylthiophenyl)-1,3-thiazol-5-yl]-2-pyridyl]benzamide (Reference Example D Compound No.16-15),
   N-[4-[4-(3-methylphenyl)-2-(4-methylthiophenyl)-1,3-thiazol-5-yl]-2-pyridyl]-3-phenylpropionamide (Reference Example D Compound No.16-16),
   N-benzyl-N-[4-[2-ethyl-4-(3-methylphenyl)-1,3-thiazol-5-yl]-2-pyridyl]amine (Reference Example D Compound No.19-2),
   N-[4-[2-ethyl-4-(3-methylphenyl)-1,3-thiazol-5-yl]-2-pyridyl]-N-(2-phenylethyl)amine (Reference Example D Compound No.19-3),
   N-[4-[2-ethyl-4-(3-methylphenyl)-1,3-thiazol-5-yl]-2-pyridyl]-N-(3-phenylpropyl)amine (Reference Example D Compound No.19-4),
   N-benzyl-N-[4-[4-(3-methylphenyl)-2-propyl-1,3-thiazol-5-yl]-2-pyridyl]amine (Reference Example D Compound No.19-5),
   N-[4-[4-(3-methylphenyl)-2-propyl-1,3-thiazol-5-yl]-2-pyridyl]-N-(2-phenylethyl)amine (Reference Example D Compound No.19-6),
   N-[4-[4-(3-methylphenyl)-2-propyl-1,3-thiazol-5-yl]-2-pyridyl]-N-(3-phenylpropyl)amine (Reference Example D Compound No.19-7),
   N-benzyl-N-[4-[2-butyl-4-(3-methylphenyl)-1,3-thiazol-5-yl]-2-pyridyl]amine (Reference Example D Compound No.19-8),
   N-[4-[2-butyl-4-(3-methylphenyl)-1,3-thiazol-5-yl]-2-pyridyl]-N-(2-phenylethyl)amine (Reference Example D Compound No.19-9),
   N-[4-[2-butyl-4-(3-methylphenyl)-1,3-thiazol-5-yl]-2-pyridyl]-N-(3-phenylpropyl)amine (Reference Example D Compound No.19-10),
   N-benzyl-N-[4-[4-(3-methylphenyl)-2-(4-methylthiophenyl)-1,3-thiazol-5-yl]-2-pyridyl]amine (Reference Example D Compound No.19-17),
   N-[4-[4-(3-methylphenyl)-2-(4-methylthiophenyl)-1,3-thiazol-5-yl]-2-pyridyl]-N-(2-phenylethyl)amine (Reference Example D Compound No.19-18),
   N-[4-[4-(3-methylphenyl)-2-(4-methylthiophenyl)-1,3-thiazol-5-yl]-2-pyridyl]-N-(3-phenylpropyl)amine (Reference Example D Compound No.19-19),
   N-[4-[4-(3-methylphenyl)-2-(4-methylsulfonylphenyl)-1,3-thiazol-5-yl]-2-pyridyl]benzamide (Reference Example D Compound No.20),
   N-[4-[4-(3-methylphenyl)-2-(4-methylsulfonylphenyl)-1,3-thiazol-5-yl]-2-pyridyl]phenylacetamide (Reference Example D Compound No.21-1),
   N-[4-[4-(3-methylphenyl)-2-(4-methylsulfonylphenyl) -1,3-thiazol-5-yl]-2-pyridyl]-3-phenylpropionamide (Reference Example D Compound No.21-2),
   N-benzyl-N-[4-[4-(3-methylphenyl)-2-(4-methylsulfonylphenyl)-1,3-thiazol-5-yl]-2-pyridyl]amine (Reference Example D Compound No.21-5),
   N-[4-[4-(3-methylphenyl)-2-(4-methylsulfonylphenyl)-1,3-thiazol-5-yl]-2-pyridyl]-N-(3-phenylpropyl)amine (Reference Example D Compound No.21-6),
   N-[4-[4-(3-methylphenyl)-2-(4-methylsulfonylphenyl)-1,3-thiazol-5-yl]-2-pyridyl]-N-(2-phenylethyl)amine (Reference Example Compound No.25-1),
   N-(4-fluorobenzyl)-N-[4-[4-(3-methylphenyl)-2-(4-methylsulfonylphenyl)-1,3-thiazol-5-yl]-2-pyridyl]amine (Reference Example D Compound No.25-2), or salts thereof,

In the aforementioned formula, R^{1a} represents a hydrogen atom, a hydrocarbon group optionally having substituents, a heterocyclic group optionally having substituents, an amino group optionally having substituents or acyl group.

As "acyl group" represented by R^{1a}, for example, there are an acyl group represented by the formula:

-(C=O)-R^{5a}, - (C=O)-OR^{5a}, -(C=O)-NR^{5a}R^{6a}, -(C=S)-NHR^{5a} or -SO₂-R^{7a}

(wherein R^{5a} represents a hydrogen atom, a hydrocarbon group optionally having substituents or a heterocyclic group optionally having substituents, R^{6a} represents a hydrogen atom or a C₁₋₆ alkyl, R^{7a} represents a hydrocarbon group optionally having substituents or a heterocyclic group optionally having substituents) and the like.

In the aforementioned formula, as "hydrocarbon group" represented by R^{5a} of "hydrocarbon group optionally having substituents", for example, there are an acyclic or cyclic hydrocarbon group (for example, alkyl, alkenyl, alkynyl, cycloalkyl, aryl, aralkyl and the like) and the like. Among them, C₁₋₁₆ acyclic or cyclic hydrocarbon groups are preferable.

As "alkyl", for example, C₁₋₆ alkyl (for example, methyl, ethyl, propyl, isopropyl, butyl, isobutyl, sec-butyl, tert-butyl, pentyl, hexyl and the like) is preferable and, in particular, C₁₋₃ alkyl (for example, methyl, ethyl, propyl and isopropyl) and the like are preferable.

As "alkenyl", for example, C₂₋₆ alkenyl (for example, vinyl, allyl, isopropenyl, 1-butenyl, 2-butenyl, 3-butenyl, 2-methyl-2-propenyl, 1-methyl-2-propenyl, 2-methyl-1-propenyl and the like) and the like are preferable.

As "alkynyl", for example, C₂₋₆ alkynyl (for example, ethynyl, propargyl, 1-butynyl, 2-butynyl, 3-butynyl, 1-hexynyl and the like) and the like are preferable.

As "cycloalkyl", for example, C₃₋₆ cycloalkyl (for example, cyclopropyl, cyclobutyl, cyclopentyl, cyclohexyl and the like) and the like are preferable.

As "aryl", for example, C₆₋₁₄ aryl (for example, phenyl, 1-naphthyl, 2-naphthyl, 2-biphenylyl, 3-biphenylyl, 4-biphenylyl, 2-anthryl and the like) and the like are preferable.

As "aralkyl", for example, C₇₋₁₆ aralkyl (for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl and the like) and the like are preferable.

As "substituents" of "hydrocarbon group optionally having substituents" represented by R^{5a}, for example, there are oxo, halogen atom (for example, fluorine, chlorine, bromine, iodine and the like), C₁₋₃ alkylenedioxy (for example, methylenedioxy, ethylenedioxy and the like), nitro, cyano, optionally halogenated C₁₋₆ alkyl, optionally halogenated C₂₋₆ alkenyl, carboxy C₂₋₆ alkenyl (for example, 2-carboxyethenyl, 2-carboxy-2-methylethenyl and the like), optionally halogenated C₂₋₆ alkynyl, optionally halogenated C₃₋₆ cycloalkyl, C₆₋₁₄ aryl (for example, phenyl, 1-naphthyl, 2-naphthyl, 2-biphenylyl, 3-biphenylyl, 4-biphenylyl, 2-anthryl and the like), optionally halogenated C₁₋₈ alkoxy, C₁₋₆ alkoxy-carbonyl-C₁₋₆ alkoxy (for example, ethoxycarbonylmethyloxy and the like), hydroxy, C₆₋₁₄ aryloxy (for example, phenyloxy, 1-naphthyloxy, 2-naphthyloxy and the like) , C₇₋₁₆ aralkyloxy (for example, benzyloxy, phenethyloxy and the like), mercapto, optionally halogenated C₁₋₆ alkylthio, C₆₋₁₄ arylthio (for example, phenylthio, 1-naphthylthio, 2-naphthylthio and the like), C₇₋₁₆ aralkylthio (for example, benzylthio, phenethylthio and the like), amino, mono-C₁₋₆ alkylamino (for example, methylamino, ethylamino and the like), mono-C₆₋₁₄ arylamino (for example, phenylamino, 1-naphthylamino, 2-naphthylamino and the like), di-C₁₋₆ alkylamino (for example, dimethylamino, diethylamino, ethylmethylamino and the like) , di-C₆₋₁₄ arylamino (for example, diphenylamino and the like), formyl, carboxy, C₁₋₆ alkyl-carbonyl (for example, acetyl, propionyl and the like), C₃₋₆ cycloalkyl-carbonyl (for example, cyclopropylcarbonyl, cyclopentylcarbonyl, cyclohexylcarbonyl and the like), C₁₋₆ alkoxy-carbonyl (for example, methoxycarbonyl, ethoxycarbonyl, propoxycarbonyl, tert-butoxycarbonyl and the like), C₆₋₁₄ aryl-carbonyl (for example, benzoyl, 1-naphthoyl, 2-naphthoyl and the like), C₇₋₁₆ aralkyl-carbonyl (for example, phenylacetyl, 3-phenylpropionyl and the like), C₆₋₁₄ aryloxy-carbonyl (for example, phenoxycarbonyl and the like), C₇₋₁₆ aralkyloxy-carbonyl (for example, benzyloxycarbonyl, phenethyloxycarbonyl and the like), 5 or 6 membered heterocyclic carbonyl (for example, nicotinoyl, isonicotinoyl, thenoyl, furoyl, morpholinocarbonyl, thiomorpholinocarbonyl, piperazin-1-ylcarbonyl, pyrrolidin-1-ylcarbonyl and the like), carbamoyl, thiocarbamoyl, mono-C₁₋₆ alkyl-carbamoyl (for example, methylcarbamoyl, ethylcarbamoyl and the like), di-C₁₋₆ alkyl-carbamoyl (for example, dimethylcarbamoyl, diethylcarbamoyl, ethylmethylcarbamoyl and the like) , C₆₋₁₄ aryl-carbamoyl (for example, phenylcarbamoyl, 1-naphthylcarbamoyl, 2-naphthylcarbamoyl and the like), 5 or 6 membered heterocyclic carbamoyl (for example, 2-pyridylcarbamoyl, 3-pyridylcarbamoyl, 4-pyridylcarbamoyl, 2-thienylcarbamoyl, 3-thienylcarbamoyl and the like), C₁₋₆ alkylsulfonyl (for example, methylsulfonyl, ethylsulfonyl and the like), C₆₋₁₄ arylsulfonyl (for example, phenylsulfonyl, 1-naphthylsulfonyl, 2-naphthylsulfonyl and the like), C₁₋₆ alkylsulfinyl (for example, methylsulfinyl, ethylsulfinyl and the like), C₆₋₁₄ arylsulfinyl (for example, phenylsulfinyl, 1-naphthylsulfinyl, 2-naphthylsulfinyl and the like), formylamino, C₁₋₆ alkyl-carbonylamino (for example, acetylamino and the like), C₆₋₁₄ aryl-carbonylamino (for example, benzoylamino, naphthoylamino and the like), C₁₋₆ alkoxy-carbonylamino (for example, methoxycarbonylamino, ethoxycarbonylamino, propoxycarbonylamino, butoxycarbonylamino and the like), C₁₋₆ alkylsulfonylamino (for example, methylsulfonylamino, ethylsulfonylamino and the like), C₆₋₁₄ arylsulfonylamino (for example, phenylsulfonylamino, 2-naphthylsulfonylamino, 1-naphthylsulfonylamino and the like), C₁₋₆ alkyl-carbonyloxy (for example, acetoxy, propionyloxy and the like), C₆₋₁₄ aryl-carbonyloxy (for example, benzoyloxy, naphthylcarbonyloxy and the like), C₁₋₆ alkoxy-carbonyloxy (for example, methoxycarbonyloxy, ethoxycarbonyloxy, propoxycarbonyloxy, butoxycarbonyloxy and the like), mono-C₁₋₆ alkyl-carbamoyloxy (for example, methylcarbamoyloxy, ethylcarbamoyloxy and the like), di-C₁₋₆ alkyl-carbamoyloxy (for example, dimethylcarbamoyloxy, diethylcarbamoyloxy and the like), C₆₋₁₄ aryl--carbamoyloxy (for example, phenylcarbamoyloxy, naphthylcarbamoyloxy and the like), nicotinoyloxy, 5 to 7 membered saturated cyclic amino optionally having substituents, 5 to 10 membered aromatic heterocyclic group (for example, 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), sulfo, sulfamoyl, sulfinamoyl, sulfenamoyl and the like.

The "hydrocarbon group" may have 1 to 5, preferably 1 to 3 aforementioned substituents at a substitutable position and, when the number of substituents is 2 or more, respective substituents may be the same or different.

As aforementioned "optionally halogenated C₁₋₆ alkyl", for example, there are C₁₋₆ alkyl (for example, methyl, ethyl, propyl, isopropyl, butyl, isobutyl, sec-butyl, tert-butyl, pentyl, hexyl and the like) and the like optionally having 1 to 5, preferably 1 to 3 halogen atoms (for example, fluorine, chlorine, bromine, iodine and the like). Examples thereof are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

As the aforementioned "optionally halogenated C₂₋₆ alkenyl", for example, there are C₂₋₆ alkenyl (for example, vinyl, propenyl, isopropenyl, 2-buten-1-yl, 4-penten-1-yl, 5-hexen-1-yl) and the like optionally having 1 to 5, preferably 1 to 3 halogen atoms (for example, fluorine, chlorine, bromine, iodine and the like).

As the aforementioned "optionally halogenated C₂₋₆ alkynyl", there are C₂₋₆ alkynyl (for example, 2-butyn-1-yl, 4-pentyn-1-yl, 5-hexyn-1-yl and the like) and the like optionally having 1 to 5, preferably 1 to 3 halogen atoms (for example, fluorine, chlorine, bromine, iodine and the like).

As the aforementioned "optionally halogenated C₃₋₆ cycloalkyl", for example, there are C₃₋₆ cycloalkyl (for example, cyclopropyl, cyclobutyl, cyclopentyl, cyclohexyl and the like) and the like optionally having 1 to 5, preferably 1 to 3 halogen atoms (for example, fluorine, chlorine, bromine, iodine and the like). Examples thereof are cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

As the aforementioned "optionally halogenated C₁₋₈ alkoxy", for example, there are C₁₋₈ alkoxy (for example, methoxy, ethoxy, propoxy, isopropoxy, butoxy, isobutoxy, sec-butoxy, pentyloxy, hexyloxy and the like) and the like optionally having 1 to 5, preferably 1 to 3 halogen atoms (for example, fluorine, chlorine, bromine, iodine and the like). Examples thereof are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

As the aforementioned "optionally halogenated C₁₋₆ alkylthio", for example, there are C₁₋₆ alkylthio (for example, methylthio, ethylthio, propylthio, isopropylthio, butylthio, sec-butylthio, tert-butylthio and the like) and the like optionally having 1 to 5, preferably 1 to 3 halogen atoms (for example, fluorine, chlorine, bromine, iodine and the like). Examples thereof are methylthio, difluoromethylthio, trifluoromethylthio, ethylthio, propylthio, isopropylthio, butylthio, 4,4,4-trifluorobutylthio, pentylthio, hexylthio and the like.

As "5 to 7 membered saturated cyclic amino" of the aforementioned "5 to 7 membered saturated cyclic amino optionally having substituents", there are 5 to 7 membered saturated cyclic amino optionally containing 1 to 4 heteroatoms of one or two kinds selected from a nitrogen atom, a sulfur atom and an oxygen atom in addition to one nitrogen atom and carbon atoms and examples thereof are pyrrolidin-1-yl, piperidino, piperazin-1-yl, morpholino, thiomorpholino, hexahydroazepin-1-yl and the like.

As "substituents" of the "5 to 7 membered saturated cyclic amino optionally having substituents", for example, there are 1 to 3 C₁₋₆ alkyl (for example, methyl, ethyl, propyl, isopropyl, butyl, isobutyl, sec-butyl, tert-butyl, pentyl, hexyl and the like) , C₆₋₁₄ aryl (for example, phenyl, 1-naphthyl, 2-naphthyl, 2-biphenylyl, 3-biphenylyl, 4-biphenylyl, 2-anthryl and the like), C₁₋₆ alkyl-carbonyl (for example, acetyl, propionyl and the like), 5 to 10 membered aromatic heterocyclic group (for example, 2-thienyl, 3-thienyl, 2-pyridyl, 3-pyridyl, 4-pyridyl, 2-quinolyl, 3-.quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like), oxo and the like.

As "heterocyclic group" of "heterocyclic group optionally having substituents" represented by R^{5a}, for example, there is a monovalent group obtained by removing one arbitrary hydrogen atom from a 5 to 14 membered (monocyclic, bicyclic or tricyclic) heterocycle containing 1 to 4 heteroatoms of one or two kinds selected from a nitrogen atom, a sulfur atom and an oxygen atom in addition to carbon atoms, preferably (i) a 5 to 14 membered (preferably 5 to 10 membered, particularly preferably 5 to 6 membered) aromatic heterocycle, (ii) a 5 to 10 membered (preferably 5 to 6 membered) non-aromatic heterocycle or (iii) a 7 to 10 membered bridged heterocycle.

As the aforementioned "5 to 14 membered (preferably 5 to 10 membered) aromatic heterocycle", there are an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, and a ring formed by fusing these rings (preferably monocyclic) with 1 or a plurality (preferably 1 to 2) of aromatic rings (for example, benzene ring and the like).

As the aforementioned "5 to 10 membered non-aromatic heterocycle", for example, there are pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole and the like.

As the aforementioned "7 to 10 membered bridged heterocycle", for example, there are quinuclidine, 7-azabicyclo[2.2.1]heptane and the like.

The "heterocyclic group" is preferably a 5 to 14 membered (preferably 5 to 10 membered) (monocyclic or bicyclic) heterocyclic group containing preferably 1 to 4 heteroatoms of one or two kinds selected from a nitrogen atom, a sulfur atom and an oxygen atom in addition to carbon atoms. More particularly, examples thereof are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, and a non-aromatic heterocyclic group such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

Among them, for example, a 5 or 6 membered heterocyclic group containing 1 to 3 heteroatoms selected from a nitrogen atom, a sulfur atom and an oxygen atom in addition to carbon atoms is further preferable. More particularly, examples thereof are 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino and the like.

As "substituents" of "heterocyclic group optionally having substituents", for example, there are the same "substituents" as substituents of "hydrocarbon group optionally having substituents" represented by R^{5a}.

The "heterocyclic group" may have 1 to 5, preferably 1 to 3 aforementioned substituents at a substitutable position and, when the number of substituents is 2 or more, respective substituents may be the same or different.

As "C₁₋₆ alkyl" represented by R^{6a}, for example, there are methyl, ethyl, propyl, isopropyl, butyl, isobutyl, sec-butyl, tert-butyl, pentyl, hexyl and the like.

As "hydrocarbon group optionally having substituents" and "heterocyclic group optionally having substituents" represented by R^{7a}, for example, there are the aforementioned "hydrocarbon group optionally having substituents" and "heterocyclic group optionally having substituents" represented by R^{5a}, respectively.

As "hydrocarbon group optionally having substituents" and "heterocyclic group optionally having substituents" represented by R^{1a}, for example, there are the aforementioned "hydrocarbon group optionally having substituents" and "heterocyclic group optionally having substituents" represented by R^{5a}, respectively.

As "amino group optionally having substituents" represented by R^{1a}, for example, there are (1) an amino group optionally having 1 or 2 substituents and (2) a cyclic amino group optionally having substituents and the like.

As "substituents" of "amino group optionally having 1 or 2 substituents" of the aforementioned (1), for example, there are a hydrocarbon group optionally having substituents, a heterocyclic group optionally having substituents, an acyl group, an alkylidene group optionally having substituents and the like. As these "hydrocarbon group optionally having substituents" and "heterocyclic group optionally having substituents", there are the same "hydrocarbon group optionally having substituents" and "heterocyclic group optionally having substituents" as those represented by R^{5a} described above, respectively. As the "acyl group", there is the same "acyl group" as that by represented by R^{1a} as described above.

As "alkylidene group" of "alkylidene group optionally having substituents", for example, there are a C₁₋₆ alkylidene group (for example, methylidene, ethylidene, propylidene and the like) and the like. As "substituents" of "alkylidene group optionally having substituents", there are 1 to 5, preferably 1 to 3 same substituents as "substituents" of "hydrocarbon group optionally having substituents" represented by R^{5a}.

When the number of the aforementioned "substituents" of "amino group optionally having 1 or 2 substituents" is 2, respective substituents may be the same or different.

As "cyclic amino group" of "cyclic amino group optionally having substituents" of the aforementioned (2), there are a 5 to 7 membered non-aromatic cyclic amino group optionally containing 1 to 4 heteroatoms of one or two kinds selected from a nitrogen atom, a sulfur atom and an oxygen atom in addition to one nitrogen atom and carbon atoms. More particularly, examples thereof are pyrrolidin-1-yl, piperidino, piperazin-1-yl, morpholino, thiomorpholino, hexahydroazepin-1-yl, imidazolidin-1-yl, 2,3-dihydro-1H-imidazol-1-yl, tetrahydro-1(2H)-pyrimidinyl, 3,6-dihydro-1(2H)-pyrimidinyl, 3,4-dihydro-1(2H)-pyrimidinyl and the like. As "substituents" of "cyclic amino optionally having substituents", there are 1 to 3 of the same ones as "substituents" of "5 to 7 membered saturated cyclic amino group" which were described in detail as "substituents" of "hydrocarbon group optionally having substituents" represented by R^{5a}.

Examples of the 5 to 7 membered non-aromatic cyclic amino group having 1 oxo, there are 2-oxoimidazolidin-1-yl, 2-oxo-2,3-dihydro-1H-imidazol-1-yl, 2-oxotetrahydro-1(2H)-pyrimidinyl, 2-oxo-3,6-dihydro-1(2H)-pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl and the like.

As R^{1a}, an amino group optionally having substituents, an aryl group optionally having substituents and an alkyl group optionally having substituents and the like are preferable.

As further preferable example of the "amino group optionally having substituents" is an amino group optionally having 1 or 2 acyl groups represented by the formula: -(C=O)-R^{5a}, -(C=O)-OR^{5a}, -(C=O)-NR^{5a}R^{6a}, -(C=S)-NHR^{5a} or -SO₂-R^{7a} [wherein respective symbols represent the same meanings as described above]. Particularly preferable example is an amino group optionally having 1 or 2 acyl groups represented by the formula: -C(C=O)-R^{5a} or -(C=O)-NR^{5a}R^{6a} [wherein respective symbols represent the same meanings as described above].

As the "aryl group optionally having substituents", for example, there is preferably a C₆₋₁₄ aryl group (preferably a phenyl group and the like) optionally having 1 to 5 substituents selected from C₁₋₆ alkylthio, C₆₋₁₄ arylthio, C₁₋₆ alkylsulfinyl, C₆₋₁₄ arylsulfinyl, C₁₋₆ alkylsulfonyl, C₆₋₁₄ arylsulfonyl and carboxy.

As the "alkyl group optionally having substituents", for example, a C₁₋₆ alkyl group (for example, methyl, ethyl, propyl, isopropyl, butyl, isobutyl, sec-butyl, tert-butyl and the like) optionally substituted with 1 to 3 substituents selected from halogen atom, C₁₋₆ alkoxy, hydroxy, carboxy and C₁₋₆ alkoxy-carbonyl and the like are preferable, and particularly C₁₋₃ alkyl groups such as methyl, ethyl and the like is preferable.

Among them, as R^{1a}, (i) C₁₋₆ alkyl group (for example, C₁₋₄ alkyl group such as methyl, ethyl, propyl, butyl), (ii) a C₆₋₁₄ aryl group (for example, a phenyl group) optionally substituted with substituents selected from C₁₋₆ alkylthio (for example, methylthio), C₁₋₆ alkylsulfonyl (for example, methylsulfonyl) and halogen atom (for example, chlorine atom, fluorine atom) or (iii) an amino group optionally having 1 or 2 acyl groups represented by the formula: -(C=O)-R^{5a}' (wherein R^{5a}' represents (1) a C₁₋₆ alkyl group (for example, C₁₋₃ alkyl group such as methyl) , (2) a C₆₋₁₄ aryl group (for example, a phenyl group) or (3) a 5 to 14 membered heterocyclic group containing 1 to 4 heteroatoms of one or two kinds selected from a nitrogen atom, a sulfur atom and an oxygen atom in addition to carbon atoms (for example, a 5 to 6 membered heterocyclic group containing 1 to 2 heteroatoms selected from a nitrogen atom, a sulfur atom and an oxygen atom in addition to carbon atoms such as pyridyl group) are preferable. As R^{5a}' and R^{5a}", a phenyl group or a pyridyl group is suitable.

In the aforementioned formula, R^{2a} represents an aromatic group optionally having substituents.

As "aromatic group" of "aromatic group optionally having substituents" represented by R^{2a}, for example, there are an aromatic hydrocarbon group, an aromatic heterocyclic group and the like.

As the "aromatic hydrocarbon group", examples thereof include a C₆₋₁₄ monocyclic or fused polycyclic (bicyclic or tricyclic) aromatic hydrocarbon group, etc. As examples, there are a C₆₋₁₄ aryl group and the like such as phenyl, 1-naphthyl, 2-naphthyl, 2-biphenylyl, 3-biphenylyl, 4-biphenylyl, 2-anthryl and the like and, further preferably, a C₆₋₁₀ aryl group and the like (for example, phenyl, 1-naphthyl, 2-naphthyl and the like, preferably phenyl and the like).

As the "aromatic heterocyclic group", there is a monovalent group obtained by removing one arbitrary hydrogen atom from 5 to 14 membered (preferably 5 to 10 membered) aromatic heterocycle containing 1 to 4 heteroatoms of one or two kinds selected from nitrogen atom, sulfur atom and oxygen atom in addition to carbon atoms.

As the aforementioned "5 to 14 membered (preferably 5 to 10 membered) aromatic heterocycle", for example, there are an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, and a ring formed by fusing these rings (preferably monocycle) with 1 or a plurality of (preferably 1 or 2) aromatic rings (for example, benzene ring and the like).

As the "aromatic heterocyclic group", there are preferably a 5 to 14 membered (preferably 5 to 10 membered)(monocyclic or bicyclic) aromatic heterocyclic group containing preferably 1 to 4 heteroatoms of one or two kinds selected from a nitrogen atom, a sulfur atom and an oxygen atom in addition to carbon atoms and the like and, more particularly, there are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like.

As "substituents" of "aromatic group optionally having substituents", there are 1 to 5, preferably 1 to 3 same substituents as "substituents" of "hydrocarbon group optionally having substituents" represented by R^{5a}. When the number of substituents is 2 or more, respective substituents may be the same or different.

As R^{2a}, (1) a C₆₋₁₄ aryl group optionally having substituents and (2) a 5 to 14 membered aromatic heterocyclic group containing 1 to 4 heteroatoms of one or two kinds selected from a nitrogen atom, a sulfur atom and an oxygen atom in addition to carbon atoms are preferable and, among them, (1) a C₆₋₁₄ aryl group (for example, phenyl group, naphthyl group) optionally substituted with halogen atom (for example, chlorine atom, fluorine atom) or C₁₋₆ alkoxy (for example, methoxy), (2) a 5 to 14 membered aromatic heterocyclic group containing 1 to 4 heteroatoms of one or two kinds selected from a nitrogen atom, a sulfur atom and an oxygen atom in addition to carbon atoms (for example, a 5 to 6 membered aromatic heterocyclic group containing 1 to 2 heteroatoms selected from a nitrogen atom, a sulfur atom and an oxygen atom in addition to carbon atoms such as pyridyl group, thienyl group) and the like are preferable and, in particular, a phenyl group, a pyridyl group and the like are suitable.

In the aforementioned formula, R^{3a} represents a hydrogen atom, a pyridyl group optionally having substituents or an aromatic hydrocarbon group optionally having substituents.

As "substituents" of "pyridyl group optionally having substituents" represented by R^{3a}, there are the same substituents as "substituents" of "hydrocarbon group optionally having substituents" represented by R^{5a}.

The "pyridyl group" may, for example, have 1 to 5, preferably 1 to 3 aforementioned substituents at substitutable positions and, when the number of substituents is 2 or more, respective substituents may be the same or different. In addition, an intracyclic nitrogen atom may be N-oxidized.

As "aromatic hydrocarbon group" of "aromatic hydrocarbon group optionally having substituents" represented by R^{3a}, there is the same aromatic hydrocarbon group as "aromatic hydrocarbon group" of "aromatic group optionally having substituents" represented by R^{2a} and, preferably, there are a C₆₋₁₄ aryl group and the like such as phenyl, 1-naphthyl, 2-naphthyl, 2-biphenylyl, 3-biphenylyl, 4-biphenylyl, 2-anthryl and the like and, further preferably, a C₆₋₁₀ aryl group and the like (for example, phenyl, 1-naphthyl, 2-naphthyl and the like, preferably phenyl and the like) and the like. As "substituents" of "aromatic hydrocarbon group optionally having substituents" represented by R^{3a}, there are the same substituents as substituents of "aromatic group optionally having substituents" represented by R^{2a}.

As R^{3a}, a C₆₋₁₄ aryl group optionally having substituents is preferable and, among them, a C₆₋₁₄ aryl group optionally substituted with 1 or 2 C₁₋₆ alkyl (for example, methyl, ethyl and the like) or C₁₋₆ alkoxy groups (for example, methoxy, ethoxy and the like) is preferable and, in particular, a phenyl group optionally substituted with 1 or 2 C₁₋₆ alkyl or C₁₋₆ alkoxy groups (for example, 3-methoxyphenyl, 2-methylphenyl, 2,4-dimethylphenyl and the like) is suitable.

In the aforementioned formula, X^{a} represents an oxygen atom or an optionally oxidized sulfur atom.

As "optionally oxidized sulfur atom" represented by X^{a}, there are S, SO and SO₂.

As X^{a}, there is preferably an optionally oxidized sulfur atom. Further preferably, it is S.

In the aforementioned formula, Y^{a} represents a bond, an oxygen atom, an optionally oxidized sulfur atom or the formula NR^{4a} (wherein R^{4a} represents a hydrogen atom, a hydrocarbon group optionally having substituents or an acyl group).

As "optionally oxidized sulfur atom" represented by Y^{a}, there are S, SO and SO₂.

As "hydrocarbon group optionally having substituents" represented by R^{4a}, for example, there is the same group as "hydrocarbon group optionally having substituents" represented by R^{5a}. Among them, a C₁₋₆ alkyl group such as methyl, ethyl and the like and, in particular, a C₁₋₃ alkyl group such as methyl and the like is preferable.

As "acyl group" represented by R^{4a}, there is the same group as "acyl group" represented by R^{1a}.

As Y^{a}, an oxygen atom, an optionally oxidized sulfur atom, a group represented by the formula NR^{4a} (wherein R^{4a} represents the same meaning as that described above) and the like are preferable and, among them, an oxygen atom, an optionally oxidized sulfur atom, a group represented by the formula NR^{4a}' (R^{4a}' represents a hydrogen atom or a C₁₋₆ alkyl group) and the like are preferable and, further, an oxygen atom, S, SO₂, NH, N(CH₃) and the like are preferable and, in particular, O or NH is suitable.

In the aforementioned formula, Z^{a} represents a bond or a divalent acyclic hydrocarbon group optionally having substituents.

As "divalent acyclic hydrocarbon group" of "divalent acyclic hydrocarbon group optionally having substituents" represented by Z^{a}, for example, there are a C₁₋₁₅ alkylene group (for example, methylene, ethylene, trimethylene, tetramethylene, pentamethylene, hexamethylene, heptamethylene, octamethylene and the like, preferably a C₁₋₆ alkylene group and the like), a C₂₋₁₆ alkenylene group (for example, vinylene, propenylene, 1-butenylene, 2-butenylene, 1-pentenylene, 2-pentenylene, 3-pentenylene and the like), a C₂₋₁₆ alkynylene group (ethynylene, propynylene, 1-butynylene, 2-butynylene, 1-pentynylene, 2-pentynylene, 3-pentynylene and the like) and the like, preferably, a C₁₋₁₅ alkylene group, particularly preferably, a C₁₋₆ alkylene group and the like. As "substituents" of "divalent acyclic hydrocarbon group optionally having substituents" represented by Z^{a}, for example, there are the same substituents as "substituents" of "hydrocarbon group optionally having substituents" represented by R^{5a}.

As Z^{a}, a lower alkylene group optionally having C₁₋₃ alkyl (for example, methyl), oxo and the like (for example, a C₁₋₆ alkylene group such as methylene, ethylene, trimethylene and the like, in particular, a C₁₋₃ alkylene group) is preferable and, among them, a C₁₋₆ alkylene group optionally having oxo (for example, a C₁₋₃ alkylene group such as methylene, ethylene, trimethylene, in particular, methylene) is suitable.

More particularly, as Z^{a}, -CH₂-, -(CH₂)₂-, -(CH₂)₃-, -CO-, -CH₂CO-, -(CH₂)₂CO-, -CH(CH₃)- and the like are used and, in particular, -CH₂-, -CO- and the like are suitable.

A nitrogen atom in Compound (II) may be N-oxidized. For example, a nitrogen atom which is a constituent atom of 4-pyridyl group as a substituent at 5-position of a ring represented by the formula: 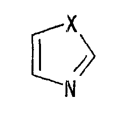 wherein a symbol in the formula represents the same meaning as that described above, may be N-oxidized. As Compound (II), for example, a compound represented by the formula: 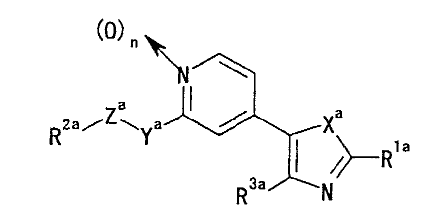 wherein n represents 0 or 1, and other symbols represents the same meanings as those described above, or salts thereof are preferable.

As Compound (II), compounds shown by the following (A) to (F) are preferably used.
(A) Compound (II) wherein R^{1a} is an amino group optionally having substituents, R^{2a} is a C₆₋₁₄ aryl group optionally having substituents, R^{3a} is a C₆₋₁₄ aryl group optionally having substituents, X is a sulfur atom, Y is an oxygen atom or a group represented by the formula NR^{4a} (wherein R^{4a} represents the same meaning as that described above) or (and) Z is a lower alkylene group optionally having substituents.
(B) Compound (II) wherein R^{1a} is (i) a C₁₋₆ alkyl group (for example, a C₁₋₄ alkyl group such as methyl, ethyl, propyl, butyl and the like),
   (ii) a C₆₋₁₄ aryl group (for example, a phenyl group) optionally substituted with substituents selected from C₁₋₆ alkylthio (for example, methylthio), C₁₋₆ alkylsulfonyl (for example, methylsulfonyl) and halogen atom (for example, chlorine atom, fluorine atom), or
   (iii) an amino group optionally having 1 or 2 acyl groups represented by the formula: -(C=O)-R^{5a}' [wherein R^{5a}' represents (1) a C₁₋₆ alkyl group (for example, C₁₋₃ alkyl group such as methyl and the like) , (2) a C₆₋₁₄ aryl group (for example, a phenyl group) or (3) a 5 to 14 membered heterocyclic group containing 1 to 4 heteroatoms of one or two kinds selected from a nitrogen atom, a sulfur atom and an oxygen atom in addition to carbon atoms (for example, a 5 to 6 membered heterocyclic group containing 1 to 2 heteroatoms selected from a nitrogen atom, a sulfur atom and an oxygen atom in addition to carbon atoms such as a pyridyl group);
      R^{2a} is a C₆₋₁₄ aryl group (for example, a phenyl group, a naphthyl group) optionally substituted with halogen atom (for example, chlorine atom, fluorine atom) or C₁₋₆ alkoxy (for example, methoxy), or a 5 to 14 membered aromatic heterocyclic group containing 1 to 4 heteroatoms of one or two kinds selected from a nitrogen atom, a sulfur atom and an oxygen atom in addition to carbon atoms (for example, a 5 to 6 membered aromatic heterocyclic group containing 1 to 2 heteroatoms selected from a nitrogen atom, a sulfur atom and an oxygen atom in addition to carbon atoms such as a pyridyl group, a thienyl group and the like);
      R^{3a} is a C₆₋₁₄ aryl group (particularly, a phenyl group) optionally substituted with 1 or 2 C₁₋₆ alkyl (for example, methyl) or C₁₋₆ alkoxy groups (for example, methoxy);
      X^{a} is a sulfur atom;
      Y^{a} is an oxygen atom, an optionally oxidized sulfur atom or a group represented by the formula NR^{4a}' (R^{4a}' is a hydrogen atom or a C₁₋₆ alkyl group) (in particular, an oxygen atom, S, SO₂, NH, N(CH₃) and the like);
      Z^{a} is a C₁₋₆ alkylene group (in particular, a C₁₋₃ alkylene group) optionally having oxo or C₁₋₆ alkyl (for example, C₁₋₃ alkyl such as methyl) or a bond.
(C) Compound (II) wherein R^{1a} is an amino group optionally having 1 or 2 acyl groups represented by the formula -(C=O)-R^{5a}" (wherein R^{5a}" represents (1) a C₆₋₁₄ aryl group (for example, phenyl group) or (2) a 5 to 14 membered heterocyclic group containing 1 to 4 heteroatoms of one or two kinds selected from a nitrogen atom, a sulfur atom and an oxygen atom in addition to carbon atoms (for example, a 5 to 6 membered heterocyclic group containing 1 to 2 heteroatoms selected from a nitrogen atom, a sulfur atom and an oxygen atom in addition to carbon atoms such as a pyridyl group);
   R^{2a} is a C₆₋₁₄ aryl group (for example, a phenyl group) or a 5 to 14 membered aromatic heterocyclic group containing 1 to 4 heteroatoms of one or two kinds selected from a nitrogen atom, a sulfur atom and an oxygen atom in addition to carbon atoms (for example, a 5 to 6 membered aromatic heterocyclic group containing 1 to 2 heteroatoms selected from a nitrogen atom, a sulfur atom and an oxygen atom in addition to carbon atoms such as a pyridyl group);
   R^{3a} is a C₆₋₁₄ aryl group (in particular, a phenyl group) optionally substituted with 1 or 2 C₁₋₆ alkyl (for example, methyl) or C₁₋₆ alkoxy groups (for example, methoxy);
   X^{a} is a sulfur atom;
   Y^{a} is O, NH or S;
   Z^{a} is a bond or a C₁₋₆ alkylene group optionally having oxo (in particular, a C₁₋₃ alkylene group, such as methylene, ethylene and the like).
(D) Compound (II) prepared in Reference Examples D 1-79.
(E) [4-(3,5-dimethylphenyl)-5-(2-phenylmethyloxy-4-pyridyl)-1,3-thiazol-2-yl]amine (Reference Example D Compound No. 1),
   N-[4-[2-benzoylamino-4-(4-methoxyphenyl)-1,3-thiazol-5-yl]-2-pyridyl]benzamide (Reference Example D Compound No. 2),
   N-[4-(4-methoxyphenyl)-5-[2-[(3-pyridylcarbonylamino)]-4-pyridyl]-1,3-thiazol-2-yl]nicotinamide (Reference Example D Compound No. 3),
   N-[4-[2-amino-4-(4-methoxyphenyl)-1,3-thiazol-5-yl]-2-pyridyl]benzamide (Reference Example D Compound No. 4),
   N-[4-[2-amino-4-(3,5-dimethylphenyl)-1,3-thiazol-5-yl]-2-pyridyl]benzamide (Reference Example D Compound No. 5),
   N-[4-[2-amino-4-(3,5-dimethylphenyl)-1,3-thiazol-5-yl]-2-pyridyl]benzylamine (Reference Example D Compound No. 6),
   N-[4-[2-amino-4-(3,5-dimethylphenyl)-1,3-thiazol-5-yl]-2-pyridyl]benzamide hydrochloride (Reference Example D Compound No. 7),
   N-[4-[2-amino-4-(3,5-dimethylphenyl)-1,3-thiazol-5-yl]-2-pyridyl]benzylamine dihydrochloride (Reference Example D Compound No. 8).
   (F) N-[5-[2-benzoylamino-4-pyridyl)-4-(3,5-dimethylphenyl)-1,3-thiazol-2-yl]acetamide (Reference Example D Compound No. 9),
   N-[5-(2-benzylamino-4-pyridyl)-4-(3,5-dimethylphenyl)-1,3-thiazol-2-yl]acetamide (Reference Example D Compound No. 10),
   N-[4-[4-(4-methoxyphenyl)-2-methyl-1,3-thiazol-5-yl]-2-pyridyl]benzamide (Reference Example D Compound No. 13),
   N-[4-[2-(4-fluorophenyl)-4-(3-methylphenyl)-1,3-thiazol-5-yl]-2-pyridyl]phenylacetamide (Reference Example D Compound No. 14),
   N-[4-[2-ethyl-4-(3-methylphenyl)-1,3-thiazol-5-yl]-2-pyridyl]phenylacetamide (Reference Example D Compound No. 15-2),
   N-[4-[4-(3-methylphenyl)-2-propyl-1,3-thiazol-5-yl]-2-pyridyl]phenylacetamide (Reference Example D Compound No. 15-3),
   N-[4-[2-butyl-4-(3-methylphenyl)-1,3-thiazol-5-yl]-2-pyridyl]phenylacetamide (Reference Example D Compound No. 15-4),
   N-[4-[4-(3-methylphenyl)-2-(4-methylthiophenyl)-1,3-thiazol-5-yl]-2-pyridyl]phenylacetamide (Reference Example D Compound No. 15-6),
   N-[4-[2-ethyl-4-(3-methylphenyl)-1,3-thiazol-5-yl]-2-pyridyl]benzamide (Reference Example D Compound No. 16-1),
   N-[4-[2-ethyl-4-(3-methylphenyl)-1,3-thiazol-5-yl]-2-pyridyl]-3-phenylpropionamide (Reference Example D Compound No. 16-2),
   N-[4-[2-ethyl-4-(3-methylphenyl)-1,3-thiazol-5-yl]-2-pyridyl]-3-(4-methoxyphenyl)propionamide (Reference Example D Compound No. 16-3),
   N-[4-[2-ethyl-4-(3-methylphenyl)-1,3-thiazol-5-yl] -2-pyridyl]-4-phenylbutyramide (Reference Example D Compound No. 16-5),
   N-[4-[4-(3-methylphenyl)-2-propyl-1,3-thiazol-5-yl]-2-pyridyl]benzamide (Reference Example D Compound No. 16-7),
   N-[4-[4-(3-methylphenyl)-2-propyl-1,3-thiazol-5-yl]-2-pyridyl]-3-phenylpropionamide (Reference Example D Compound No. 16-8),
   N-[4-[2-butyl-4-(3-methylphenyl)-1,3-thiazol-5-yl]-2-pyridyl]benzamide (Reference Example D Compound No. 16-9),
   N-[4-[2-butyl-4-(3-methylphenyl)-1,3-thiazol-5-yl]-2-pyridyl]-3-phenylpropionamide (Reference Example D Compound No. 16-10),
   N-[4-[2-(4-fluorophenyl)-4-(3-methylphenyl)-1,3-thiazol-5-yl]-2-pyridyl]benzamide (Reference Example D Compound No. 16-11),
   N-[4-[2-(4-fluorophenyl)-4-(3-methylphenyl)-1,3-thiazol-5-yl]-2-pyridyl]-3-phenylpropionamide (Reference Example D Compound No. 16-12),
   N-[4-[4-(3-methylphenyl)-2-(4-methylthiophenyl)-1,3-thiazol-5-yl]-2-pyridyl]benzamide (Reference Example D Compound No. 16-15),
   N-[4-[4-(3-methylphenyl)-2-(4-methylthiophenyl)-1,3-thiazol-5-yl]-2-pyridyl]-3-phenylpropionamide (Reference Example D Compound No. 16-16),
   N-benzyl-N-[4-[2-ethyl-4-(3-methylphenyl)-1,3-thiazol-5-yl]-2-pyridyl]amine (Reference Example D Compound No. 19-2),
   N-[4-[2-ethyl-4-(3-methylphenyl)-1,3-thiazol-5-yl]-2-pyridyl]-N-(2-phenylethyl)amine (Reference Example D Compound No. 19-3) ,
   N-[4-[2-ethyl-4-(3-methylphenyl)-1,3-thiazol-5-yl]-2-pyridyl]-N-(3-phenylpropyl)amine (Reference Example D Compound No. 19-4),
   N-benzyl-N-[4-[4-(3-methylphenyl)-2-propyl-1,3-thiazol-5-yl]-2-pyridyl]amine (Reference Example D Compound No. 19-5),
   N-[4-[4-(3-methylphenyl)-2-propyl-1,3-thiazol-5-yl]-2-pyridyl]-N-(2-phenylethyl)amine (Reference Example D Compound No. 19-6),
   N-[4-[4-(3-methylphenyl)-2-propyl-1,3-thiazol-5-yl]-2-pyridyl]-N-(3-phenylpropyl)amine (Reference Example D Compound No. 19-7),
   N-benzyl-N-[4-[2-butyl-4-(3-methylphenyl)-1,3-thiazol-5-yl]-2-pyridyl]amine (Reference Example D Compound No. 19-8),
   N-[4-[2-butyl-4-(3-methylphenyl)-1,3-thiazol-5-yl]-2-pyridyl]-N-(2-phenylethyl)amine (Reference Example D Compound No. 19-9),
   N-[4-[2-butyl-4-(3-methylphenyl)-1,3-thiazol-5-yl]-2-pyridyl]-N-(3-phenylpropyl)amine (Reference Example D Compound No. 19-10),
   N-benzyl-N-[4-[4-(3-methylphenyl)-2-(4-methylthiophenyl)-1,3-thiazol-5-yl]-2-pyridyl]amine (Reference Example D Compound No. 19-17),
   N-[4-[4-(3-methylphenyl)-2-(4-methylthiophenyl)-1,3-thiazol-5-yl]-2-pyridyl]-N-(2-phenylethyl)amine (Reference Example D Compound No. 19-18),
   N-[4-[4-(3-methylphenyl)-2-(4-methylthiophenyl)-1,3-thiazol-5-yl]-2-pyridyl]-N-(3-phenylpropyl)amine (Reference Example D Compound No. 19-19),
   N-[4-[4-(3-methylphenyl)-2-(4-methylsulfonylphenyl)-1,3-thiazol-5-yl]-2-pyridyl]benzamide (Reference Example D Compound No. 20),
   N-[4-[4-(3-methylphenyl)-2-(4-methylsulfonylphenyl)-1,3-thiazol-5-yl]-2-pyridyl]phenylacetamide (Reference Example D Compound No. 21-1),
   N-[4-[4-(3-methylphenyl)-2-(4-methylsulfonylphenyl)-1,3-thiazol-5-yl]-2-pyridyl]-3-phenylpropionamide (Reference Example D Compound No. 21-2),
   N-benzyl-N-[4-[4-(3-methylphenyl)-2-(4-methylsulfonylphenyl)-1,3-thiazol-5-yl]-2-pyridyl]amine (Reference Example D Compound No. 21-5),
   N-[4-[4-(3-methylphenyl)-2-(4-methylsulfonylphenyl)-1,3-thiazol-5-yl]-2-pyridyl]-N-(3-phenylpropyl)amine (Reference Example D Compound No. 21-6),
   N-[4-[4-(3-methylphenyl)-2-(4-methylsulfonylphenyl)-1,3-thiazol-5-yl]-2-pyridyl]-N-(2-phenylethyl)amine (Reference Example D Compound No. 25-1),
   N-(4-fluorobenzyl)-N-[4-[4-(3-methylphenyl)-2-(4-methylsulfonylphenyl)-1,3-thiazol-5-yl]-2-pyridyl]amine (Reference Example D Compound No. 25-2).

As a salt of Compound (II), for example, there are a metal salt, ammonium salt, a salt with an organic base, a salt with an inorganic acid, a salt with an organic acid, a salt with basic or acidic amino acid and the like. As a suitable metal salt, there are alkali metal salts such as sodium salt, potassium salt and the like; alkaline earth metal salts such as calcium salt, magnesium salt, barium salt and the like; aluminum salt and the like. As a suitable example of a salt with an organic base, for example, there are salts with trimethylamine, triethylamine, pyridine, picoline, 2,6-lutidine, ethanolamine, diethanolamine, triethanolamine, cyclohexylamine, dicyclohexylamine, N,N'-dibe:nzylethylenediamine and the like. As a suitable example of a salt with an inorganic acid, for example, there are salts with hydrochloric acid, hydrobromic acid, nitric acid, sulfuric acid, phosphoric acid and the like. As a suitable example of a salt with an organic acid, for example, there are salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. As a suitable example of a salt with a basic amino acid, for example, there are salts with arginine, lysine, ornithine and the like. As a suitable example of a salt with an acidic amino acid, for example, there are salts with aspartic acid, glutamic acid and the like.

Among them, pharmaceutically acceptable salts are preferable. For example, when a compound has an acidic functional group therein, there are inorganic salts such as alkali metal salts (for example, sodium salt, potassium salt and the like), alkaline earth metal salts (for example, calcium salt, magnesium salt, barium salt and the like), ammonium salts and the like and, when a compound has a basic functional group therein, there are salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like.

The compound (II) and a salt thereof can be produced according to the method described in WO00/64894.

In the above-mentioned formulas, R^{1a}, R^{2a}, R^{3a}, X^{a}, Y^{a} and Z^{a} are each correspond to R¹, R², R³, X, Y and Z, described in WO00/64894.

### [Compound (III)]

A compound represented by the formula 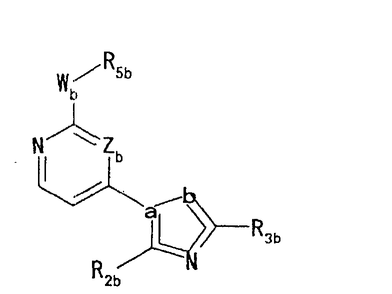 wherein
- a: is N or C;
- b: is CH when a is N, or O when a is C;
- =: denotes a single or a double bond dependent upon whether the azole ring is an imidazole or an oxazole ring;
- Z_{b}: is N or CH;
- W_{b}: is -NR_{6b}-Y_{b}-, -O- or -S-,
where R_{6b} is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y_{b}- is C₁₋₄ alkylene group or a bond;
- R_{2b}: is phenyl group, optionally substituted by one or more substituents selected from a halogen atom, trifluoromethyl, cyano, amido, thioamido, carboxylate, thiocarboxylate, C₁₋₄ alkoxy, C₁₋₄ alkyl, amino, and mono-or di-C₁₋₄ alkylamino;
- R_{3b}: is a hydrogen atom, a halogen atom, C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂ (wherein C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group and -CH=N-NH-C(NH)NH₂ are each optionally substituted by 1 to 4 substituents selected from C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5 to 7 membered N-heterocyclic group optionally further containing heteroatom(s));
- R_{5b}: is C₆₋₁₈ aryl group, C₃₋₁₈ heteroaryl group or C₃₋₁₂ cycloalkyl group each of which is optionally substituted by 1 to 4 substituents selected from C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5 to 7 membered N-heterocyclic group optionally further containing heteroatom(s), or a salt thereof.

The compound (III) and a salt thereof can be produced according to WO00/63204, and specifically, the compounds produced in Examples can be used.

In the above-mentioned formulas, R_{2b}, R_{3b}, R_{5b}, R_{6b}, Z_{b} and W_{b} respectively correspond to R₂, R₃, R₅, R₆, Z and W described in WO00/63204, pages 1-2.

A prodrug for the above-mentioned compounds (I) - (III) is a compound which is converted into compounds (I) - (III) under a physiological condition *in vivo* as a result of a reaction with an enzyme, gastric acid etc., thus a compound undergoing an enzymatic oxidation, reduction, hydrolysis etc. to convert into compounds (I) - (III) and a compound subjected to hydrolysis and the like by gastric acid etc. to convert into compounds (I) - (III). A prodrug for compounds (I)-(III) may be a compound obtained by subjecting an amino group in compounds (I) - (III) to an acylation, alkylation or phosphorylation (e.g., a compound obtained by subjecting an amino group in compounds (I) - (III) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group in compounds (I) - (III) to an acylation, alkylation, phosphorylation and boration (e.g., a compound obtained by subjecting a hydroxy group in compounds (I) - (III) to an acetylation, palmitoylation, propanoylation, pivaloylation, succinylation, fumarylation, alanylation, dimethylaminomethylcarbonylation, etc.); a compound obtained by subjecting a carboxyl group in compounds (I) - (III) to an esterification or amidation (e.g., a compound obtained by subjecting a carboxyl group in compounds (I) - (III) to an ethylesterification, phenylesterification, carboxymethylesterification, dimethylamino-methylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation, etc.) and the like. Any of these compounds can be produced from compounds (I) - (III) by a method known per se.

A prodrug for compounds (I) - (III) may also be one which is converted into compounds (I) - (III) under a physiological condition, such as those described in "IYAKUHIN no KAIHATSU (Development of Pharmaceuticals)", Vol.7, Design of Molecules, p.163-198, Published by HIROKAWA SHOTEN (1990).

In addition, as the p38 MAP kinase inhibitor and/or the TNF-α production inhibitor to be used in the present invention, the compounds described in WO98/57966, WO98/56377, WO98/25619, WO98/07425, WO98/06715, US5739143, WO97/35855, WO97/33883, WO97/32583, WO97/25048, WO97/25046, WO96/10143, WO96/21654, WO95/07922, WO2000/09525, , WO99/17776, WO99/01131, WO98/28292, WO97/25047, WO97/25045, US5658903, WO96/21452, WO99/18942, US5756499, US5864036, US6046208, US5716955, US5811549, US5670527, US5969184, WO2000/31072, WO2000/31063, WO2000/20402, WO2000/18738, WO2000/17175, WO2000/12497, WO2000/12074, WO2000/07991, WO2000/07980, WO2000/02561, US6096711, WO99/64400, WO99/61440, WO99/59959, WO99/58523, WO99/58502, WO99/57101, WO99/32111, WO99/32110, WO99/26657, WO99/20624, WO99/18942, WO99/15164, WO99/00357, WO98/52940, WO98/52937, WO98/52558, WO98/06715, WO97/22256, WO96/21452, WO2000/43366, WO2000/42003, WO2000/42002, WO2000/41698, WO2000/41505, WO2000/40243, WO2000/34303, WO2000/25791, WO2000/17204, WO2000/10563, US6080546, WO99/61426, WO99/32463, WO99/32121, WO99/17776, WO98/28292, WO98/27098, WO98/25619, WO98/20868, WO97/35855, WO97/32583, WO97/25048, WO97/25047, WO97/25046, WO97/25045, US5658903, WO96/40143, WO96/21654, WO2000/55153, WO2000/55120, WO2000/26209, US6046208, US5756499, US5864036, JP-A-2000-86657, WO99/59960, WO99/21859, WO99/03837 WO99/01449, WO99/01136, WO/, WO99/01130, US5905089, WO98/57966, WO98/52941, WO98/47899, WO98/07425, WO97/33883, WO2000/42213, WO99/58128, WO2000/04025, WO2000/40235, WO2000/31106, WO97/46228, WO2000/59904, WO2000/42003, WO2000/42002, WO2000/41698, WO2000/10563, WO99/61426, WO99/32463, US6002008, WO98/43960, WO98/27098, WO97/35856, WO97/35855, WO96/22985, JP-A-61-145167 and the like, and the like can be used.

In the present specification, the above-mentioned p38 MAP kinase inhibitor and/or the TNF-α inhibitor may sometimes be abbreviated as the compound of the present invention.

The p38 MAP kinase inhibitor and/or the TNF-α inhibitor such as the compounds (I)-(III) to be used in the present invention have a superior p38 MAP kinase inhibitory activity, TNF-α inhibitory activity (TNF-α production inhibitory activity, TNF-α action inhibitory activity), Interleukin-1 (IL-1) inhibitory activity, Interleukin-6 (IL-6) inhibitory activity, phosphodiesterase IV (PDE IV) inhibitory activity and the like, show low toxicity, and cause fewer side effects. Therefore, they are useful as a safe pharmaceutical product, p38 MAP kinase inhibitor, TNF-α production inhibitor, IL-1 inhibitor, IL-6 inhibitor, PDE IV inhibitor and the like.

The p38 MAP kinase inhibitor and/or the TNF-α inhibitor such as the compounds (I)-(III) to be used in the present invention show an excellent p38 MAP kinase inhibitory activity and a TNF-α inhibitory activity and are also excellent in (oral) absorption, (metabolism) stability and the like to a mammal (e.g., mouse, rat, hamster, rabbit, cat, dog, cow, sheep, monkey, human and the like) and, therefore, can be used as an agent for the prophylaxis or treatment of p38 MAP kinase related diseases and TNF-α production related diseases, such as asthma, chronic obstructive pulmonary disease (COPD), allergic disease (e.g., allergic dermatitis, allergic rhinitis), atopic dermatitis, inflammation, inflammatory eye disease, Addison's disease, autoimmune hemolytic anemia, systemic lupus erythematosus, Crohn's disease, psoriasis, rheumatism, central nervous disease (e.g., cerebrovascular disease such as cerebral hemorrhage and cerebral infarction, head trauma, spinal cord injury, brain edema, multiple sclerosis and the like), neurodegenerative disease (e.g., Alzheimer's disease, Parkinson's disease, amyotrophic lateral sclerosis (ALS), AIDS encephalopathy), meningitis, diabetes, arthritis (e.g., chronic rheumatoid arthritis, osteoarthritis, rheumatoid-like spondylitis, urarthritis, synovitis), osteoporosis, toxemia (e.g., sepsis, septic shock, endotoxic shock, Gram negative sepsis, toxic shock syndrome), inflammatory bowel disease (e.g., Crohn's disease, ulcerative colitis), inflammatory pulmonary disease (e.g., chronic pneumonia, pulmonary silicosis, pulmonary sarcoidosis, pulmonary tuberculosis) or cachexia (e.g., infectious cachexia, cancerous cachexia, cachexia by acquired immunodeficiency syndrome (AIDS)), arteriosclerosis, Creutzfeldt-Jakob disease, virus infection (e.g., infection with cytomegalovirus, influenzavirus, herpesvirus and the like), angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension, disseminated intravascular coagulation, and the like. Particularly, they can be used as an agent for the prophylaxis or treatment of chronic rheumatoid arthritis, osteoarthritis and the like.

In addition, the p38 MAP kinase inhibitor and/or the TNF-α inhibitor such as the compounds (I)-(III) to be used in the present invention have a PDE IV inhibitory activity and can be used as a prophylactic or therapeutic agent of diseases caused by inflammation, such as bronchial asthma, chronic obstructive pulmonary disease (COPD), chronic rheumatoid arthritis, autoimmune disease, diabetes, graft versus host disease, multiple sclerosis, sepsis, psoriasis, osteoporosis, depression, central hypergasia after cerebrovascular obstruction, cerebrovascular dementia, Alzheimer's dementia, obesity, cardiac failure and the like.

Therefore, the pharmaceutical agent of the present invention comprising the compound of the present invention and a concomitant drug to be explained later has a superior p38 MAP kinase inhibitory activity, TNF-α inhibitory activity (TNF-α production inhibitory activity, TNF-α action inhibitory activity), IL-1 inhibitory activity, IL-6 inhibitory activity, PDE IV inhibitory activity and the like, show low toxicity, and causes fewer side effects. Therefore, it is useful as a safe pharmaceutical product, p38 MAP kinase inhibitor, TNF-α production inhibitor, IL-1 inhibitor, IL-6 inhibitor, PDE IV inhibitor and the like.

The pharmaceutical agent of the present invention comprising the compound of the present invention and a concomitant drug to be explained later shows an excellent p38 MAP kinase inhibitory activity and a TNF-α inhibitory activity and is also excellent in (oral) absorption, (metabolism) stability and the like to a mammal (e.g., mouse, rat, hamster, rabbit, cat, dog, cow, sheep, monkey, human and the like) and, therefore, can be used as an agent for prophylaxis or treatment of p38 MAP kinase related diseases and TNF-α production related diseases, such as asthma, chronic obstructive pulmonary disease (COPD), allergic disease (e.g., allergic dermatitis, allergic rhinitis), atopic dermatitis, inflammation, inflammatory eye disease, Addison's disease, autoimmune hemolytic anemia, systemic lupus erythematosus, Crohn's disease, psoriasis, rheumatism, central nervous disease (e.g., cerebrovascular disease such as cerebral hemorrhage and cerebral infarction, head trauma, spinal cord injury, brain edema, multiple sclerosis and the like), neurodegenerative disease (e.g., Alzheimer's disease, Parkinson's disease, amyotrophic lateral sclerosis (ALS), AIDS encephalopathy), meningitis, diabetes, arthritis (e.g., chronic rheumatoid arthritis, osteoarthritis, rheumatoid-like spondylitis, urarthritis, synovitis), osteoporosis, toxemia (e.g., sepsis, septic shock, endotoxic shock, Gram negative sepsis, toxic shock syndrome), inflammatory bowel disease (e.g., Crohn's disease, ulcerative colitis), inflammatory pulmonary disease (e.g., chronic pneumonia, pulmonary silicosis, pulmonary sarcoidosis, pulmonary tuberculosis) or cachexia (e.g., infectious cachexia, cancerous cachexia, cachexia by acquired immunodeficiency syndrome (AIDS)), arteriosclerosis, Creutzfeldt-Jakob disease, virus infection (e.g., infection with cytomegalovirus, influenzavirus, herpesvirus and the like), angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension, disseminated intravascular coagulation, and the like. Particularly, it can be used as an agent for the prophylaxis or treatment of chronic rheumatoid arthritis, osteoarthritis and the like.

In addition, the pharmaceutical agent of the present invention comprising the compound of the present invention and a concomitant drug to be explained later has a PDE IV inhibitory activity and can be used as a prophylactic or therapeutic agent of diseases caused by inflammation, such as bronchial asthma, chronic obstructive pulmonary disease (COPD), chronic rheumatoid arthritis, autoimmune disease, diabetes, graft versus host disease, multiple sclerosis, sepsis, psoriasis, osteoporosis, depression, central hypergasia after cerebrovascular obstruction, cerebrovascular dementia, Alzheimer's dementia, obesity, cardiac failure and the like.

As the drugs that can be used in combination with the compound of the present invention (hereinafter the drug is sometimes abbreviated as a concomitant drug) includes, for example, the following.

### (1) non-steroidal antiinflammatory drugs (NSAIDs)

(i) classical NSAIDs
   alcofenac, aceclofenac, sulindac, tolmetin, etodolac, fenoprofen, thiaprofenic acid, meclofenamic acid, meloxicam, tenoxicam, lornoxicam, nabumeton, acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesilate, camostat mesilate, ulinastatin, colchicine, probenecid, sulfinpyrazone, benzbromarone, allopurinol, sodium aurothiomalate, hyaluronate sodium, sodium salicylate , morphine hydrochloride, salicylic acid, atropine, scopolamine, morphine, pethidine, levorphanol, oxymorphone or a salt thereof and the like.
(ii) cyclooxygenase inhibitor (COX-1 selective inhibitor, COX-2 selective inhibitor and the like)
   salicylic acid derivatives (e.g., celecoxib, rofecoxib, aspirin), MK-663, valdecoxib, SC-57666, tiracoxib, S-2474, diclofenac, indomethacin, loxoprofen and the like.
(iii) drug concurrently having COX inhibitory activity and 5-lipoxygenase inhibitory activity
   ML-3000, p54 (COX inhibitor & 5-lipoxygenase inhibitor) and the like.
(iv) nitric oxide-releasing NSAIDs

### (2) disease-modifying anti-rheumatic drugs (DMARDs)

(i) gold preparation
   Auranofin and the like.
(ii) penicillamine
   D-penicillamine
(iii) sulfasalazine
(iv) antimalarial drug
   chloroquine and the like.
(v) pyrimidine synthesis inhibitor
   leflunomide and the like.
(vi) prograf

### (3) anti-cytokine drug

### (I) protein drug

(i) TNF inhibitor
   etanercept, infliximab, D2E7, CDP-571, PASSTNF-α, soluble TNF-α receptor, TNF-α binding protein, anti-TNF-α antibody and the like.
(ii) interleukin-1 inhibitor
   anakinra (interleukin-1 receptor antagonist), soluble interleukin-1 receptor and the like.
(iii) interleukin-6 inhibitor
   MRA (anti-interleukin-6 receptor antibody), anti-interleukin-6 antibody and the like.
(iv) interleukin-10 drug
   interleukin-10 and the like.
(v) interleukin-12 inhibitor
   anti-interleukin-12 antibody and the like.
(vi) drug concurrently having interferon-α and -γ inhibitory activity and TNF-α inhibitory activity (polyclonal antibody) AGT-1

### (II) non-protein drug

(i) MAP kinase inhibitor
   PD-98059 and the like.
(ii) gene modulator
   SP-100030, inhibitor of molecule involved in signal transduction, such as NF-κ, NF-κB, IKK-1, IKK-2, AP-1 and the like
(iii) cytokine production inhibitor
   T-614, SR-31747, sonatimod and the like.
(iv) TNF-α converting enzyme inhibitor
(v) interleukin-1β converting enzyme inhibitor
   HMR3480/VX-740 and the like.
(vi) interleukin-6 antagonist
   SANT-7 and the like.
(vii) interleukin-8 inhibitor
   IL-8 antagonist, CXCR1 & CXCR2 antagonist and the like.
(viii) chemokine antagonist
   MCP-1 antagonist and the like.
(ix) interleukin-2 receptor antagonist
   denileukin diftitox and the like.
(x) therapeutic vaccines
   TNF-α vaccine and the like.
(xi) gene therapy drug
   gene therapy drugs aiming at promoting the expression of gene having an anti-inflammatory action such as interleukin-4, interleukin-10, soluble interleukin-1 receptor, soluble TNF-α receptor and the like.
(xii) antisense compound
   ISIS-104838 and the like.

### (4) immunomodulator (immunosuppressant)

(i) T cell differentiation modulator
   ethyl 6,7-dimethoxy-4-(3,4-dimethoxyphenyl)-2-(1,2,4-triazol-1-ylmethyl)quinoline-3-carboxylate (JP-A-7-118266)
(ii) others
   methotrexate, cyclophosphamide, MX-68, atiprimod dihydrochloride, BMS-188667, CKD-461, rimexolone, cyclosporine, tacrolimus, gusperimus, azathiopurine, antilymphocyte serum, freeze-dried sulfonated normal immunoglobulin, erythropoietin, colony stimulating factor, interleukin, interferon and the like.

### (5) steroid

dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclomethasone dipropionate, estriol and the like.

### (6) c-Jun N terminal kinase (JNK) inhibitor

compounds described in WO00/35906, WO00/35909, WO00/35921, WO00/64872 or WO00/75118 and the like.

### (7) angiotensin converting enzyme inhibitor

enalapril, captopril, ramipril, lisinopril, cilazapril, perindopril and the like.

### (8)angiotensin II receptor antagonist

candesartan cilexetil (TCV-116), valsartan, irbesartan, olmesartan, eprosartan and the like.

### (9) diuretic drug

hydrochlorothiazide, spironolactone, furosemide, indapamide, bendrofluazide, cyclopenthiazide and the like.

### (10) cardiotonic drug

digoxin, dobutamine and the like.

### (11) β receptor antagonist

carvedilol, metoprolol, atenolol and the like.

### (12) Ca sensitizer

MCC-135 and the like.

### (13) Ca channel antagonist

nifedipine, diltiazem, verapamil and the like.

### (14) anti-platelet drug, anticoagulator

heparin, aspirin, warfarin and the like.

### (15) HMG-CoA reductase inhibitor

atorvastatin, simvastatin and the like.

### (16) contraceptive

(i) sex hormone or derivatives thereof
   gestagen or a derivative thereof (progesterone, 17α-hydroxy progesterone, medroxyprogesterone, medroxyprogesterone acetate, norethisterone, norethisterone enanthate, norethindrone, norethindrone acetate, norethynodrel, levonorgestrel, norgestrel, ethynodiol diacetate, desogestrel, norgestimate, gestodene, progestin, etonogestrel, drospirenone, dienogest, trimegestone, nestorone, chlormadinone acetate, mifepristone, nomegestrol acetate, Org-30659, TX-525, EMM-310525) or a combination of a gestagen or a derivative thereof and an estrogen or a derivative thereof (estradiol, estradiol benzoate, estradiol cypionate, estradiol dipropionate, estradiol enanthate, estradiol hexahydrobenzoate, estradiol phenylpropionate, estradiol undecylate, estradiol valerate, estrone, ethinylestradiol, mestranol) and the like.
(ii) antiestrogen
   ormeloxifene, mifepristone, Org-33628 and the like.
(iii) spermatocide
   ucarcide and the like.

### (17) others

(i) T cell inhibitors
   IR-501 (T cell receptor peptide) and the like.
(ii) inosine monophosphate dehydrogenase (IMPDH) inhibitor mycophenolate mofetil, VX-497 and the like.
(iii) adhesion molecule inhibitor
   ISIS-2302, selectin inhibitor, ELAM-1, VCAM-1, ICAM-1 and the like.
(iv) thalidomide
(v) cathepsin inhibitor
(vi) matrix metalloprotease (MMPs) inhibitor
   BB-3644, CGS-27023A, Bay-12-9566, KB-R7785, L-758354, POL-641 and the like.
(vii) glucose-6-phosphate dehydrogenase inhibitor
   CBF-BS2 and the like.
(viii) hydroorotate dehydrogenase (DHODH) inhibitor
(ix) phosphodiesterase IV (PDE IV) inhibitor
   CG-1088 and the like.
(x) phospholipase A₂ inhibitor
(xi) iNOS inhibitor
   NOX-200 and the like.
(xii) microtubule stimulating drug
   paclitaxel and the like.
(xiii) microtubule inhibitor
   reumacon and the like.
(xiv) MHC class II antagonist
   ZD-2315 and the like.
(xv) prostacyclin agonist
   iloprost and the like.
(xvi) CD4 antagonist
   4162W94, keliximab and the like.
(xvii) CD23 antagonist
(xviii) LTB4 receptor antagonist
   CGS-25019C and the like.
(xix) 5-lipoxygenase inhibitor
   zileuton and the like.
(xx) cholinesterase inhibitor
   galanthamine and the like.
(xxi) tyrosine kinase inhibitor
   YT-146 and the like.
(xxii) cathepsin B inhibitor
(xxiii) adenosine deaminase inhibitor
   pentostatin and the like.
(xxiv) osteogenesis stimulator
   (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide or a salt thereof (JP-A-8-231659) and the like.
(xxv) dipeptidylpeptidase inhibitor
   TMC-2A and the like.
(xxvi) TRK-530, TOK-8801
(xxvii) collagen agonist
   AI-200 and the like.
(xxviii) capsaicin cream
(xxix) hyaluronic acid derivative
   synvisc (hylan G-F 20), orthovisc and the like.
(xxx) glucosamine sulfate
(xxxi) amiprilose

Other concomitant drugs besides the above-mentioned include, for example, antibacterial agent, antifungal agent, antiprotozoal agent, antibiotic, antitussive and expectorant drug, sedative, anesthetic, antiulcer drug, antiarrhythmic agent, hypotensive diuretic drug, anticoagulant, tranquilizer, antipsychotic, antitumor drug, hypolipidemic drug, muscle relaxant, anticonvulsant, antidepressant, antiallergic drug, cardiac, antiarrhythmic agent, vasodilator, vasoconstrictor, hypotensive diuretic drug, antidiabetic drug, antinarcotic, vitamin, vitamin derivative, antiasthmatic, therapeutic agent for pollakisuria/anischuria, therapeutic agent for atopic dermatitis, therapeutic agent for allergic rhinitis, hypertensor, endotoxin-antagonist or -antibody, signal transduction inhibitor, inhibitor of inflammatory mediator activity, antibody to inhibit inflammatory mediator activity, inhibitor of anti-inflammatory mediator activity, antibody to inhibit anti-inflammatory mediator activity and the like. Specific examples thereof include the following.

### (1) antibacterial agent

(1) sulfa drug
   sulfamethizole, sulfisoxazole, sulfamonomethoxine, sulfamethizole, salazosulfapyridine, silver sulfadiazine and the like.
(2) quinoline antibacterial agent
   nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
(3) antiphthisic
   isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
(4) antiacidfast bacterium drug
   diaphenylsulfone, rifampicin and the like.
(5) antiviral drug
   idoxuridine, acyclovir, vidarabine, gancyclovir and the like.
(6) anti-HIV agent
   zidovudine, didanosine, zalcitabine, indinavir sulfate ethanolate, ritonavir and the like.
(7) antispirochetele
(8) antibiotic
   tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)], azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone, fluconazole, itraconazole] and the like.

### (2) antifungal agent

(1) polyethylene antibiotic (e.g., amphotericin B, nystatin, trichomycin)
(2) griseofulvin, pyrrolnitrin and the like.
(3) cytosine metabolism antagonist (e.g., flucytosine)
(4) imidazole derivative (e.g., econazole, clotrimazole, miconazole nitrate, bifonazole, croconazole)
(5) triazole derivative (e.g. fluconazole, itraconazole)
(6) thiocarbamic acid derivative (e.g. trinaphthol)

### (3) antiprotozoal agent

metronidazole, tinidazole, diethylcarbamazine citrate, quinine hydrochloride, quinine sulfate and the like.

### (4) antitussive and expectorant drug

ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, cloperastine, protokylol, isoproterenol, salbutamol, terbutaline, oxymetebanol, morphine hydrochloride, dextromethorfan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.

### (5) sedative

chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.

### (6) anesthetic

### (6-1) local anesthetic

cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine) and the like.

### (6-2) general anesthetic

(A) inhalation anesthetic (e.g., ether, halothane, nitrous oxide, isoflurane, enflurane),
(B) intravenous anesthetic (e.g., ketamine hydrochloride, droperidol, thiopental sodium, thiamylal sodium, pentobarbital) and the like.

### (7) antiulcer drug

histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.

### (8) antiarrhythmic agent

(1) Na channel blocker (e.g., quinidine, procainamide, disopyramide, ajmaline, lidocaine, mexiletine, phenytoin),
(2) β-blocker (e.g., propranolol, alprenolol, bufetolol hydrochloride, oxprenolol, atenolol, acebutolol, metoprolol, bisoprolol, pindolol, carteolol, arotinolol,
(3) K channel blocker (e.g., amiodarone),
(4) Ca channel blocker (e.g., verapamil, diltiazem) and the like.

### (9) hypotensive diuretic drug

hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophylline and the like.

### (10) anticoagulant

heparin sodium, sodium citrate, activated protein C, tissue factor pathway inhibitor, antithrombin III, dalteparin sodium, warfarin potassium, argatroban, gabexate, sodium citrate, ozagrel sodium, ethyl icosapentate, beraprost sodium, alprostadil, ticlopidine hydrochloride, pentoxifylline, dipyridamole, tisokinase, urokinase, streptokinase and the like.

### (11) tranquilizer

diazepam, lorazepam, oxazepam, chlordiazepoxide, medazepam, oxazolam, cloxazolam, clotiazepam, bromazepam, etizolam, fludiazepam, hydroxyzine and the like.

### (12) antipsychotic

chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.

### (13) antitumor drug

6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuprorelin acetate, buserelin acetate and the like.

### (14) antihypolipidemic drug

clofibrate, ethyl 2-chloro-3-[4-(2-methyl-2-phenylpropoxy)-phenyl]propionate [Chemical and Pharmaceutical Bulletin (Chem. Pharm. Bull), 38, 2792-2796 (1990)], pravastatin, simvastatin, probucol, bezafibrate, clinofibrate, nicomol, cholestyramine, dextran sulfate sodium and the like.

### (15) muscle relaxant

pridinol, tubocurarine, pancuronium, tolperisone hydrochloride, chlorphenesin carbamate, baclofen, chlormezanone, mephenesin, chlorzoxazone, eperisone, tizanidine and the like.

### (16) anticonvulsant

phenytoin, ethosuximide, acetazolamide, chlordiazepoxide, trimethadione, carbamazepine, phenobarbital, primidone, sulthiame, sodium valproate, clonazepam, diazepam, nitrazepam and the like.

### (17) antidepressant

imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.

### (18) antiallergic drug

diphenhydramine, chlorpheniramine, tripelennamine, metodilamine, clemizole, diphenylpyraline, methoxyphenamine, sodium cromoglicate, tranilast, repirinast, amlexanox, ibudilast, ketotifen, terfenadine, mequitazine, azelastine hydrochloride, epinastine, ozagrel hydrochloride, pranlukast hydrate, seratrodast and the like.

### (19) cardiac

trans-π-oxocamphor, terephyllol, aminophylline, etilefrine, dopamine, dobutamine, denopamine, aminophylline, bencirin, amrinone, pimobendan, ubidecarenone, digitoxin, digoxin, methyldigoxin, lanatoside C, G-strophanthin and the like.

### (20) vasodilator

oxyfedrine, diltiazem, tolazoline, hexobendine, bamethan, clonidine, methyldopa, guanabenz and the like.

### (21) vasoconstrictor

dopamine, dobutamine denopamine and the like.

### (22) hypotensive diuretic drug

hexamethonium bromide, pentolinium, mecamylamine, ecarazine, clonidine, diltiazem, nifedipine and the like.

### (23) antidiabetic drug

tolbutamide, chlorpropamide, acetohexamide, glibenclamide, tolazamide, acarbose, epalrestat, troglitazone, glucagon, glymidine, glipizide, phenformin, buformin, metformin and the like.

### (24) antinarcotic

levallorphan, nalorphine, naloxone or a salt thereof and the like.

### (25) fat-soluble vitamin

(1) vitamin A: vitamin A₁, vitamin A₂ and retinol palmitate
(2) vitamin D: vitamin D₁, D₂, D₃, D₄ and D₅
(3) vitamin E: α-tocopherol, β-tocopherol, γ-tocopherol, δ-tocopherol, dl-α-tocopherol nicotinate
(4) vitamin K: vitamin K₁, K₂, K₃ and K₄
(5) folic acid (vitamin M) and the like.

### (26) vitamin derivative

various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.

### (27) antiasthmatic

isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglicate, tranilast, repirinast, amlexanox, ibudilast, ketotifen, terfenadine, mequitazine, azelastine, epinastine, ozagrel hydrochloride, pranlkast hydrate, seratrodast, dexamethasone, prednisolone, hydrocortisone, hydrocortisone sodium succinate, beclometasone dipropionate and the like.

### (28) therapeutic agent for pollakisuria/anischuria

flavoxate hydrochloride and the like.

### (29) therapeutic agent for atopic dermatitis

sodium cromoglicate and the like.

### (30) therapeutic agent for allergic rhinitis

sodium cromoglicate, chlorpheniramine maleate, alimemazine tartrate, clemastine fumarate, homochlorcyclizine hydrochloride, terfenadine, mequitazine and the like.

### (31) hypertensive drug

dopamine, dobutamine, denopamine, digitoxin, digoxin, methyldigoxin, lanatoside C, G-strophanthin and the like.

### (32) Others

hydroycam, diacerein, megestrol acetate, nicergoline, prostaglandins and the like.

By combining the compound of the present invention and a concomitant drug, a superior effect such as
(1) the dose can be reduces as compared to single administration of the compound of the present invention or a combination drug,
(2) the drug to be combined with the compound of the present invention can be selected according to the condition of patients (mild case, severe case and the like),
(3) the period of treatment can be set longer by selecting a combination drug having different action and mechanism from the compound of the present invention,
(4) a sustained treatment effect can be designed by selecting a combination drug having different action and mechanism from the compound of the present invention,
(5) a synergistic effect can be afforded by a combined use of the compound of the present invention and a combination drug, and the like, can be achieved.

In the present specification, a pharmaceutical agent comprising the compound of the present invention and a concomitant drug may be referred to as the "combination agent of the present invention".

As regards the use of the combination agent of the present invention, the administration time of the compound of the present invention and the concomitant drug is not restricted, and the compound of the present invention or the concomitant drug can be administered to an administration subject simultaneously, or may be administered at different times. In addition, the combination agent of the present invention can be used after synovectomy, after treatment with Prosorba column, after mononuclear cell therapy, and the like. The dosage of the concomitant drug may be determined according to the dose clinically used, and can be appropriately selected depending on an administration subject, administration route, disease, combination and the like.

The administration mode of the compound of the present invention and the concomitant drug of the present invention is not particularly restricted, and it is sufficient that the compound of the present invention and the concomitant drug are combined in administration. Examples of such administration mode include the following methods:
(1) The compound of the present invention and the concomitant drug are simultaneously produced to give a single preparation which is administered. (2) The compound of the present invention and the concomitant drug are separately produced to give two kinds of preparations which are administered simultaneously by the same administration route. (3) The compound of the present invention and the concomitant drug are separately produced to give two kinds of preparations which are administered by the same administration route only at the different times. (4) The compound of the present invention and the concomitant drug are separately produced to give two kinds of preparations which are administered simultaneously by the different administration routes. (5) The compound of the present invention and the concomitant drug are separately produced to give two kinds of preparations which are administered by the different administration routes only at different times (for example, the compound of the present invention and the concomitant drug are administered in this order, or in the reverse order).

A combination agent of the present invention has low toxicity, and for example, the compound of the present invention or (and) the above-mentioned concomitant drug can be mixed, according to a method known per se, with a pharmacologically acceptable carrier to give pharmaceutical compositions, for example, tablets (including a sugar-coated tablet, film-coated tablet), powders, granules, capsules (including a soft capsule), solutions, injections, suppositories, sustained release agents and the like which can be safely administered orally or parenterally (e.g., local, rectum, vein, and the like). An injection can be administered by intravenous, intramuscular, subcutaneous or intraorgan route, or directly to the lesion.

As a pharmacologically acceptable carrier which may be used for preparing a preparation of a combination agent of the present invention, there are the various conventional organic or inorganic carriers as pharmaceutical materials, for example, excipient, lubricant, binder and disintegrating agent in solid preparations, or solvent, solubilizing agent, suspending agent, isotonizing agent, buffer and soothing agent in liquid preparations. Further, if needed, additives such as the conventional preservative, antioxidant, colorant, sweetening agent, adsorbing agent, wetting agent and the like can be appropriately used in an appropriate amount.

As an excipient, for example, there are lactose, sucrose, D-mannitol, starch, corn starch, microcrystalline cellulose, light anhydrous silicic acid and the like.

As a lubricant, for example, there are magnesium stearate, calcium stearate, talc, colloidal silica and the like.

As a binder, for example, there are microcrystalline cellulose, sucrose, D-mannitol, dextrin, hydroxypropylcellulose, hydroxypropylmethylcellulose, polyvinylpyrrolidone, starch, saccharose, gelatin, methylcellulose, sodium carboxymethylcellulose and the like.

As a disintegrating agent, for example, there are starch, carboxymethylcellulose, calcium carboxymethylcellulose, sodium carboxymethylstarch, L-hydroxypropylcellulose and the like.

As a solvent, for example, there are water for injection, alcohol, propylene glycol, macrogol, sesame oil, corn oil, olive oil and the like.

As a solubilizing agent, for example, there are polyethylene glycol, propylene glycol, D-mannitol, benzyl benzoate, ethanol, tris-aminomethane, cholesterol, triethanolamine, sodium carbonate, sodium citrate and the like.

As a suspending agent, for example, there are surfactants such as stearyl triethenolamine, sodium lauryl sulfate, lauryl aminopropionate, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydoxypropylcellulose and the like.

As an isotonizing agent, for example, there are glucose, D-sorbitol, sodium chloride, glycerin, D-mannitol and the like.

As a buffer, for example, there are buffering solutions such as phosphate, acetate, carbonate, citrate and the like.

As a soothing agent, for example, there are benzyl alcohol and the like.

As a preservative, for example, there are p-hydroxybenzoates, chlorobutanol, benzyl alcohol, phenethyl alcohol, dehydroacetic acid, sorbic acid and the like.

As an antioxidant, for example, there are sulfites, ascorbic acid, α-tocopherol and the like.

The compounding ratio of the compound of the present invention to the concomitant drug in the combination agent of the present invention can be appropriately selected depending on an administration subject, administration route, diseases and the like.

For example, the content of the compound of the present invention in the combination agent of the present invention differs depending on the form of a preparation, and usually from about 0.01 to 100% by weight, preferably from about 0.1 to 50% by weight, further preferably from about 0.5 to 20% by weight, based on the preparation.

The content of the concomitant drug in the combination agent of the present invention differs depending on the form of a preparation, and usually from about 0.01 to 100% by weight, preferably from about 0.1 to 50% by weight, further preferably from about 0.5 to 20% by weight, based on the preparation.

The content of additives such as a carrier and the like in the combination agent of the present invention differs depending on the form of a preparation, and usually from about 1 to 99.99% by weight, preferably from about 10 to 90% by weight, based on the preparation.

In the case when the compound of the present invention and the combination drug are separately prepared respectively, the same contents may be adopted.

These preparations can be produced by a method known per se usually used in a preparation process.

For example, the compound of the present invention and the concomitant drug can be made into an aqueous injection together with a dispersing agent (e.g., Tween 80 (manufactured by Atlas Powder, US), HCO 60 (manufactured by Nikko Chemicals), polyethylene glycol, carboxymethylcellulose, sodium alginate, hydroxypropylmethylcellulose, dextrin and the like), a stabilizer (e.g., ascorbic acid, sodium pyrosulfite, and the like), a surfactant (e.g., Polysorbate 80, macrogol and the like), a solubilizer (e.g., glycerin, ethanol and the like), a buffer (e.g., phosphoric acid and alkali metal salt thereof, citric acid and alkali metal salt thereof, and the like), an isotonizing agent (e.g., sodium chloride, potassium chloride, mannitol, sorbitol, glucose and the like), a pH regulator (e.g., hydrochloric acid, sodium hydroxide and the like), a preservative (e.g., ethyl p-hydroxybenzoate, benzoic acid, methyl p-hydroxybenzoate, propyl p-hydroxybenzoate, benzyl alcohol and the like), a dissolving agent (e.g., conc. glycerin, meglumine and the like), a dissolution aid (e.g., propylene glycol, sucrose and the like), a soothing agent (e.g., glucose, benzyl alcohol and the like), and the like, or can be dissolved, suspended or emulsified in a vegetable oil such as olive oil, sesame oil, cotton seed oil, corn oil and the like or a dissolution aid such as propylene glycol and molded into an oily injection.
In the case of a preparation for oral administration, an excipient (e.g., lactose, sucrose, starch and the like), a disintegrating agent (e.g., starch, calcium carbonate and the like), a binder (e.g., starch, acacia, carboxymethylcellulose, polyvinylpyrrolidone, hydroxpropylcellulose and the like), a lubricant (e.g., talc, magnesium stearate, polyethylene glycol 6000 and the like) and the like, for example, can be added to the compound of the present invention or the combination drug, according to a method known per se, and the mixture can be compression-molded, then if desirable, the molded product can be coated by a method known per se for the purpose of masking of taste, enteric property or durability, to obtain a preparation for oral administration. As this coating agent, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (methacrylic acid·acrylic acid copolymer, manufactured by Rohm, DE), pigment (e.g., iron oxide red, titanium dioxide, et.) and the like can be used. The preparation for oral administration may be any of a quick release preparation and a sustained release preparation.

For example, in the case of a suppository, the compound of the present invention and the combination drug can be made into an oily or aqueous solid, semisolid or liquid suppository according to a method known per se. As the oily substrate used in the above-mentioned composition, for example, glycerides of higher fatty acids [e.g., cacao butter, Witepsols (manufactured by Dynamite Novel, DE), etc.], intermediate grade fatty acids [e.g., Miglyols (manufactured by Dynamite Nobel, DE), etc.], or vegetable oils (e.g., sesame oil, soy bean oil, cotton seed oil and the like), and the like are listed. Further, as the aqueous substrate, for example, polyethylene glycols, propylene glycol are listed, and as the aqueous gel substrate, for example, natural gums, cellulose derivatives, vinyl polymers, acrylic acid polymers and the like are listed.

As the above-mentioned sustained release agent, sustained release microcapsules and the like are listed.

For obtaining a sustained release microcapsule, a method known per se can be adopted, and for example, it is preferably molded into a sustained release preparation shown in the following [2] before administration.

A compound of the present invention is preferably molded into an oral administration preparation such as a solid preparation (e.g., powder, granule, tablet, capsule) and the like, or molded into a rectal administration preparation such as a suppository. Particularly, an oral administration preparation is preferable.

The concomitant drug can be made into the above-mentioned drug form depending on the kind of the drug.
[1] An injection of the compound of the present invention or the concomitant drug, and preparation thereof, [2] a sustained release preparation or quick release preparation of the compound of the present invention or the concomitant drug, and preparation thereof, [3] a sublingual, buccal or intraoral quick integrating agent of the compound of the present invention or the concomitant drug, and preparation thereof, will be described below specifically.

### [1] Injection and preparation thereof

An injection prepared by dissolving the compound of the present invention or the concomitant drug into water is preferable. This injection may be allowed to contain a benzoate and/or salicylate.

The injection is obtained by dissolving the compound of the present invention or the concomitant drug, and if desirable, a benzoate and/or salicylate, into water.

As the above-mentioned salts of benzoic acid and salicylic acid, for example, salts of alkali metals such as sodium, potassium and the like, salts of alkaline earth metals such as calcium, magnesium and the like, ammonium salts, meglumine salts, organic acid salts such as tromethamol and the like, etc. are listed.

The concentration of the compound of the present invention or the concomitant drug in an injection is from 0.5 to 50 w/v%, preferably from about 3 to 20 w/v%. The concentration of a benzoate salt or/and salicylate salt is from 0.5 to 50 w/v%, preferably from 3 to 20 w/v%.

Into a preparation of the present invention, additives usually used in an injection, for example, a stabilizer (ascorbic acid, sodium pyrosulfite, and the like), a surfactant (Polysorbate 80, macrogol and the like), a solubilizer (glycerin, ethanol and the like), a buffer (phosphoric acid and alkali metal salt thereof, citric acid and alkali metal salt thereof, and the like), an isotonizing agent (sodium chloride, potassium chloride, and the like), a dispersing agent (hydroxypropylmethylcellulose, dextrin), a pH regulator (hydrochloric acid, sodium hydroxide and the like), a preservative (ethyl p-hydroxybenzoate, benzoic acid and the like), a dissolving agent (conc. glycerin, meglumine and the like), a dissolution aid (propylene glycol, sucrose and the like), a soothing agent (glucose, benzyl alcohol and the like), and the like, can be appropriately compounded. These additives are generally compounded in a proportion usually used in an injection.

It is advantageous that pH of an injection is controlled from 2 to 12, preferably from 2.5 to 8.0 by addition of a pH regulator.

An injection is obtained by dissolving the compound of the present invention or the concomitant drug and if desirable, a benzoate and/or a salicylate, and if necessary, the above-mentioned additives into water. These may be dissolved in any order, and can be appropriately dissolved in the same manner as in a conventional method of producing an injection.

An aqueous solution for injection may be advantageously be heated, alternatively, for example, filter sterilization, high pressure heat sterilization and the like can be conducted in the same manner as for a usual injection, to provide an injection.

It may be advantageous that an aqueous solution for injection is subjected to high pressure heat sterilization at 100 to 121°C for 5 to 30 minutes.

Further, a preparation endowed with an antibacterial property of a solution may also be produced so that it can be used as a preparation which is divided and administered multiple times.

### [2] Sustained release preparation or quick release preparation, and preparation thereof

A sustained release preparation is preferable which is obtained, if desirable, by coating a nucleus containing the compound of the present invention or the concomitant drug with a film agent such as a water-insoluble substance, swellable polymer and the like. For example, a sustained release preparation for oral administration for a single administration per day type is preferable.

As the water-insoluble substance used in a film agent, there are listed, for example, cellulose ethers such as ethylcellulose, butylcellulose ad the like, cellulose esters such as cellulose stearate, cellulose propionate and the like, polyvinyl esters such as polyvinyl acetate, polyvinyl butyrate and the like, acrylic acid/methacrylic acid copolymers, methyl methacrylate copolymers, ethoxyethyl methacrylate/cinnamoethyl methacrylate/aminoalkyl methacrylate copolymers, polyacrylic acid, polymethacrylic acid, methacrylic acid alkylamide copolymers, poly(methyl methacrylate), polymethacrylate, polymethacrylamide, aminoalkyl methacrylate copolymers, poly(methacrylic anhydride), glycidyl methacrylate copolymer, particularly, acrylic acid-based polymers such as Eudragits (Rohm Pharma) such as Eudragit RS-100, RL-100, RS-30D, RL-30D, RL-PO, RS-PO (ethyl acrylate•methyl methacrylate•trimethyl chloride methacrylate•ammoniumethyl copolymer), Eudragit NE-30D (methyl methacrylate•ethyl acrylate copolymer), and the like, hardened oils such as hardened castor oil (e.g., Lovery wax (Freunt) and the like), waxes such as carnauba wax, fatty acid glycerin ester, paraffin and the like, polyglycerin fatty esters, and the like.

As the swellable polymer, polymers having an acidic dissociating group and showing pH dependent swelling are preferable, and polymers manifesting slight swelling in acidic regions such as in the stomach and greater swelling in neutral regions such as in the small intestine and the large intestine are preferable.

As such a polymer having an acidic dissociating group and showing pH dependent swelling, cross-linkable polyacrylic acid copolymers such as, for example, Carbomer 934P, 940, 941, 974P, 980, 1342 and the like, polycarbophil, calcium polycarbophil (last two are manufactured by BF Goodrich), Hibiswako 103, 104, 105, 304 (all are manufactured by Wako Purechemical Co., Ltd.), and the like, are listed.

The film agent used in a sustained release preparation may further contain a hydrophilic substance.

As the hydrophilic substance, for example, polysaccharides which may contain a sulfate group such as pullulan, dextrin, alkali metal alginate and the like, polysaccharides having a hydroxyalkyl group or carboxyalkyl group such as hydroxypropylcellulose, hydroxypropylmethylcellulose, carboxymethylcellulose sodium and the like, methylcellulose, polyvinylpyrrolidone, polyvinyl alcohol, polyethylene glycol and the like.

The content of a water-insoluble substance in the film agent of a sustained release preparation is from about 30 to 90% (w/w), preferably from about 35 to 80% (w/w), further preferably from about 40 to 75% (w/w), the content of a swellable polymer is from about 3 to 30% (w/w), preferably from about 3 to 15% (w/w). The film agent may further contain a hydrophilic substance, and in which case, the content of a hydrophilic substance in the film agent is about 50% (w/w) or less, preferably about 5 to 40% (w/w), further preferably from about 5 to 35% (w/w). This % (w/w) indicates % by weight based on a film agent composition which is obtained by removing a solvent (e.g., water, lower alcohols such as methanol, ethanol and the like) from a film agent solution.

The sustained release preparation is produced by preparing a nucleus containing a drug as exemplified below, then, coating the resulting nucleus with a film agent solution prepared by heat-solving a water-insoluble substance, swellable polymer and the like or by dissolving or dispersing it in a solvent.

### I. Preparation of nucleus containing drug

The form of nucleus containing a drug to be coated with a film agent (hereinafter, sometimes simply referred to as nucleus) is not particularly restricted, and preferably, the nucleus is formed into particles such as a granule or fine particle.

When the nucleus is composed of granules or fine particles, the average particle size thereof is preferably from about 150 to 2000 µm, further preferably, from about 500 to 1400 µm.

Preparation of the nucleus can be effected by a usual production method. For example, a suitable excipient, binding agent, integrating agent, lubricant, stabilizer and the like are mixed into a drug, and the mixture is subjected to a wet extrusion granulating method, fluidized bed granulating method or the like, to prepare a nucleus.

The content of drugs in a nucleus is from about 0.5 to 95% (w/w), preferably from about 5.0 to 80% (w/w), further preferably from about 30 to 70% (w/w).

As the excipient contained in the nucleus, for example, saccharides such as sucrose, lactose, mannitol, glucose and the like, starch, crystalline cellulose, calcium phosphate, corn starch and the like are used. Among them, crystalline cellulose and corn starch are preferable.

As the bonder, for example, polyvinyl alcohol, hydroxypropyl cellulose, polyethylene glycol, polyvinyl pyrrolidone, Pluronic F68, gum Arabic, gelatin, starch and the like are used. As the disintegrating agent, for example, carboxymethylcelulose calcium (ECG505), crosscarmelose sodium (Ac-Di-Sol), crosslinked polyvinylpyrrolidone (Crospovidone), lower substituted hydroxypropylcellulose (L-HPC) and the like are used. Among them, hydroxypropylcellulose, polyvinylpyrrolidone, lower substituted hydroxypropylcellulose are preferable. As the lubricant and coagulation inhibitor, for example, talc, magnesium stearate and inorganic salts thereof are used, and as the lubricant, polyethylene glycol and the like are used. As the stabilizer, acids such as tartaric acid, citric acid, succinic acid, fumaric acid, maleic acid and the like, are used.

A nucleus can also be prepared by, in addition to the above-mentioned, for example, a rolling granulation method in which a drug or a mixture of a drug with an excipient, lubricant and the like is added portionwise onto an inert carrier particle which is the core of the nucleus while spraying a binder dissolved in a suitable solvent such as water, lower alcohol (e.g., methanol, ethanol and the like) and the like, a pan coating method, a fluidized bed coating method or a melt granulating method. As the inert carrier particle, for example, those made of sucrose, lactose, starch, crystalline cellulose, waxes can be used, and the average particle size thereof is preferably from about 100 µm to 1500 µm.

For separating a drug and a film agent contained in a nucleus, the surface of the nucleus may be coated with a protective agent. As the protective agent, for example, the above-mentioned hydrophilic substances, water-insoluble substances and the like are used. As the protective agent, preferably polyethylene glycol, and polysaccharides having a hydroxyalkyl group or carboxyalkyl group are used, more preferably, hydroxypropylmethylcellulose and hydroxypropyplcellulose are use. The protective agent may contain, as a stabilizer, acids such as tartaric acid, citric acid, succinic acid, fumaric acid, maleic acid and the like, and lubricants such as talc and the like. When the protective agent is used, the coating amount is from about 1 to 15% (w/w), preferably from about 1 to 10% (w/w), further preferably from about 2 to 8% (w/w), based on the nucleus.

The protective agent can be coated by a usual coating method, and specifically, the protective agent can be coated, for example, by a fluidized bed coating method, pan coating method and the like.

### II. Coating of nucleus with film agent

A nucleus obtained in the above-mentioned step I is coated with a film agent solution obtained by heat-solving the above-mentioned water-insoluble substance and pH-dependent swellable polymer, and a hydrophilic substance, or by dissolving or dispersing them in a solvent, to give a sustained release preparation.

As the method for coating a nucleus with a film agent solution, for example, a spray coating method and the like are listed.

The composition ratio of a water-insoluble substance, swellable polymer and hydrophilic substance in a film agent solution is appropriately selected so that the contents of these components in a coated film are the above-mentioned contents, respectively.

The coating amount of a film agent is from about 1 to 90% (w/w), preferably from about 5 to 50% (w/w), further preferably from about 5 to 35% (w/w), based on a nucleus (not including coating amount of protective agent).

As the solvent in a film agent solution, water or an organic solvent can be used alone or in admixture thereof. In the case of use in admixture, the mixing ratio of water to an organic solvent (water/organic solvent: by weight) can be varied in the range from 1 to 100%, and preferably from 1 to about 30%. The organic solvent is not particularly restricted providing it dissolves a water-insoluble substance, and for example, lower alcohols such as methyl alcohol, ethyl alcohol, isopropyl alcohol, n-butyl alcohol and the like, lower alkanone such as acetone and the like, acetonitrile, chloroform, methylene chloride and the like are used. Among them, lower alcohols are preferable, and ethyl alcohol and isopropyl alcohol are particularly preferable. Water, and a mixture of water with an organic solvent are preferably used as a solvent for a film agent. In this case, if necessary, an acid such as tartaric acid, citric acid, succinic acid, fumaric acid, maleic acid and the like may also be added into a film agent solution for stabilizing the film agent solution.

An operation of coating by spray coating can be effected by a usual coating method, and specifically, it can be effected by spray-coating a film agent solution onto a nucleus by a fluidized bed coating method, pan coating method and the like. In this case, if necessary, talc, titanium oxide, magnesium stearate, calcium stearate, light anhydrous silicic acid and the like may also be added as a lubricant, and glycerin fatty ester, hardened castor oil, triethyl citrate, cetyl alcohol, stearyl alcohol and the like may also be added as a plasticizer.

After coating with a film agent, if necessary, an antistatic agent such as talc and the like may be mixed.

The quick release preparation may be liquid (solution, suspension, emulsion and the like) or solid (particle, pill, tablet and the like). Oral agents and parenteral agents such as an injection and the like are used, and oral agents are preferable.

The quick release preparation, usually, may contain, in addition to an active component drug, also carriers, additives and excipients conventionally used in the production field (hereinafter, sometimes abbreviated as excipient). The preparation excipient used is not particularly restricted providing it is an excipient ordinarily used as a preparation excipient. For example, as the excipient for an oral solid preparation, lactose, starch, corn starch, crystalline cellulose (Acevil PH101, manufactured by Asahi Chemical Industry Co., Ltd., and the like), powder sugar, granulated sugar, mannitol, light anhydrous silicic acid, magnesium carbonate, calcium carbonate, L-cysteine and the like are listed, and preferably, corn starch and mannitol and the like are listed. These excipients can be used alone or in combination of two or more. The content of the excipient is, for example, from about 4.5 to 99.4 w/w%, preferably from about 20 to 98.5 w/w%, further preferably from about 30 to 97 w/w%, based on the total amount of the quick release preparation.

The content of a drug in the quick release preparation can be appropriately selected in the range from about 0.5 to 95%, preferably from about 1 to 60% based on the total amount of the quick release preparation.

When the quick release preparation is an oral solid preparation, it usually contains, in addition to the above-mentioned components, also an integrating agent. As this integrating agent, there are used, for example, carboxymethylcellulose calcium (ECG-505, manufactured by Gotoku Yakuhin), crosscarmelose sodium (for example, Actisol, manufactured by Asahi Chemical Industry Co., Ltd.), crosspovidone (for example, Colicone CL, manufactured by BASF), lower substitution hydroxypropylcellulose (manufactured by Shin-Etsu Chemical Co., Ltd.), carboxymethylstarch (manufactured by Matsutani Kagaku K.K.), carboxymethylstarch sodium (Exprotab, manufactured by Kimura Sangyo), partially pregelatinized starch (PCS, manufactured by Asahi Chemical Industry Co., Ltd.), and the like are used, and for example, those which disintegrate a granule by adsorbing water in contact with water, causing swelling, or making a channel between an effective ingredient constituting the nucleus and an excipient, can be used. These disintegrating agents can be used alone or in combination of two or more. The amount of the disintegrating agent used is appropriately selected depending on the kind and compounding amount of a drug used, design of releasing property, and the like, and for example, from about 0.05 to 30 w/w%, preferably from about 0.5 to 15 w/w%, based on the total amount of the quick releasing agent.

When the quick release preparation is an oral solid preparation, it may further contain, in addition to the above-mentioned composition, if desired, additives conventional in solid preparations. As such an additive, there are used, for example, a binder (e.g., sucrose, gelatin, gum Arabic powder, methylcellulose, hydroxypropylcellulose, hydroxypropylmethylcellulose, carboxylmethylcellulose, polyvinylpyrrolidone, pullulan, dextrin and the like), a lubricant (e.g., polyethylene glycol, magnesium stearate, talc, light anhydrous silicic acid (e.g., aerosil (Nippon Aerosil)), a surfactant (e.g., anionic surfactants such as sodium alkylsulfate and the like, nonionic surfactants such as polyoxyethylene fatty acid ester and polyoxyethylene sorbitan fatty acid ester, polyoxyethylene castor oil derivatives and the like), a coloring agent (e.g., tar coloring matter, caramel, iron oxide red, titanium oxide, riboflavins), if necessary, an appetizing agent (e.g., sweetening agent, aroma and the like), an adsorbent, preservative, wetting agent, antistatic agent, and the like. Further, as the stabilizer, an organic acid such as tartaric acid, citric acid, succinic acid, fumaric acid and the like may also be added.

As the above-mentioned binder, hydroxypropylcellulose, polyethylene glycol and polyvinylpyrrolidone and the like are preferably used.

The quick releasing preparation can be prepared by, based on a usual technology of producing preparations, mixing the above-mentioned components, and if necessary, further kneading the mixture, and molding it. The above-mentioned mixing is conducted by generally used methods, for example, mixing, kneading and the like. Specifically, when a quick release preparation is formed, for example, into a particle, it can be prepared, according to the same means as in the above-mentioned method for preparing a nucleus of a sustained release preparation, by mixing the components using a vertical granulator, universal kneader (manufactured by Hata Tekkosho), fluidized bed granulator FD-5S (manufactured by Pulek), and the like, then, subjecting the mixture to a wet extrusion granulation method, fluidized bed granulation method and the like.

Thus obtained quick releasing preparation and sustained releasing preparation may be themselves made into products or made into products appropriately together with preparation excipients and the like, separately, by an ordinary method, then, may be administered simultaneously or may be administered in combination at any administration interval, or they may be themselves made into one oral preparation (e.g., granule, fine particle, tablet, capsule and the like) or made into one oral preparation together with preparation excipients and the like. It may also be permissible that they are made into granules or fine particles, and filled in the same capsule to be used as a preparation for oral administration.

### [3] Sublingual, buccal or intraoral quick disintegrating agent and preparation thereof

Sublingual, buccal or intraoral quick disintegrating agents may be a solid preparation such as tablet and the like, or may be an oral mucosa membrane patch (film).

As the sublingual, buccal or intraoral quick disintegrating agent, a preparation containing the compound of the present invention or the concomitant drug and an excipient is preferable. It may contain also auxiliary agents such as a lubricant, isotonizing agent, hydrophilic carrier, water-dispersible polymer, stabilizer and the like. Further, for easy absorption and increase in *in vivo* use efficiency, β-cyclodextrin or β-cyclodextrin derivatives (e.g., hydroxypropyl-β-cyclodextrin and the like) and the like may also be contained.

As the above-mentioned excipient, lactose, sucrose, D-mannitol, starch, crystalline cellulose, light anhydrous silicic acid and the like are listed. As the lubricant, magnesium stearate, calcium stearate, talc, colloidal silica and the like are listed, and particularly, magnesium stearate and colloidal silica are preferable. As the isotonizing agent, sodium chloride, glucose, fructose, mannitol, sorbitol, lactose, saccharose, glycerin, urea and the like are listed, and particularly, mannitol is preferable. As the hydrophilic carrier, swellable hydrophilic carriers such as crystalline cellulose, ethylcellulose, crosslinkable polyvinylpyrrolidone, light anhydrous silicic acid, silicic acid, dicalcium phosphate, calcium carbonate and the like are listed, and particularly, crystalline cellulose (e.g., fine crystalline cellulose and the like) is preferable. As the water-dispersible polymer, gums (e.g., gum tragacanth, acacia gum, cyamoposis gum), alginates (e.g., sodium alginate), cellulose derivatives (e.g., methylcellulose, carboxymethylcellulose, hydroxymethylcellulose, hydroxypropylcellulose, hydroxypropylmethylcellulose), gelatin, water-soluble starch, polyacrylic acids (e.g., Carbomer), polymethacylic acid, polyvinyl alcohol, polyethylene glycol, polyvinylpyrrolidone, polycarbofil, ascorbate palmitates and the like are listed, and hydroxypropylmethylcellulose, polyacrylic acid, alginate, gelatin, carboxymethylcellulose, polyvinylpyrrolidone, polyethylene glycol and the like are preferable. Particularly, hydroxypropylmethylcellulose is preferable. As the stabilizer, cysteine, thiosorbitol, tartaric acid, citric acid, sodium carbonate, ascorbic acid, glycine, sodium sulfite and the like are listed, and particularly, citric acid and ascorbic acid are preferable.

The sublingual, buccal or intraoral quick disintegrating agent can be produced by mixing the compound of the present invention or the concomitant drug and an excipient by a method known per se. Further, is desirable, auxiliary agents such as a lubricant, isotonizing agent, hydrophilic carrier, water-dispersible polymer, stabilizer, coloring agent, sweetening agent, preservative and the like may be mixed. The sublingual, buccal or intraoral quick disintegrating agent is obtained by mixing the above-mentioned components simultaneously or at a time interval, then subjecting the mixture to tablet-making molding under pressure. For obtaining suitable hardness, it may also be permissible that the materials are moistened by using a solvent such as water, alcohol and the like if desired before and after the tablet making process, and after the molding, the materials are dried, to obtain a product.

In the case of molding into a mucosa membrane patch (film), the compound of the present invention or the concomitant drug and the above-mentioned water-dispersible polymer (preferably, hydroxypropylcellulose, hydroxypropylmethylcellulose), excipient and the like are dissolved in a solvent such as water and the like, and the resulted solution is cast, to give a film. Further, additives such as a plasticizer, stabilizer, antioxidant, preservative, coloring agent, buffer, sweetening agent and the like may also be added. For imparting suitable elasticity to the film, glycols such as polyethylene glycol, propylene glycol and the like may be contained, or for enhancing adhesion of the film to an intraoral mucosa membrane lining, a bio-adhesive polymer (e.g., polycarbofil, carbopol) may also be contained. In the casting, a solution is poured on the non-adhesive surface, spread to uniform thickness (preferably, about 10 to 1000 micron) by an application tool such as a doctor blade and the like, then, the solution is dried to form a film. It may be advantageous that thus formed film is dried at room temperature or under heat, and cut into given area.

As the preferable intraoral quick disintegrating agent, there are listed solid quick scattering dose agents composed of a network body comprising the compound of the present invention or the concomitant drug, and a water-soluble or water-diffusible carrier which is inert to the compound of the present invention or combination drug, are listed. This network body is obtained by sublimating a solvent from the solid composition constituted of a solution prepared by dissolving the compound of the present invention or the concomitant drug in a suitable solvent.

It is preferable that the composition of an intraoral quick disintegrating agent contains a matrix forming agent and a secondary component, in addition to the compound of the present invention or the concomitant drug.

Examples of the matrix forming agent include animal proteins or vegetable proteins such as gelatins, dextrins and, soybean, wheat and psyllium seed protein and the like; rubber substances such as gum Arabic, guar gum, agar, xanthan gum and the like; polysaccharides; alginic acids; carboxymethylcelluloses; carageenans; dextrans; pectins; synthetic polymers such as polyvinylpyrrolidone and the like; substances derived from a gelatin-gum Arabic complex, and the like. Further, saccharides such as mannitol, dextrose, lactose, galactose, trehalose and the like; cyclic saccharides such as cyclodextrin and the like; inorganic salts such as sodium phosphate, sodium chloride and aluminum silicate and the like; amino acids having 2 to 12 carbon atoms such as glycine, L-alanine, L-aspartic acid, L-glutamic acid, L-hydroxyproline, L-isoleucine, L-leucine, L-phenylalanine and the like, are contained.

One or more of the matrix forming agents can be introduced in a solution or suspension before solidification. Such as matrix forming agent may be present in addition to a surfactant, or may be present while a surfactant being excluded. The matrix forming agent aids to maintain the compound of the present invention or the concomitant drug in the solution or suspension in diffused condition, in addition to formation of the matrix.

The composition may contain secondary components such as a preservative, antioxidant, surfactant, thickening agent, coloring agent, pH controlling agent, flavoring agent, sweetening agent, food taste masking agent and the like. As the suitable coloring agent, there are listed red, black and yellow iron oxides, and FD & C dyes such as FD & C Blue 2, FD & C Red 40 and the like manufactured by Elis and Eberald. Examples of the suitable flavoring agent include mint, raspberry, licorice, orange, lemon, grape fruit, caramel, vanilla, cherry, grape flavor and combinations thereof. Examples of the suitable pH controlling agent include citric acid, tartaric acid, phosphoric acid, hydrochloric acid and maleic acid. Examples of the suitable sweetening agent include aspartame, acesulfame K and thaumatin and the like. Examples of the suitable food taste masking agent include sodium bicarbonate, ion exchange resin, cyclodextrin-containing compounds, adsorbent substances and microcapsulated apomorphine.

The preparation contains the compound of the present invention or the concomitant drug in an amount usually from about 0.1 to 50% by weight, preferably from about 0.1 to 30% by weight, and preferable are preparations (such as the above-mentioned sublingual agent, buccal and the like) which can dissolve 90% or more the compound of the present invention or the concomitant drug (into water) within the time range of about 1 to 60 minutes, preferably of about 1 to 16 minutes, more preferably of about 2 to 5 minutes, and intraoral quick disintegrating preparations which are disintegrated within the range of 1 to 60 seconds, preferably of 1 to 30 seconds, further preferably of 1 to 10 seconds after place in an oral cavity.

The content of the above-mentioned excipient in the whole preparation is from about 10 to 99% by weight, preferably from about 30 to 90% by weight. The content of β-cyclodextrin or β-cyclodextrin derivative in the whole preparation is from 0 to about 30% by weight. The content of the lubricant in the whole preparation is from about 0.01 to 10% by weight, preferably from about 1 to 5% by weight. The content of the isotonizing agent in the whole preparation is from about 0.1 to 90% by weight, preferably, from about 10 to 70% by weight. The content of the hydrophilic carrier agent in the whole preparation is from about 0.1 to 50% by weight, preferably, from about 10 to 30% by weight. The content of the water-dispersible polymer in the whole preparation is from about 0.1 to 30% by weight, preferably, from about 10 to 25% by weight. The content of the stabilizer in the whole preparation is from about 0.1 to 10% by weight, preferably, from about 1 to 5% by weight. The above-mentioned preparation may further contain additives such as a coloring agent, sweetening agent, preservative and the like, if necessary.

The dosage of a combination agent of the present invention differs depending on the kind of a compound (I), age, body weight, condition, drug form, administration method, administration period and the like, and for example, for one sepsis patient (adult, body weight: about 60 kg), the combination agent is administered intravenously, at a dose of about 0.01 to 1000 mg/kg/day, preferably about 0.01 to 100 mg/kg/day, more preferably about 0.1 to 100 mg/kg/day, particularly about 0.1 to 50 mg/kg/day, especially about 1.5 to 30 mg/kg/day, in terms of the compound of the present invention or the concomitant drug, respectively, once or divided several times in a day. Of course, since the dose as described above varies depending on various conditions, amounts smaller than the above-mentioned dosage may sometimes be sufficient, further, amounts over that range sometimes have to be administered.

The amount of the concomitant drug can be set at any value unless side effects are problematical. The daily dosage in terms of the combination drug differs depending on the severity, age, sex, body weight, sensitivity difference of the subject, administration period, interval, and nature, pharmacology, kind of the pharmaceutical preparation, kind of effective ingredient, and the like, and not particularly restricted, and the amount of a drug is, in the case of oral administration for example, usually from about 0.001 to 2000 mg, preferably from about 0.01 to 500 mg, further preferably from about 0.1 to 100 mg, per 1 kg of a mammal and this is usually administered once to 4-times divided in a day.

In administration of a medicine of the present invention, the compound of the present invention may be administered after administration of the concomitant drug or the concomitant drug may be administered after administration of the compound of the present invention, though they may be administered simultaneously. When administered at a time interval, the interval differs depending on the effective ingredient, drug form and administration method, and for example, when the concomitant drug is administered first, a method in which the compound of the present invention is administered within time range of from 1 minute to 3 days, preferably from 10 minutes to 1 day, more preferably from 15 minutes to 1 hour after administration of the concomitant drug is exemplified. When the compound of the present invention is administered first, a method in which the concomitant drug is administered within time range of from 1 minute to 1 day, preferably from 10 minutes to 6 hours, more preferably from 15 minutes to 1 hour after administration of the compound of the present invention is exemplified.

In a preferable administration method, for example, the concomitant drug which has been formed into an oral administration preparation is administered orally at a daily dose of about 0.001 to 200 mg/kg, and 15 minutes after, the compound of the present invention which has been formed into an oral administration preparation is administered orally at a daily dose of about 0.005 to 100 mg/kg.

### Examples

The present invention is explained in detail by way of the following Reference Example, Examples, Preparation Examples and Test Examples but these are mere examples and do not limit the present invention and can be varied without departing the scope of the present invention.

"Room temperature" in the following Reference Example and Examples indicates normally about 10°C to about 35°C. "%" indicates percentage by weight unless otherwise indicated, provided that yield represents mol/mol%.

Abbreviations used elsewhere indicate the following meanings:
- s:: singlet
- d:: doublet
- t:: triplet
- q:: quartet
- dd:: double doublet
- ddd:: double double doublet
- dt:: double triplet
- br:: broad
- J:: coupling constant
- Hz:: Hertz
- CDCl₃:: deuterated chloroform
- ¹H-NMR:: proton nuclear magnetic resonance
- Me:: methyl

### Reference Example A 1

### 1-(4-methoxyphenyl)-2-(3-pyridyl)ethanone

A solution of diisopropylamine (33.2 mL) in anhydrous tetrahydrofuran (300 mL) was cooled to -78°C and a 1.6 M n-butyllithium/hexane solution (148 mL) was added dropwise with stirring. After completion of dropwise addition, the mixture was stirred for 10 min at the same temperature, and then β-picoline (20 g) was added dropwise. The temperature was raised to -10-0°C, and after stirring for 20 min, a solution of ethyl p-anisate (19.4 g) in anhydrous tetrahydrofuran (40 mL) was added dropwise. After completion of dropwise addition, the mixture was stirred at room temperature for 1 h, and water (100 mL) was added. The organic solvent was evaporated under reduced pressure and an oily product was extracted with ethyl acetate. The extract was washed with water, and after drying, the solvent was evaporated. The remaining crude crystals were recrystallized from ethyl acetate-isopropyl ether to give the title compound (20.8 g, yield 85%).
melting point: 71-72°C.

### Reference Example A 2:

In accordance with the above-mentioned Reference Example A 1 and respectively using, instead of ethyl p-anisate, ethyl benzoate, ethyl 3,4-dimethoxybenzoate, ethyl 3,4,5-trimethoxybenzoate, ethyl 4-(methoxymethoxy)benzoate, ethyl 4-fluorobenzoate, ethyl 4-ethylbenzoate, ethyl 3,4-methylenedioxybenzoate, methyl 5-indanylcarboxylate, methyl 5,6,7,8-tetrahydro-2-naphthoate, methyl 1,4-benzodioxane-6-carboxylate and methyl 2-naphthoate, the following Reference Example A compounds 2-1 to 2-11 were synthesized.
Reference Example compound A 2-1: 1-phenyl-2-(3-pyridyl)ethanone melting point: 44.5-45.5°C.
Reference Example A compound 2-2: 1-(3,4-dimethoxyphenyl)-2-(3-pyridyl)ethanone melting point: 114-115°C.
Reference Example A compound 2-3: 2-(3-pyridyl)-1-(3,4,5-trimethoxyphenyl)ethanone melting point: 104-105°C.
Reference Example A compound 2-4: 1-(4-methoxymethoxyphenyl)-2-(3-pyridyl)ethanone melting point: 43-44°C.
Reference Example A compound 2-5: 1-(4-fluorophenyl)-2-(3-pyridyl)ethanone oil.
Reference Example A compound 2-6: 1-(4-ethylphenyl)-2-(3-pyridyl)ethanone melting point: 80-81°C.
Reference Example A compound 2-7: 1-(3,4-methylenedioxyphenyl)-2-(3-pyridyl)ethanone melting point: 98-99°C.
Reference Example A compound 2-8: 1-(5-indanyl)-2-(3-pyridyl)ethanone melting point: 55-56°C.
Reference Example A compound 2-9: 2-(3-pyridyl)-1-(5,6,7,8-tetrahydro-2-naphthyl)ethanone melting point: 65-66°C.
Reference Example A compound 2-10: 1-(1,4-benzodioxan-6-yl)-2-(3-pyridyl)ethanone melting point: 89-90°C.
Reference Example A compound 2-11: 1-(2-naphthyl)-2-(3-pyridyl)ethanone melting point: 69-70°C.

### Reference Example A 3

In accordance with the above-mentioned Reference Example A 2 and respectively using α-picoline, γ-picoline and 3,5-lutidine instead of β-picoline, the following Reference Example A compounds 3-1 to 3-3 were synthesized.
Reference Example A compound 3-1: 1-phenyl-2-(2-pyridyl)ethanone melting point: 59-60°C.
Reference Example A compound 3-2: 1-(4-methoxyphenyl)-2-(2-pyridyl)ethanone melting point: 77-78°C.
Reference Example A compound 3-3: 1-phenyl-2-(4-pyridyl)ethanone melting point: 109-110°C.

### Reference Example A 4

### 1-(4-methoxyphenyl)-2-(4-pyridyl)ethanone

A solution of diisopropylamine (33.2 mL) in anhydrous tetrahydrofuran (300 mL) was cooled to -78°C and 1.6 M n-butyllithium-hexane solution (148 mL) was added dropwise with stirring. After completion of dropwise addition, the mixture was stirred for 10 min at the same temperature, then γ-picoline (20 g) was added dropwise. The temperature was raised to -10-0°C, and after stirring for 20 min, a solution of ethyl p-anisate (19.4 g) in anhydrous tetrahydrofuran (40 mL) was added dropwise. After completion of dropwise addition, the mixture was stirred at room temperature for 1 h, and water (100 mL) was added. The organic solvent was evaporated under reduced pressure and an oily product was extracted with ethyl acetate. The extract was washed with water, and after drying, the solvent was evaporated. The remaining crude crystals were recrystallized from ethyl acetate-isopropyl ether to give the title compound (16.2 g, yield 66 %).
melting point: 103-104°C.

### Reference Example A 5

### 2-(5-methyl-3-pyridyl)-1-phenylethanone

A solution of diisopropylamine (20.2 mL) in anhydrous tetrahydrofuran (180 mL) was cooled to -78°C, and a 1.6 M n-butyllithium-hexane solution (90 mL) was added dropwise with stirring. After completion of dropwise addition, the mixture was stirred for 10 min at the same temperature, and then 3,5-lutidine (14 g) was added dropwise. The temperature was raised to -10-0°C, and after stirring for 20 min, a solution of ethyl benzoate (9.8 g) in anhydrous tetrahydrofuran (20 mL) was added dropwise. After completion of dropwise addition, the mixture was stirred at room temperature for 1 h, and water (100 mL) was added. The organic solvent was evaporated under reduced pressure and an oily product was extracted with ethyl acetate. The extract was washed with water, and after drying, the solvent was evaporated. The remaining crude crystals were recrystallized from ethyl acetate-isopropyl ether to give the title compound (10 g, yield 70%).
melting point: 53-54°C.

### Reference Example A 6

### 2-bromo-1-(4-methoxyphenyl)-2-(3-pyridyl)ethanone hydrobromide

1-(4-Methoxyphenyl)-2-(3-pyridyl)ethanone (6.9 g) was dissolved in acetic acid (36 mL), bromine (1.7 mL) was added, and the mixture was stirred at 80°C for 3 h. The reaction mixture was cooled with iced water and the precipitated crude crystals were collected by filtration. The crude crystals were recrystallized from ethanol-ethyl ether to give the title compound (10 g, yield 89%).
melting point: 188-195°C.

### Reference Example A 7

In accordance with the above-mentioned Reference Example A 6, 1-phenyl-2-(3-pyridyl)ethanone, 1-(3,4-dimethoxyphenyl)-2-(3-pyridyl)ethanone, 2-(3-pyridyl)-1-(3,4,5-trimethoxyphenyl) ethanone, 1-(4-methoxymethoxyphenyl)-2-(3-pyridyl)ethanone, 1-(4-fluorophenyl)-2-(3-pyridyl)ethanone, 1-phenyl-2- (2-pyridyl) ethanone, 1-(4-methoxyphenyl)-2-(2-pyridyl)ethanone, 1-phenyl-2-(4-pyridyl)ethanone, 1-(4-methoxyphenyl)-2-(4-pyridyl)ethanone, 2-(5-methyl-3-pyridyl)-1-phenylethanone, 1-(4-ethylphenyl)-2-(3-pyridyl)ethanone, 1-(3,4-methylenedioxyphenyl)-2-(3-pyridyl)ethanone, 1-(5-indanyl)-2-(3-pyridyl)ethanone, 2-(3-pyridyl)-1-(5,6,7,8-tetrahydro-2-naphthyl)ethanone, 1-(1,4-benzodioxan-6-yl)-2-(3-pyridyl)ethanone, 1-(2-naphthyl)-2-(3-pyridyl)ethanone and 1-(4-methoxyphenyl)-2-(2-pyridyl)ethanone were respectively used instead of 1-(4-methoxyphenyl)-2-(3-pyridyl)ethanone, the following Reference Example A compounds 7-1 to 7-17 were synthesized.
Reference Example A compound 7-1: 2-bromo-1-phenyl-2-(3-pyridyl)ethanonehydrobromide melting point: 208-215°C.
Reference Example A compound 7-2:
   2-bromo-1-(3,4-dimethoxyphenyl)-2-(3-pyridyl)ethanonehydrobromide melting point: 191-193°C.
Reference Example A compound 7-3: 2-bromo-2-(3-pyridyl)-1-(3,4,5-trimethoxyphenyl)ethanone hydrobromide melting point: 184-186°C.
Reference Example A compound 7-4: 2-bromo-1-(4-hydroxyphenyl)-2-(3-pyridyl)ethanone hydrobromide
   Used in the next reaction without purification.
Reference Example A compound 7-5: 2-bromo-1-(4-fluorophenyl)-2-(3-pyridyl)ethanone hydrobromide melting point: 189-191°C.
Reference Example A compound 7-6: 2-bromo-1-phenyl-2-(2-pyridyl)ethanone hydrobromide melting point: 180-181°C.
Reference Example A compound 7-7: 2-bromo-1-(4-methoxyphenyl)-2-(2-pyridyl)ethanone hydrobromide melting point: 170-171°C.
Reference Example A compound 7-8: 2-bromo-1-phenyl-2-(4-pyridyl)ethanone hydrobromide melting point: 230-232°C.
Reference Example A compound 7-9: 2-bromo-1-(4-methoxyphenyl)-2-(4-pyridyl)ethanone hydrobromide melting point: 207-209°C.
Reference Example A compound 7-10: 2-bromo-2-(5-methyl-3-pyridyl)-1-phenylethanone hydrobromide melting point: 189-193°C.
Reference Example A compound 7-11: 2-bromo-1-(4-ethylphenyl)-2-(3-pyridyl)ethanone hydrobromide melting point: 145-146°C.
Reference Example A compound 7-12: 2-bromo-1-(3,4-methylenedioxyphenyl)-2-(3-pyridyl)ethanone hydrobromide melting point: 174-175°C.
Reference Example A compound 7-13: 2-bromo-1-(5-indanyl)-2-(3-pyridyl)ethanone hydrobromide melting point: 177-178°C.
Reference Example A compound 7-14: 2-bromo-2-(3-pyridyl)-1-(5,6,7,8-tetrahydro-2-naphthyl)ethanone hydrobromide melting point: 160-162°C.
Reference Example A compound 7-15: 1-(1,4-benzodioxan-6-yl)-2-bromo-2-(3-pyridyl)ethanone hydrobromide oil.
Reference Example A compound 7-16: 2-bromo-1-(2-naphthyl)-2-(3-pyridyl)ethanone hydrobromide melting point: 197-199°C.
Reference Example A compound 7-17: 2-bromo-1-(4-methoxyphenyl)-2-(2-pyridyl)ethanone hydrobromide melting point: 170-171°C.

### Reference Example A 8

### [4-(4-methoxyphenyl)-5-(3-pyridyl)-1,3-thiazol-2-yl]amine

To a suspension of thiourea (0.52 g) in acetonitrile (40 mL) was added 2-bromo-1-(4-methoxyphenyl)-2-(3-pyridyl)ethanone hydrobromide (2.5 g) and triethylamine (0.95 mL) was slowly added dropwise with stirring. After completion of dropwise addition, the mixture was stirred at a refluxing temperature for 3 h, and after allowing to cool, the precipitated crystals were collected by filtration. The crystals were washed successively with saturated sodium hydrogencarbonate solution, water, ethanol and ethyl ether and dried. The obtained crude crystals were recrystallized from tetrahydrofuran to give the title compound (1.5 g, yield 90%). melting point: 265-266°C.

### Reference Example A 9

### N-methyl [4-(4-methoxyphenyl)-5-(3-pyridyl)-1,3-thiazol-2-yl]amine

To a suspension of N-methylthiourea (0.24 g) in acetonitrile (18 mL) was added 2-bromo-1-(4-methoxyphenyl)-2-(3-pyridyl)ethanone hydrobromide (1.0 g) and triethylamine (0.4 mL) was slowly added dropwise with stirring. After completion of dropwise addition, the mixture was stirred at a refluxing temperature for 3 h, and the solvent was evaporated. To the residue was added saturated aqueous sodium hydrogencarbonate and the mixture was extracted with ethyl acetate, and the extract was washed with water and dried, and the solvent was evaporated. The remaining crude crystals were recrystallized from ethyl acetate-isopropyl ether to give the title compound (0.65 g, yield 85%).
melting point: 158-159°C.

### Reference Example A 10

### N-[4-(4-methoxyphenyl)-5-(3-pyridyl)-1,3-thiazol-2-yl]acetamide

Using [(4-methoxyphenyl)-5-(3-pyridyl)-1,3-thiazol-2-yl]amine as a starting compound and according to a method similar to Reference Example A 23-128 to be mentioned below, the title compound was obtained (yield 82%).
melting point: 208-210°C.

### Reference Example A 11

### 2-(4-acetylpiperazin-1-yl)-4-(4-methoxyphenyl)-5-(3-pyridyl)-1,3-thiazole

In a solution of 1-piperazinecarbothioamide (0.39 g) in acetonitrile (15 mL) was suspended 2-bromo-1-(4-methoxyphenyl)-2-(3-pyridyl)ethanone hydrobromide (1.0 g) and triethylamine (0.4 mL) was slowly added dropwise with stirring. After completion of dropwise addition, the mixture was stirred at a refluxing temperature for 3 h, and the solvent was evaporated. To the residue was added saturated aqueous sodium hydrogencarbonate and the mixture was extracted with ethyl acetate, and the extract was washed with water and dried, and the solvent was evaporated. The residue was dissolved in pyridine (2 mL) and cooled with ice. Acetyl chloride (0.3 mL) was added, and the mixture was left standing at room temperature for 1 h. The reaction mixture was poured into iced water, and the resulting product was extracted with ethyl acetate. The extract was washed with water, and after drying, the solvent was evaporated. The residue was purified by silica gel column chromatography (ethyl acetate-methanol=9:1) to give the title compound (0.30 g, yield 28%). oil.

### Reference Example A 12

### [4-(4-methoxyphenyl)-5-(3-pyridyl)-1,3-thiazol-2-yl]amine hydrochloride

[4-(4-Methoxyphenyl)-5-(3-pyridyl)-1,3-thiazol-2-yl]amine (200 mg) was dissolved in 1% hydrochloric acid-methanol (3.2 mL) and the solvent was evaporated. The obtained crude crystals were recrystallized from methanol-ethyl acetate to give the title compound (180 mg, yield 80%).
melting point: 145-150°C.

The chemical structural formulas of the compounds obtained in Reference Examples A8 to 12 are shown in the following Table 1.

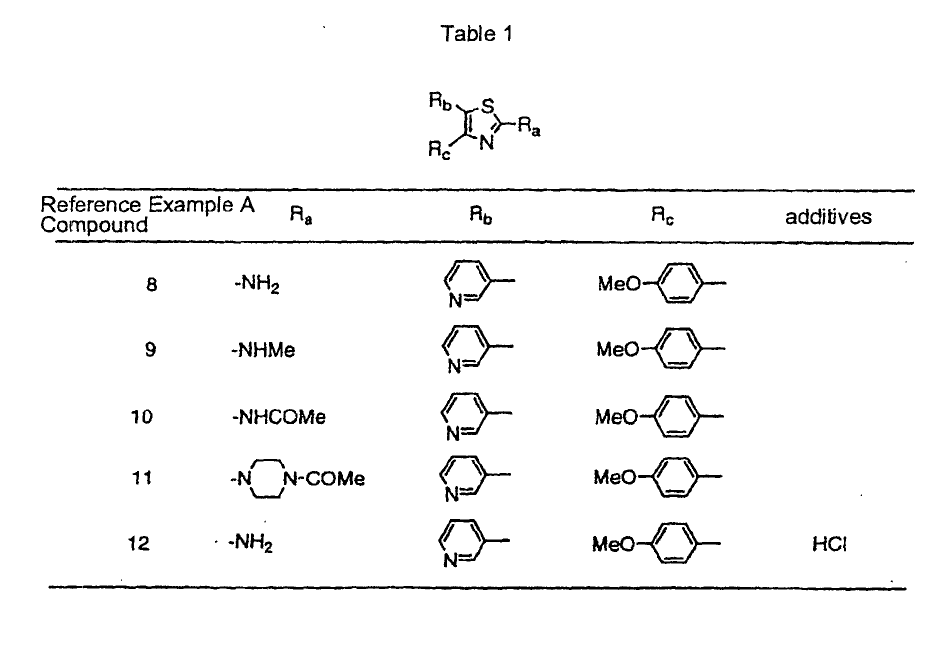

### Reference Example A 13

Reference Example A compounds 13-1 to 13-102 shown in the following Tables 2-7 were synthesized in accordance with the methods described in Reference Example A 8-12, JP-A-61-10580 and USP 4,612,321.

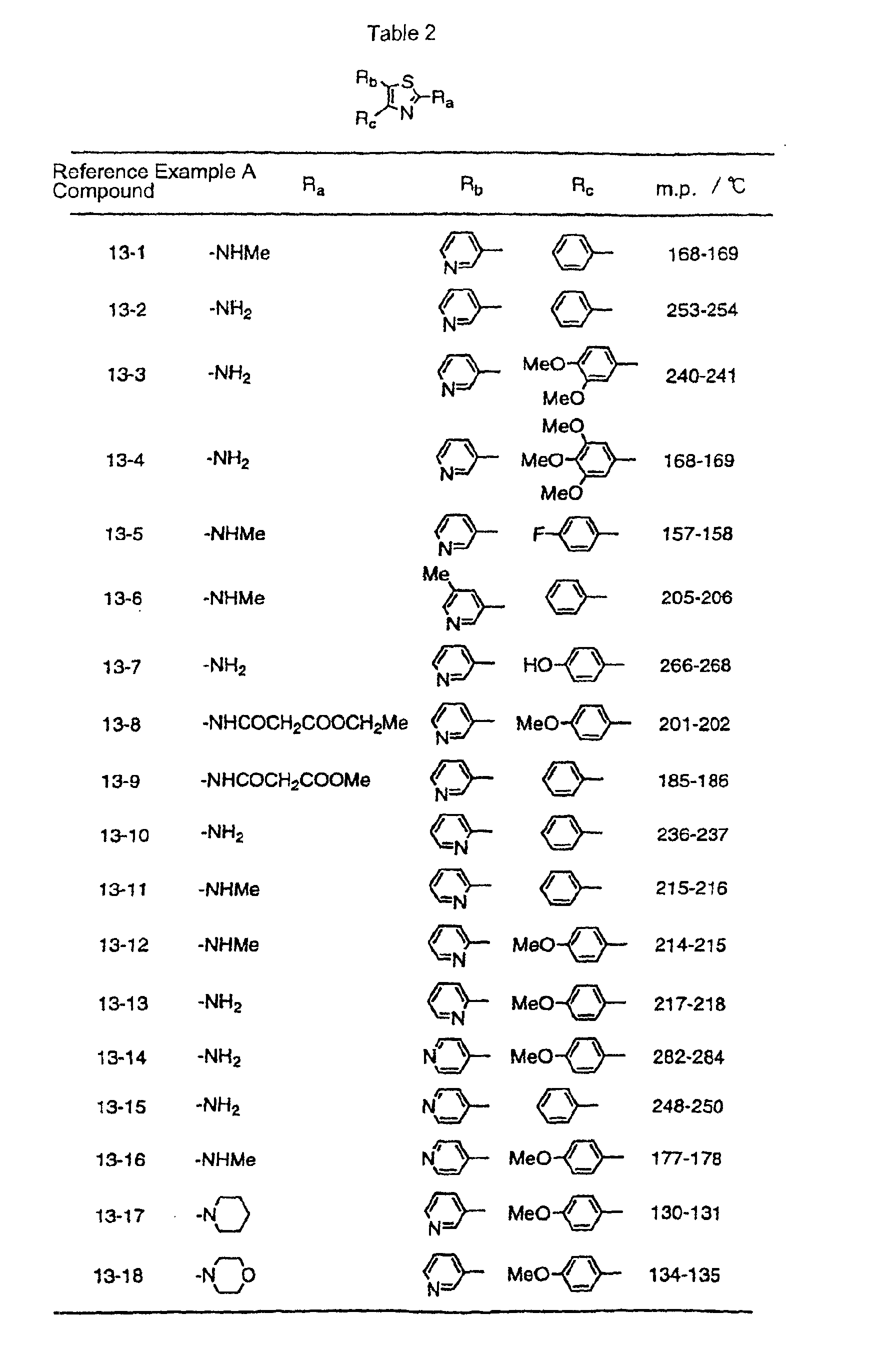

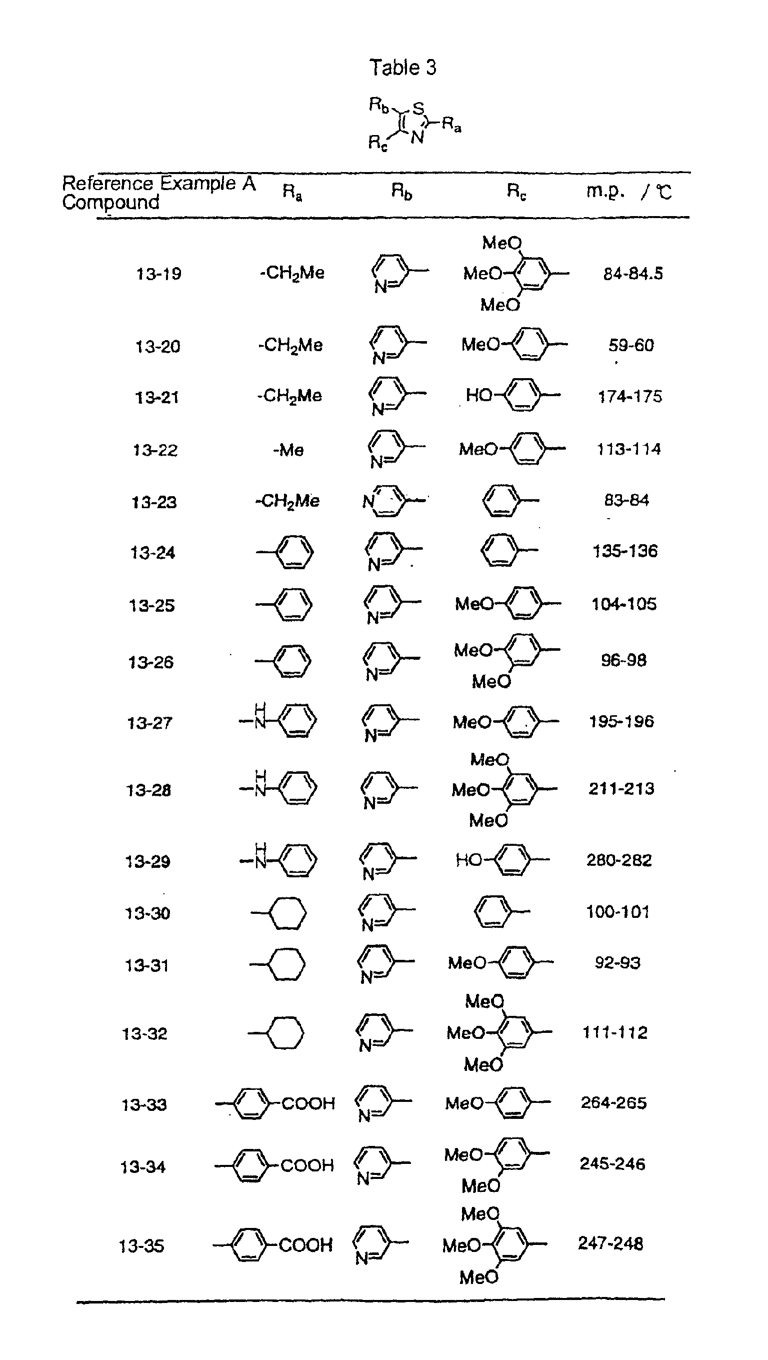

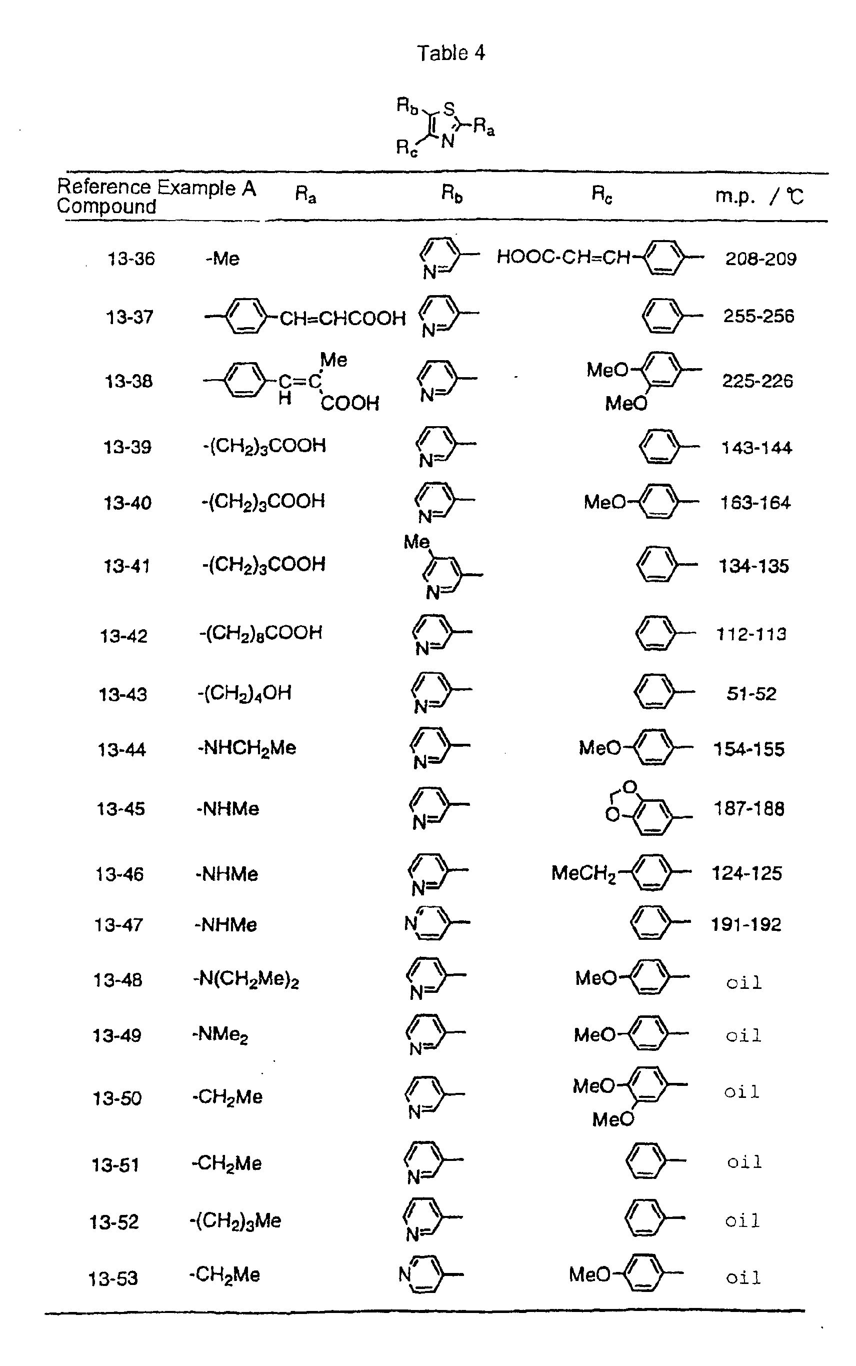

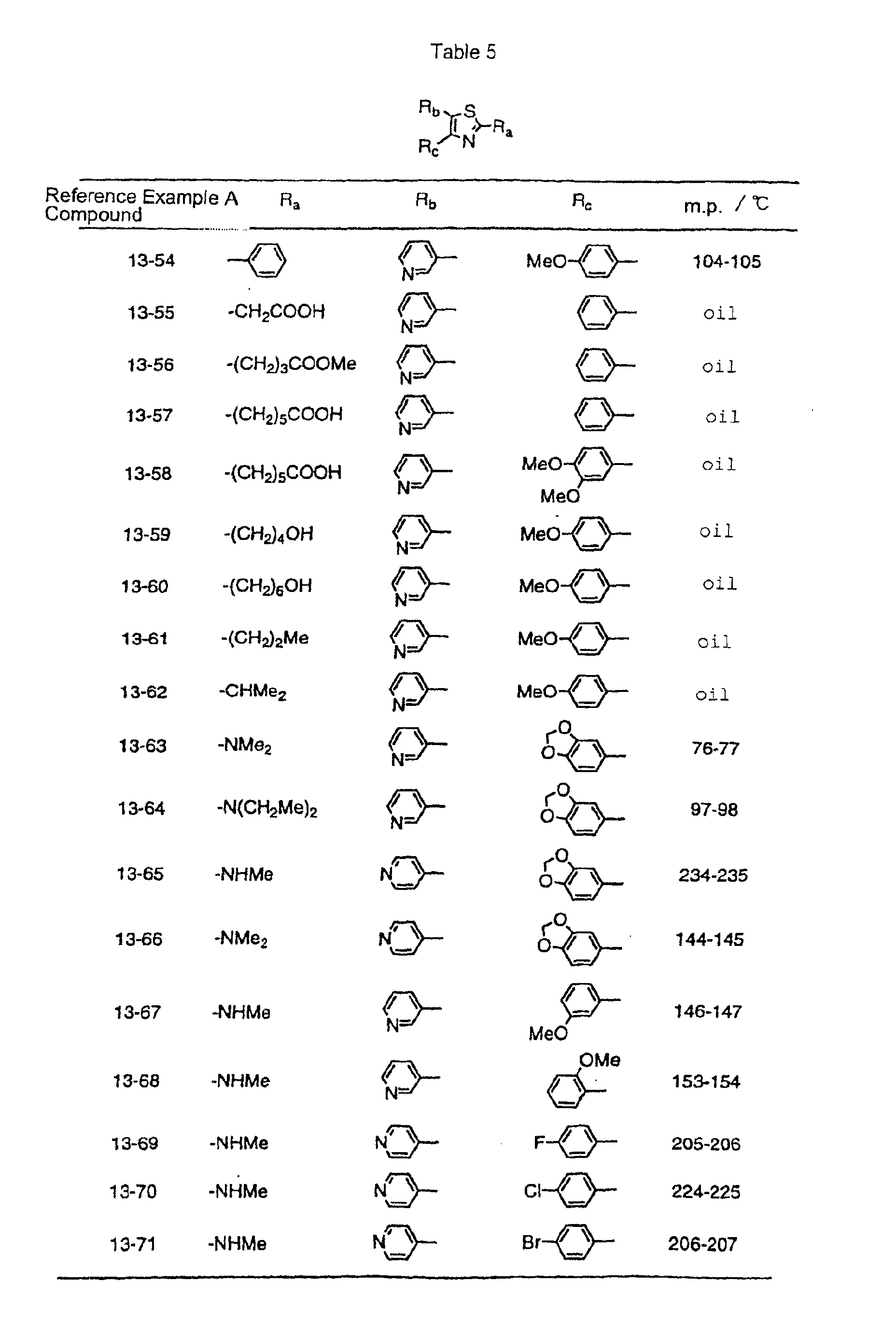

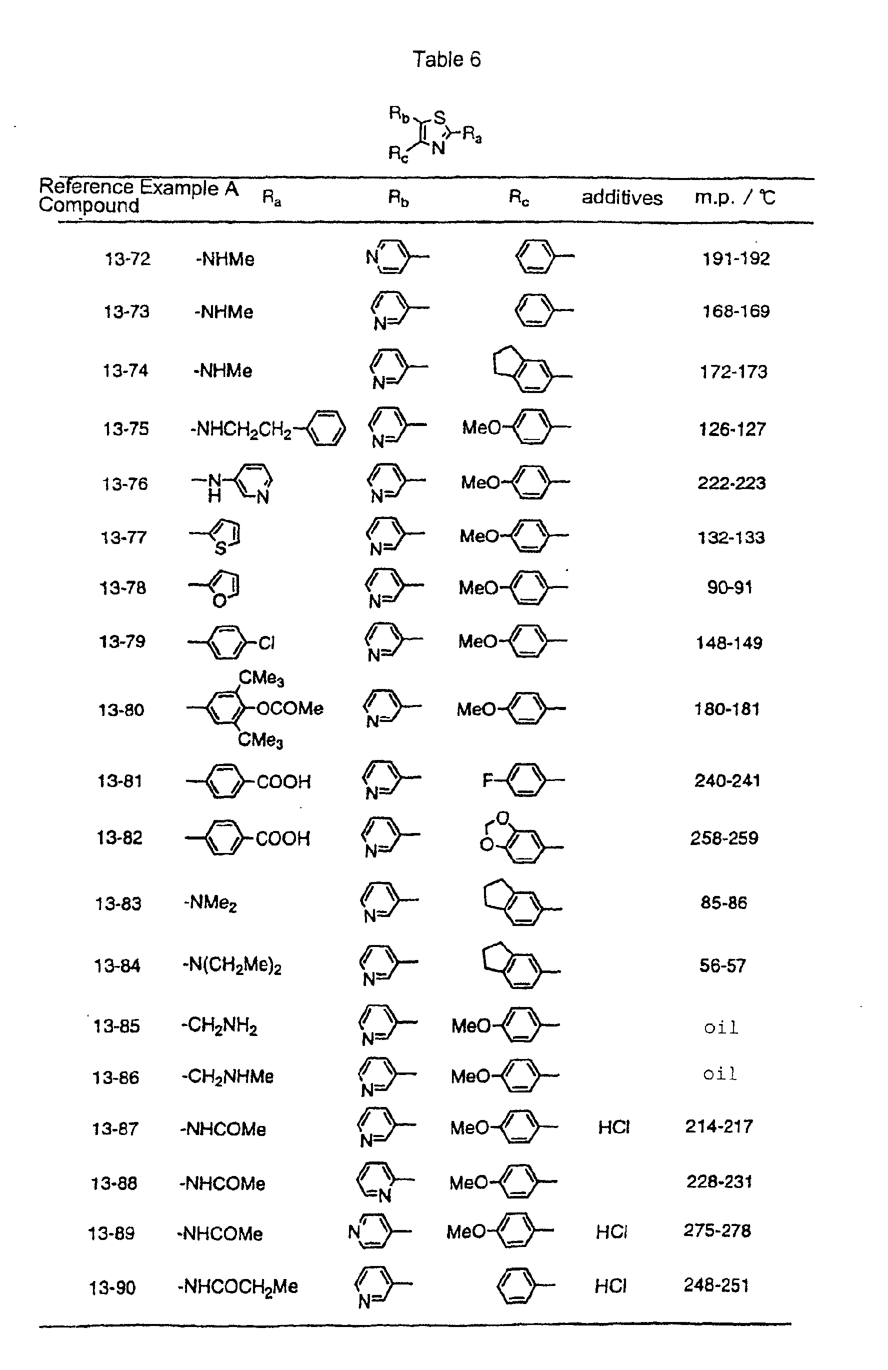

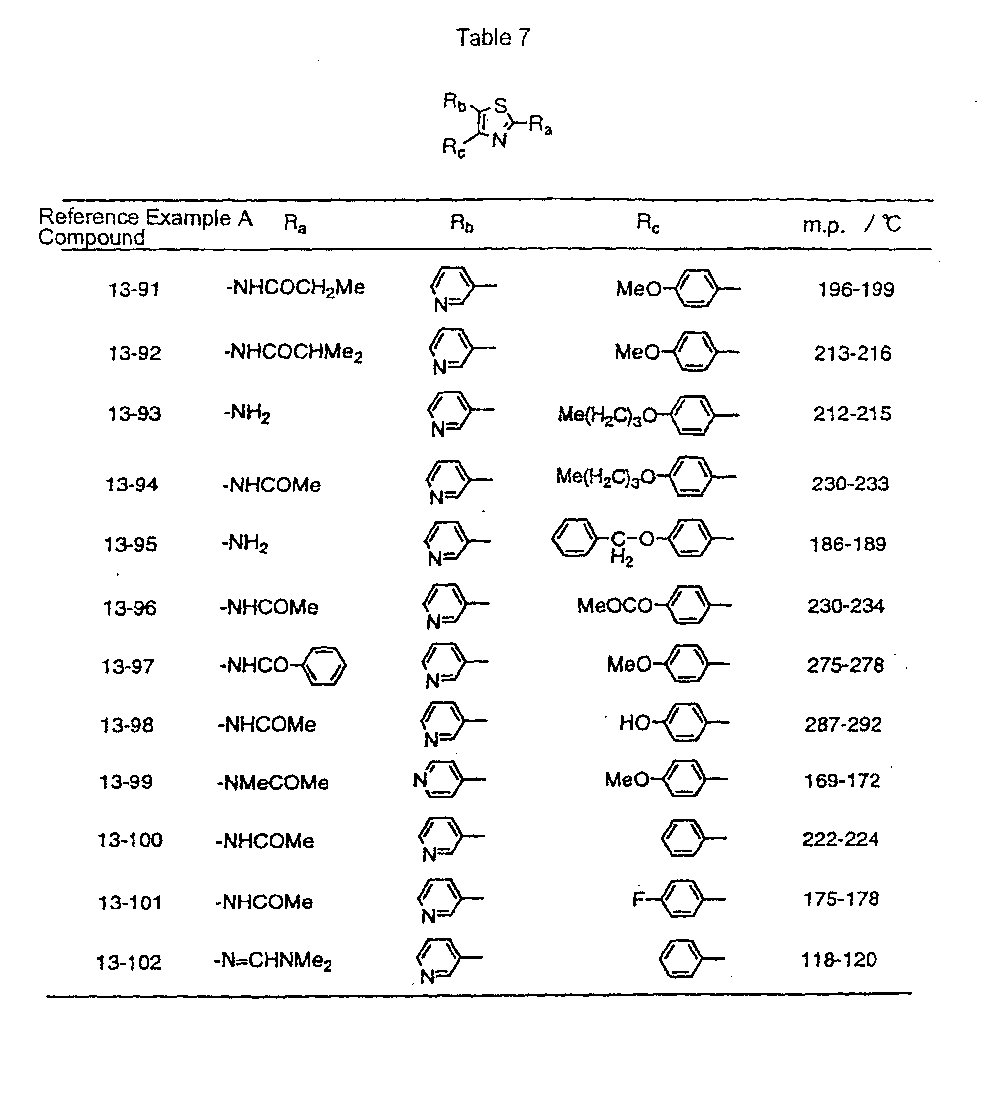

### Reference Example A 14

### N-(4-chlorobenzoyl)propyleneimine

A solution of propyleneimine (12.3 mL) in tetrahydrofuran (160 mL) was added to 1N aqueous sodium hydroxide solution. To this mixture was added dropwise 4-chlorobenzoyl chloride (25 g) at 0°C. After completion of dropwise addition, the mixture was stirred for further 30 min. The reaction mixture was extracted with ethyl acetate. The extract was dried, and the solvent was evaporated to give the title compound (24.9 g, yield 89%).
oil.
¹H-NMR (CDCl₃) δ: 1.39 (3H, d, J= 5.5 Hz), 2.15 (1H, d, J= 2.9 Hz), 2.51-2.66 (2H, m), 7.39-7.47 (2H, m), 7.93-8.01 (2H, m).

### Reference Example A 15

In accordance with Reference Example A 14, 3-chlorobenzoyl chloride, 2-chlorobenzoyl chloride, 2-methylbenzoyl chloride, 3-methylbenzoyl chloride, 4-methylbenzoyl chloride, 2-methoxybenzoyl chloride, 3-methoxybenzoyl chloride, 4-ethylbenzoyl chloride, 4-(1-methylethyl)benzoyl chloride, 4-(1,1-dimethylethyl)benzoyl chloride, 4-propylbenzoyl chloride, 4-butylbenzoyl chloride, 4-hexylbenzoyl chloride, 4-trifluoromethoxybenzoyl chloride, 4-trifluoromethylbenzoyl chloride, 3,4-dimethoxybenzoyl chloride, 3,4-dimethylbenzoyl chloride, 3,5-dimethylbenzoyl chloride, 3,4-methylenedioxybenzoyl chloride, 2-naphthoyl chloride, 4-fluorobenzoyl chloride and 3-cyclopentyloxy-4-methoxybenzoyl chloride were respectively used instead of 4-chlorobenzoyl chloride, the following Reference Example A compounds 15-1 to 15-22 were synthesized.
Reference Example A compound 15-1: N-(3-chlorobenzoyl)-propyleneimine
   oil.
   ¹H-NMR (CDCl₃) δ: 1.40 (3H, d, J= 5.1 Hz), 2.17 (1H, d, J= 3.3 Hz), 2.53-2.68 (2H, m), 7.40 (1H, dd, J= 8.1, 7.7 Hz), 7.53 (1H, ddd, J= 8.1, 2.2, 1.5 Hz), 7.90 (1H, dt, J= 7.7, 1.5 Hz), 8.00 (1H, dd, J= 2.2, 1.5 Hz).
Reference Example A compound 15-2: N-(2-chlorobenzoyl)-propyleneimine
   oil.
   ¹H-NMR (CDCl₃) δ: 1.30 (3H, d, J= 5.1 Hz), 2.12 (1H, d, J= 3.3 Hz), 2.53 (1H, d, J= 5.5 Hz), 2.56-2.68 (1H, m), 7.28-7.48 (3H, m), 7.75-7.81 (1H, m).
Reference Example A compound 15-3: N-(2-methylbenzoyl)-propyleneimine
   oil.
   ¹H-NMR (CDCl₃) δ: 1.30 (3H, d, J= 5.5 Hz), 2.08 (1H, d, J= 3.3 Hz), 2.43-2.57 (5H, m), 7.20-7.31 (2H, m), 7.33-7.43 (1H, m), 7.89 (1H, d, J= 7.7 Hz).
Reference Example A compound 15-4: N-(3-methylbenzoyl)-propyleneimine
   oil.
   ¹H-NMR (CDCl₃) δ: 1.39 (3H, d, J= 5.5 Hz), 2.14 (1H, d, J= 3.3 Hz), 2.41 (3H, s), 2.51-2.66 (2H, m), 7.32-7.39 (2H, m), 7.79-7.87 (2H, m) .
Reference Example A compound 15-5: N-(4-methylbenzoyl)-propyleneimine
   oil.
   ¹H-NMR (CDCl₃) δ: 1.39 (3H, d, J= 5.5 Hz), 2.12 (1H, d, J= 2.9 Hz), 2.42 (3H, s), 2.50-2.62 (2H, m), 7.25 (2H, d, J= 8.1 Hz), 7.92 (2H, d, J= 8.1 Hz).
Reference Example A compound 15-6: N-(2-methoxybenzoyl)-propyleneimine
   oil.
   ¹H-NMR (CDCl₃) δ: 1.30 (3H, d, J= 5.5 Hz), 2.10 (1H, d, J= 3.3 Hz), 2.50 (1H, d, J= 5.9Hz), 2.53-2.65 (1H, m), 3.90 (3H, s), 6.95-7.05 (2H, m), 7.41-7.52 (1H, m), 7.81-7.88 (1H, m). Reference Example A compound 15-7: N-(3-methoxybenzoyl)-propyleneimine
   oil.
   ¹H-NMR (CDCl₃) δ: 1.40 (3H, d, J= 5.9 Hz), 2.14 (1H, d, J= 2.9 Hz), 2.52-2.65 (2H, m), 3.86 (3H, s), 7.10 (1H, ddd, J= 8.4, 2.6, 1.1 Hz), 7.37 (1H, dd, J= 8.4, 7.3 Hz), 7.55 (1H, dd, J= 2.6, 1.5 Hz), 7.63 (1H, ddd, J= 7.3, 1.5, 1.1 Hz).
Reference Example A compound 15-8: N-(4-ethylbenzoyl)-propyleneimine
   oil.
   ¹H-NMR (CDCl₃) δ: 1.27 (3H, t, J= 7.6 Hz), 1.39 (3H, d, J= 5.5 Hz), 2.13 (1H, d, J= 3.3 Hz), 2.50-2.61 (2H, m), 2.71 (2H, q, J= 7.6 Hz), 7.28 (2H, d, J= 7.7 Hz), 7.95 (2H, d, J= 7.7 Hz).
Reference Example A compound 15-9: N-[4-(1-methylethyl)-benzoyl]propyleneimine
   oil.
   ¹H-NMR (CDCl₃) δ: 1.28 (6H, d, J= 7.0 Hz), 1.40 (3H, d, J= 5.5 Hz), 2.13 (1H, d, J= 3.3 Hz), 2.50-2.64 (2H, m), 2.90-3.05 (1H, m), 7.31 (2H, d, J= 8.2 Hz), 7.96 (2H, d, J= 8.2 Hz).
Reference Example A compound 15-10: N-[4-(1,1-dimethylethyl)-benzoyl]propyleneimine
   A solution of propyleneimine (11 mL, 0.14 mol) in tetrahydrofuran (160 mL) was added to 2N aqueous sodium hydroxide solution (70 mL). To this mixture was added dropwise 4-(1,1-dimethylethyl)benzoyl chloride (25 g, 0.13 mol) at 0°C. After completion of dropwise addition, the mixture was stirred further for 30 min. The reaction mixture was extracted with ethyl acetate. The extract was dried, and the solvent was evaporated to give the title compound (27 g, 0.13 mol, yield 99%).
   oil.
   ¹H-NMR (CDCl₃)δ: 1.35 (9H, s), 1.41 (3H, d, J= 5.5 Hz), 2.12 (1H, d, J= 2.9 Hz), 2.51-2.64 (2H, m), 7.47 (2H, d, J= 8.8 Hz), 7.96 (2H, d, J= 8.8 Hz).
Reference Example A compound 15-11: N-(4-propylbenzoyl)-propyleneimine
   oil.
   ¹H-NMR (CDCl₃) δ: 0.96 (3H, t, J= 7.3 Hz), 1.39 (3H, d, J= 5.5 Hz), 1.57-1.75 (2H, m), 2.12 (1H, d, J= 3.3 Hz), 2.50-2.59 (2H, m), 2.65 (2H, t, J= 7.7 Hz), 7.26 (2H, d, J= 8.1 Hz), 7.94 (2H, d, J= 8.1 Hz).
Reference Example A compound 15-12: N-(4-butylbenzoyl)-propyleneimine
   oil.
   ¹H-NNR (CDCl₃) δ: 0.94 (3H, t, J= 7.1 Hz), 1.26-1.47 (5H, m), 1.54-1.73 (2H, m), 2.12 (1H, d, J= 2.9 Hz), 2.51-2.62 (2H, m), 2.67 (2H, t, J= 7.7 Hz), 7.26 (2H, d, J= 8.1 Hz), 7.94 (2H, d, J= 8.1 Hz).
Reference Example A compound 15-13: N-(4-hexylbenzoyl)-propyleneimine
   oil.
   ¹H-NMR (CDCl₃) δ: 0.89 (3H, t, J= 6.6 Hz), 1.24-1.38 (6H, m), 1.39 (3H, d, J= 5.5 Hz), 1.56-1.68 (2H, m), 2.12 (1H, d, J= 3.3 Hz), 2.51-2.61 (2H, m), 2.66 (2H, t, J= 7.7 Hz), 7.26 (2H, d, J= 8.1 Hz), 7.94 (2H, d, J= 8.1 Hz).
Reference Example A compound 15-14: N-(4-trifluoromethoxybenzoyl)propyleneimine
   oil.
   ¹H-NMR (CDCl₃) δ: 1.40 (3H, d, J= 5.5 Hz), 2.16 (1H, d, J= 3.3 Hz), 2.53-2.68 (2H, m), 7.29 (2H, d, J= 9.0 Hz), 8.08 (2H, d, J= 9.0 Hz).
Reference Example A compound 15-15: N-(4-trifluoromethylbenzoyl)propyleneimine
   oil.
   ¹H-NMR (CDCl₃) δ:1.40 (3H, d, J= 5.5 Hz), 2.19 (1H, d, J= 3.7 Hz), 2.54-2.70 (2H, m), 7.73 (2H, d, J= 8.0 Hz), 8.13 (2H, d, J= 8.0 Hz).
Reference Example A compound 15-16: N-(3,4-dimethoxybenzoyl)-propyleneimine
   oil.
   ¹H-NMR (CDCl₃) δ: 1.41 (3H, d, J= 5.5 Hz), 2.12 (1H, d, J= 3.3 Hz), 2.51-2.63 (2H, m), 3.94 (3H, s), 3.95 (3H, s), 6.92 (1H, d, J= 8.5 Hz), 7.56 (1H, d, J= 2.2 Hz), 7.69 (1H, dd, J= 8.5, 2.2 Hz).
Reference Example A compound 15-17: N-(3,4-dimethylbenzoyl)-propyleneimine
   oil.
   ¹H-NMR (CDCl₃) δ: 1.39 (3H, d, J= 5.5 Hz), 2.12 (1H, d, J= 3.3 Hz), 2.32 (6H, s), 2.49-2.61 (2H, m), 7.21 (1H, d, J= 7.7 Hz), 7.77 (1H, dd, J= 7.7, 1.8 Hz), 7.80 (1H, d, J= 1.8 Hz).
Reference Example A compound 15-18: N-(3,5-dimethylbenzoyl)-propyleneimine
   3,5-Dimethylbenzoic acid (25 g, 0.17 mol) and dimethylformamide (0.1 mL) were added to thionyl chloride (50 mL) at 0°C successively. The mixture was refluxed under heating for 2 h. The excess thionyl chloride was evaporated under reduced pressure and to the residue was added toluene (50 mL). Toluene was evaporated under reduced pressure to give oily 3,5-dimethylbenzoyl chloride. A solution of propyleneimine (14 mL, 0.18 mol) in tetrahydrofuran (160 mL) was added to 1N aqueous sodium hydroxide solution (180 mL). 3,5-Dimethylbenzoyl chloride was added dropwise to this mixture at 0°C. After completion of dropwise addition, the mixture was stirred further for 30 min. The reaction mixture was extracted with ethyl acetate. The extract was dried, and the solvent was evaporated to give the title compound (31 g, 0.16 mol, yield 99%).
   oil.
   ¹H-NMR (CDCl₃)δ: 1.39 (3H, d, J= 5.5 Hz), 2.13 (1H, d, J= 3.7 Hz), 2.37 (6H, s), 2.47-2.62 (2H, m), 7.19 (1H, s), 7.64 (2H, s) .
Reference Example A compound 15-19: N-(3,4-methylenedioxybenzoyl)propyleneimine
   oil.
   ¹H-NMR (CDCl₃) δ: 1.38 (3H, d, J= 4.9 Hz), 2.11 (1H, d, J= 3.1 Hz), 2.48-2.64 (2H, m), 6.05 (2H, s), 6.86 (1H, d, J= 8.2 Hz), 7.48 (1H, d, J= 1.7 Hz), 7.65 (1H, dd, J= 8.2, 1.7 Hz).
Reference Example A compound 15-20: N-(2-naphthoyl)-propyleneimine
   oil.
   ¹H-NMR (CDCl₃) δ: 1.44 (3H, d, J= 5.5 Hz), 2.22 (1H, d, J= 3.3 Hz), 2.57-2.84 (2H, m), 7.50-7.65 (2H, m), 7.85-8.00 (3H, m), 8.06 (1H, dd, J= 8.6, 1.5 Hz), 8.59 (1H, s).
Reference Example A compound 15-21: N-(4-fluorobenzoyl)-propyleneimine
   oil.
   ¹H-NMR (CDCl₃) δ: 1.39 (3H, d, J= 5.2 Hz), 2.14-2.15 (1H, m), 2.52-2.63 (2H, m), 7.08-7.19 (2H, m), 8.00-8.10 (2H, m).
Reference Example compound A 15-22: N-(3-cyclopentyloxy-4-methoxybenzoyl)propyleneimine
   oil.
   ¹H-NMR (CDCl₃) δ: 1.40 (3H, d, J= 5.1 Hz), 1.54-1.68 (2H, m), 1.73-2.06 (6H, m), 2.11 (1H, d, J= 3.3 Hz), 2.51-2.63 (2H, m), 3.91 (3H, s), 4.79-4.90 (1H, m), 6.90 (1H, d, J= 8.4 Hz), 7.55 (1H, d, J= 1.8 Hz), 7.65 (1H, dd, J= 8.4, 1.8 Hz).

### Reference Example A 16

### 1-(2-chlorophenyl)-2-(4-pyridyl)ethanone

A solution of diisopropylamine (15 mL) in anhydrous tetrahydrofuran (100 mL) was cooled at -50°C and 1.6 M n-butyllithium/hexane solution (69 mL) was added dropwise with stirring. After completion of dropwise addition, the mixture was stirred for 10 min and a solution of γ-picoline (20 g) in anhydrous tetrahydrofuran (10 mL) was added dropwise at -30°C. The mixture was stirred for 1 h and a solution of N-(2-chlorobenzoyl)propyleneimine (20 g) in anhydrous tetrahydrofuran (10 mL) was added dropwise at -10°C. After completion of dropwise addition, the mixture was stirred for at room temperature for 2 h. To the reaction mixture was added water (100 mL) and the mixture was extracted with ethyl acetate. The extract was washed with water, and after drying, the solvent was evaporated. The residue was purified by silica gel column chromatography (hexane-ethyl acetate=1:1) to give the title compound (16 g, yield 71%).
oil.
¹H-NMR (CDCl₃) δ: 4.28 (2H, s), 7.20 (2H, d, J= 6.2 Hz), 7.28-7.39 (1H, m), 7.41-7.48 (3H, m), 8.56 (2H, d, J= 6.2 Hz).

### Reference Example A 17

In accordance with Reference Example A 16, N-(3-chlorobenzoyl)propyleneimine, N-(4-chlorobenzoyl)-propyleneimine, N-(2-methylbenzoyl)propyleneimine, N-(3-methylbenzoyl)propyleneimine, N-(4-methylbenzoyl)-propyleneimine, N-(2-methoxybenzoyl)propyleneimine, N-(3-methoxybenzoyl)propyleneimine, N-(4-ethylbenzoyl)-propyleneimine, N-[4-(1-methylethyl)benzoyl]propyleneimine, N-[4-(1,1-dimethylethyl)benzoyl]propyleneimine, N-(4-propylbenzoyl)propyleneimine, N-(4-butylbenzoyl)propyleneimine, N-(4-hexylbenzoyl)propyleneimine, N-(4-trifluoromethoxybenzoyl)propyleneimine, N-(4-trifluoromethylbenzoyl)propyleneimine, N-(3,4-dimethoxybenzoyl)propyleneimine, N-(3,4-dimethylbenzoyl)-propyleneimine, N-(3,5-dimethylbenzoyl)propyleneimine, N-(3,4-methylenedioxybenzoyl)propyleneimine, N-(2-naphthoyl)-propyleneimine and N-(3-cyclopentyloxy-4-methoxybenzoyl)-propyleneimine, instead of N-(2-chlorobenzoyl)propyleneimine, the following Reference Example A compounds 17-1 to 17-21 were synthesized.
Reference Example A compound 17-1: 1-(3-chlorophenyl)-2-(4-pyridyl) ethanone
   melting point: 79-80°C.
Reference Example A compound 17-2: 1-(4-chlorophenyl)-2-(4-pyridyl)ethanone
   melting point: 93-94°C.
Reference Example A compound 17-3: 1-(2-methylphenyl)-2-(4-pyridyl)ethanone
   oil.
   ¹H-NMR (CDCl₃) δ: 2.48 (3H, s), 4.23 (2H, s), 7.19 (2H, d, J= 6.2 Hz), 7.24-7.47 (3H, m), 7.73 (1H, d, J= 7.7 Hz), 8.56 (2H, d, J= 6.2 Hz).
Reference Example A compound 17-4: 1-(3-methylphenyl)-2-(4-pyridyl)ethanone
   melting point: 115-116°C.
Reference Example A compound 17-5: 1-(4-methylphenyl)-2-(4-pyridyl)ethanone
   melting point: 110-111°C.
Reference Example A compound 17-6: 1-(2-methoxyphenyl)-2-(4-pyridyl)ethanone
   oil.
   ¹H-NMR (CDCl₃) δ: 3.92 (3H, s), 4.30 (2H, s), 6.95-7.07 (2H, m), 7.17 (2H, d, J= 5.9 Hz), 7.50 (1H, ddd, J= 8.4, 7.3, 1.8 Hz), 7.73 (1H, dd, J= 7.7, 1.8 Hz), 8.53 (2H, d, J= 5.9 Hz).
Reference Example A compound 17-7: 1-(3-methoxyphenyl)-2-(4-pyridyl)ethanone
   oil.
   ¹H-NMR (CDCl₃) δ: 3.86 (3H, s), 4.28 (2H, s), 7.14 (1H, ddd, J= 8.1, 2.6, 1.1 Hz), 7.20 (2H, d, J= 6.2 Hz), 7.36 (1H, dd, J= 8.1, 7.7 Hz), 7.51 (1H, dd, J= 2.6, 1.5 Hz), 7.58 (1H, ddd, J= 7.7, 1.5, 1.1 Hz), 8.57 (2H, d, J= 6.2 Hz).
Reference Example A compound 17-8: 1-(4-ethylphenyl)-2-(4-pyridyl) ethanone
   melting point: 87-89°C.
Reference Example A compound 17-9: 1-[4-(1-methylethyl)phenyl]-2-(4-pyridyl)ethanone
   melting point: 86-88°C.
Reference Example A compound 17-10: 1-[4-(1,1-dimethylethyl)-phenyl]-2-(4-pyridyl)ethanone
   A solution of diisopropylamine (15 mL, 0.11 mol) in anhydrous tetrahydrofuran (100 mL) was cooled to -50°C, 1.6 M n-butyllithium-hexane solution (69 mL, 0.11 mol) was added dropwise with stirring. After completion of dropwise addition, the mixture was stirred for 10 min, and then a solution of γ-picoline (9.3 g, 0.10 mol) in anhydrous tetrahydrofuran (10 mL) was added dropwise at -30°C. The mixture was stirred for 1 h, a solution of N-[4-(1,1-dimethylethyl)benzoyl]-propyleneimine (22 g, 0.10 mol) in anhydrous tetrahydrofuran (10 mL) was added dropwise at -30°C. After completion of dropwise addition, the temperature of the mixture was increased gradually to room temperature and the mixture was stirred for 2 h. To the reaction mixture was added water (100 mL), the mixture was extracted with ethyl acetate. The extract was washed with water, and after drying, the solvent was evaporated. The residue was purified by silica gel column chromatography (hexane-ethyl acetate, 1:1) and recrystallized from diisopropyl ether-hexane to give the title compound (11 g, yield 43%).
   melting point: 75-76°C.
Reference Example A compound 17-11: 1-(4-propylphenyl)-2-(4-pyridyl)ethanone
   melting point: 71-72°C.
Reference Example A compound 17-12: 1-(4-butylphenyl)-2-(4-pyridyl)ethanone
   melting point: 41-43°C.
Reference Example A compound 17-13: 1-(4-hexylphenyl)-2-(4-pyridyl)ethanone
   melting point: 57-58°C.
Reference Example A compound 17-14: 2-(4-pyridyl)-1-(4-trifluoromethoxyphenyl)ethanone
   melting point: 65-66°C.
Reference Example A compound 17-15: 2-(4-pyridyl)-1-(4-trifluoromethylphenyl)ethanone
   melting point: 94-95°C.
Reference Example A compound 17-16: 1-(3,4-dimethoxyphenyl)-2-(4-pyridyl) ethanone
   melting point: 110-111°C.
Reference Example A compound 17-17: 1-(3,4-dimethylphenyl)-2-(4-pyridyl)ethanone
   melting point: 81-83°C.
Reference Example A compound 17-18
   1-(3,5-dimethylphenyl)-2-(4-pyridyl)ethanone
   A solution of diisopropylamine (15 mL, 0.11 mol) in anhydrous tetrahydrofuran (100 mL) was cooled to -50°C, 1.6 M n-butyllithium-hexane solution (69 mL, 0.11 mol) was added dropwise with stirring. After completion of dropwise addition, the mixture was stirred for 10 min, and a solution of γ-picoline (9.3 g, 0.10 mol) in anhydrous tetrahydrofuran (10 mL) was added dropwise at -30°C. The mixture was stirred for 1 h, a solution of N-(3,5-dimethylbenzoyl)propyleneimine (19 g, 0.10 mol) in anhydrous tetrahydrofuran (10 mL) was added dropwise at -30°C. After completion of dropwise addition, the temperature of the mixture was gradually raised to room temperature and the mixture was stirred for 2 h. To the reaction mixture was added water (100 mL) and the mixture was extracted with ethyl acetate. The extract was washed with water, and after drying, the solvent was evaporated. The residue was crystallized from diisopropyl ether-hexane to give the title compound (13 g, yield 58%).
   melting point: 90-91°C.
Reference Example A compound 17-19: 1-(3,4-methylenedioxyphenyl)-2-(4-pyridyl)ethanone
   melting point: 126-127°C.
Reference Example A compound 17-20: 1-(2-naphthyl)-2-(4-pyridyl)ethanone
   melting point: 114-115°C.
Reference Example A compound 17-21: 1-(3-cyclopentyloxy-4-methoxyphenyl)-2-(4-pyridyl)ethanone
   melting point: 87-89°C.

### Reference Example A 18

In accordance with Reference Example A 17, the following Reference Example A compound 18-1-18-9 were synthesized using γ-picoline instead of β-picoline.
Reference Example A compound 18-1: 1-(2-chlorophenyl)-2-(3-pyridyl)ethanone
   oil.
   ¹H-NMR (CDCl₃) δ: 4.28 (2H, s), 7.18-7.49 (5H, m), 7.59-7.67 (1H, m), 8.47-8.56 (2H, m).
Reference Example A compound 18-2: 1-(3-chlorophenyl)-2-(3-pyridyl)ethanone
   oil.
   ¹H-NMR (CDCl₃) δ: 4.29 (2H, s), 7.25-7.34 (1H, m), 7.44 (1H, t, J= 7.7 Hz), 7.54-7.63 (2H, m), 7.90 (1H, dt, J= 7.7, 1.5 Hz), 8.00 (1H, dd, J= 1.8, 1.5 Hz), 8.49-8.57 (2H, m).
Reference Example A compound 18-3: 1-(4-chlorophenyl)-2-(3-pyridyl)ethanone
   ¹H-NMR (CDCl₃) δ: 4.27 (2H, s), 7.24-7.31 (1H, m), 7.47 (2H, d, J= 8.8 Hz), 7.55-7.63 (1H, m), 7.96 (2H, d, J= 8.8 Hz), 8.46-8.53 (2H, m) .
Reference Example A compound 18-4: 1-(2-methylphenyl)-2-(3-pyridyl)ethanone
   oil.
   ¹H-NMR (CDCl₃) δ: 2.47 (3H, s), 4.23 (2H, s), 7.18-7.47 (5H, m), 7.73 (1H, d, J= 7.7 Hz), 8.47-8.56 (2H, m).
Reference Example A compound 18-5: 1-(3-methylphenyl)-2-(3-pyridyl)ethanone
   oil.
   ¹H-NMR (CDCl₃) δ: 2.43 (3H, s), 4.29 (2H, s), 7.17-7.36 (1H, m), 7.36-7.46 (2H, m), 7.58-7.65 (1H, m), 7.78-7.86 (2H, m) , 8.50-8.56 (2H, m).
Reference Example A compound 18-6: 1-(4-methylphenyl)-2-(3-pyridyl)ethanone
   melting point: 72-74°C.
Reference Example A compound 18-7: 1-(3-methoxyphenyl)-2-(3-pyridyl)ethanone
   oil.
   ¹H-NMR (CDCl₃) δ: 3.86 (3H, s), 4.29 (2H, s), 7.14 (1H, ddd, J= 8.1, 2.6, 1.8 Hz), 7.28 (1H, dd, J= 7.3, 4.8 Hz), 7.40 (1H, dd, J= 8.1, 7.7 Hz), 7.53 (1H, dd, J= 2.6, 1.8 Hz), 7.58-7.65 (2H, m), 8.50-8.55 (2H, m).
Reference Example A compound 18-8: 1-[4-(1,1-dimethylethyl)phenyl]-2-(3-pyridyl)ethanone
   oil.
   ¹H-NMR (CDCl₃) δ: 1.34 (9H, s), 4.28 (2H, s), 7.22-7.31 (1H, m), 7.50 (2H, d, J= 8.4 Hz), 7.56-7.65 (1H, m), 7.96 (2H, d, J= 8.4 Hz), 8.48-8.55 (2H, m).
Reference Example A compound 18-9: 1-(3,5-dimethylphenyl)-2-(3-pyridyl)ethanone
   oil.
   ¹H-NMR (CDCl₃) δ: 2.38 (6H, s), 4.27 (2H, s), 7.24-7.30 (2H, m), 7.58-7.63 (3H, m), 8.50-8.52 (2H, m).

### Reference Example A 19

In accordance with Reference Example A 1, the following Reference Example A compound 19 was synthesized using ethyl 4-dimethylaminobenzoate instead of ethyl p-anisate.
Reference Example A compound 19: 1-(4-dimethylaminophenyl)-2-(4-pyridyl)ethanone
   melting point: 189-192°C.

### Reference Example A 20

### 1-(4-fluorophenyl)-2-(4-pyridyl)ethanone

A solution of diisopropylamine (29 mL) in anhydrous tetrahydrofuran (300 mL) was cooled to -78°C, and 1.6 M n-butyllithium/hexane solution (140 mL) was added dropwise with stirring. After completion of dropwise addition, the mixture was stirred for 10 min, and then a solution of γ-picoline (21 g) in anhydrous tetrahydrofuran (50 mL) was added. The reaction mixture was stirred at -10°C for 30 min. The reaction solution was cooled to -78°C and a solution of N-(4-fluorobenzoyl)propyleneimine (36 g) in anhydrous tetrahydrofuran (50 mL) was added dropwise. After completion of dropwise addition, the mixture was stirred at room temperature for 3 h. To the reaction mixture was added water (100 mL) and extracted with ethyl acetate. The extract was washed with water, and after drying, the solvent was evaporated. The residue was crystallized from diisopropyl ether to give the title compound (28 g, yield 66%).
melting point: 90-91°C.

### Reference Example A 21

### 4-(methylthio)thiobenzamide

4-Methylthiobenzonitrile (12 g) was dissolved in a solution (130 mL) of 4N hydrogen chloride in ethyl acetate. To this solution was added O,O-diethyl dithiophosphate (15 mL) and the mixture was stirred at room temperature for 22 h. To the reaction mixture was added water (100 mL), and the mixture was extracted with ethyl acetate. The insoluble material was filtered off and the filtrate was washed with saturated brine, dried and the solvent was evaporated. The residue was recrystallized from ethyl acetate to give the title compound (10 g, yield 67%).
melting point: 176-178°C.

### Reference Example A 22

In accordance with Reference Example A 6 and respectively using 1-(2-chlorophenyl)-2-(3-pyridyl)ethanone, 1-(3-chlorophenyl)-2-(3-pyridyl)ethanone, 1-(4-chlorophenyl)-2-(3-pyridyl)ethanone, 1-(2-methylphenyl)-2-(3-pyridyl)ethanone, 1-(3-methylphenyl)-2-(3-pyridyl)ethanone, 1-(4-methylphenyl)-2-(3-pyridyl) ethanone, 1-(3-methoxyphenyl)-2-(3-pyridyl)ethanone, 1-[4-(1,1-dimethylethyl)phenyl]-2-(3-pyridyl)ethanone, 1-(3,5-dimethylphenyl)-2-(3-pyridyl)ethanone, 1-(2-chlorophenyl)-2-(4-pyridyl)ethanone, 1-(3-chlorophenyl)-2-(4-pyridyl)ethanone, 1-(4-chlorophenyl)-2-(4-pyridyl)ethanone, 1-(2-methylphenyl)-2-(4-pyridyl)ethanone, 1-(3-methylphenyl)-2-(4-pyridyl)ethanone, 1-(4-methylphenyl)-2-(4-pyridyl) ethanone, 1-(2-methoxyphenyl)-2-(4-pyridyl) ethanone, 1-(3-methoxyphenyl)-2-(4-pyridyl)ethanone, 1-(4-ethylphenyl)-2-(4-pyridyl)ethanone, 1-[4-(1-methylethyl)phenyl]-2-(4-pyridyl)ethanone, 1-[4-(1,1-dimethylethyl)phenyl]-2-(4-pyridyl)ethanone, 1-(4-propylphenyl)-2-(4-pyridyl)ethanone, 1-(4-butylphenyl)-2-(4-pyridyl)ethanone, 1-(4-hexylphenyl)-2-(4-pyridyl)ethanone, 2-(4-pyridyl)-1-(4-trifluoromethoxyphenyl)ethanone, 2-(4-pyridyl)-1-(4-trifluoromethylphenyl)ethanone, 1-(4-dimethylaminophenyl)-2-(4-pyridyl)ethanone hydrobromide, 1-(3,4-dimethoxyphenyl)-2-(4-pyridyl)ethanone, 1-(3,4-dimethylphenyl)-2-(4-pyridyl)ethanone, 1-(3,5-dimethylphenyl)-2-(4-pyridyl)ethanone, 1-(3,4-methylenedioxyphenyl)-2-(4-pyridyl)ethanone, 1-(2-naphthyl)-2-(4-pyridyl)ethanone, 1-(4-fluorophenyl)-2-(4-pyridyl)ethanone and 1-(3-cyclopentyloxy-4-methoxyphenyl)-2-(4-pyridyl) ethanone instead of 1-(4-methoxyphenyl)-2-(3-pyridyl)ethanone, the following Reference Example A compounds 22-1 to 22-33 were synthesized.
Reference Example A compound 22-1: 2-bromo-1-(2-chlorophenyl)-2-(3-pyridyl)ethanone hydrobromide
   melting point: 88-90°C.
Reference Example A compound 22-2: 2-bromo-1-(3-chlorophenyl)-2-(3-pyridyl)ethanone hydrobromide
   melting point: 164-166°C
Reference Example A compound 22-3: 2-bromo-1-(4-chlorophenyl)-2-(3-pyridyl)ethanone hydrobromide
   Used in the next reaction without purification.
Reference Example A compound 22-4: 2-bromo-1-(2-methylphenyl)-2-(3-pyridyl)ethanone hydrobromide
   Used in the next reaction without purification.
Reference Example A compound 22-5: 2-bromo-1-(3-methylphenyl)-2-(3-pyridyl)ethanone hydrobromide
   Used in the next reaction without purification.
Reference Example A compound 22-6: 2-bromo-1-(4-methylphenyl)-2- (3--pyridyl) ethanone hydrobromide
   melting point: 96-98°C.
Reference Example A compound 22-7: 2-bromo-1-(3-methoxyphenyl)-2-(3-pyridyl)ethanone hydrobromide
   Used in the next reaction without purification.
Reference Example A compound 22-8: 2-bromo-1-[4-(1,1-dimethylethyl)phenyl]-2-(3-pyridyl)ethanone hydrobromide
   melting point: 190-194°C.
Reference Example A compound 22-9: 2-bromo-1-(3,5-dimethylphenyl)-2-(3-pyridyl)ethanone hydrobromide
   melting point: 195-197°C.
Reference Example A compound 22-10: 2-bromo-1-(2-chlorophenyl)-2-(4-pyridyl)ethanone hydrobromide
   melting point: 157-159°C.
Reference Example A compound 22-11: 2-bromo-1-(3-chlorophenyl)-2-(4-pyridyl)ethanone hydrobromide
   melting point: 178-181°C.
Reference Example A compound 22-12: 2-bromo-1-(4-chlorophenyl)-2-(4-pyridyl)ethanone hydrobromide
   melting point: 189-193°C.
Reference Example A compound 22-13: 2-bromo-1-(2-methylphenyl)-2-(4-pyridyl)ethanone hydrobromide
   melting point: 183-186°C.
Reference Example A compound 22-14: 2-bromo-1-(3-methylphenyl)-2-(4-pyridyl)ethanone hydrobromide
   Used in the next reaction without purification.
Reference Example A compound 22-15: 2-bromo-1-(4-methylphenyl)-2-(4-pyridyl)ethanone hydrobromide
   melting point: 111-113°C.
Reference Example A compound 22-16: 2-bromo-1-(2-methoxyphenyl)-2-(4-pyridyl)ethanone hydrobromide
   melting point: 168-171°C.
Reference Example A compound 22-17: 2-bromo-1-(3-methoxyphenyl)-2-(4-pyridyl)ethanone hydrobromide
   Used in the next reaction without purification.
Reference Example A compound 22-18: 2-bromo-1-(4-ethylphenyl)-2-(4-pyridyl)ethanone hydrobromide
   melting point: 170-173°C.
Reference Example A compound 22-19: 2-bromo-1-[4-(1-methylethyl)phenyl]-2-(4-pyridyl)ethanone hydrobromide melting point: 185-188°C.
Reference Example A compound 22-20: 2-bromo-1-[4-(1,1-dimethylethyl)phenyl]-2-(4-pyridyl)ethanone hydrobromide
   1-[4-(1,1-Dimethylethyl)phenyl]-2-(4-pyridyl)ethanone (10 g, 39 mmol) was dissolved in acetic acid (40 mL) and bromine (2.0 mL, 39 mmol) was added. The mixture was stirred at 80°C for 3 h. The reaction mixture was cooled with iced water and the precipitated crude crystals were collected by filtration. The crude crystals were washed with ethyl acetate to give the title compound (9.6 g, yield 81%).
   melting point: 209-212°C.
Reference Example A compound 22-21: 2-bromo-1-(4-propylphenyl)-2-(4-pyridyl)ethanone hydrobromide
   melting point: 167-170°C.
Reference Example A compound 22-22: 2-bromo-1-(4-butylphenyl)-2-(4-pyridyl)ethanone hydrobromide
   melting point: 158-161°C.
Reference Example A compound 22-23: 2-bromo-1-(4-hexylphenyl)-2-(4-pyridyl)ethanone hydrobromide
   melting point: 153-155°C.
Reference Example A compound 22-24: 2-bromo-2-(4-pyridyl)-1-(4-trifluoromethoxyphenyl)ethanone hydrobromide
   Used in the next reaction without purification.
Reference Example A compound 22-25: 2-bromo-2-(4-pyridyl)-1-(4-trifluoromethylphenyl)ethanone hydrobromide
   melting point: 190-194°C.
Reference Example A compound 22-26: 2-bromo-1-(4-dimethylaminophenyl)-2-(4-pyridyl)ethanone dihydrobromide
   melting point: 163-167°C.
Reference Example A compound 22-27: 2-bromo-1-(3,4-dimethoxyphenyl)-2-(4-pyridyl)ethanone hydrobromide
   melting point: 174-175°C.
Reference Example A compound 22-28: 2-bromo-1-(3,4-dimethylphenyl)-2-(4-pyridyl)ethanone hydrobromide
   melting point: 196-199°C.
Reference Example A compound 22-29: 2-bromo-1-(3,5-dimethylphenyl)-2-(4-pyridyl)ethanone hydrobromide
   1-(3,5-Dimethylphenyl)-2-(4-pyridyl)ethanone (7.0 g, 31 mmol) was dissolved in acetic acid (35 mL) and bromine (1.6 mL, 31 mmol) was added. The mixture was stirred at 80°C for 3 h. Ethyl acetate was added to the residue and the precipitated crude crystals were collected by filtration. The crude crystals were washed with ethyl acetate to give the title compound (16 g, yield 96%).
   melting point: 216-219°C.
Reference Example A compound 22-30: 2-bromo-1-(3,4-methylenedioxyphenyl)-2-(4-pyridyl)ethanone hydrobromide
   melting point: 211-214°C.
Reference Example A compound 22-31: 2-bromo-1-(2-naphthyl)-2-(4-pyridyl)ethanone hydrobromide
   melting point: 149-152°C.
Reference Example A compound 22-32: 2-bromo-1-(4-fluorophenyl)-2-(4-pyridyl)ethanone hydrobromide
   melting point: 185-189°C.
Reference Example A compound 22-33: 2-bromo-1-(3-cyclopentyloxy-4-methoxyphenyl)-2-(4-pyridyl)ethanone hydrobromide
   melting point: 168-170°C.

### Reference Example A 23

In accordance with the method described in Reference Examples A 8-12, JP-A-61-10580 and USP 4,612,321, Reference Example A compounds 23-1 to 23-294 and 23-295 to 23-349 shown in the following Tables 8 to 31 were synthesized.

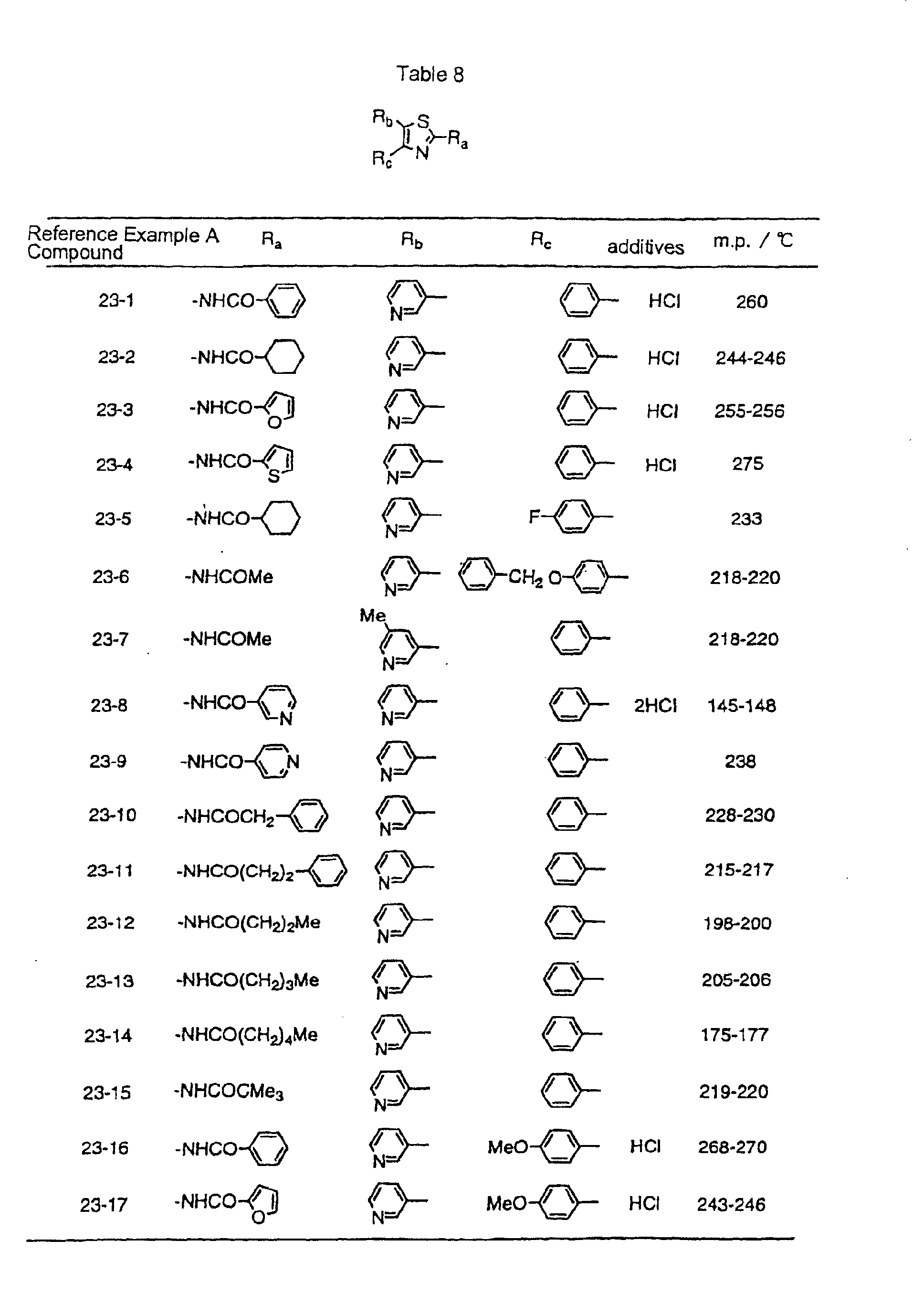

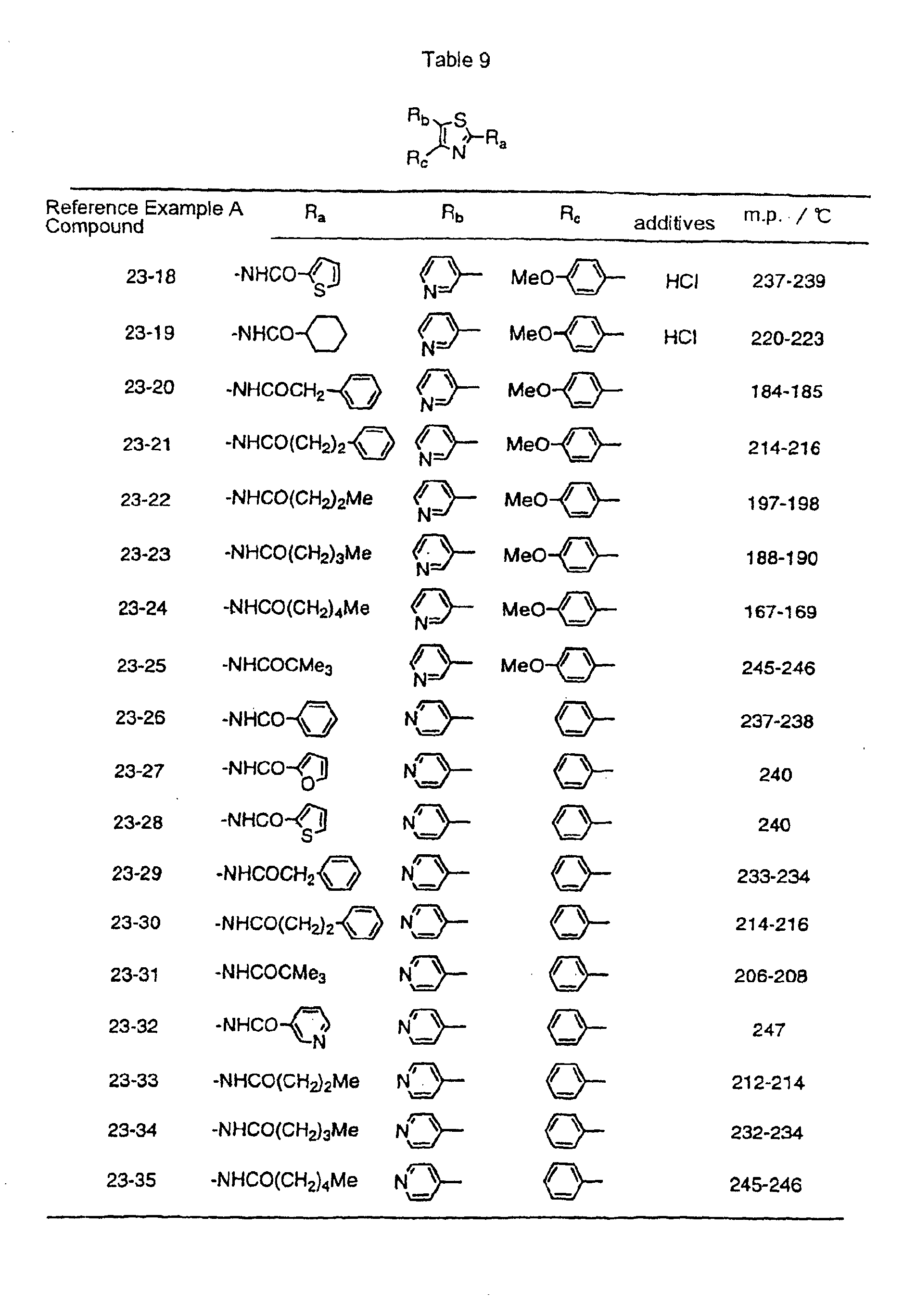

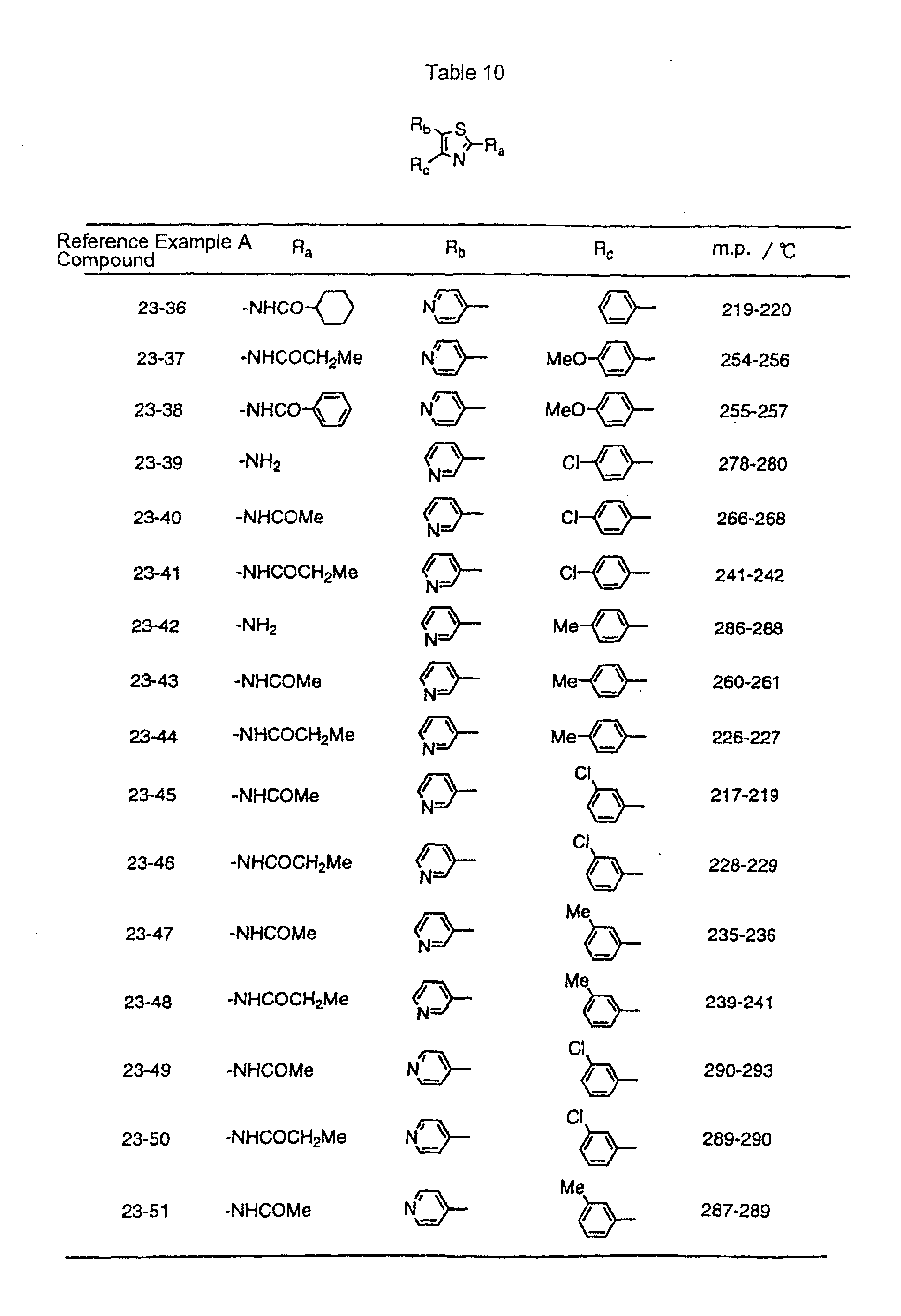

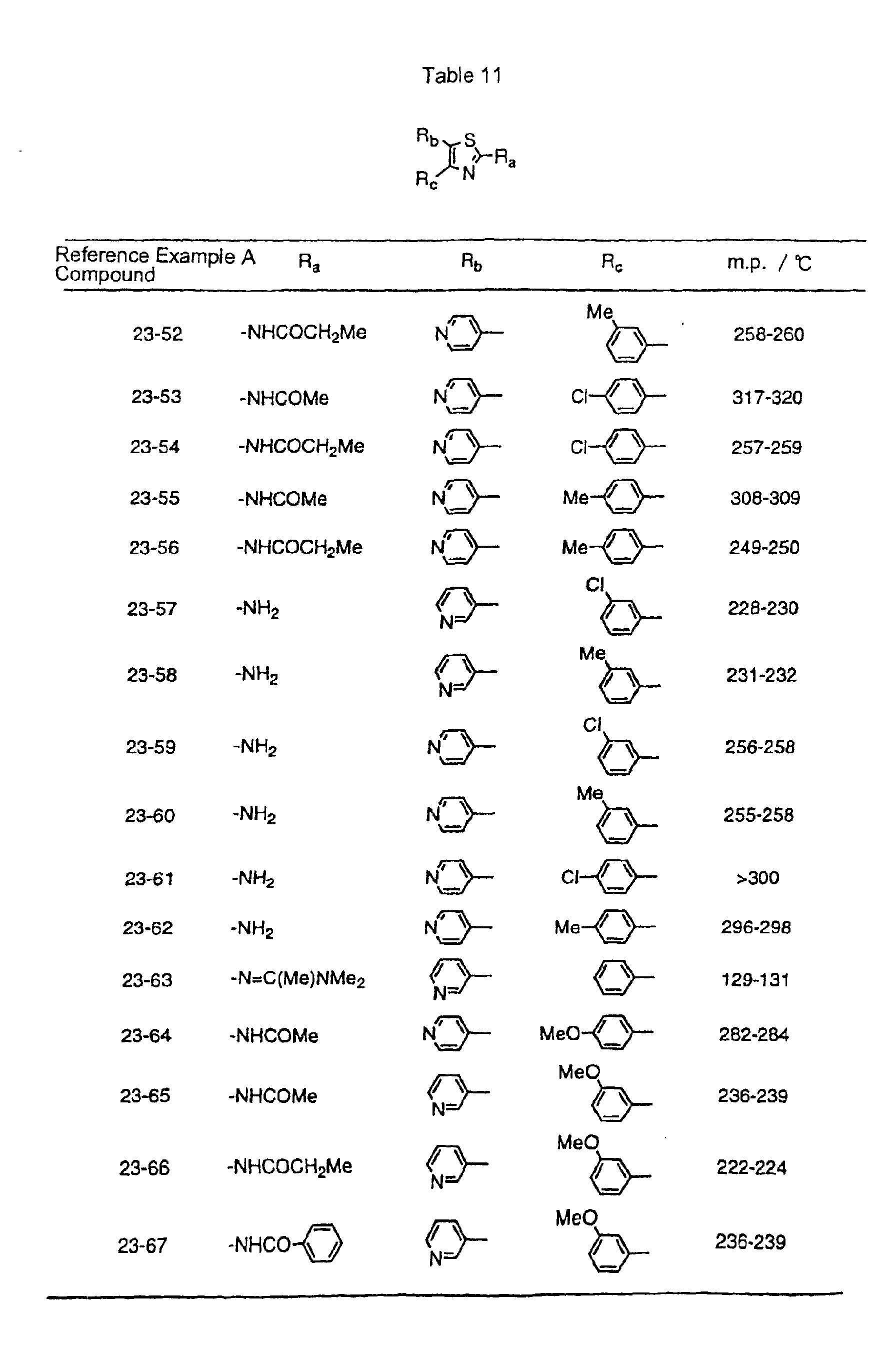

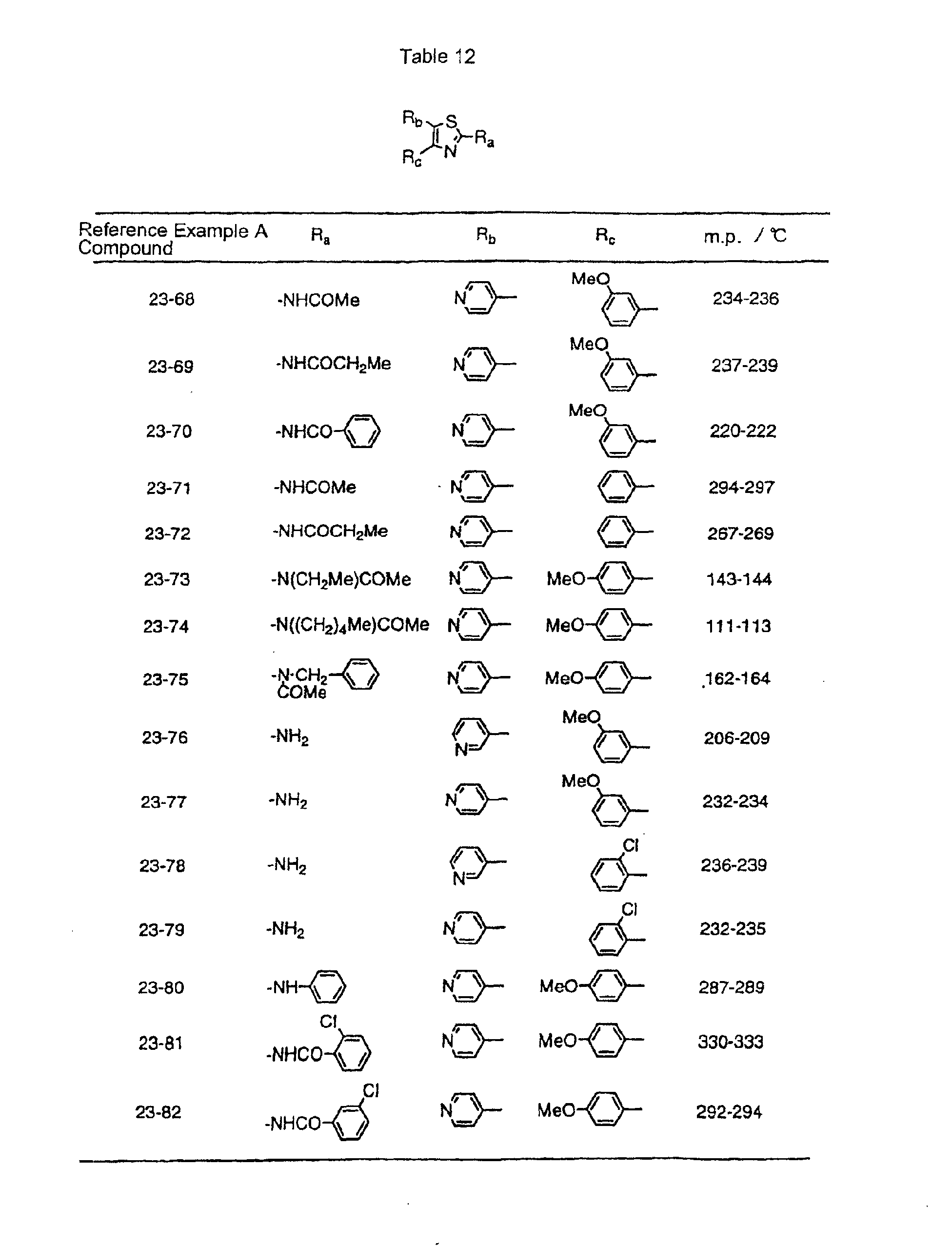

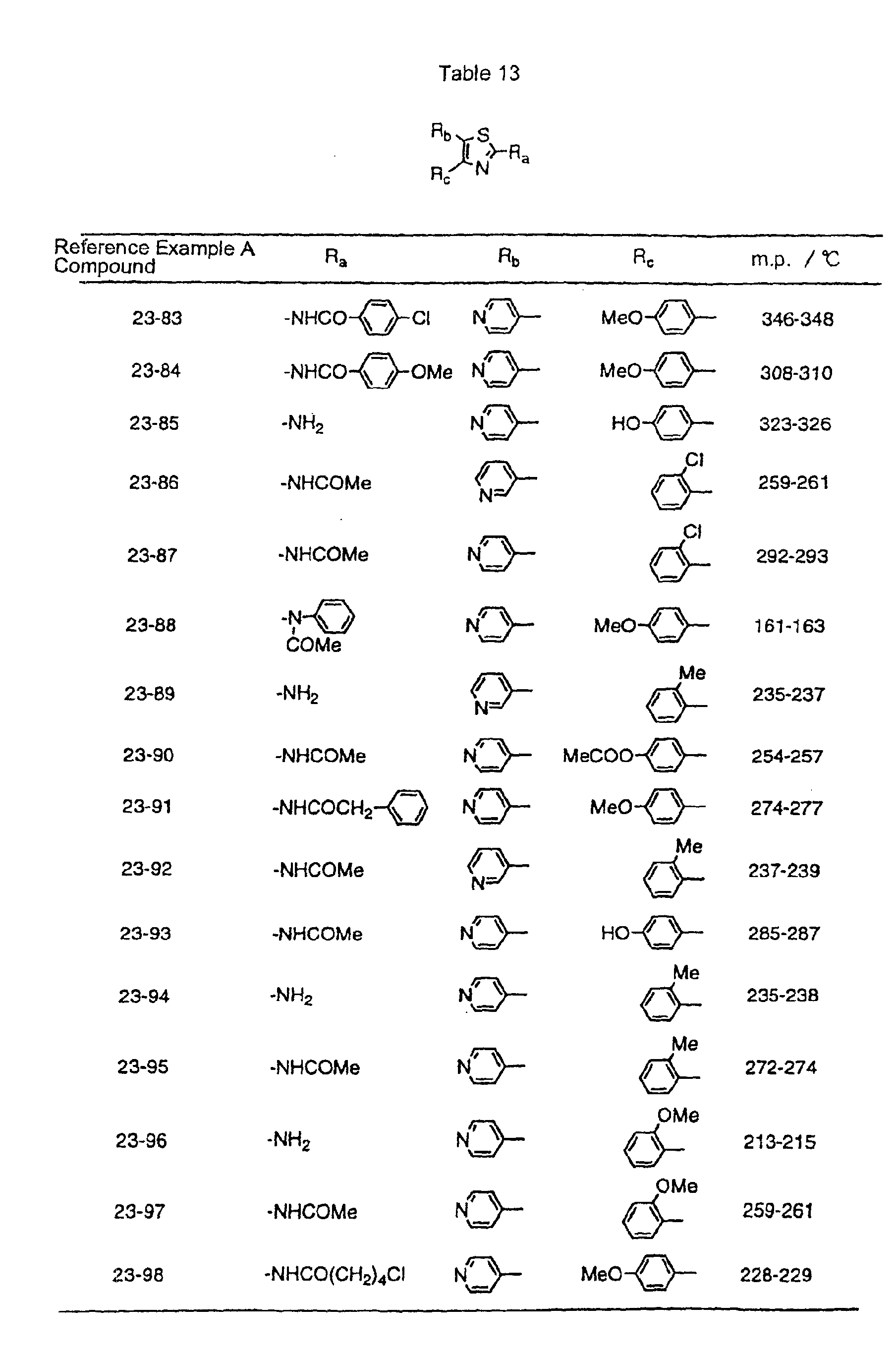

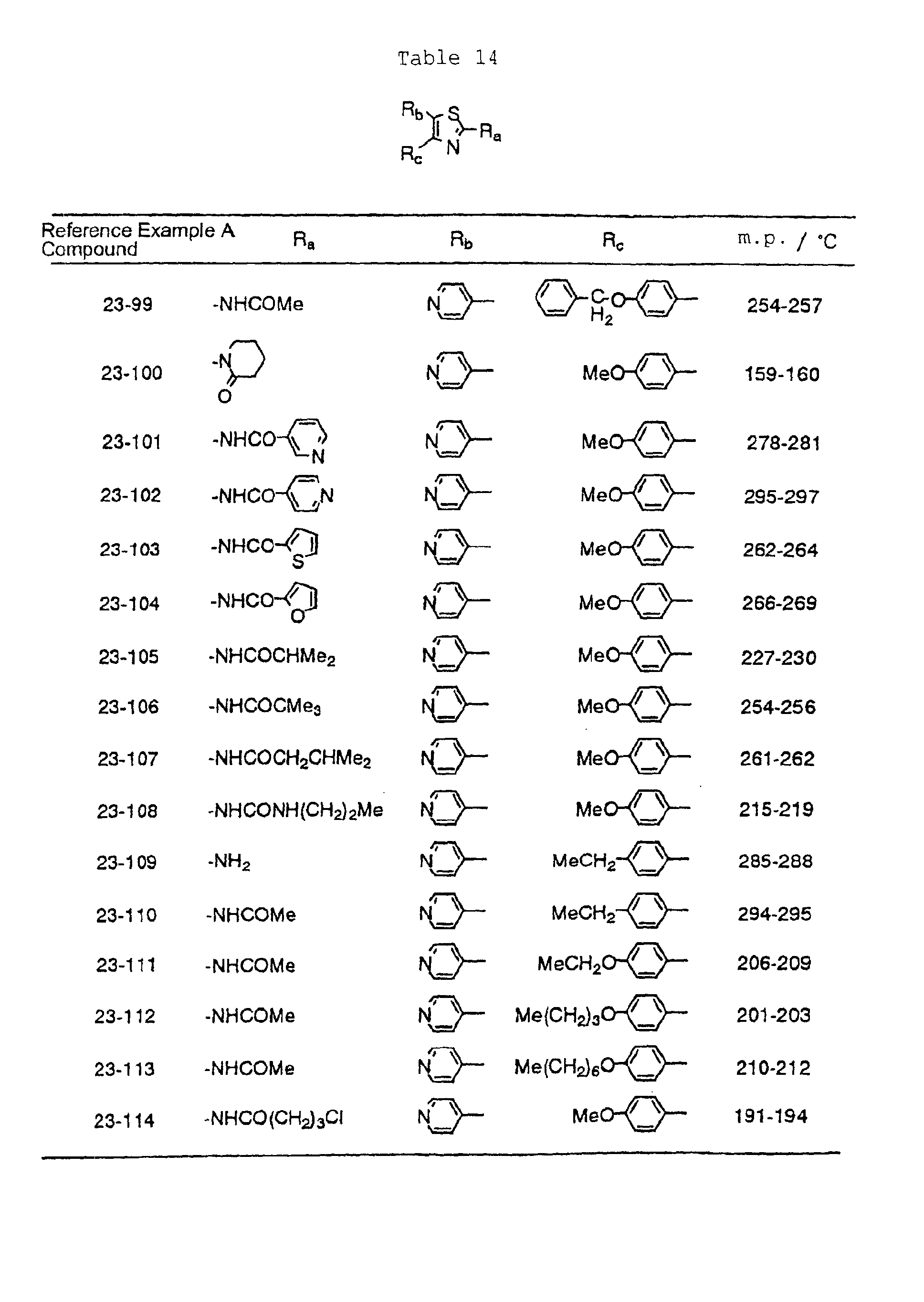

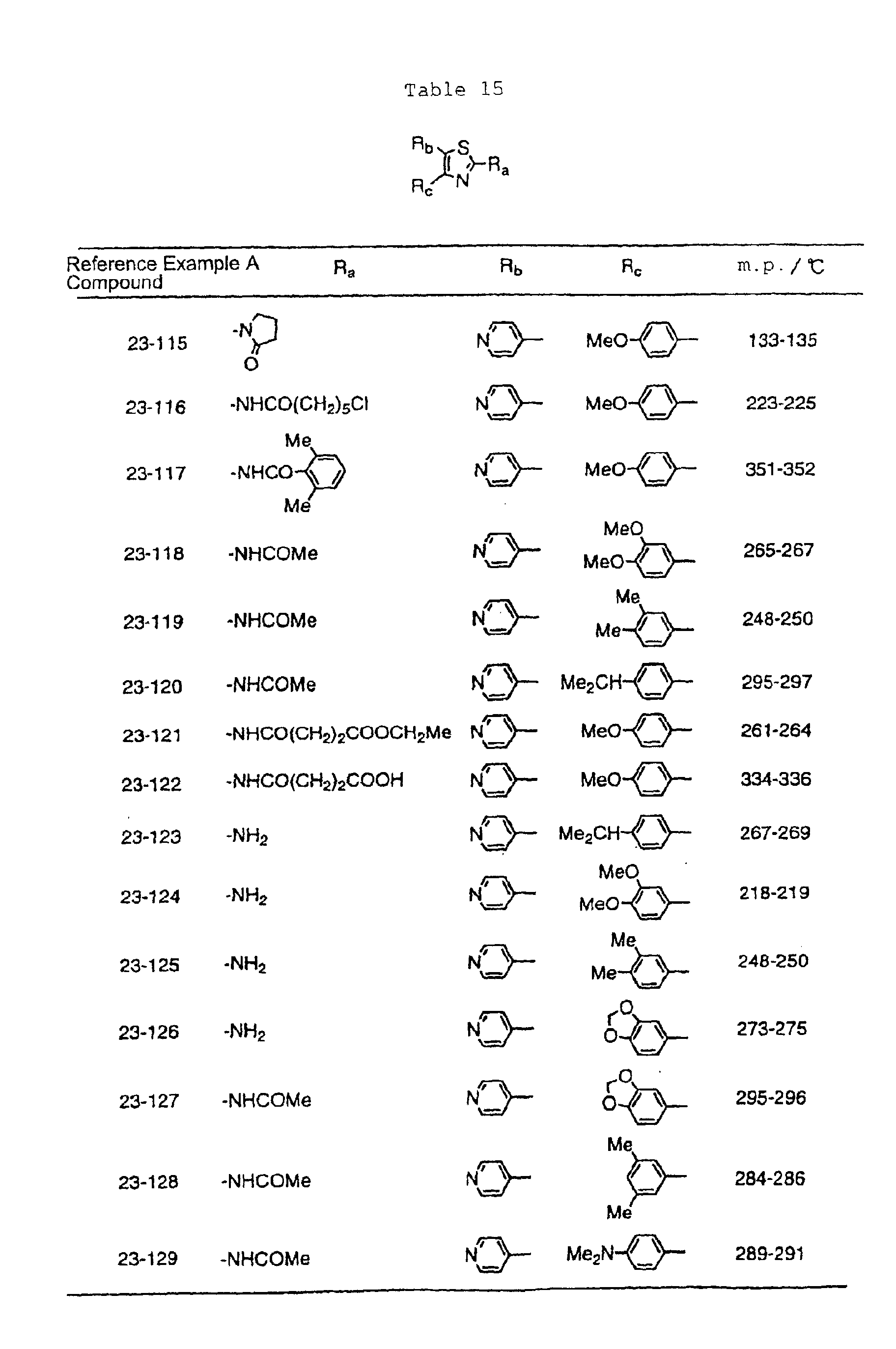

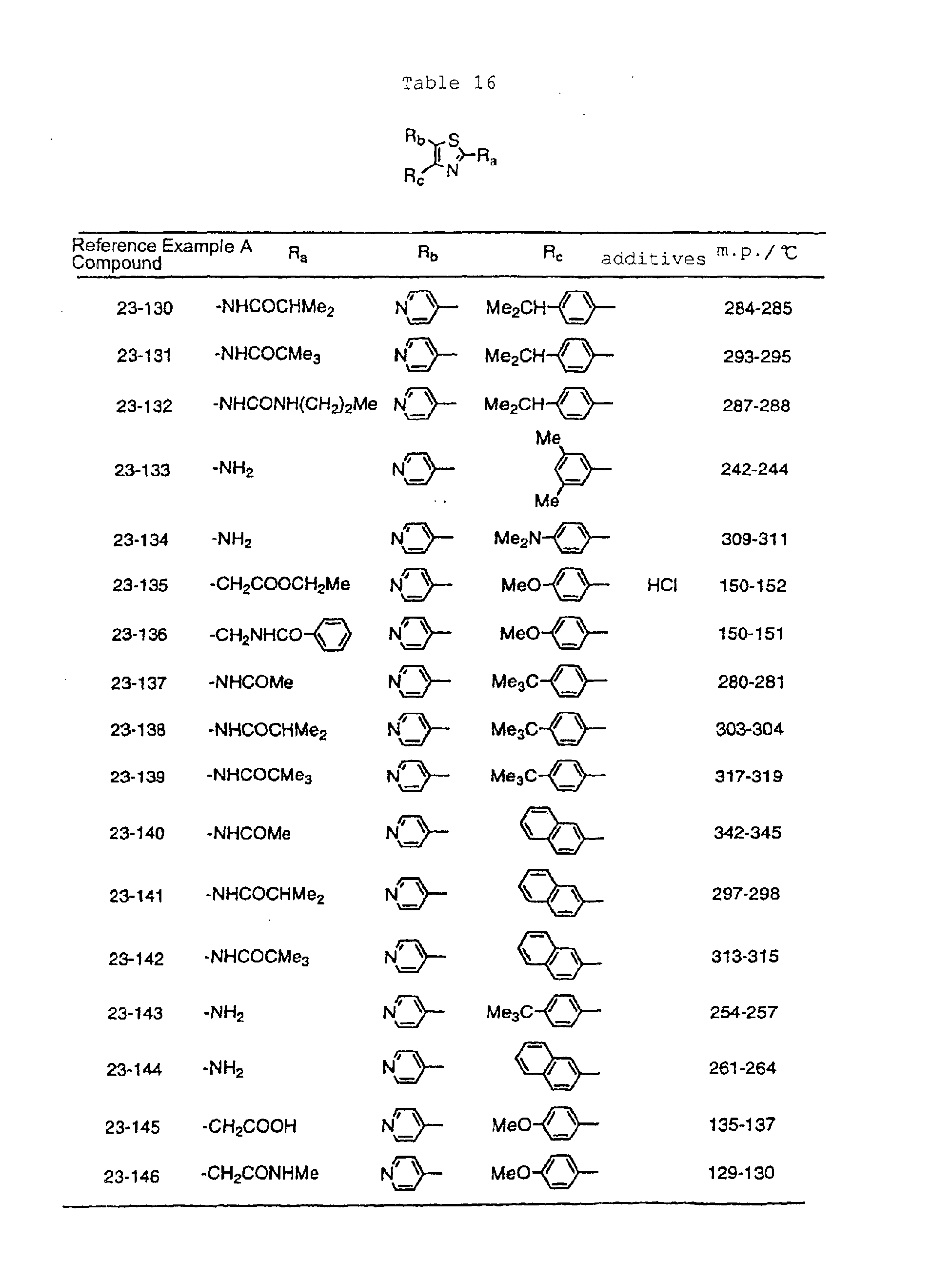

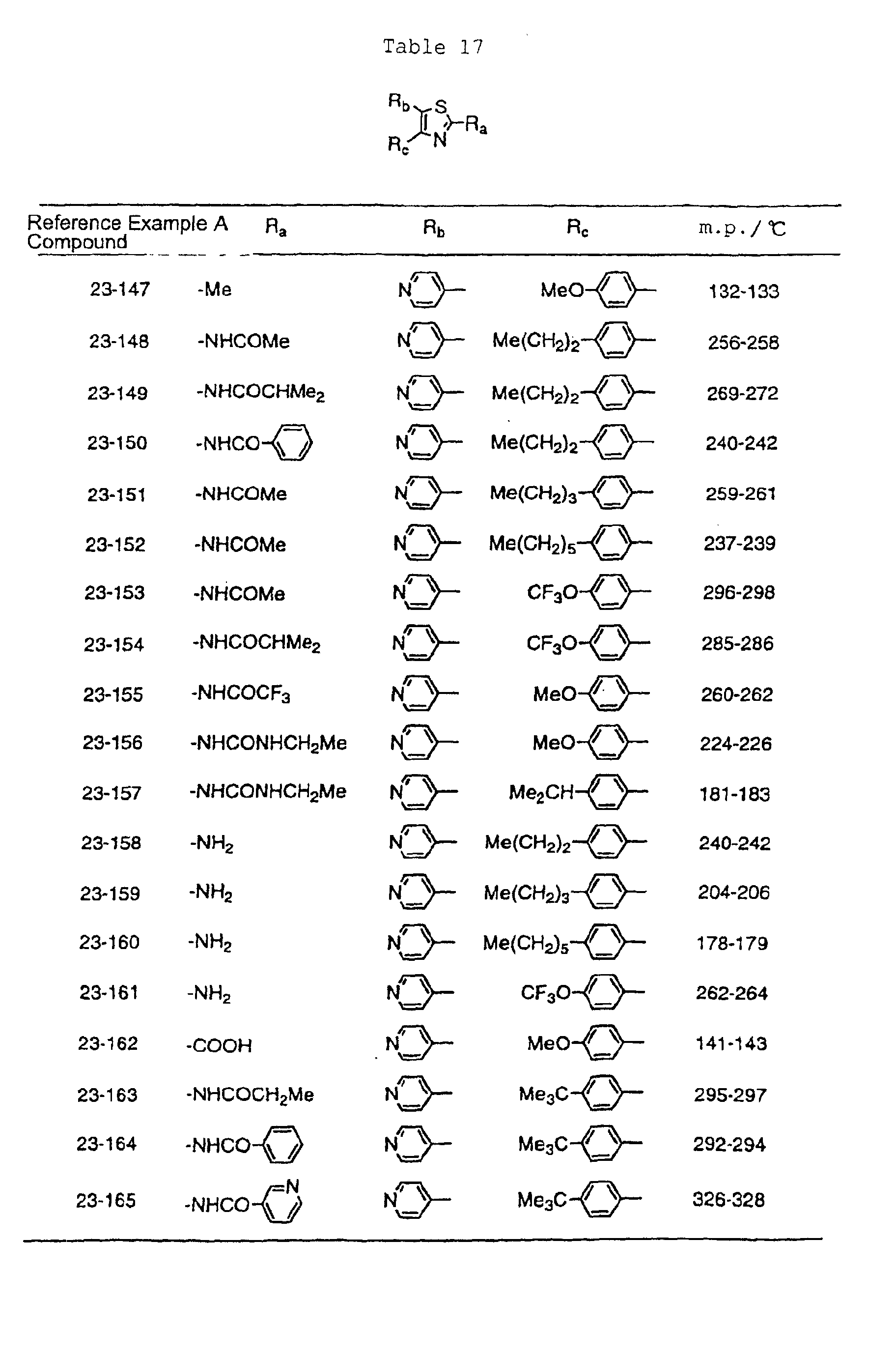

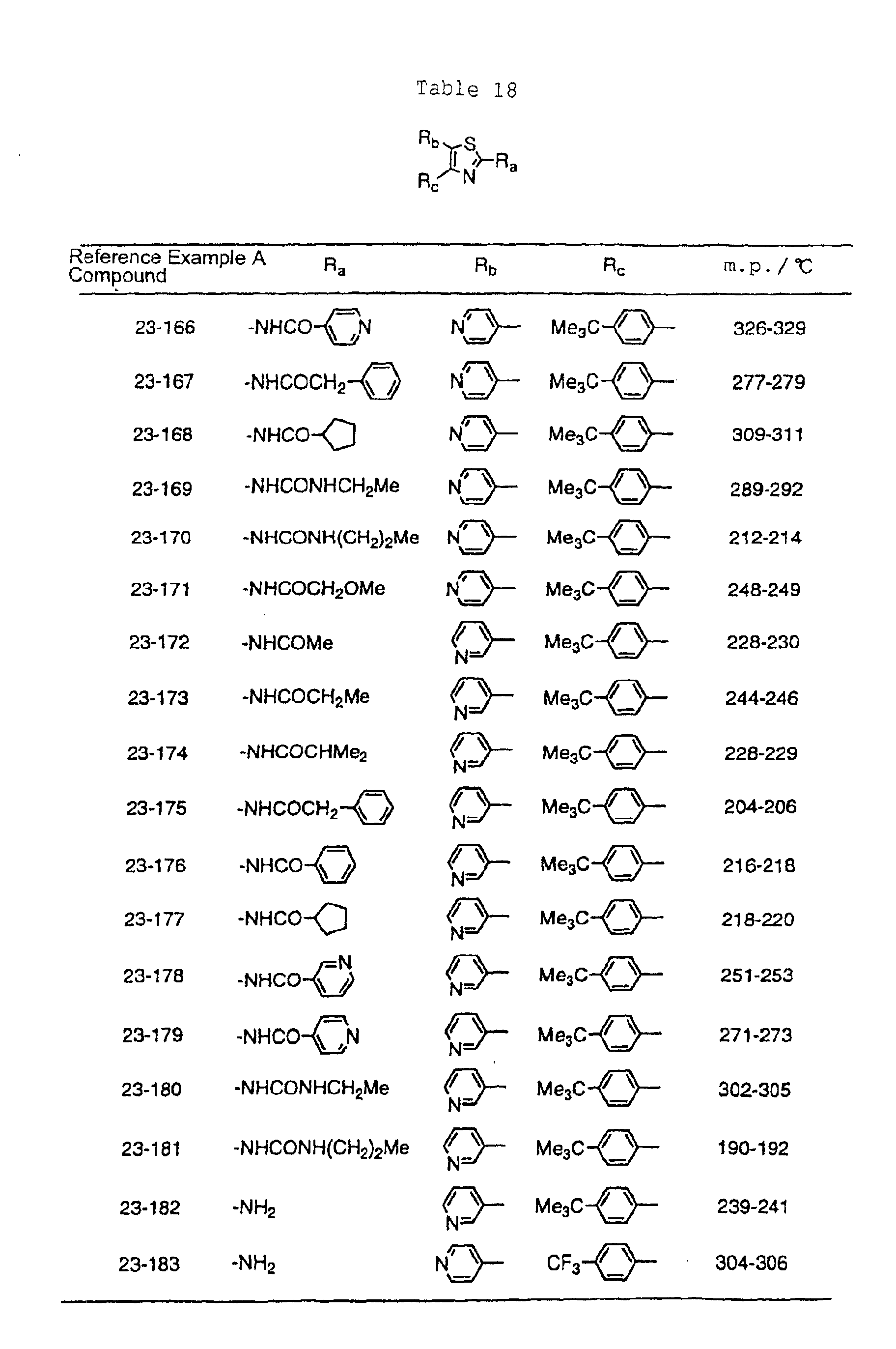

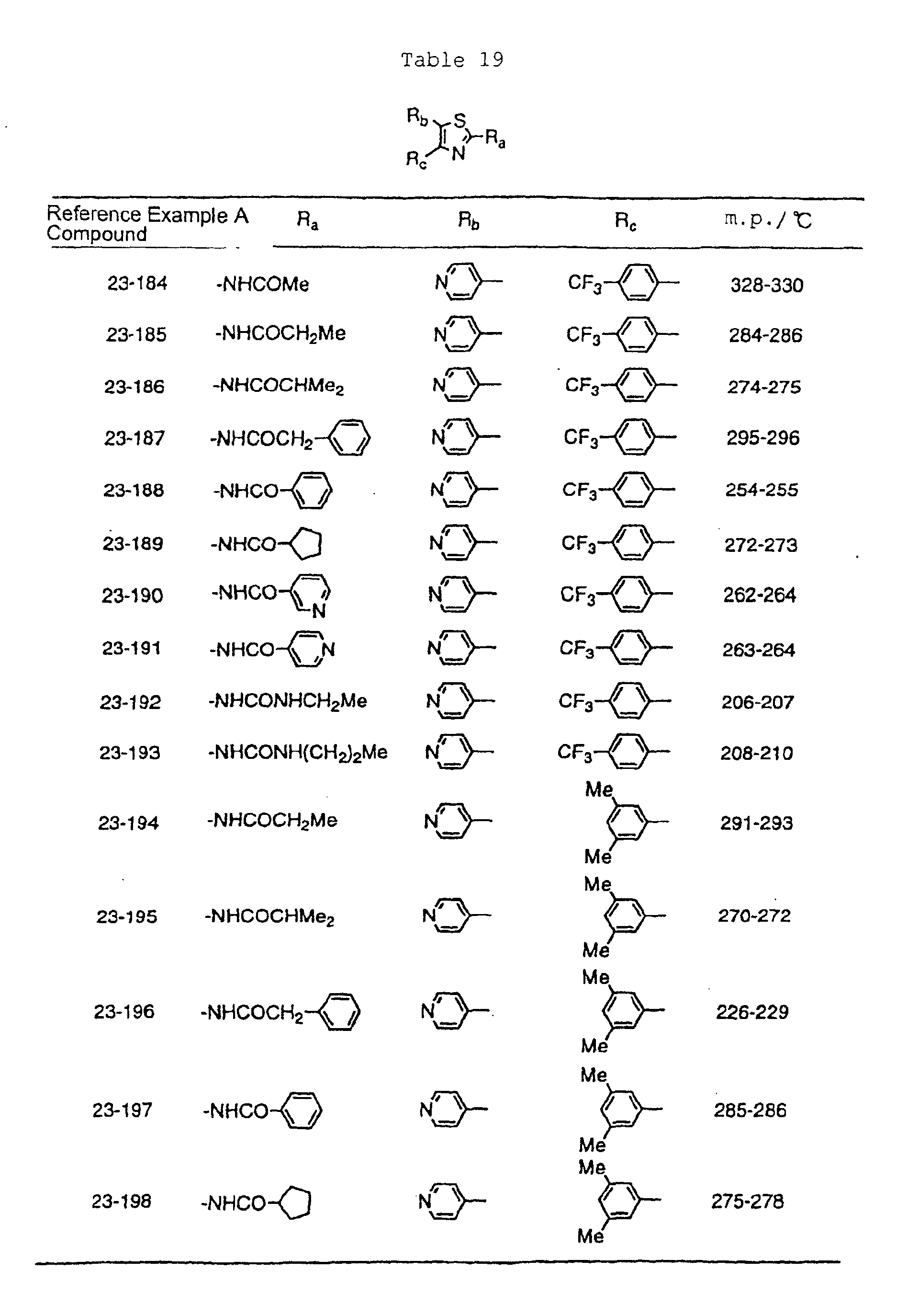

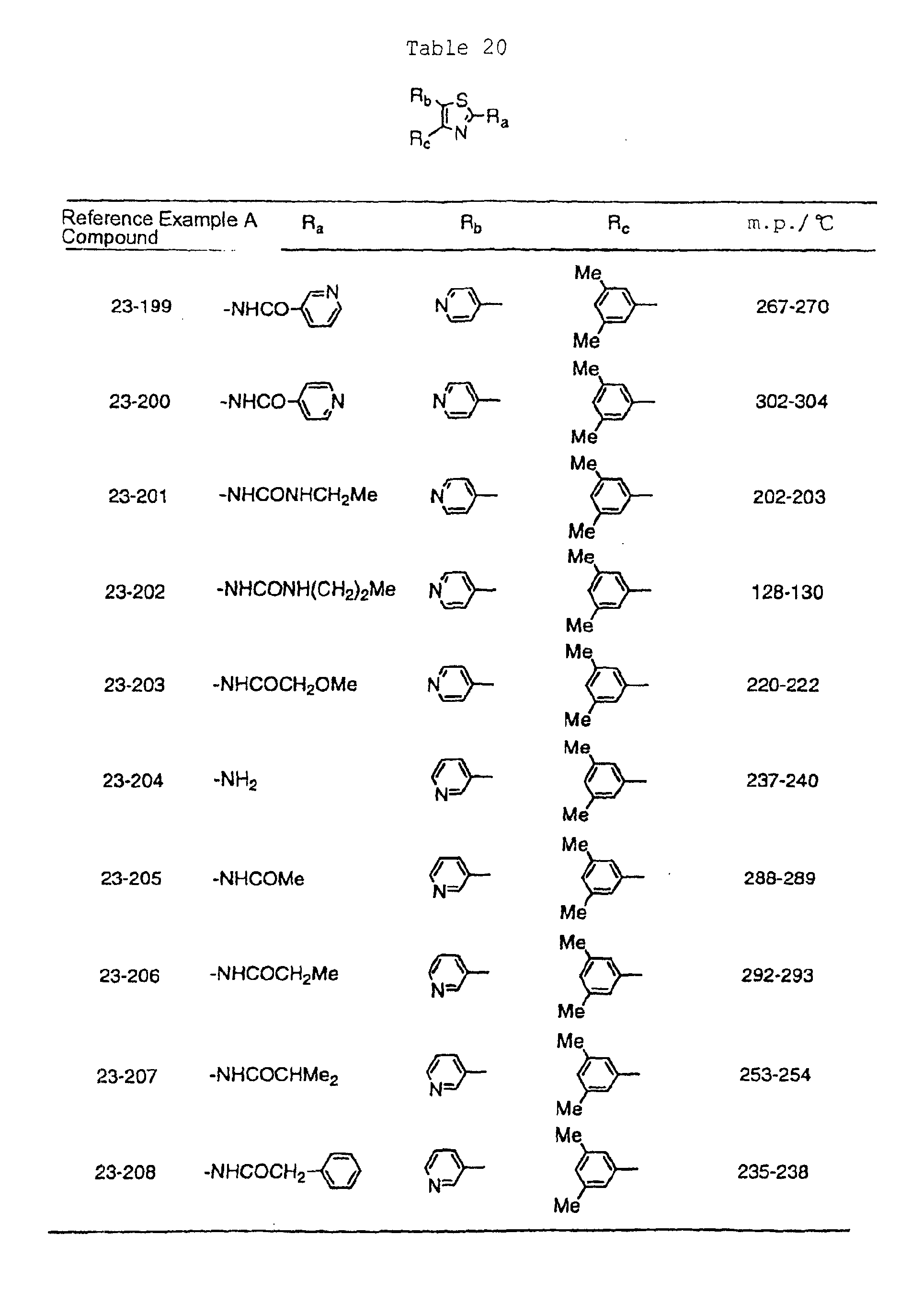

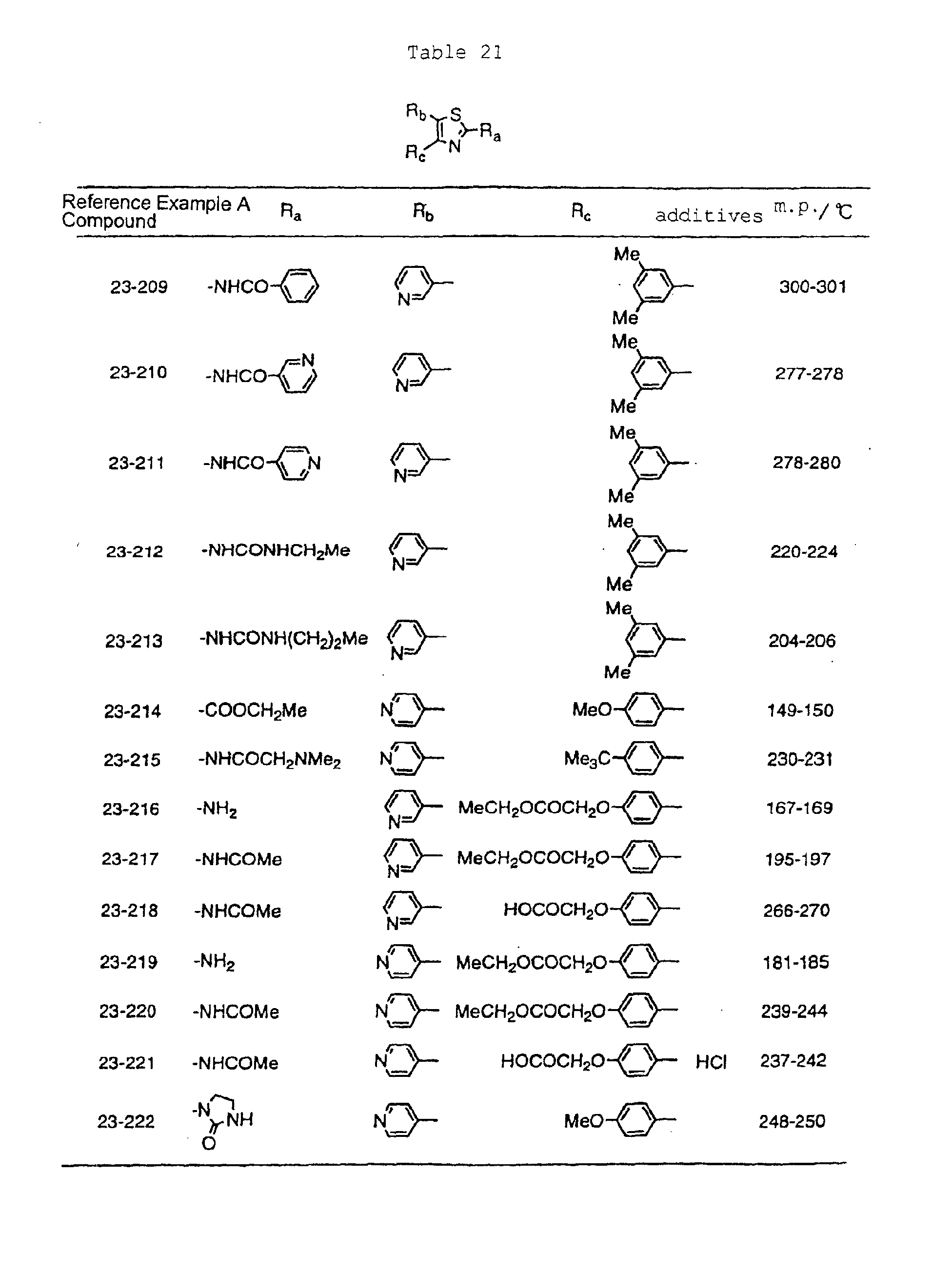

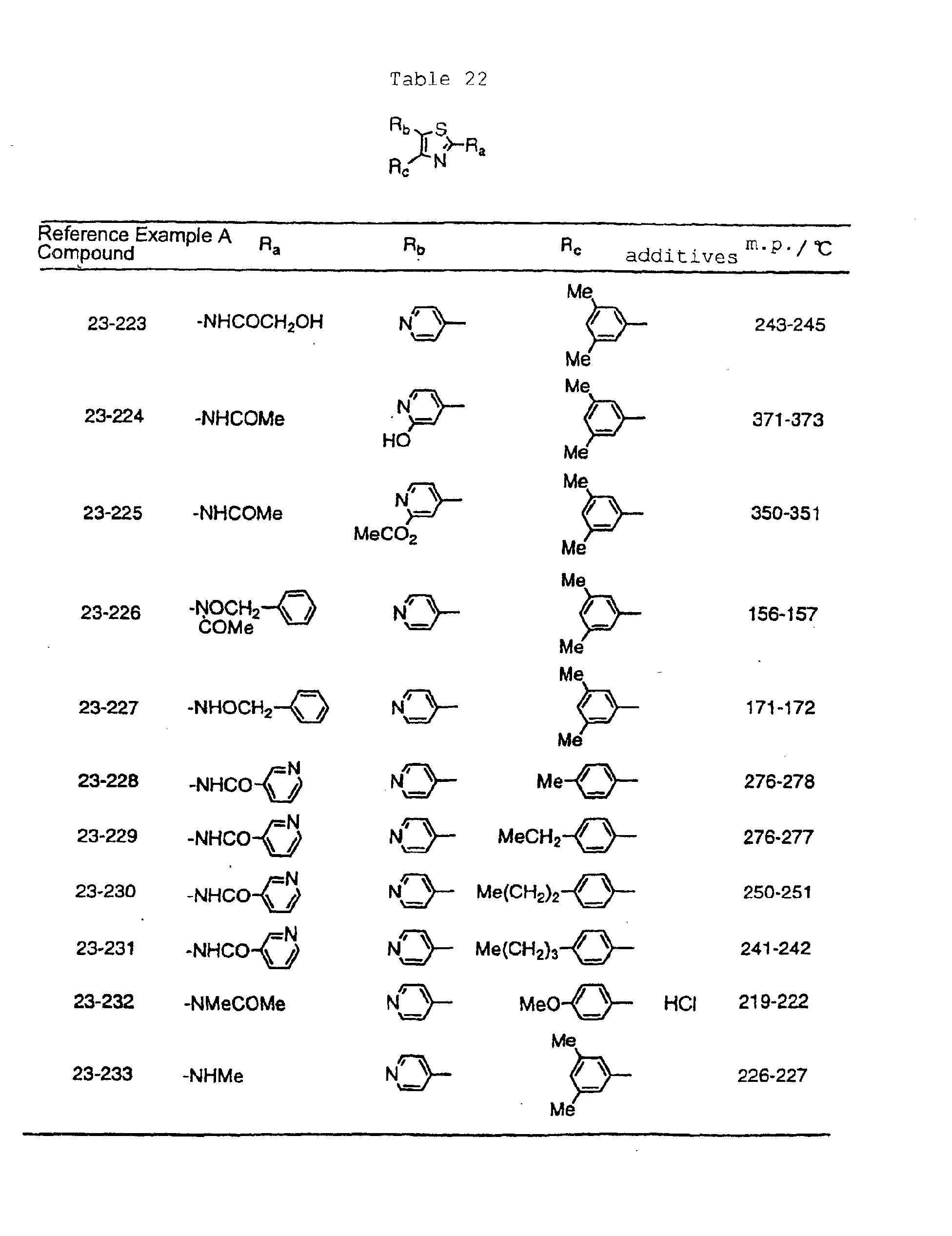

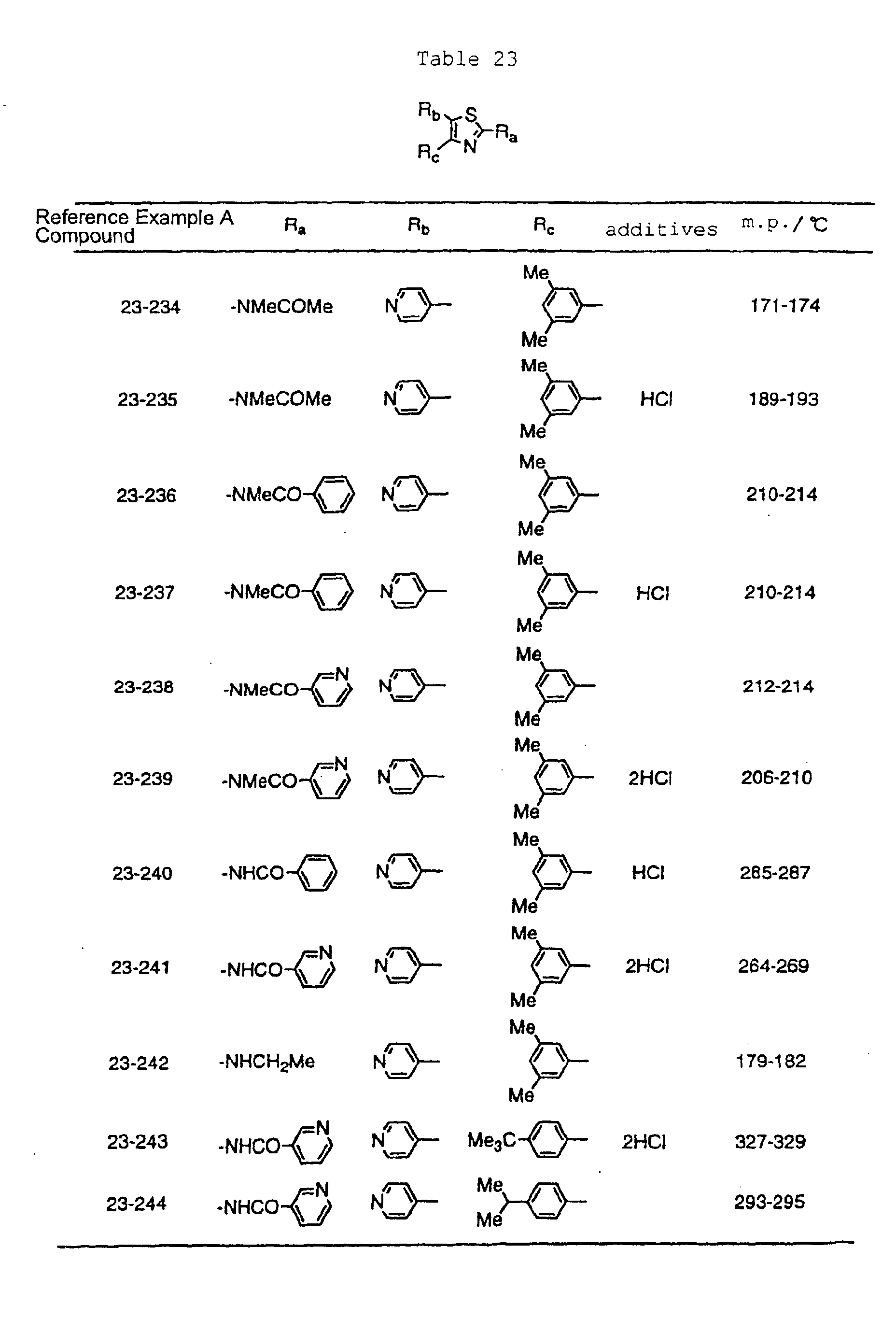

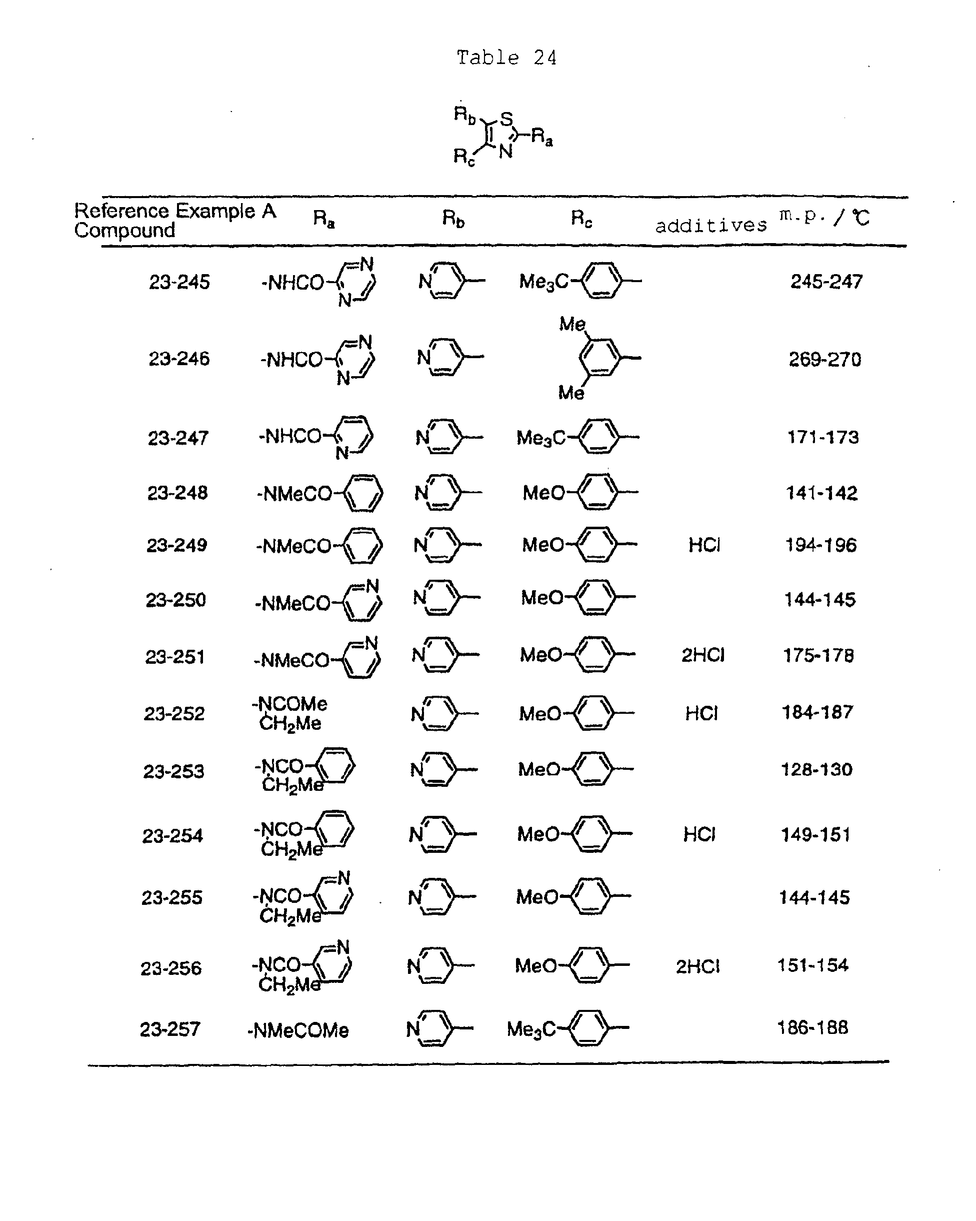

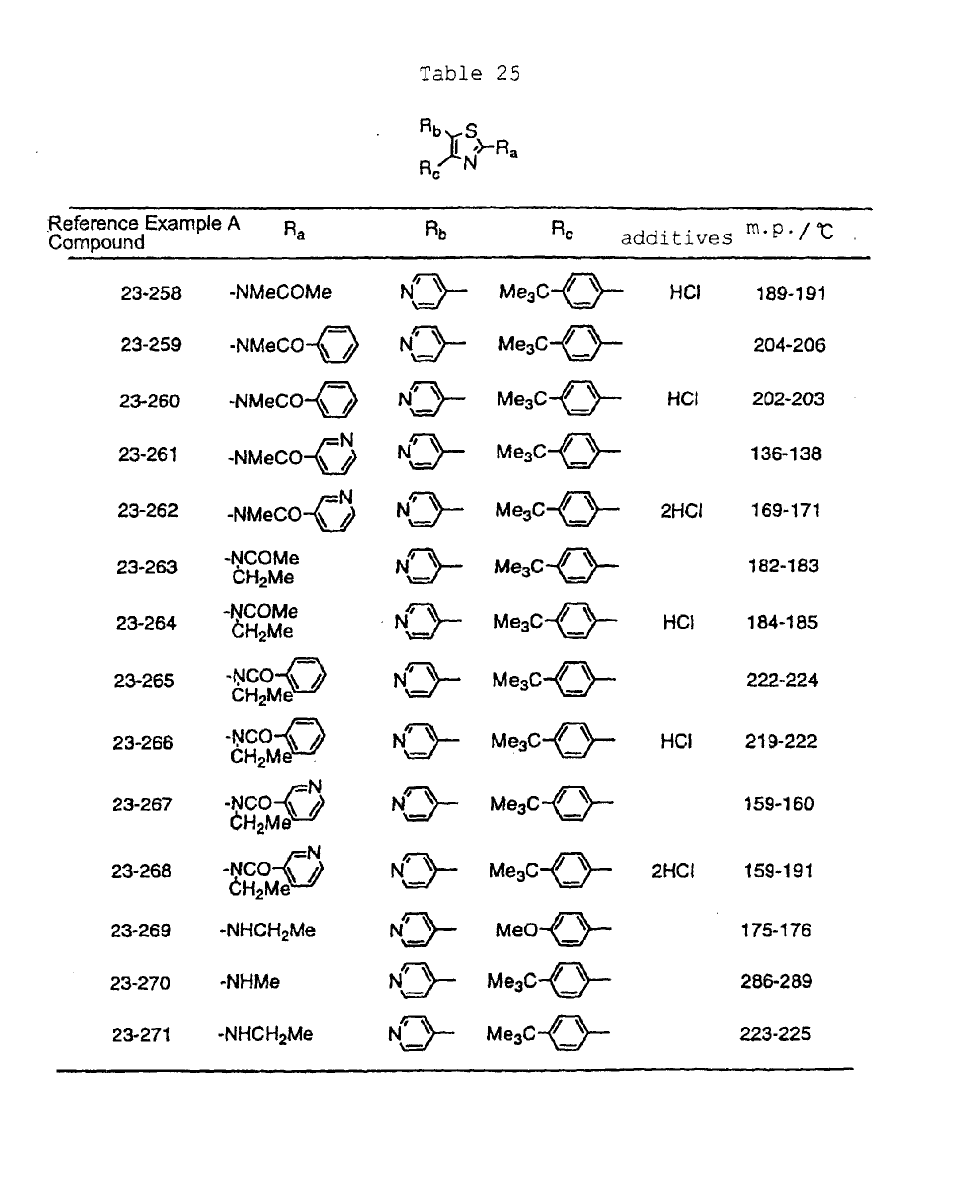

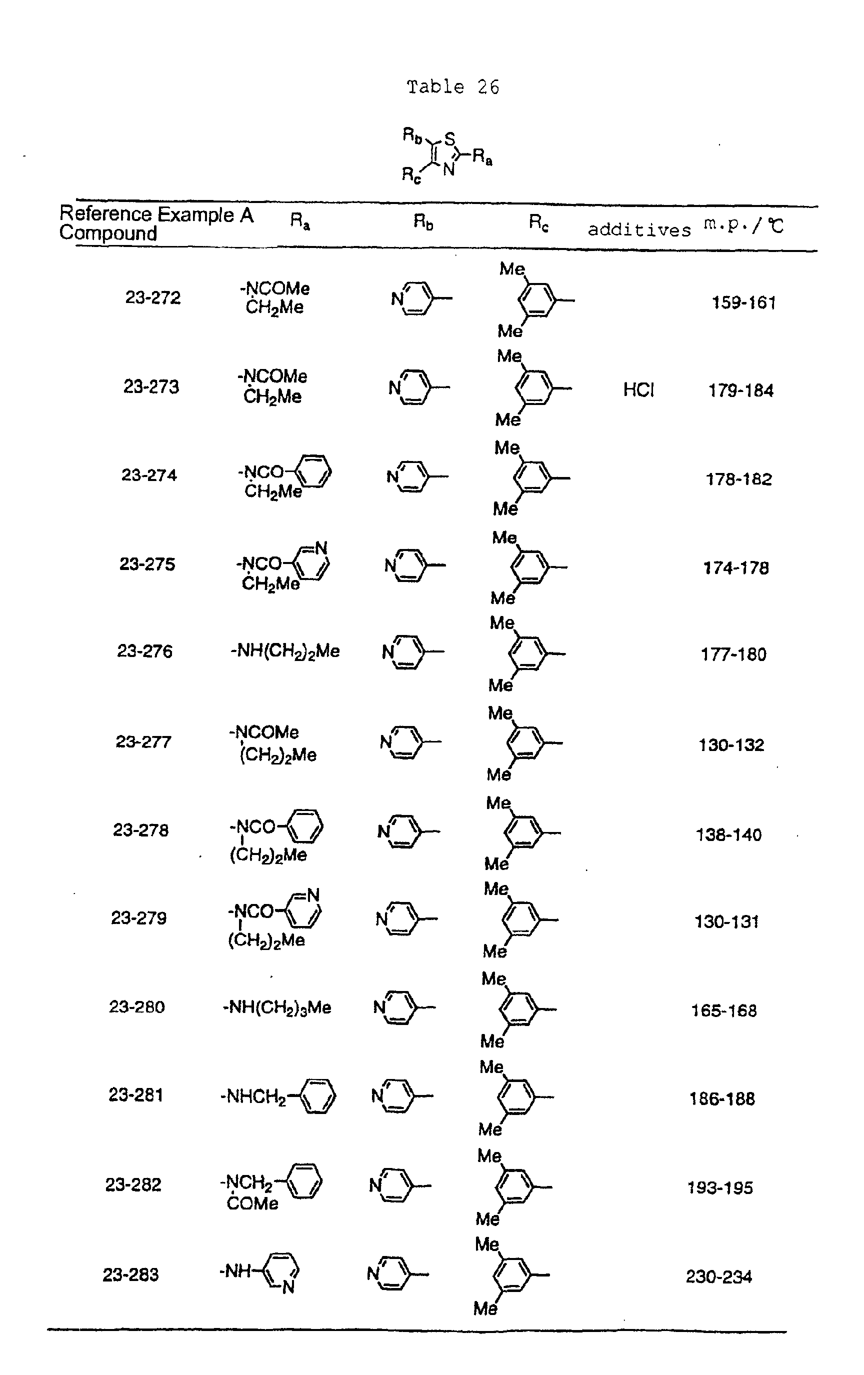

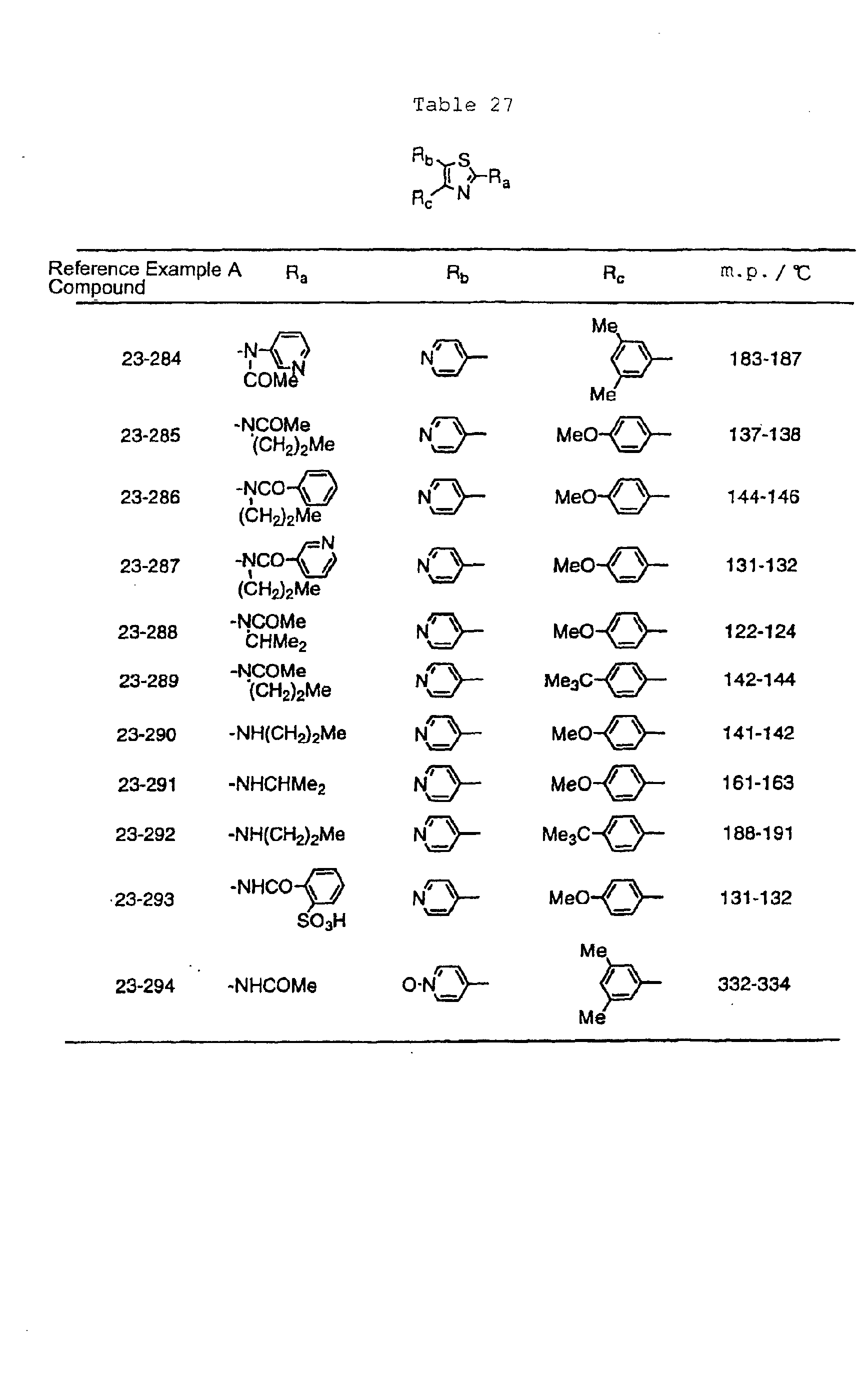

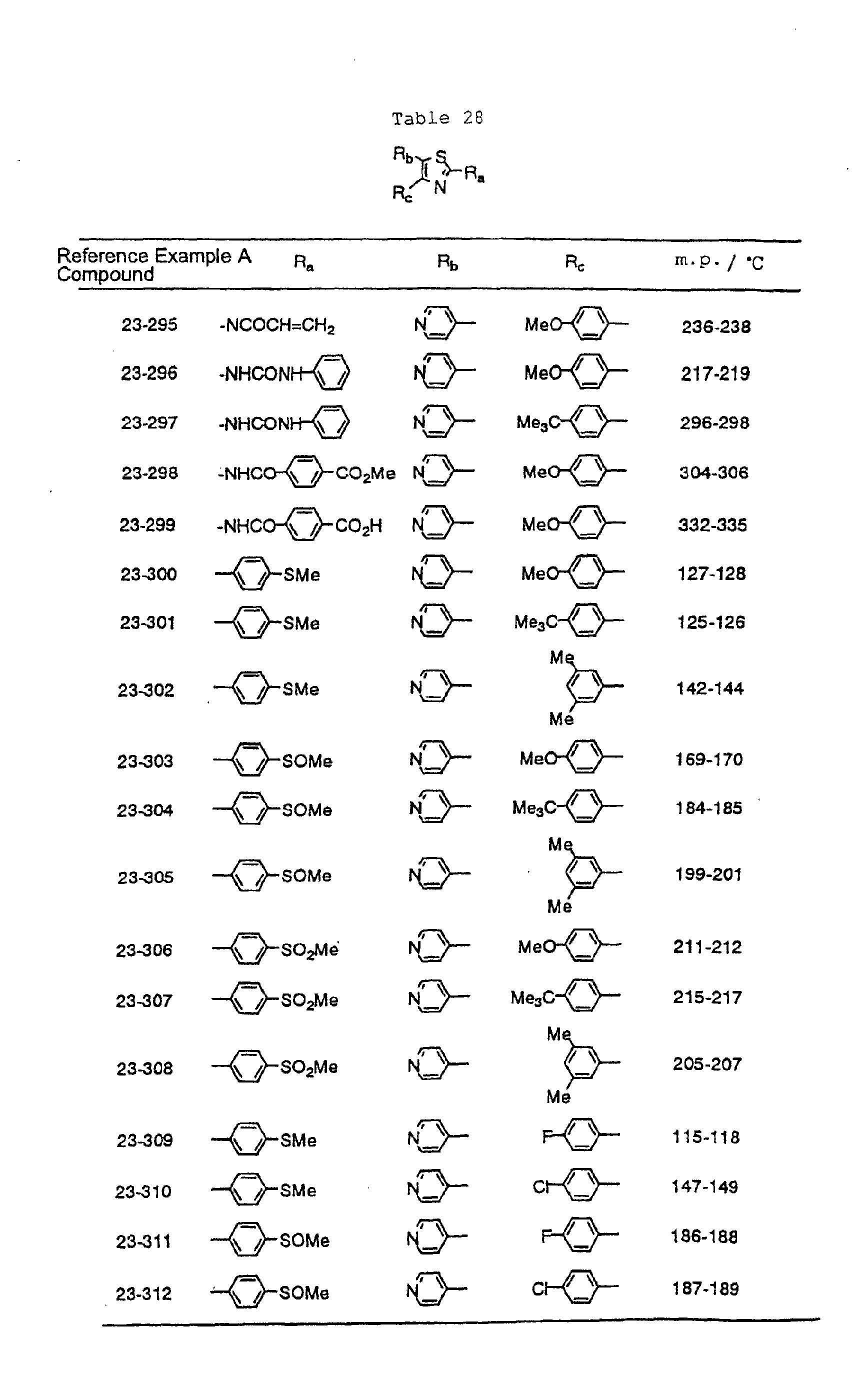

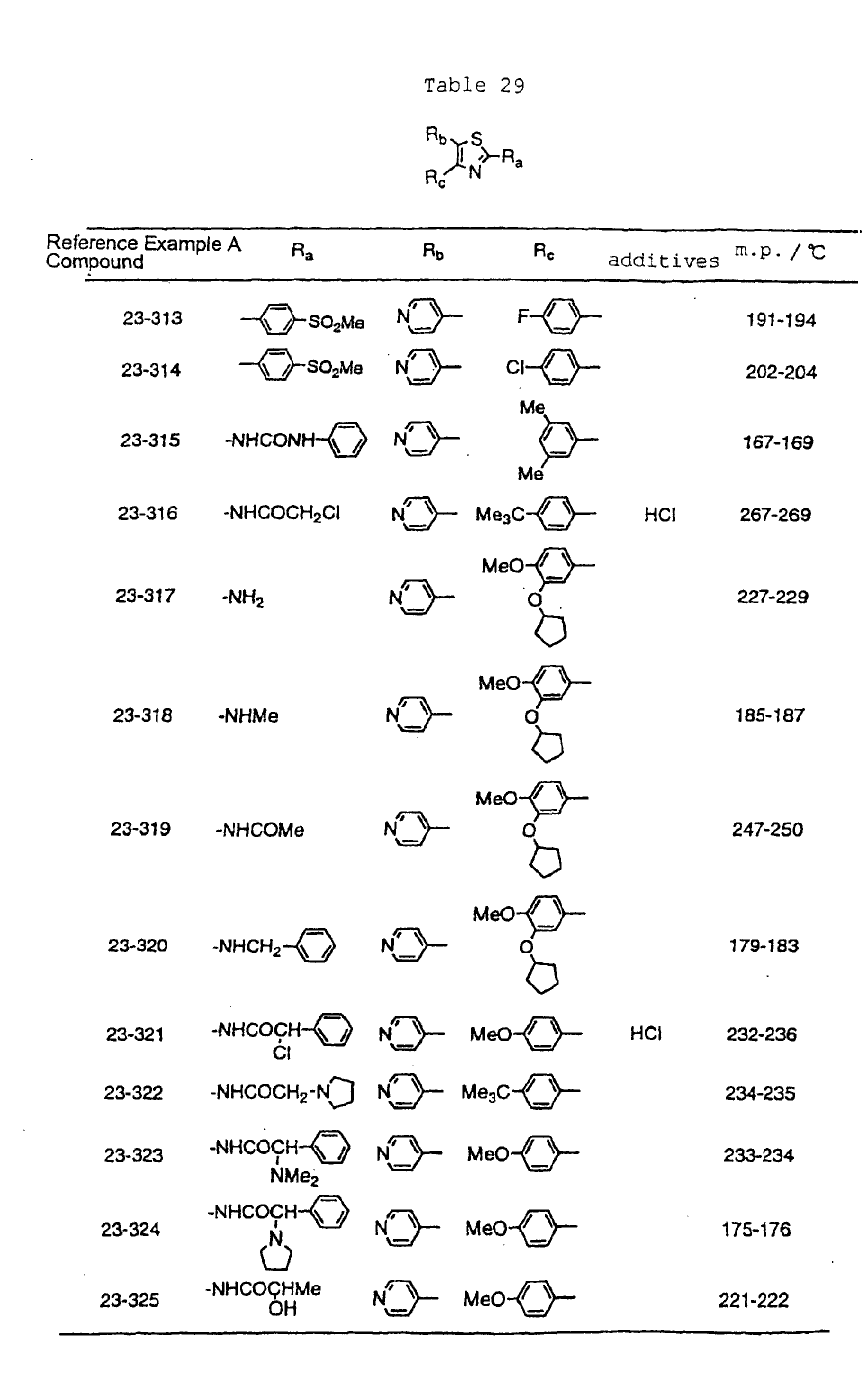

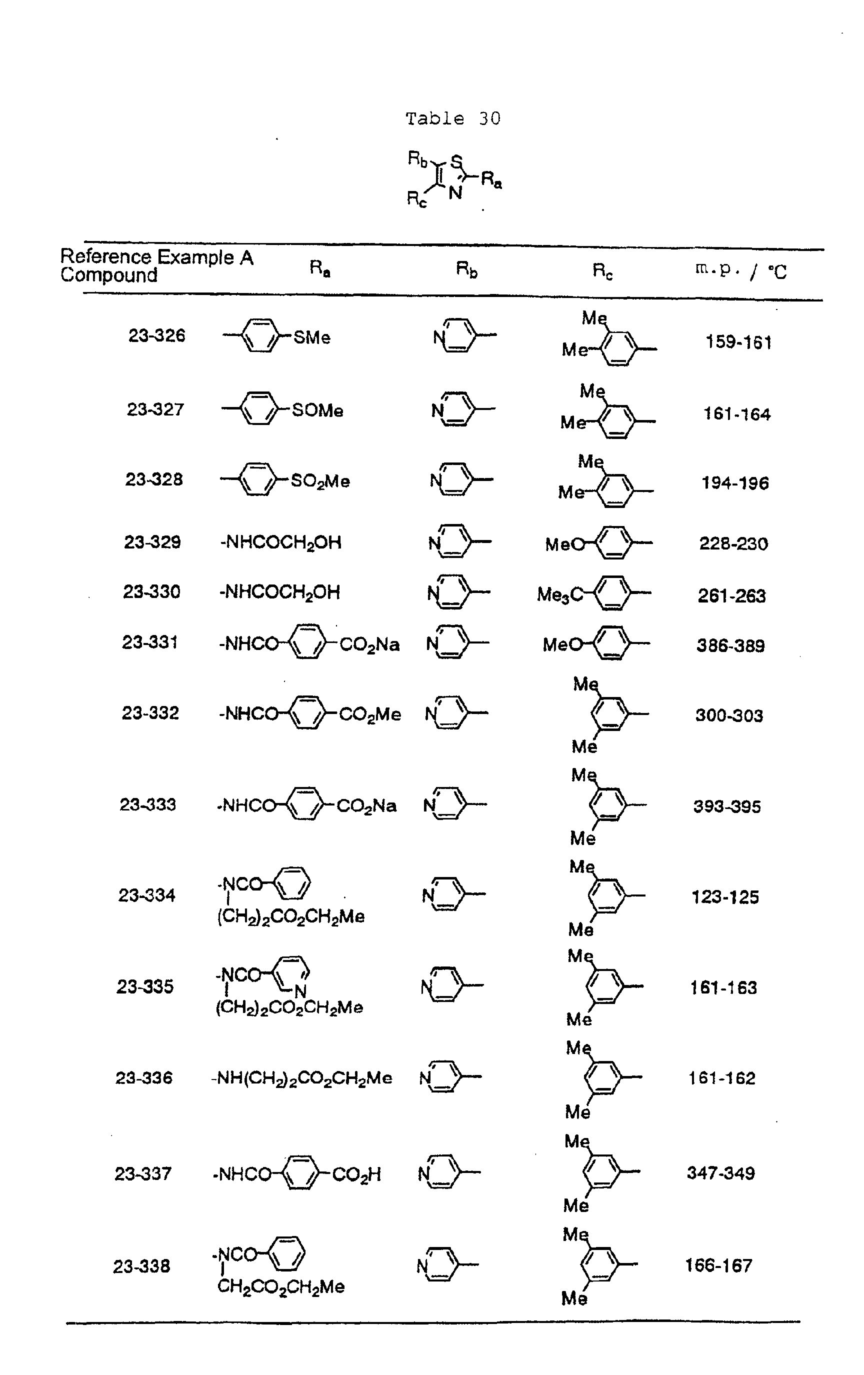

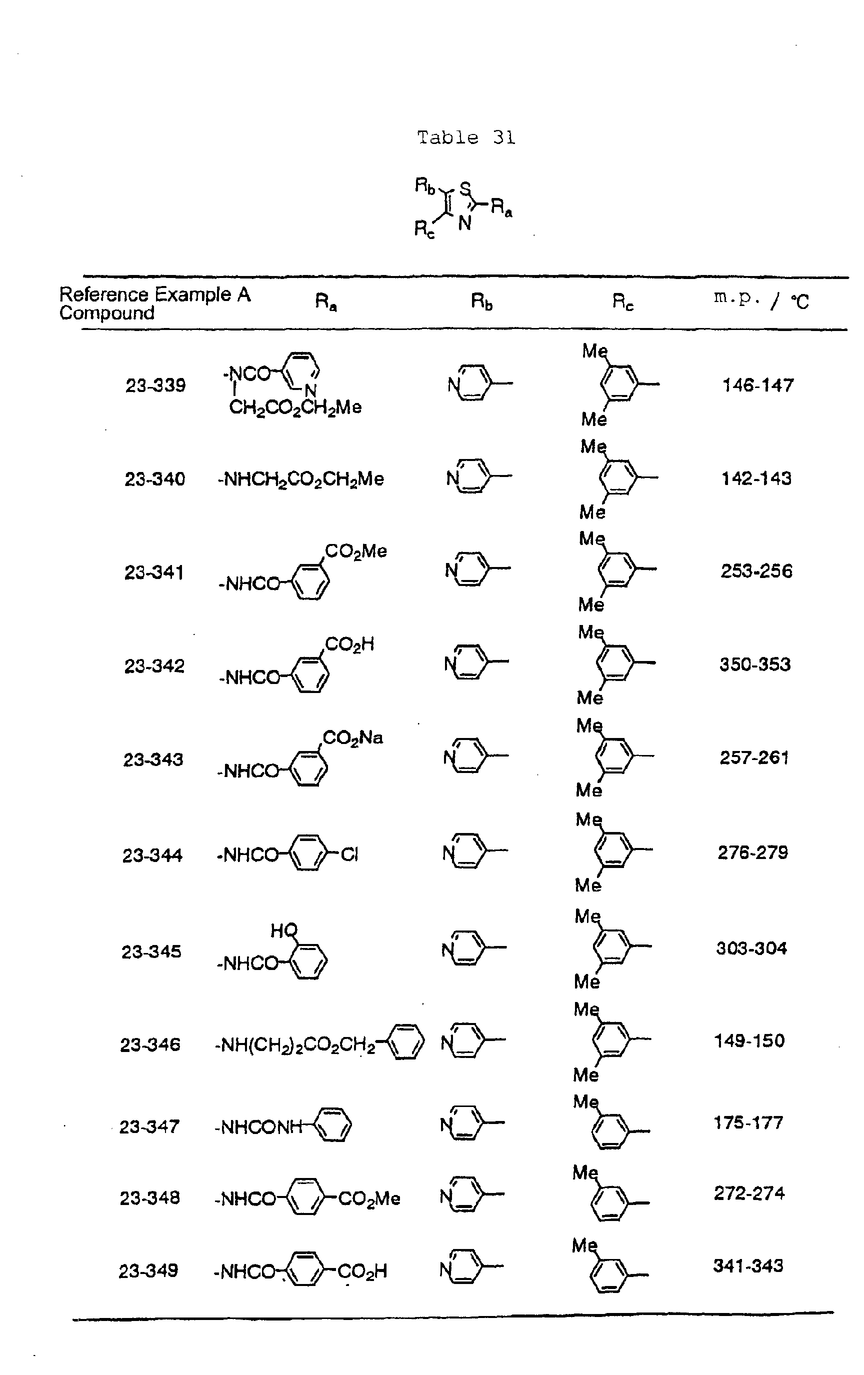

### Reference Example A 23-128

### N-[4-(3,5-dimethylphenyl)-5-(4-pyridyl)-1,3-thiazol-2-yl]acetamide

To a solution of [4-(3,5-dimethylphenyl)-5-(4-pyridyl)-1,3-thiazol-2-yl]amine (0.50 g, 1.78 mmol) and 4-dimethylaminopyridine (0.06 g, 0.51 mmol) in N,N-dimethylacetamide (5 mL) was added acetyl chloride (0.21 g, 2.67 mmol) and the mixture was stirred at 80°C for 14 h. To the reaction mixture was poured aqueous sodium hydrogencarbonate. The precipitated solid was collected by filtration. The obtained solid was washed with water and dried. The crude crystals were recrystallized from ethanol to give the title compound (0.17 g, yield 29%).
melting point: 284-286°C.

### Reference Example A 23-133

### [4-(3,5-dimethylphenyl)-5-(4-pyridyl)-1,3-thiazol-2-yl]amine

To a solution of 2-bromo-1-(3,5-dimethylphenyl)-2-(4-pyridyl)ethanone hydrobromide (5.0 g, 13 mmol) and thiourea (1.0 g, 14 mmol) in acetonitrile (60 mL) was added dropwise triethylamine (1.9 ml, 14 mmol) and the mixture was stirred at room temperature for 3 h. The solvent was concentrated under reduced pressure and a saturated aqueous sodium hydrogencarbonate solution was added to the residue. The mixture was extracted with ethyl acetate. The organic layer was washed with water and the solvent was evaporated. The obtained crude crystals were recrystallized from ethyl acetate to give the title compound (2.0 g, 7.2 mmol, yield 55%).
melting point: 242-244°C.

### Reference Example A 23-137

### N-[4-[4-(1,1-dimethylethyl)phenyl]-5-(4-pyridyl)-1,3-thiazol-2-yl]acetamide

To a solution of [4-[4-(1,1-dimethylethyl)phenyl]-5-(4-pyridyl)-1,3-thiazol-2-yl]amine (0.40 g, 1.29 mmol) and 4-dimethylaminopyridine (0.05 g, 0.39 mmol) in N,N-dimethylacetamide (4 mL) was added acetyl chloride (0.15 g, 1.94 mmol) and the mixture was stirred at 80°C for 14 h. To the reaction mixture was poured aqueous sodium hydrogencarbonate and the precipitated solid was collected by filtration. The obtained solid was washed with water and dried. Crude crystals were recrystallized from ethanol to give the title compound (0.23 g, yield 50%).
melting point: 280-281°C.

### Reference Example A 23-143

### [4-[4-(1,1-dimethylethyl)phenyl]-5-(4-pyridyl)-1,3-thiazol-2-yl]amine

To a solution of 2-bromo-1-[4-(1,1-dimethylethyl)-phenyl]-2-(4-pyridyl)ethanone hydrobromide (5.0 g, 12 mmol) and thiourea (0.95 g, 13 mmol) in acetonitrile (60 mL) was added dropwise triethylamine (1.8 ml, 13 mmol) and the mixture was refluxed for 3 h. The solvent was evaporated under reduced pressure and saturated aqueous sodium hydrogencarbonate solution was added to the residue. The precipitated solid was collected by filtration. The obtained crude crystal was recrystallized from ethanol to give the title compound (2.6 g, 8.4 mmol, yield 69%).
melting point: 254-257°C.

### Reference Example A 23-164

### N-[4-[4-(1,1-dimethylethyl)phenyl]-5-(4-pyridyl)-1,3-thiazol-2-yl]benzamide

To a solution of [4-[4-(1,1-Dimethylethyl)phenyl]-5-(4-pyridyl)-1,3-thiazol-2-yl]amine (0.50 g, 1.62 mmol) and 4-dimethylaminopyridine (0.05 g, 0.39 mmol) in N,N-dimethylacetamide (5 mL) was added benzoyl chloride (0.15 g, 1.94 mmol), and the mixture was stirred at 80°C for 14 h. To the reaction mixture was poured an aqueous sodium hydrogencarbonate and the precipitated solid was collected by filtration. The obtained solid was washed with water and dried. The crude crystals were recrystallized from ethanol to -give the title compound (0.44 g, yield 66%).
melting point: 292-294°C.

### Reference Example A 23-165

### N-[4-[4-(1,1-dimethylethyl)phenyl]-5-(4-pyridyl)-1,3-thiazol-2-yl]nicotinamide

To a solution of [4-[4-(1,1-dimethylethyl)phenyl]-5-(4-pyridyl)-1,3-thiazol-2-yl]amine (0.50 g, 1.62 mmol) and 4-dimethylaminopyridine (0.06 g, 0.49 mmol) in N,N-dimethylacetamide (5 mL) was added nicotinoyl chloride hydrochloride (0.43 g, 2.42 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.49 g, yield 73%).
melting point: 326-328°C.

### Reference Example A 23-168

### N-[4-[4-(1,1-dimethylethyl)phenyl]-5-(4-pyridyl)-1,3-thiazol-2-yl]cyclopentanecarboxamide

To a solution of [4-[4-(1,1-dimethylethyl)phenyl]-5-(4-pyridyl)-1,3-thiazol-2-yl]amine (0.50 g, 1.62 mmol) and 4-dimethylaminopyridine (0.06 g, 0.49 mmol) in N,N-dimethylacetamide (5 mL) was added cyclopentanecarbonyl chloride (0.32 g, 2.42 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.43 g, yield 66%).
melting point: 309-311°C.

### Reference Example A 23-194

### N-[4-(3,5-dimethylphenyl)-5-(4-pyridyl)-1,3-thiazol-2-yl]propionamide

To a solution of [4-(3,5-dimethylphenyl)-5-(4-pyridyl)-1,3-thiazol-2-yl]amine (0.51 g, 1.8 mmol) and 4-dimethylaminopyridine (0.06 g, 0.52 mmol) in N,N-dimethylacetamide (20 mL) was added propionyl chloride (0.18 g, 1.96 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.41 g, yield 67%).
melting point: 291-293°C.

### Reference Example A 23-195

### N-[4-(3,5-dimethylphenyl)-5-(4-pyridyl)-1,3-thiazol-2-yl]-2-methylpropionamide

To a solution of [4-(3,5-dimethylphenyl)-5-(4-pyridyl)-1,3-thiazol-2-yl]amine (0.50 g, 1.8 mmol) and 4-dimethylaminopyridine (0.06 g, 0.53 mmol) in N,N-dimethylacetamide (20 mL) was added 2-methylpropionyl chloride (0.20 g, 1.91 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.52 g, yield 83%).
melting point: 270-272°C.

### Reference Example A 23-196

### N-[4-(3,5-dimethylphenyl)-5-(4-pyridyl)-1,3-thiazol-2-yl]-2-phenylacetamide

To a solution of [4-(3,5-dimethylphenyl)-5-(4-pyridyl)-1,3-thiazol-2-yl]amine (0.51 g, 1.8 mmol) and 4-dimethylaminopyridine (0.06 g, 0.52 mmol) in N,N-dimethylacetamide (15 mL) was added 2-phenylacetyl chloride (0.32 g, 2.0 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.33 g, yield 46%).
melting point: 226-229°C.

### Reference Example A 23-197

### N-[4-(3,5-dimethylphenyl)-5-(4-pyridyl)-1,3-thiazol-2-yl]benzamide

To a solution of [4-(3,5-dimethylphenyl)-5-(4-pyridyl)-1,3-thiazol-2-yl]amine (0.51 g, 1.8 mmol) and 4-dimethylaminopyridine (0.06 g, 0.52 mmol) in N,N-dimethylacetamide (20 mL) was added benzoyl chloride (0.30 g, 2.15 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.18 g, yield 26%).
melting point: 285-286°C.

### Reference Example A 23-198

### N-[4-(3,5-dimethylphenyl)-5-(4-pyridyl)-1,3-thiazol-2-yl]cyclopentanecarboxamide

To a solution of [4-(3,5-dimethylphenyl)-5-(4-pyridyl)-1,3-thiazol-2-yl]amine (0.51 g, 1.8 mmol) and 4-dimethylaminopyridine (0.07 g, 0.56 mmol) in N,N-dimethylacetamide (10 mL) was added cyclopentanecarbonyl chloride (0.33 g, 2.47 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.41 g, yield 59%).
melting point: 275-278°C.

### Reference Example A 23-199

### N-[4-(3,5-dimethylphenyl)-5-(4-pyridyl)-1,3-thiazol-2-yl]nicotinamide

To a solution of [4-(3,5-dimethylphenyl)-5-(4-pyridyl)-1,3-thiazol-2-yl]amine (0.52 g, 1.9 mmol) and 4-dimethylaminopyridine (0.07 g, 0.56 mmol) in N,N-dimethylacetamide (10 mL) was added nicotinoyl chloride hydrochloride (0.51 g, 2.86 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.44 g, yield 61%).
melting point: 267-270°C.

### Reference Example A 23-200

### N-[4-(3,5-dimethylphenyl)-5-(4-pyridyl)-1,3-thiazol-2-yl]isonicotinamide

To a solution of [4-(3,5-dimethylphenyl)-5-(4-pyridyl)-1,3-thiazol-2-yl]amine (0.51 g, 1.8 mmol) and 4-dimethylaminopyridine (0.07 g, 0.56 mmol) in N,N-dimethylacetamide (10 mL) was added isonicotinoyl chloride hydrochloride (0.48 g, 2.72 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.22 g, yield 32%).
melting point: 302-304°C.

### Reference Example A 23-201

### N-[4(3,5-dimethylphenyl)-5-(4-pyridyl)-1,3-thiazol-2-yl]-N'-ethylurea

To a solution of [4-(3,5-dimethylphenyl)-5-(4-pyridyl)-1,3-thiazol-2-yl]amine (0.51 g, 1.8 mmol) in N,N-dimethylacetamide (10 mL) was added ethyl isocyanate (0.20 g, 2.8 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.27 g, yield 42%).
melting point: 202-203°C.

### Reference Example A 23-202

### N-[4-(3,5-dimethylphenyl)-5-(4-pyridyl)-1,3-thiazol-2-yl]-N'-propylurea

To a solution of [4-(3,5-dimethylphenyl)-5-(4-pyridyl)-1,3-thiazol-2-yl]amine (0.51 g, 1.8 mmol) in N,N-dimethylacetamide (15 mL) was added propyl isocyanate (0.23 g, 2.67 mmol) and the mixture was stirred at 80°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.23 g, yield 33%).
melting point: 128-130°C.

### Reference Example A 23-246

### N-[4-(3,5-dimethylphenyl)-5-(4-pyridyl)-1,3-thiazol-2-yl]pyrazinecarboxamide

To a solution of [4-(3,5-dimethylphenyl)-5-(4-pyridyl)-1,3-thiazol-2-yl]amine (0.50 g, 1.8 mmol) and 4-dimethylaminopyridine (0.06 g, 0.53 mmol) in N,N-dimethylacetamide (5 mL) was added pyrazinecarbonyl chloride (0.44 g, 2.7 mmol) and the mixture was stirred at 70°C for 14 h. To the reaction mixture was poured aqueous sodium hydrogencarbonate solution and the precipitated solid was collected by filtration. The obtained solid was washed with water and dried. The crude crystals were recrystallized from ethanol to give the title compound (0.41 g, yield 59%).
melting point: 269-270°C.

### Reference Example A 24

### 1-bromo-3-ethylbenzene

To a 50% aqueous sulfuric acid solution (43.6 g) of 3-ethylaniline (10.0 g, 82.5mmol) was added dropwise at 0°C an aqueous solution (16.5 mL) of sodium nitrite (6.83 g, 99.0 mmol) over 30 min. The obtained reaction mixture was stirred at 0°C for 45 min. This diazonium salt solution was added by small portions to a 48% hydrobromic acid solution (82.5 mL) of copper(I) bromide (12.4 g, 86.6 mmol) being gently refluxed under heating. After the addition, the reaction mixture was refluxed under heating for 30 min. The reaction mixture was cooled to room temperature and extracted with ether. The extract was washed successively with 1N aqueous sodium hydroxide solution and saturated brine, filtrated, dried and concentrated. The residue was purified by silica gel column chromatography (hexane-ethyl acetate = 20:1) to give the title compound (6.13 g, yield 40%).
oil.
¹H-NMR (CDCl₃) δ: 1.23 (3H, t, J= 7.5 Hz), 2.63 (2H, q, J= 7.5 Hz), 7.11-7.20 (2H, m), 7.28-7.38 (2H, m).

### Reference Example A 25

In accordance with Reference Example A 24, the following Reference Example compound A 25 was synthesized using 3-(1-methylethyl)aniline instead of 3-ethylaniline.

### Reference Example compound 25: 1-bromo-3-(1-methylethyl)benzene oil.

¹H-NMR (CDCl₃) δ: 1.24 (6H, d, J= 7.0 Hz), 2.77-2.99 (1H, m), 7.03-7.16 (2H, m), 7.27-7.34 (1H, m), 7.37 (1H, s).

### Reference Example A 26

### 3-ethylbenzoic acid

A solution (45 mL) of 1-bromo-3-ethylbenzene (5.1 g, 28 mmol) in tetrahydrofuran was added dropwise to a mixture (5.0 mL) of magnesium turnings (0.74 g, 31 mmol) and tetrahydrofuran under an argon atmosphere, and the mixture was stirred as it was for 30 min. The reaction mixture was added to the crushed dry ice and the mixture was stirred as it was for 1 h. 1N Hydrochloric acid was added to the reaction mixture and the mixture was extracted with ethyl acetate. The extract was dried, filtrated and concentrated. The residue was purified by silica gel column chromatography (hexane-ethyl acetate = 5:1) to give the title compound (3.87 g, yield 93%).
oil.
¹H-NMR (CDCl₃) δ: 1.28 (3H, t, J= 7.5 Hz), 2.73 (2H, q, J= 7.5 Hz), 7.34-7.50 (2H, m), 7.92-7.98 (2H, m).

### Reference Example A 27

In accordance with Reference Example 26, the following Reference Example A compounds 27-1 and 27-2 were synthesized using 1-bromo-3-(1-methylethyl)benzene or 1-bromo-4-fluoro-3-methylbenzene instead of 1-bromo-3-ethylbenzene.
Reference Example A compound 27-1: 3-(1-methylethyl)benzoic acid oil.
   ¹H-NMR (CDCl₃) δ: 1.29 (6H, d, J= 7.0 Hz), 2.98-3.06 (1H, m), 7.38-7.54 (2H, m), 7.90-8.02 (2H, m).
Reference Example A compound 27-2: 4-fluoro-3-methylbenzoic acid
   melting point: 165-167°C.

### Reference Example A 28

### 3-ethylbenzoyl chloride

3-Ethylbenzoic acid (9.40 g, 62.6 mmol) was added slowly to thionyl chloride (45 mL) at 0°C, and N,N-dimethylformamide (3 drops) was added dropwise. The obtained reaction mixture was refluxed under heating as it was for 2 h. The reaction mixture was concentrated and used without purification in the next reaction.

### Reference Example A 29

In accordance with Reference Example A 28, the following Reference Example A compounds 29-1 to 29-3 were synthesized using 3-(1-methylethyl)benzoic acid, 4-fluoro-3-methylbenzoic acid or 4-cyclohexylbenzoic acid instead of 3-ethylbenzoic acid.
Reference Example A compound 29-1: 3-(1-methylethyl)benzoyl chloride
   Used in the next reaction without purification.
Reference Example A compound 29-2: 4-fluoro-3-methylbenzoyl chloride
   Used in the next reaction without purification.
Reference Example A compound 29-3: 4-cyclohexylbenzoyl chloride
   Used in the next reaction without purification.

### Reference Example A 30

In accordance with Reference Example A 14, the following Reference Example A compounds 30-1 to 30-7 were synthesized respectively using 3-trifluoromethylbenzoyl chloride, 3,5-dichlorobenzoyl chloride, 3-ethylbenzoyl chloride, 3-(1-methylethyl)benzoyl chloride, 4-fluoro-3-methylbenzoyl chloride, 4-cyclohexylbenzoyl chloride and 3-fluorobenzoyl chloride instead of 4-chlorobenzoyl chloride.
Reference Example A compound 30-1: N-(3-trifluoromethylbenzoyl)propyleneimine
   oil.
   ¹H-NMR (CDCl₃) δ: 1.42 (3H, d, J= 5.5 Hz), 2.20 (1H, d, J= 3.3 Hz), 2.56-2.67 (2H, m), 7.61 (1H, t, J= 7.7 Hz), 7.81 (1H, d, J= 7.7 Hz), 8.21 (1H, d, J= 7.7 Hz), 8.30 (1H, s).
Reference Example A compound 30-2: N-(3,5-dichlorobenzoyl)-propyleneimine
   oil.
   ¹H-NMR (CDCl₃) δ: 1.40 (3H, d, J= 5.1 Hz), 2.19 (1H, d, J= 3.3 Hz), 2.57 (1H, t, J= 5.5 Hz), 2.57-2.70 (1H, m), 7.54 (1H, t, J= 1.8 Hz), 7.88 (2H, d, J= 1.8 Hz).
Reference Example A compound 30-3: N-(3-ethylbenzoyl)-propyleneimine
   oil.
   ¹H-NMR (CDCl₃) δ: 1.27 (3H, t, J= 7.5 Hz), 1.40 (3H, d, J= 5.5 Hz), 2.14 (1H, d, J= 2.9 Hz), 2.52-2.61 (2H, m), 2.71 (2H, q, J= 7.5 Hz), 7.32-7.41 (2H, m), 7.81-7.89 (2H, m).
Reference Example A compound 30-4: N-[3-(1-methylethyl)benzoyl]propyleneimine
   oil.
   ¹H-NMR (CDCl₃) δ: 1.29 (6H, d, J= 7.0 Hz), 1.40 (3H, d, J= 5.9 Hz), 2.14 (1H, d, J= 3.7 Hz), 2.51-2.64 (2H, m), 2.87-3.10 (1H, m), 7.33-7.46 (2H, m), 7.84 (1H, dt, J= 7.0, 1.8 Hz), 7.91 (1H, s).
Reference Example A compound 30-5: N-(4-fluoro-3-methylbenzoyl)propyleneimine
   oil.
   ¹H-NMR (CDCl₃) δ: 1.39 (3H, d, J= 5.4 Hz), 2.14 (1H, d, J= 3.4 Hz), 2.33 (s, 3H), 2.51-2.61 (2H, m), 7.06 (1H, t, J= 8.8 Hz), 7.81-7.90 (2H, m).
Reference Example A compound 30-6: N-(4-cyclohexylbenzoyl)-propyleneimine
   oil.
   ¹H-NMR (CDCl₃) δ: 1.22-1.54 (7H, m), 1.67-1.89 (6H, m), 2.12 (1H, d, J= 3.2 Hz), 2.52-2.60 (3H, m), 7.28 (2H, d, J= 8.3 Hz), 7.95 (2H, d, J= 8.3 Hz).
Reference Example A compound 30-7: N-(3-fluorobenzoyl)-propyleneimine
   oil.
   ¹H-NMR (CDCl₃) δ: 1.40 (3H, d, J= 5.5 Hz), 2.16 (1H, d, J= 3.3 Hz), 2.52-2.68 (2H, m), 7.25 (1H, ddd, J= 8.4, 2.6, 1.1 Hz), 7.43 (1H, ddd, J= 8.1, 7.7, 5.5 Hz), 7.69 (1H, ddd, J= 8.1, 2.6, 1.5 Hz), 7.81 (1H, ddd, J= 7.7, 1.5, 1.1 Hz).

### Reference Example A 31

In accordance with Reference Example A 16, the following Reference Example A compounds 31-1 to 31-7 were synthesized respectively using N-(3-trifluoromethylbenzoyl)propyleneimine, N-(3,5-dichlorobenzoyl)propyleneimine, N-(3-ethylbenzoyl)-propyleneimine, N-[3-(1-methylethyl)benzoyl]propyleneimine, N-(4-fluoro-3-methylbenzoyl)propyleneimine, N-(4-cyclohexylbenzoyl)propyleneimine and N-(3-fluorobenzoyl)-propyleneimine instead of N-(2-chlorobenzoyl)propyleneimine. Reference Example A compound 31-1: 2-(4-pyridyl)-1-(3-trifluoromethylphenyl)ethanone
oil.
¹H-NMR (CDCl₃) δ: 4.33 (2H, s), 7.21 (2H, d, J= 6.0 Hz), 7.65 (1H, dd, J= 8.4, 7.7 Hz), 7.87 (1H, d, J= 7.7 Hz), 8.18 (1H, d, J= 8.4 Hz), 8.26 (1H, s), 8.59 (2H, d, J= 6.0 Hz).
Reference Example A compound 31-2: 1-(3,5-dichlorophenyl)-2-(4-pyridyl)ethanone
melting point: 163-164°C.
Reference Example A compound 31-3: 1-(3-ethylphenyl)-2-(4-pyridyl)ethanone
   melting point: 102-103°C.
Reference Example A compound 31-4: 1-[3-(1-methylethyl)phenyl]-2-(4-pyridyl)ethanone
   melting point: 50-52°C.
Reference Example A compound 31-5: 1-(4-fluoro-3-methylphenyl)-2-(4-pyridyl)ethanone
   melting point: 86-88°C.
Reference Example A compound 31-6: 1-(4-cyclohexylphenyl)-2-(4-pyridyl)ethanone
   oil.
   ¹H-NMR (CDCl₃) δ: 1.32-1.52 (5H, m) , 1.77-1.89 (5H, m), 2.58 (1H, m), 4.26 (2H, s), 7.20 (2H, d, J= 6.3 Hz), 7.32 (2H, d, J= 8.4 Hz), 7.93 (2H, d, J= 8.4 Hz), 8.56 (2H, d, J= 6.3 Hz).
   Reference Example A compound 31-7: 1-(3-fluorophenyl)-2-(4-pyridyl)ethanone
   Amorphous powder.
   ¹H-NMR (CDCl₃) δ: 4.28 (2H, s), 7.20 (2H, d, J= 6.2 Hz), 7.33 (1H, ddd, J= 8.1, 2.6, 1.1 Hz), 7.49 (1H, ddd, J= 8.1, 7.7, 5.5 Hz), 7.68 (1H, ddd, J= 9.5, 2.6, 1.5 Hz), 7.79 (1H, ddd, J= 7.7, 1.5, 1.1 Hz), 8.58 (2H, d, J= 6.2 Hz).

### Reference Example A 32

In accordance with Reference Example A 17, the following Reference Example A compounds 32-1 to 32-4 were synthesized using 2,4-lutidine or γ-collidine instead of γ-picoline.
Reference Example A compound 32-1: 1-(3-methylphenyl)-2-(2-methyl-4-pyridyl)ethanone
melting point: 56-57°C.
Reference Example A compound 32-2: 1-(3,5-dimethylphenyl)-2-(2-methyl-4-pyridyl)ethanone
   oil.
   ¹H-NMR (CDCl₃) δ: 2.38 (6H, s), 2.54 (3H, s), 4.21 (2H, s), 6.98-7.10 (1H, m), 7.01 (1H, m), 7.06 (1H, s), 7.23 (1H, s), 7.60 (2H, s), 8.42-8.45 (1H, m).
Reference Example A compound 32-3: 2-(2,6-dimethyl-4-pyridyl)-1-(3-methylphenyl)ethanone
   melting point: 46-48°C.
Reference Example A compound 32-4: 1-(3,5-dimethylphenyl)-2-(2,6-dimethyl-4-pyridyl)ethanone
   melting point: 135-136°C.

### Reference Example A 33

### 2-(2-tert-butoxycarbonylamino-4-pyridyl)-1-(4-methoxyphenyl)ethanone

A solution of 2-tert-butoxycarbonylamino-4-methylpyridine (20 g, 97 mmol) in anhydrous tetrahydrofuran (300 mL) was cooled to -78°C and 1.6 M n-butyllithium/hexane solution (140 mL, 0.23 mol) was added dropwise with stirring. After completion of the dropwise addition, the mixture was stirred at room temperature for 30 min and cooled to -78°C. A solution of N-(4-methoxybenzoyl)propyleneimine (25 g, 0.13 mol) in anhydrous tetrahydrofuran (50 mL) was added dropwise. After completion of the dropwise addition, the mixture was stirred at room temperature for 2 h. To the reaction mixture were added water (100 mL) and isopropyl ether (300 mL), and the obtained crude crystals were collected by filtration. The crude crystals were recrystallized from tetrahydrofuran-hexane to give the title compound (23 g, yield 69%).
melting point: 187-190°C.

### Reference Example A 34

In accordance with Reference Example A 33, the following Reference Example A compound 34-1 and 34-2 were synthesized respectively using N-(3-methylbenzoyl)propyleneimine and N-(3,5-dimethylbenzoyl)propyleneimine instead of N-(4-methoxybenzoyl)propyleneimine.
Reference Example A compound 34-1: 2-(2-tert-butoxycarbonylamino-4-pyridyl)-1-(3-methylphenyl)ethanone melting point: 144-146°C.
Reference Example A compound 34-2: 2-(2-tert-butoxycarbonylamino-4-pyridyl)-1-(3,5-dimethylphenyl)ethanone melting point: 133-136°C.

### Reference Example A 35

### 2-fluoro-4-methylpyridine

Synthesized in accordance with the method described in Journal of Medicinal Chemistry, vol. 33, pp. 1667-1675 (1990).
boiling point: 82-86°C (10 kPa).

### Reference Example A 36

### 2-(2-fluoro-4-pyridyl)-1-(3-methylphenyl)ethanone

A solution of diisopropylamine (44 mL, 0.31 mol) in anhydrous tetrahydrofuran (300 mL) was cooled to -78°C under an argon atmosphere, and 1.6 M n-butyllithium/hexane solution (190 mL, 0.31 mol) was added dropwise with stirring. After completion of the dropwise addition, the mixture was stirred for 10 min, and a solution of 2-fluoro-4-methylpyridine (34.5 g, 0.31 mol) in anhydrous tetrahydrofuran (30 mL) was added. The reaction mixture was stirred at -10°C for 30 min. The reaction solution was cooled to -78°C and a solution of N-(3-methylbenzoyl)propyleneimine (52 g, 0.30 mol) in anhydrous tetrahydrofuran (30 mL) was added dropwise. After completion of dropwise addition, the mixture was stirred at room temperature for 2 h. To the reaction mixture was added water (100 mL), and the mixture was extracted with ethyl acetate. The extract was washed with water, dried and the solvent was evaporated. The residue was recrystallized from isopropyl ether to give the title compound (35 g, yield 52%).
melting point: 66-67°C.

### Reference Example A 37

In accordance with Reference Example A 36, the following
Reference Example A compound 37 was synthesized using N-(3-methoxybenzoyl)propyleneimine instead of N-(3-methylbenzoyl)propyleneimine.
Reference Example A compound 37: 2-(2-fluoro-4-pyridyl)-1-(3-methoxyphenyl)ethanone
   oil
   ¹H-NMR (CDCl₃) δ: 3.86 (3H, s), 4.31 (2H, s), 6.86 (1H, s), 7.03-7.19 (2H, m), 7.31-7.59 (3H, m), 8.18 (1H, d, J= 5.6 Hz).

### Reference Example A 38

In accordance with Reference Example A 21, the following Reference Example compounds 38-1 to 38-21 were synthesized respectively using 2-methylbenzonitrile, 3-methylbenzonitrile, 4-methylbenzonitrile, 2-chlorobenzonitrile, 3-chlorobenzonitrile, 4-chlorobenzonitrile, 3-methoxybenzonitrile, 4-methoxybenzonitrile, 2-fluorobenzonitrile, 3-fluorobenzonitrile, 4-fluorobenzonitrile, 4-nitrobenzonitrile, piperonylonitrile, 3-methoxycarbonylbenzonitrile, 4-methoxycarbonylbenzonitrile, butyronitrile, isobutyronitrile, valeronitrile, hexanenitrile, 3-phenylpropionitrile and 4-phenylbutyronitrile instead of 4-methylthiobenzonitrile.
Reference Example A compound 38-1: 2-methyl(thiobenzamide)
   oil
   ¹H-NMR (CDCl₃) δ: 2.37 (3H, s), 6.88 (1H, br s), 7.06-7.23 (3H, m), 7.24-7.31 (1H, m), 7.88 (1H, br s).
Reference Example A compound 38-2: 3-methyl(thiobenzamide) melting point: 88-89°C.
Reference Example A compound 38-3: 4-methyl(thiobenzamide) melting point: 172-174°C.
Reference Example A compound 38-4: 2-chlorothiobenzamide melting point: 58-59°C.
Reference Example A compound 38-5: 3-chlorothiobenzamide melting point: 114-115°C.
Reference Example A compound 38-6: 4-chlorothiobenzamide melting point: 130-131°C.
Reference Example A compound 38-7: 3-methoxythiobenzamide
   oil
   ¹H-NMR (CDCl₃) δ: 3.86 (3H, s), 7.02-7.08 (1H, m), 7.31-7.36 (3H, m), 7.46-7.49 (1H, m), 7.76 (1H, br s).
Reference Example A compound 38-8: 4-methoxythiobenzamide melting point: 148-149°C.
Reference Example A compound 38-9: 2-fluorothiobenzamide melting point: 113-114°C.
Reference Example A compound 38-10: 3-fluorothiobenzamide melting point: 151-152°C.
Reference Example A compound 38-11: 4-fluorothiobenzamide melting point: 156-157°C.
Reference Example A compound 38-12: 4-nitrothiobenzamide melting point: 159-160°C.
Reference Example A compound 38-13: thiopiperonylamide melting point: 188-189°C.
Reference Example A compound 38-14: 3-methoxycarbonyl-thiobenzamide
   melting point: 140-141°C.
Reference Example A compound 38-15: 4-methoxycarbonylthiobenzamide
   melting point: 191-192°C.
Reference Example A compound 38-16: thiobutylamide
   oil
   ¹H-NMR (CDCl₃) δ: 0.99 (3H, t, J= 7.6 Hz), 1.72-1.93 (2H, m), 2.64 (2H, t, J= 7.6 Hz), 7.02 (1H, br s), 7.77 (1H, br s).
Reference Example compound A 38-17: thioisobutylamide
   oil
   ¹H-NMR (CDCl₃) δ: 1.28 (6H, d, J= 5.8 Hz), 2.79-2.96 (1H, m), 6.99 (1H, br s), 7.71 (1H, br s).
Reference Example A compound 38-18: thiovaleramide
   oil
   ¹H-NMR (CDCl₃) δ: 0.94 (3H, t, J= 7.3 Hz), 1.31-1.49 (2H, m), 1.68-1.83 (2H, m), 2.67 (2H, t, J= 7.7 Hz), 6.92 (1H, br s), 7.73 (1H, br s).
Reference Example A compound 38-19: hexanethioamide
   oil
   ¹H-NMR (CDCl₃) δ: 0.90 (3H, t, J= 6.9 Hz), 1.22-1.45 (4H, m), 1.70-1.84 (2H, m), 2.66 (2H, t, J= 7.5 Hz), 7.05 (1H, br s), 7.91 (1H, br s).
Reference Example A compound 38-20: 3-phenyl(thiopropionamide) melting point: 83-84°C.
Reference Example A compound 38-21: 4-phenyl(thiobutylamide) melting point: 60-61°C.

### Reference Example A 39

In accordance with Reference Example A 6, the following Reference Example A compounds 39-1 to 39-13 were synthesized respectively using 2-(4-pyridyl)-1-(3-trifluoromethylphenyl)-ethanone, 1-(3,5-dichlorophenyl)-2-(4-pyridyl)ethanone, 1-(3-ethylphenyl)-2-(4-pyridyl)ethanone, 1-[3-(1-methylethyl)-phenyl]-2-(4-pyridyl)ethanone, 1-(4-fluoro-3-methylphenyl)-2-(4-pyridyl)ethanone, 1-(4-cyclohexylphenyl)-2-(4-pyridyl)-ethanone, 1-(3-fluorophenyl)-2-(4-pyridyl)ethanone, 2-(2-fluoro-4-pyridyl)-1-(3-methylphenyl)ethanone, 2-(2-fluoro-4-pyridyl)-1-(3-methoxyphenyl)ethanone, 1-(3-methylphenyl)-2-(2-methyl-4-pyridyl)ethanone, 1-(3,5-dimethylphenyl)-2-(2-methyl-4-pyridyl)ethanone, 2-(2,6-dimethyl-4-pyridyl)-1-(3-methylphenyl)ethanone and 1-(3,5-dimethylphenyl)-2-(2,6-dimethyl-4-pyridyl)ethanone instead of 1-(4-methoxyphenyl)-2-(3-pyridyl)ethanone.
Reference Example A compound 39-1: 2-bromo-2-(4-pyridyl)-1-(3-trifluoromethylphenyl)ethanone hydrobromide
   Used in the next reaction without purification.
Reference Example A compound 39-2: 2-bromo-1-(3,5-dichlorophenyl)-2-(4-pyridyl)ethanone hydrobromide
   melting point: 253-254°C
Reference Example A compound 39-3: 2-bromo-1-(3-ethylphenyl)-2-(4-pyridyl)ethanone hydrobromide
   melting point: 146-148°C.
Reference Example A compound 39-4: 2-bromo-1-[3-(1-methylethyl)phenyl]-2-(4-pyridyl)ethanone hydrobromide
   melting point: 143-144°C.
Reference Example A compound 39-5: 2-bromo-1-(4-fluoro-3-methylphenyl) -2-(4-pyridyl)ethanone hydrobromide
   melting point: 211-214°C.
Reference Example A compound 39-6: 2-bromo-1-(4-cyclohexylphenyl)-2-(4-pyridyl)ethanone hydrobromide
   melting point: 189-191°C.
Reference Example A compound 39-7: 2-bromo-l-(3-fluorophenyl)-2-(4-pyridyl)ethanone hydrobromide
   melting point: 191-194°C.
Reference Example A compound 39-8: 2-bromo-2-(2-fluoro-4-pyridyl)-1-(3-methylphenyl)ethanone hydrobromide
   Used in the next reaction without purification.
Reference Example A compound 39-9: 2-bromo-2-(2-fluoro-4-pyridyl)-1-(3-methoxyphenyl)ethanone hydrobromide
   Used in the next reaction without purification.
Reference Example A compound 39-10: 2-bromo-1-(3-methylphenyl)-2-(2-methyl-4-pyridyl)ethanone hydrobromide melting point: 144-146°C.
Reference Example A compound 39-11: 2-bromo-1-(3,5-dimethylphenyl)-2-(2-methyl-4-pyridyl)ethanone hydrobromide Used in the next reaction without purification.
Reference Example A compound 39-12: 2-bromo-2-(2,6-dimethyl-4-pyridyl)-1-(3-methylphenyl)ethanone hydrobromide Used in the next reaction without purification.
Reference Example A compound 39-13: 2-bromo-1-(3,5-dimethylphenyl)-2-(2,6-dimethyl-4-pyridyl)ethanone hydrobromide
   melting point: 208-212°C.

### Reference Example A 40

### 2-bromo-2-(2-tert-butoxycarbonylamino-4-pyridyl)-1-(4-methoxyphenyl)ethanone hydrobromide

To a solution of 2-(2-tert-butoxycarbonylamino-4-pyridyl)-1-(4-methoxyphenyl)ethanone (0.36 g, 1.1 mmol) in acetic acid (5 mL) was added bromine (0.058 mL, 1.1 mmol) and the mixture was stirred at room temperature for 1 h. The reaction mixture was concentrated and the residue was washed with isopropyl ether to give the title compound (0.44 g, yield 82%).

### Amorphous powder

¹H-NMR (CDCl₃) δ: 1.55 (6H, s), 3.92 (3H, s), 6.35 (1H, s), 6.99-7.03 (2H, m), 7.66 (1H, dd, J= 6.6, 1.8 Hz), 8.02-8.07 (2H, m), 8.20 (1H, d, J= 6.6 Hz), 8.70 (2H, d, J= 1.8 Hz), 11.02 (1H, br s).

### Reference Example A 41

In accordance with Reference Example A 40, the following Reference Example A compounds 41-1 and 41-2 were synthesized respectively using 2-(2-tert-butoxycarbonylamino-4-pyridyl)-1-(3-methylphenyl)ethanone and 2-(2-tert-butoxycarbonylamino-4-pyridyl)-1-(3,5-dimethylphenyl)ethanone instead of 2-(2-tert-butoxycarbonylamino-4-pyridyl)-1-(4-methoxyphenyl)ethanone. Reference Example A compound 41-1: 2-bromo-2-(2-tert-butoxycarbonylamino-4-pyridyl)-1-(3-methylphenyl)ethanone hydrobromide
Used in the next reaction without purification.
Reference Example A compound 41-2: 2-bromo-2-(2-tert-butoxycarbonylamino-4-pyridyl)-1-(3,5-dimethylphenyl)ethanone hydrobromide
   Used in the next reaction without purification.

### Reference Example A 42

### ethyl (4-phenyl-1-piperazinyl)carbothioylcarbamate

1-Phenylpiperazine (10 g, 62 mmol) was added to a solution of ethyl isothiocyanatoformate (8.1 g, 62 mmol) in acetone (30 mL) and the mixture was refluxed under heating for 1 h. The reaction mixture was concentrated and the crude crystals were recrystallized from ethyl acetate to give the title compound (13 g, yield 73%).
melting point: 134-135°C.

### Reference Example A 43

### 4-phenyl-1-piperazinecarbothioamide

Ethyl (4-phenyl-1-piperazinyl)carbothioylcarbamate (13 g, 44 mmol) was added to conc. hydrochloric acid (44 mL) and the mixture was stirred at 80°C for 2 h. The reaction mixture was made basic with 8N aqueous sodium hydroxide solution and the crystals were collected by filtration. The crystals were washed with water and dried to give the title compound (6.1 g, yield 63%).
melting point: 178-179°C.

### Reference Example A 44

In accordance with the methods described in Reference Examples A 8 to 12, Reference Example A 44-1, JP-A-61-10580 and USP 4,612,321, Reference Example compounds A 44-1 to 44-129 shown in the following Tables 32-42 were synthesized.

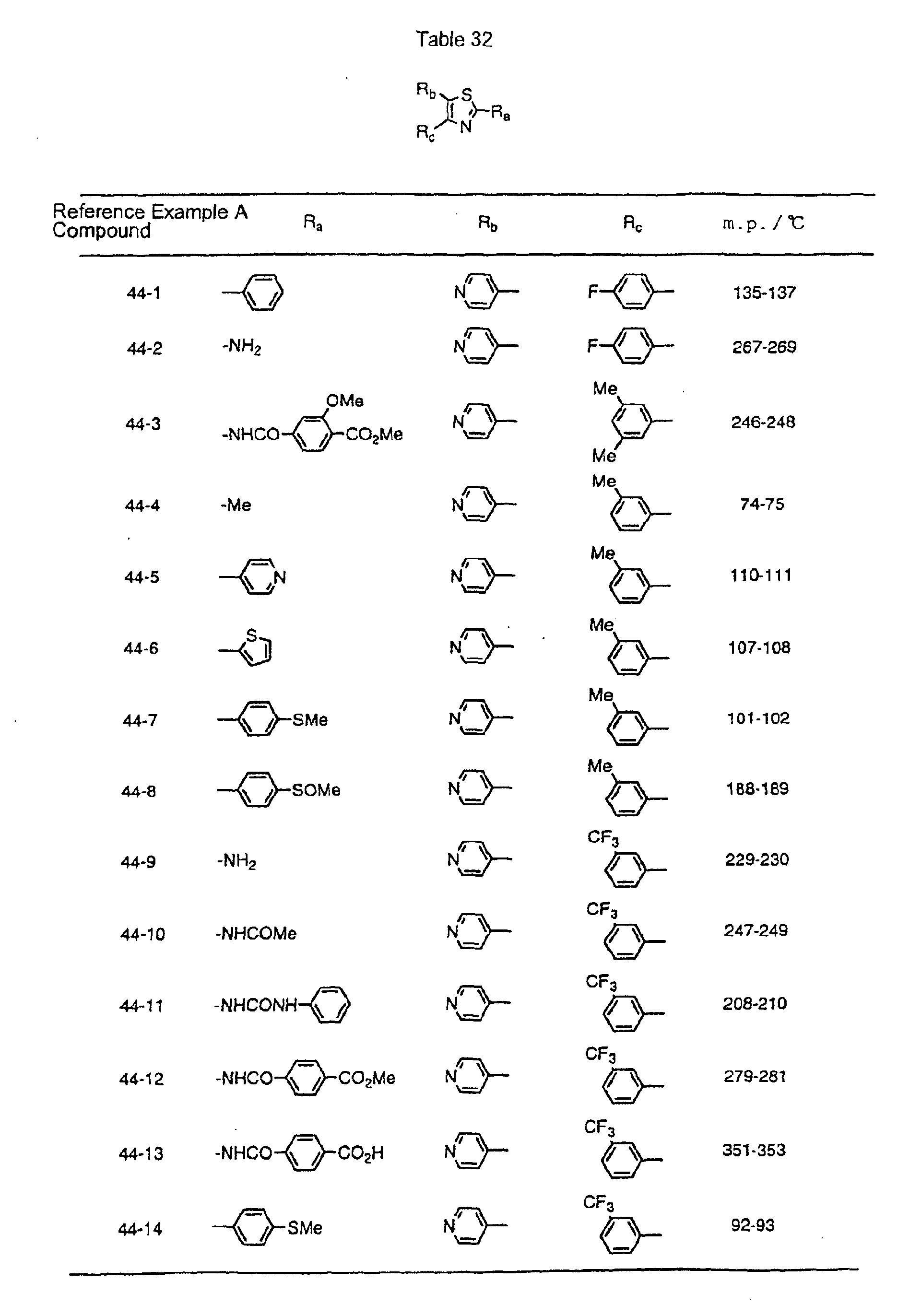

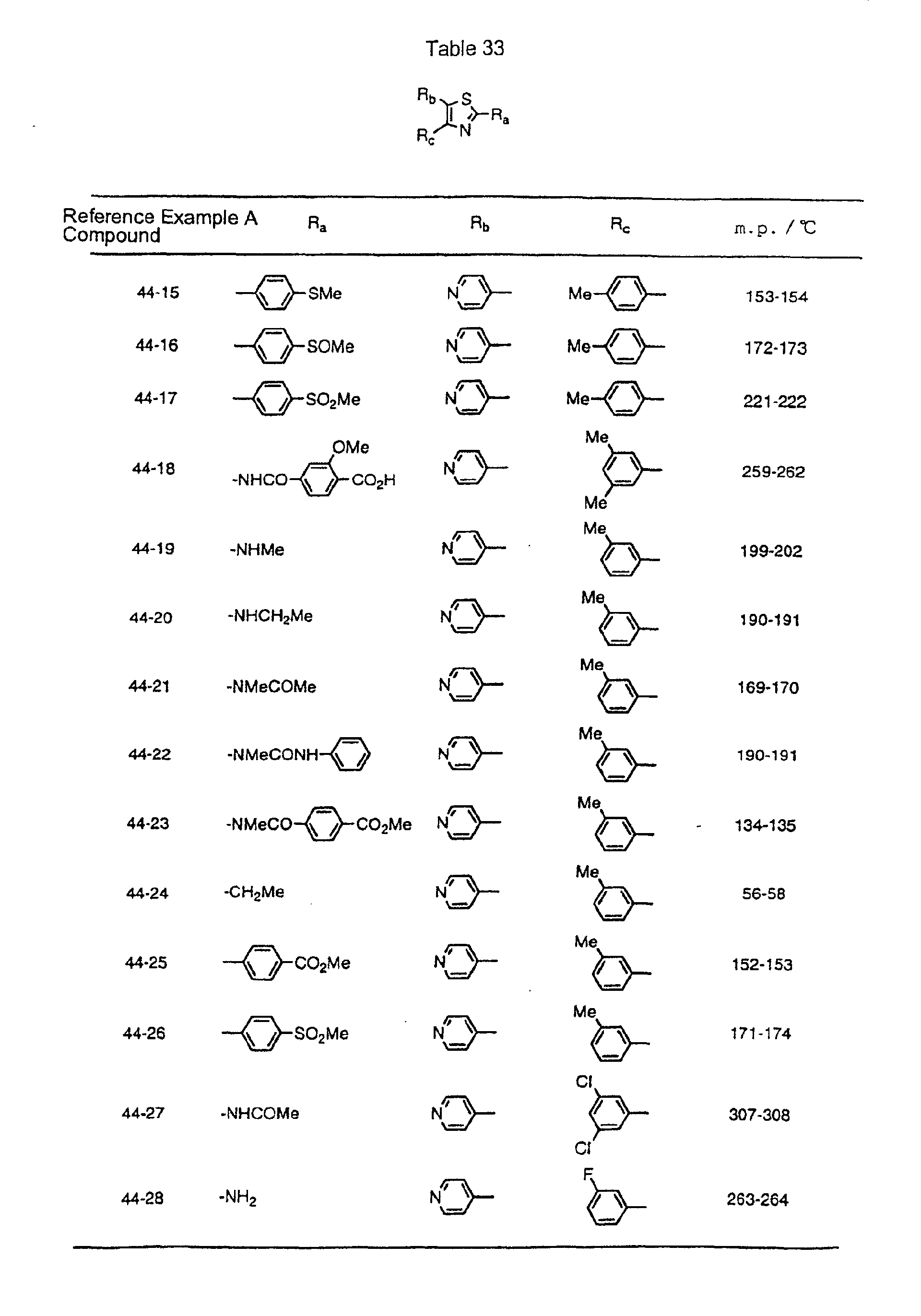

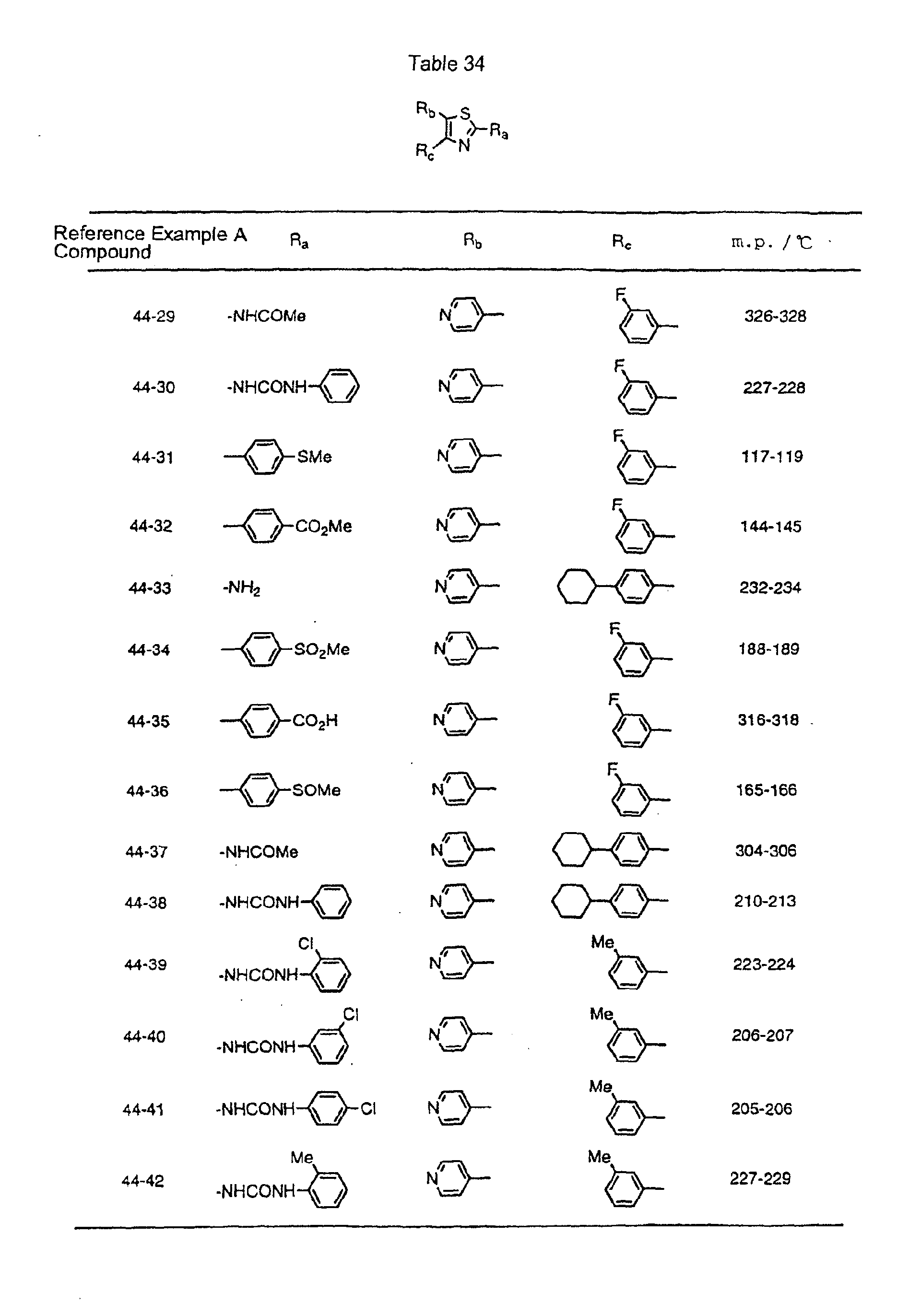

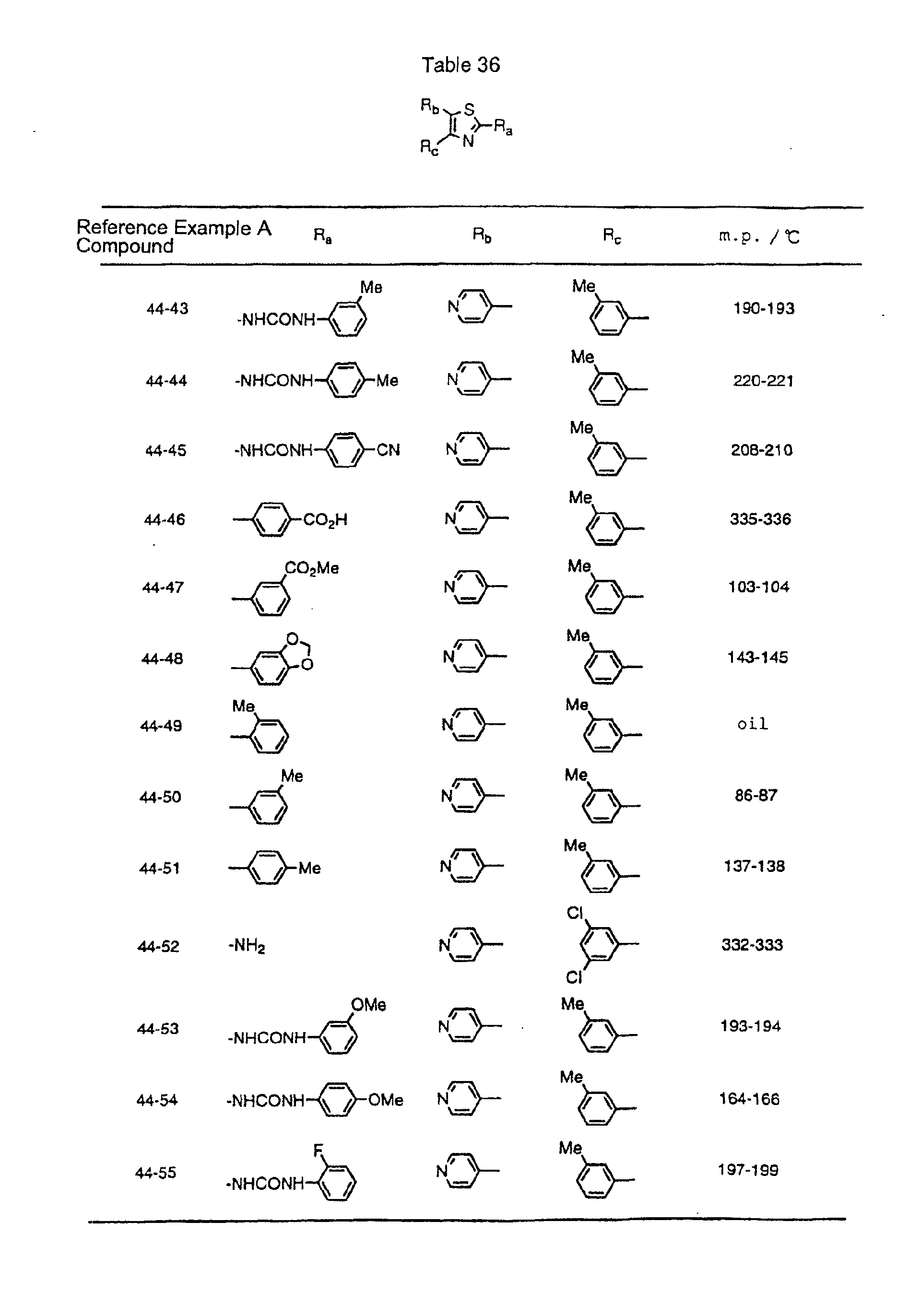

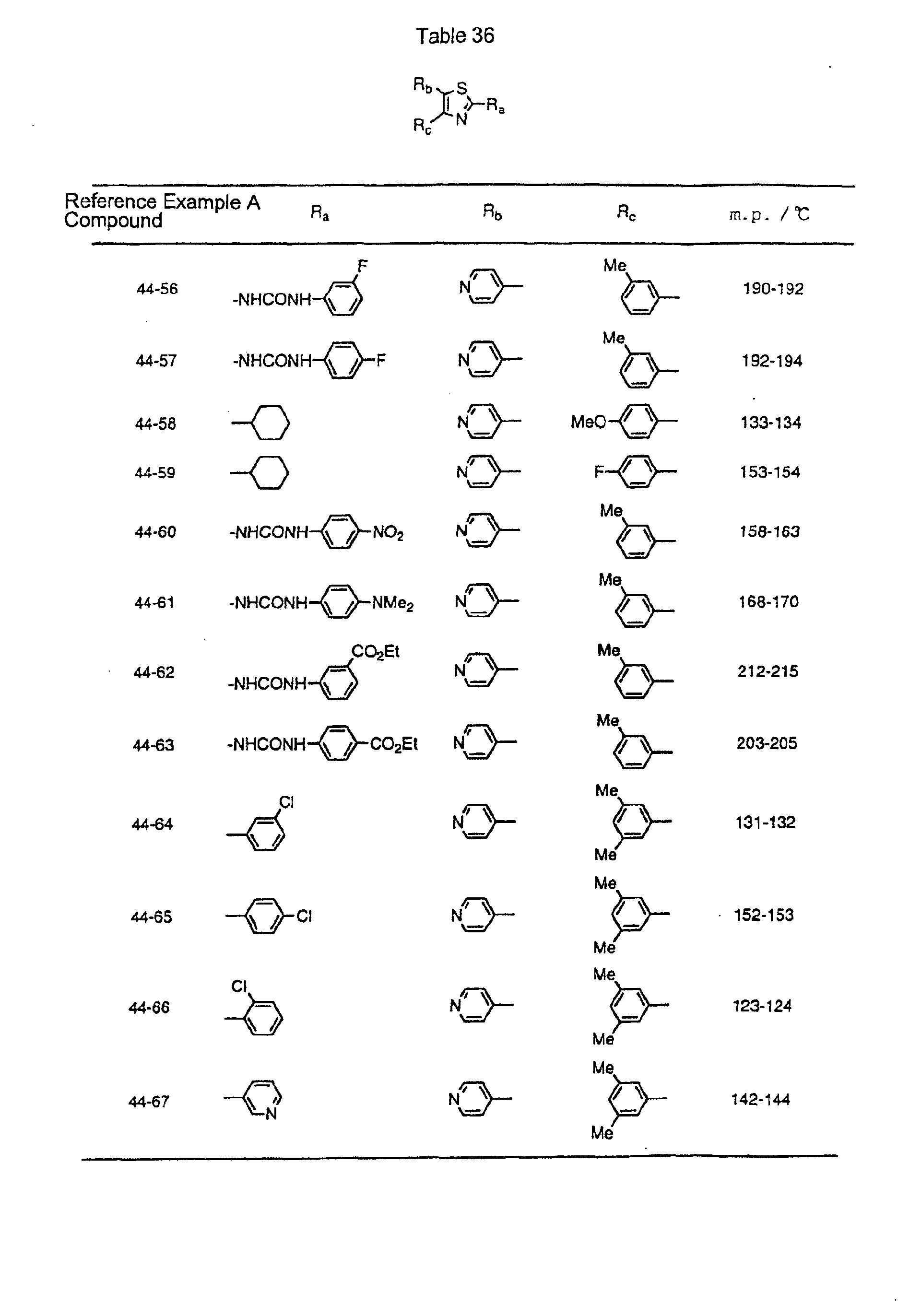

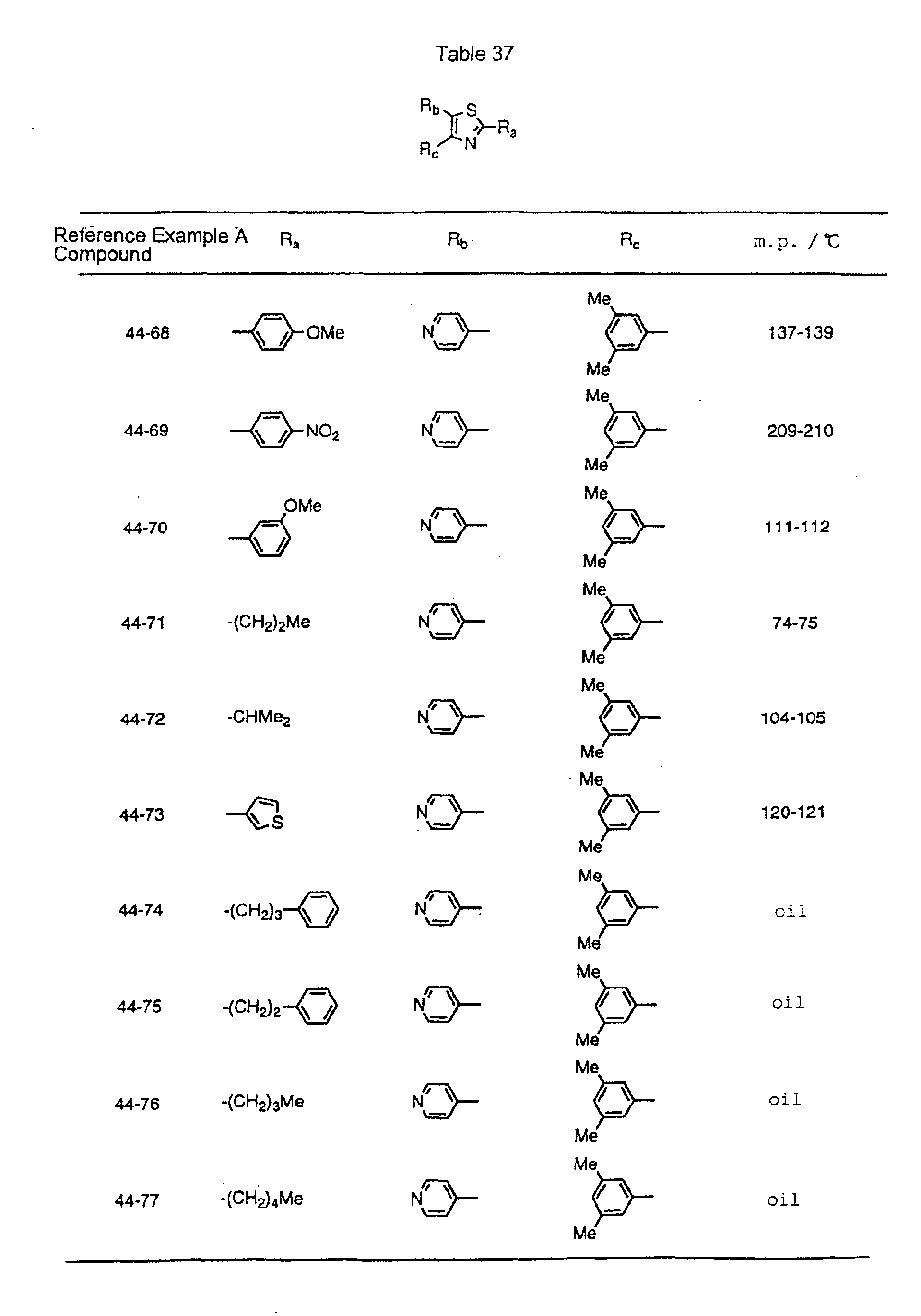

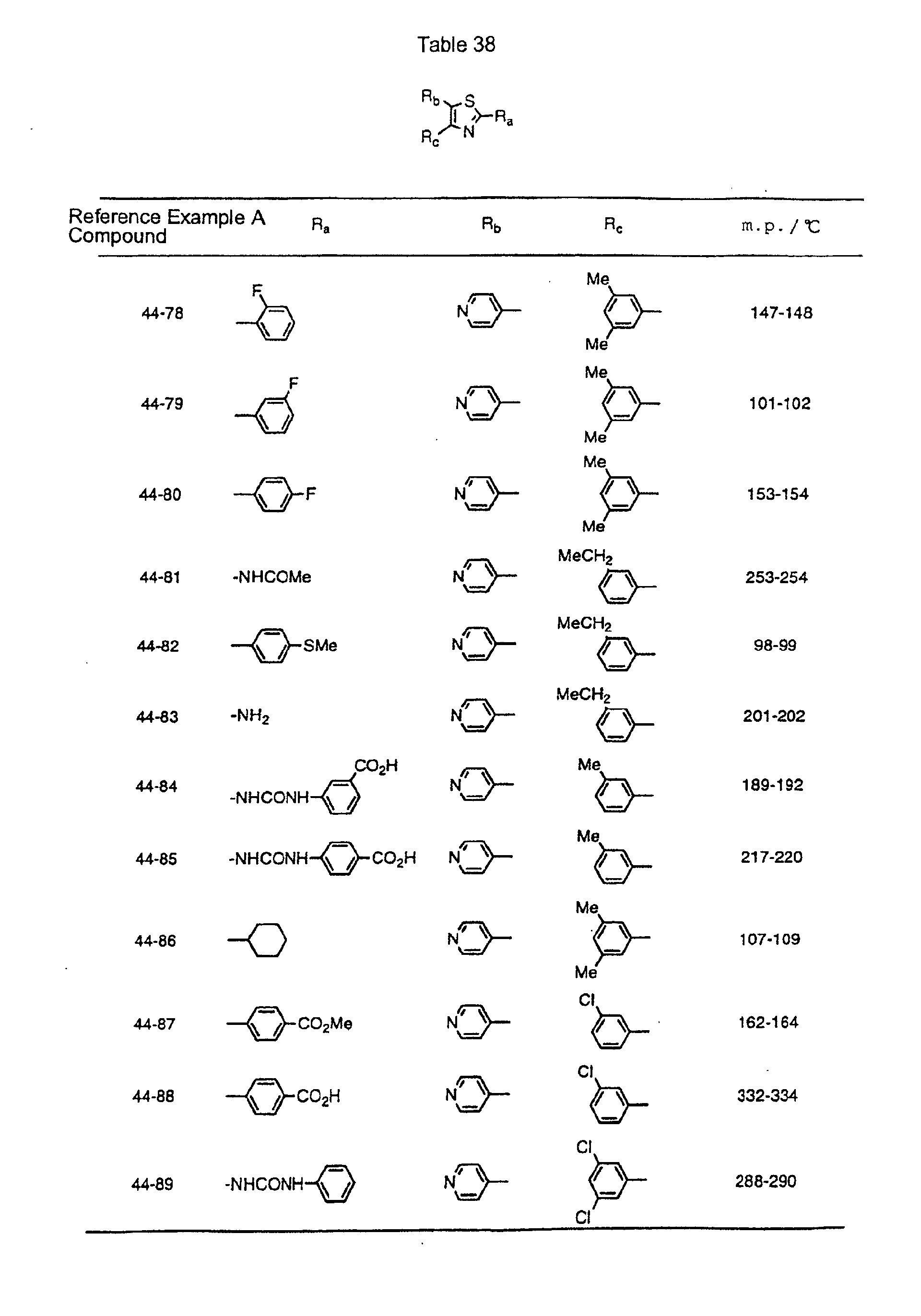

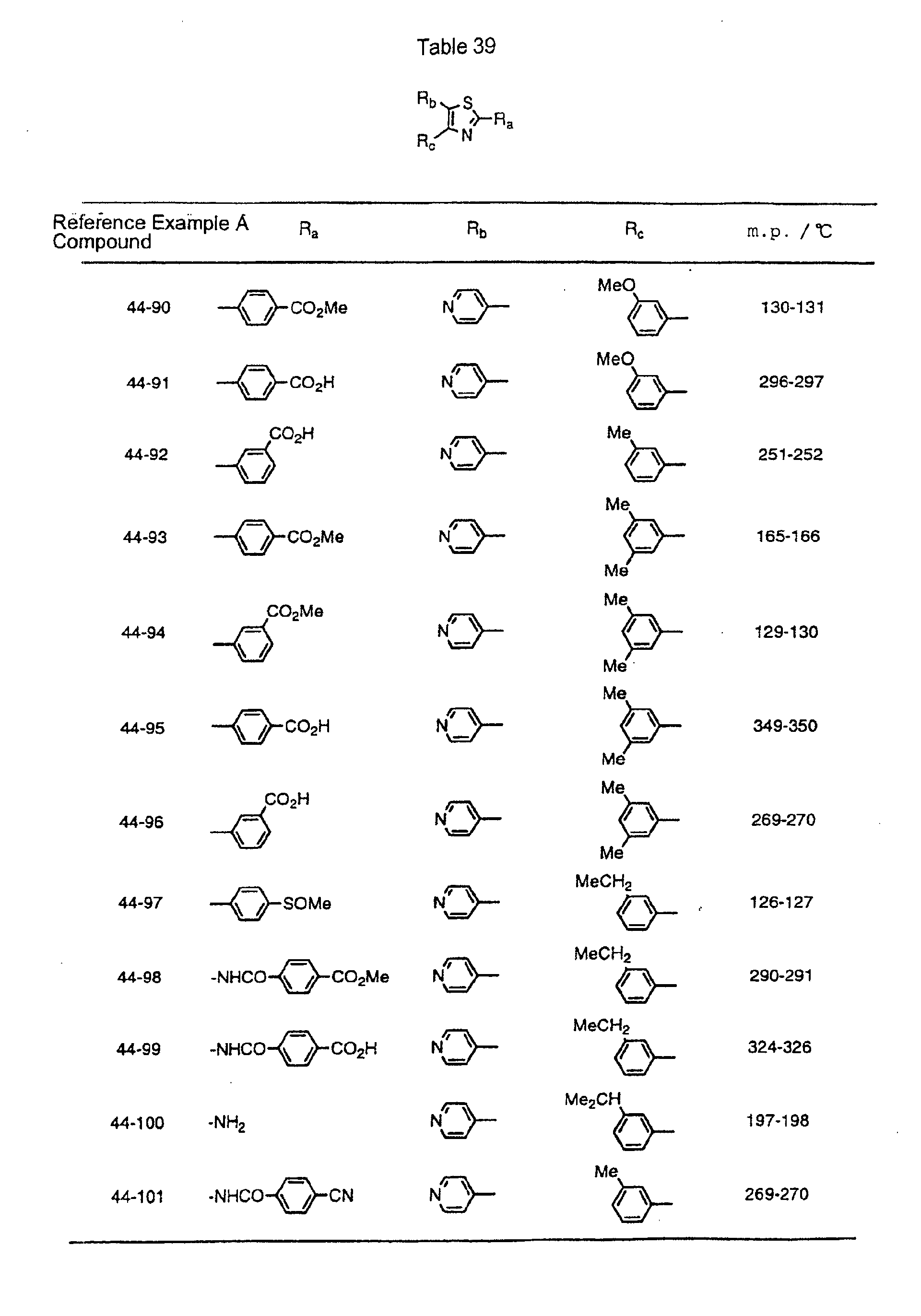

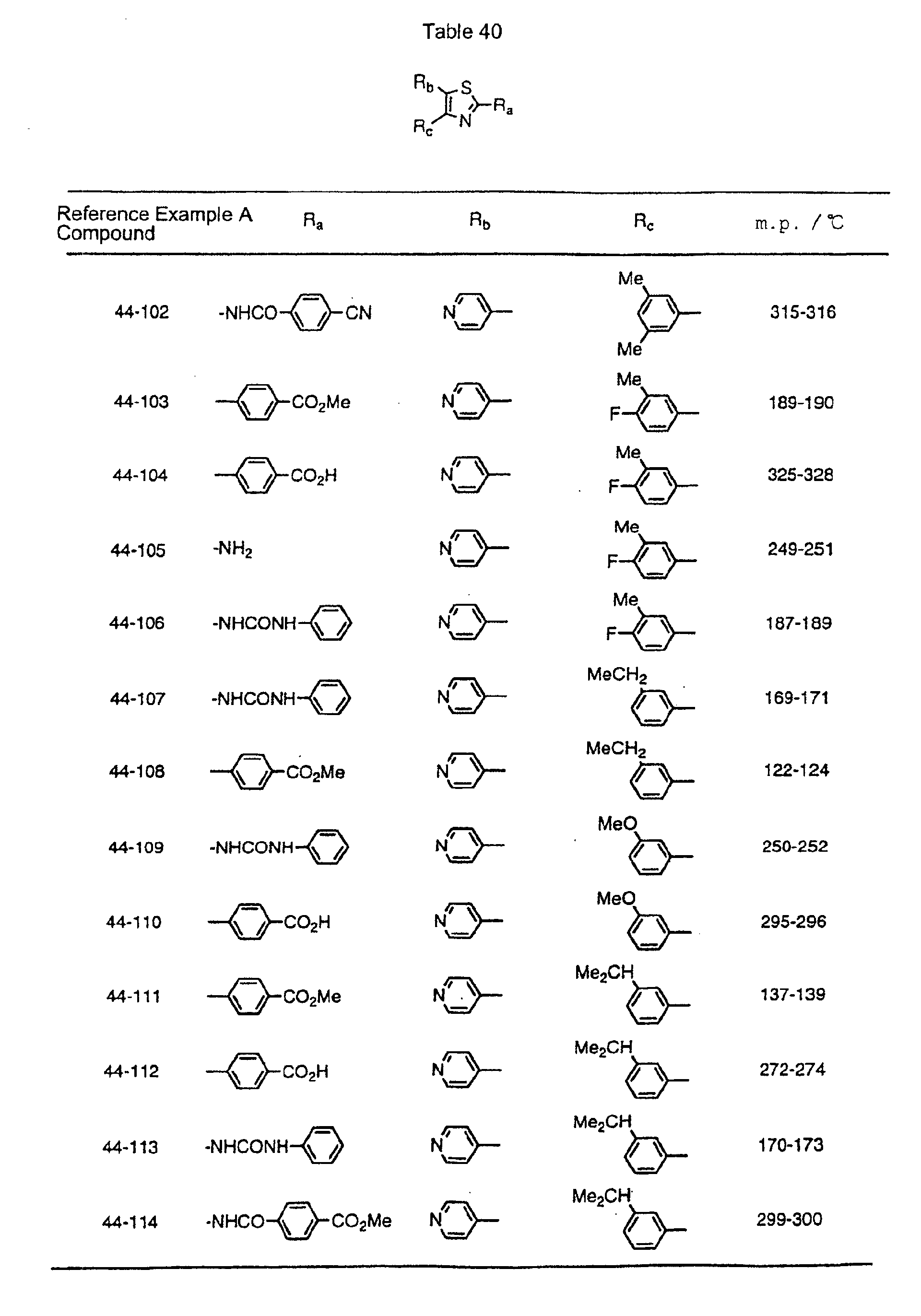

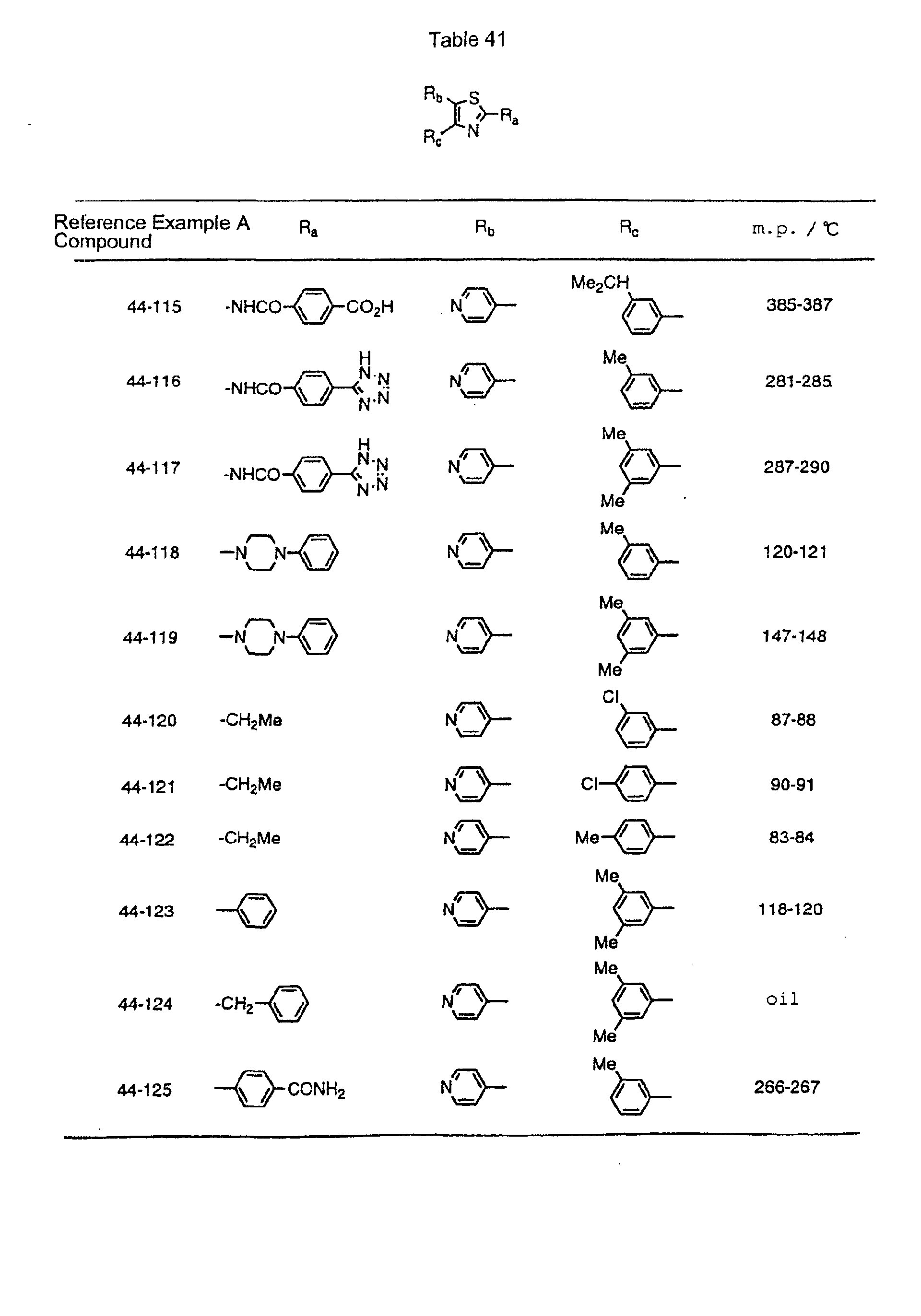

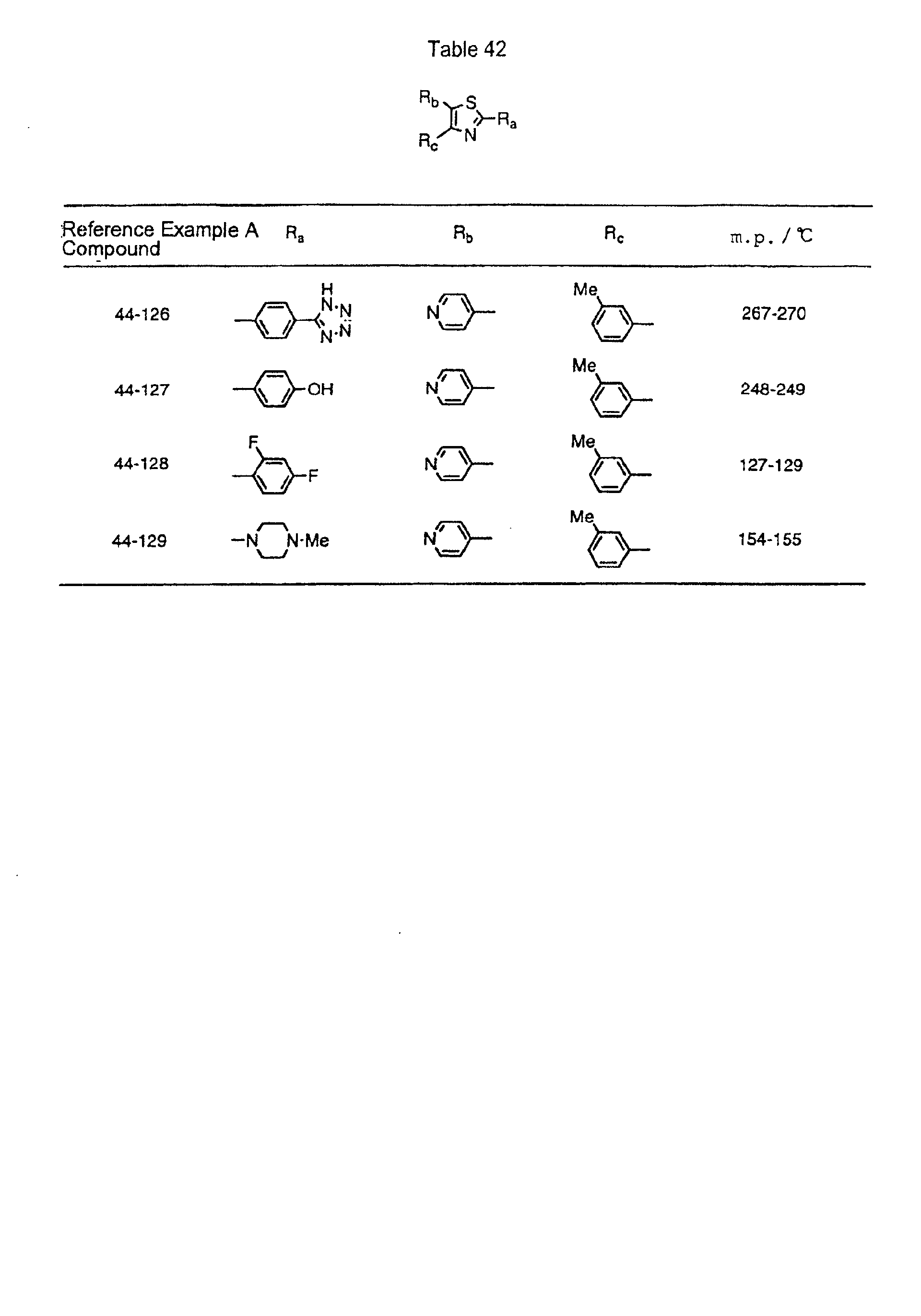

### Reference Example A 44-1

### 4-(4-fluorophenyl)-2-phenyl-5-(4-pyridyl)-1,3-thiazole

A solution of 2-bromo-1-(4-fluorophenyl)-2-(4-pyridyl)ethanone hydrobromide (1.6 g, 4.1 mmol) and thiobenzamide (0.57 g, 4.2 mmol) in N,N-dimethylformamide (5 mL) was stirred at room temperature for 14 h. To the reaction mixture was poured aqueous sodium hydrogencarbonate solution and the precipitated solid was collected by filtration. The obtained solid was washed with water and dried. The crude crystals were recrystallized from ethyl acetate to give the title compound (0.27 g, yield 19%).
melting point: 135-137°C.

The proton nuclear magnetic resonance spectrum of the aforementioned Reference Example A 44 is shown in the following Table 43.

**Table 43**

| Reference Example A Compound No. | Proton Nuclear Magnetic Resonance Spectrum |
|---|---|
| 44-49 | ¹H-NMR (CDCl₃) δ : 2.34 (3H, s), 2.70 (3H, s), 7.14-7.38 (8H, m), 7.46 (1H, s), 7.81 (1H, ddd, J= 6.6, 1.8, 1.1 Hz), 8.56 (2H, d, J= 6.0 Hz). |
| 44-74 | ¹H-NMR (CDCl₃) δ : 2.04-2.26 (8H, m), 2.79 (2H, t, J= 7.5 Hz), 3.08 (2H, t, J= 7.6 Hz), 6.97 (1H, s), 7.08 (2H, s), 7.17-7.35 (7H, m), 8.50 (2H, dd, J= 4.6, 1.8 Hz). |
| 44-75 | ¹H-NMR (CDCl₃) δ : 2.27 (6H, s), 3.13-3.23 (2H, m), 3.31-3.41 (2H, m), 6.98 (1H, s), 7.08 (2H, s), 7.19 (2H, dd, J= 4.5, 1.7 Hz), 7.24-7.37 (5H, m), 8.50 (2H, dd, J= 4.5, 1.7 Hz). |
| 44-76 | ¹H-NMR (CDCl₃) δ : 0.98 (3H, t, J= 7.3 Hz), 1.43-1.55 (2H, m), 1.76-1.88 (2H, m), 2.26 (6H, m), 3.05 (2H, t, J= 7.7 Hz), 6.97 (1H, s), 7.08 (2H, s), 7.21 (2H, dd, J= 4.6, 1.8 Hz), 8.50 (2H, dd, J= 4.6, 1.8 Hz). |
| 44-77 | ¹H-NMR (CDCl₃) δ : 0.90-0.97 (3H, m), 1.38-1.49 (4H. m), 1.78-1.89 (2H, m), 2.26 (6H, s), 3.04 (2H, t, J= 7.9 Hz), 6.97 (1H, s), 7.08 (2H, s), 7.21 (2H, dd, J= 4.5, 1.8 Hz), 8.50 (2H, dd, J= 4.5, 1.8 Hz). |
| 44-124 | ¹H-NMR (CDCl₃) δ : 2.27 (6H, s), 4.38 (2H, s), 6.99 (1H, s), 7.10 (2H, s), 7.16 (2H, dd, J= 4.9, 1.6 Hz), 7.34-7.41 (5H, m), 8.47 (2H, dd, J=4.9, 1.6 Hz). |

### Reference Example A 45

In accordance with Reference Example A 21, the following Reference Example A compound 45 was synthesized using pivalonitrile instead of 4-methylthiobenzonitrile.
Reference Example A compound 45: thiopivaloamide
melting point: 117-119°C.

### Reference Example A 46

In accordance with the methods described in Reference Examples A 8 to 12, Reference Example A 44-1, JP-A-61-10580 and USP 4,612,321, Reference Example A compounds 46-1 to 46-7 shown in the following Table 44 were synthesized.

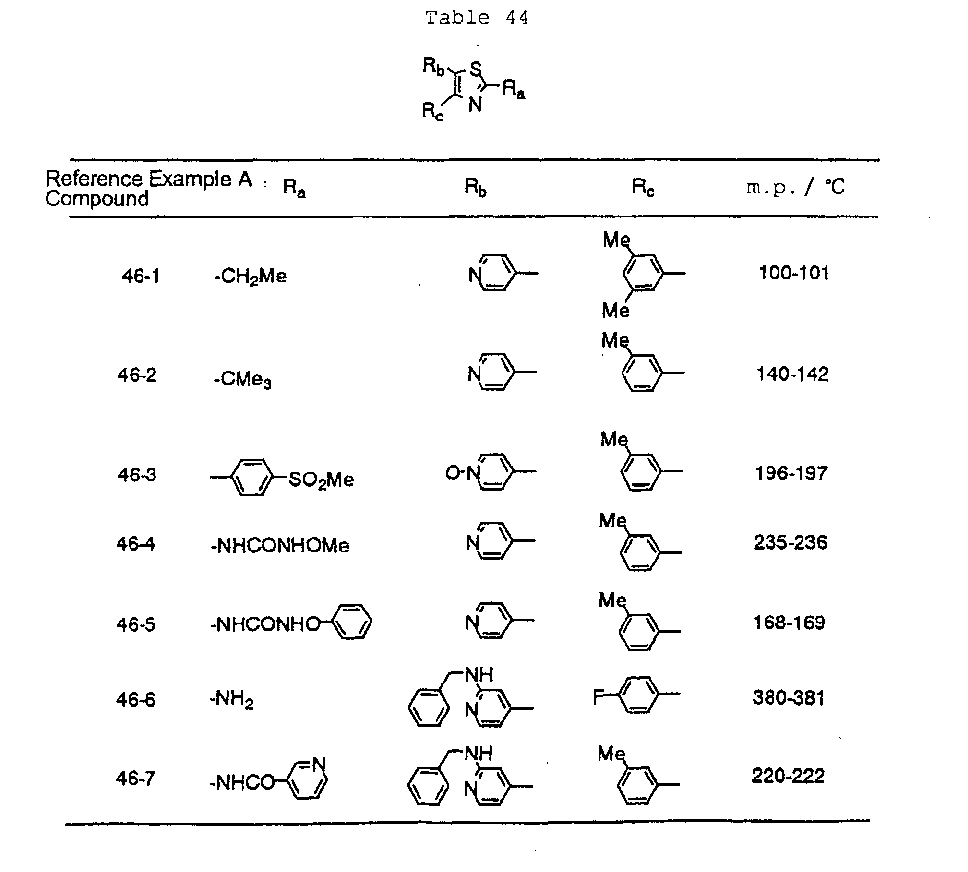

### Reference Example B1

| | | |
|---|---|---|
| (1) | Reference Example A compound 23-313 | 10.0 mg |
| (2) | lactose | 60.0 mg |
| (3) | cornstarch | 35.0 mg |
| (4) | gelatin | 3.0 mg |
| (5) | magnesium stearate | 2.0 mg |

A mixture of Reference Example A compound 23-313 (10.0 mg), lactose (60.0 mg) and cornstarch (35.0 mg) is granulated using 10% aqueous gelatin solution (0.03 ml, 3.0 mg as gelatin) and passing through a 1 mm mesh sieve. The granules are dried at 40°C and passed through the sieve again. The granules thus obtained are mixed with magnesium stearate (2.0 mg) and compressed. The obtained core tablet is coated with sugar coating made of an aqueous suspension of sucrose, titanium dioxide, talc and gum arabic. The coated tablet is polished with bee wax to give a coated tablet.

### Reference Example B2

| | | |
|---|---|---|
| (1) | Reference Example A compound 23-313 | 10.0 mg |
| (2) | lactose | 70.0 mg |
| (3) | cornstarch | 50.0 mg |
| (4) | soluble starch | 7.0 mg |
| (5) | magnesium stearate | 3.0 mg |

Reference Example A compound 23-313 (10.0 mg) and magnesium stearate (3.0 mg) are granulated using an aqueous solution (0.07 ml) of soluble starch (7.0 mg as soluble starch), dried and mixed with lactose (70.0 mg) and cornstarch (50.0 mg). The mixture is compressed to give tablets.

### Reference Example B3

| | | |
|---|---|---|
| (1) | Reference Example A compound 23-313 | 5.0 mg |
| (2) | sodium chloride | 20.0 mg |
| (3) | distilled water to total | 2 ml |

Reference Example A compound 23-313 (5.0 mg) and sodium chloride (20.0 mg) are dissolved in distilled water and water is added to make the total amount 2.0 ml. The solution is filtrated and aseptically filled in a 2 ml ampoule. The ampoule is sterilized and sealed to give a solution for injection.

### Reference Example B4

| | | |
|---|---|---|
| (1) | Reference Example A compound 23-331 | 10.0 mg |
| (2) | lactose | 60.0 mg |
| (3) | cornstarch | 35.0 mg |
| (4) | gelatin | 3.0 mg |
| (5) | magnesium stearate | 2.0 mg |

A mixture of Reference Example A compound 23-331 (10.0 mg), lactose (60.0 mg) and cornstarch (35.0 mg) is granulated using 10% aqueous gelatin solution (0.03 ml, 3.0 mg as gelatin) and passing through a 1 mm mesh sieve. The granules are dried at 40°C and passed through the sieve again. The granules thus obtained are mixed with magnesium stearate (2.0 mg) and compressed. The obtained core tablet is coated with sugar coating made of an aqueous suspension of sucrose, titanium dioxide, talc and gum arabic. The coated tablet is polished with bee wax to give a coated tablet.

### Reference Example B5

| | | |
|---|---|---|
| (1) | Reference Example A compound 23-331 | 10.0 mg |
| (2) | lactose | 70.0 mg |
| (3) | cornstarch | 50.0 mg |
| (4) | soluble starch | 7.0 mg |
| (5) | magnesium stearate | 3.0 mg |

Reference Example A compound 23-331 (10.0 mg) and magnesium stearate (3.0 mg) are granulated using an aqueous solution (0.07 ml) of soluble starch (7.0 mg as soluble starch), dried and mixed with lactose (70.0 mg) and cornstarch (50.0 mg). The mixture is compressed to give tablets.

### Reference Example B6

| | | |
|---|---|---|
| (1) | Reference Example A compound 23-331 | 5.0 mg |
| (2) | sodium chloride | 20.0 mg |
| (3) | distilled water to total | 2 ml |

Reference Example A compound 23-331 (5.0 mg) and sodium chloride (20.0 mg) are dissolved in distilled water and water is added to make the total 2.0 ml. The solution is aseptically filtered and filled into a 2 ml ampoule. The ampoule is sterilized and sealed to give a solution for injection.

### Reference Example C1:

The genetic manipulations described below were according to a method described in the book (Maniatis et al., Molecular Cloning, Cold Spring Harbor Laboratory, 1989) or methods described in the protocols attached to the reagents.

### (1) Cloning of human p38 MAP kinase gene and preparation of recombinant baculovirus

Cloning of human p38 MAP kinase gene was performed by a PCR method using a primer set P38-U: 5'-ACCACTCGAGATGGACTACAAGGACGACGATGACAAGTCTCAGGAGAGGCCCACGTTCTACC -3' [SEQ ID NO:1] and PAG-L: 5'-ACCCGGTACCACCAGGTGCTCAGGACTCCATCTCT-3' [SEQ ID NO:2] made by the use of kidney cDNA (Toyobo, QUICK-Clone cDNA) as a template and referring to the base sequence of p38 MAP kinase gene reported by Han et al. (Science 265 (5173), 808-811 (1994)).

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solutions, and 10 µL sterile distilled water were mixed. As the upper mixed solution, 1 µL human cardiac cDNA (1 ng/mL) as a template, 3 µL 10×LA PCR Buffer, 1 µL 2.5 mM dNTP solution, 0.5 µL TaKaRa LA Taq DNA polymerase (Takara Shuzo), and 24.5 µL sterile distilled water were mixed. One AmpliWax PCR Gem 100 (Takara Shuzo) was added to the prepared lower mixed solution and the mixture was treated at 70°C for 5 min and for 5 min in an ice and, thereafter, the upper mixed solution was added to prepare a reaction solution for PCR. A tube containing the reaction solution was set at a thermal cycler (Perkin Elmer), which was treated at 95°C for 2 min. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.1 kb DNA fragment containing p38 MAP kinase gene was recovered from the gel and, thereafter, which was inserted into pT7Blue-T vector (Takara Shuzo) to make the plasmid pHP38.

The 4.8 kb XhoI-KpnI fragment of the plasmid pFASTBAC1 (CIBCOBRL) and the 1.1 kb XhoI-Kpn fragment of the above plasmid pHP38 were ligated to make the plasmid pFBHP38.

The plasmid pFBHP38 and BAC-TO-BAC Baculovirus Expression System (GIBCOBRL) were used to prepare the recombinant baculovirus virusstock BAC-HP38.

### (2) Cloning of human MKK3 gene and preparation of recombinant baculovirus

Cloning of human MKK3 gene was performed by a PCR method using a primer set MKK-U: 5'-ACAAGAATTCATAACATATGGCTCATCATCATCATCATCATTCCAAGCCACCCGCACCCAA-3' [SEQ ID NO:3] and MKK-L: 5'-TCCCGTCTAGACTATGAGTCTTCTCCCAGGAT-3' [SEQ ID NO:4] made by the use of kidney cDNA (Toyobo, QUICK-Clone cDNA) as a template and referring to the base sequence of MKK3 gene reported by Derijard, B. et al., Science 267 (5198), 682-685 (1995).

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solutions, and 10 µL sterile distilled water were mixed. As the upper mixed solution, 1 µL human kidney cDNA (1 ng/mL) as a template, 3 µL 10×LA PCR Buffer, 1 µL 2.5 mM dNTP solution, 0.5 µL TaKaRa LA Taq DNA polymerase (Takara Shuzo) and 24.5 µL sterile distilled water were mixed. One AmpliWax PCR Gem 100 (Takara Shuzo) was added to the prepared lower mixed solution and the mixture was treated at 70°C for 5 minutes and for 5 minutes in an ice and, thereafter, the upper mixed solution was added to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.0 kb DNA fragment containing MKK3 gene was recovered from the gel and, thereafter, which was inserted into pT7Blue-T vector (Takara Shuzo) to make the plasmid pHMKK3.

In order to mutate MKK3 into a constitutive active form (from Ser to Glu at 189 position, from Thr to Glu at position 193), a primer set SER-U: 5'-GGCTACTTGGTGGACGAGGTGGCCAAGGAGATGGATGCCGGCTGC-3' [SEQ ID NO:5] and SER-L: 5'-GCAGCCGGCATCCATCTCCTTGGCCACCTCGTCCACCAAGTAGCC-3' [SEQ ID NO:6] was used to introduce a mutation by QuikChange Site-Directed Mutagenesis Kit (Stratagene), to obtain pcaMKK3.

4.8 kb EcoRI-XbaI fragment of the plasmid pFASTBAC1 (CIBCOBRL) and the 1.0 kb EcoRI-XbaI fragment of the above plasmid pcaMKK 3 were ligated to make the plasmid pFBcaMKK3.

The plasmid pFBcaMKK3 and BAC-TO-BAC Baculovirus Expression System (GIBCOBRL) were used to prepare the recombinant baculovirus virusstock BAC-caMKK3.

### (3) Preparation of active form p38 MAP kinase

The Sf-21 cells were seeded on 100 mL Sf-900II SFM medium (GIBCOBRL) to 1×10⁶ cells/mL and cultured at 27°C for 24 hours. After each 0.2 mL of the virusstock BAC-HP38 and BAC-caMKK3 of recombinant baculovirus were added, the culturing was further performed for 48 hours. After the cells were separated from the culturing solution by centrifugation (3000 rpm, 10 min), the cells were washed twice with PBS. After the cells were suspended in 10 ml Lysis buffer (25 mM HEPES (pH 7.5), 1% Triton X, 130 mM NaCl, 1 mM EDTA, 1 mM DTT, 25 mM β-glycerophosphate, 20 mM leupeptin, 1 mM APMSF, 1 mM Sodium orthovanadate), the cells were lysed by treating twice in a homogenizer (POLYTRON) at 20000 rpm for 2 minutes. From the supernatant obtained by centrifugation (40000 rpm, 45 minutes), active form p38 MAP kinase was purified using Anti-FLAG M2 Affinity Gel (Eastman Chemical).

### (4) Measurement of the enzyme inhibitory activity

2.5 µL of a test compound dissolved in DMSO was added to 37.5 µL reaction solution (25 mM HEPES (pH 7.5), 10 mM Magnesium Acetate) containing 260 ng active form p38 MAP kinase and 1 µg Myelin Basic Protein, which was maintained at 30°C for 5 minutes. The reaction was initiated by adding 10 µL ATP solution (2.5 µM ATP, 0.1 µCi [g-³²P]ATP). After the reaction was performed at 30°C for 60 minutes, the reaction was stopped by adding 50 µL 20% TCA solution. After the reaction solution was allowed to stand at 0°C for 20 minutes, an acid insoluble fraction was transferred to GF/C filter (Packard Japan) using Cell Harvester (Packard Japan) and washed with 250 mM H₃PO₄. After drying at 45°C for 60 minutes, 40 µL Microscint 0 (Packard Japan) was added and the radioactivity was measured with a TopCount (Packard Japan). The concentration (IC₅₀ value) of the test compound necessary for inhibiting uptake of ³²P into an acid insoluble fraction by 50% was calculated with PRISM 2.01 (Graphpad Software). The results are shown in Table 45.

**Table 45**

| Reference Example A Compound No. | IC₅₀ (µM) |
|---|---|
| 13-14 | 0.086 |
| 13-15 | 0.081 |
| 13-16 | 0.060 |
| 13-70 | 0.026 |
| 13-74 | 0.63 |

### Experimental Example C2

### Measurement of inhibiting activity of TNF-α production

After THP-1 cells which had been cultured on PRMI 1640 medium (manufactured by Life Technologies, Inc.) containing 1% inactivated bovine fetal serum (manufactured by Life Technologies, Inc., U.S.A.) and 10 mM HEPES (pH 7.5) seeded on a 96-well plate to 1×10⁵ cells/well, 1 µL test compound dissolved in DMSO was added. After incubation at 37°C for 1 hour in a CO₂ incubator, LPS (Wako Pure Chemicals) was added to the final concentration 5 µg/mL. After cultured at 37°C for 4 hours in a CO₂ incubator, the supernatant was obtained by centrifugation. The concentration of TNF-α in the supernatant was measured by ELISA (R&D Systems, Quantikine Kit). The concentration (IC₅₀ value) of the test compound necessary for inhibiting TNF-α production by 50% was calculated using PRIMS 2.01 (Graphpad Software). The results are shown in Table 46.

**Table 46**

| Reference Example A Compound No. | IC₅₀ (µM) |
|---|---|
| 13-16 | 0.14 |
| 13-70 | 0.18 |
| 23-60 | 0.046 |

From the above results, it can be seen that Compound (I) has an excellent inhibitory activity against p38 MAP kinase and TNF-α production.

The following Reference Example D can be produced according to Examples of WO00/64894.

### Reference Example D 1

[4-(3,5-dimethylphenyl)-5-(2-phenylmethyloxy-4-pyridyl)-1,3-thiazol-2-yl]amine

### Reference Example D 2

N-[4-[2-benzoylamino-4-(4-methoxyphenyl)-1,3-thiazol-5-yl]-2-pyridyl]benzamide

### Reference Example D 3

N-[4-(4-methoxypheny)-5-[2-[(3-pyridylcarbonylamino)]-4-pyridyl]-1,3-thiazol-2-yl]nicotinamide

### Reference Example D 4

N-[4-[2-amino-4-(4-methoxyphenyl)-1,3-thiazol-5-yl]-2-pyridyl]benzamide

### Reference Example D 5

N-[4-[2-amino-4-(3,5-dimethylphenyl)-1,3-thiazol-5-yl]-2-pyridyl]benzamide

### Reference Example D 6

N-[4-[2-amino-4-(3,5-dimethylphenyl)-1,3-thiazol-5-yl]-2-pyridyl]benzylamine

### Reference Example D 7

N-[4-[2-amino-4-(3,5-dimethylphenyl)-1,3-thiazol-5-yl]-2-pyridyl]benzamide hydrochloride

### Reference Example D 8

N-[4-[2-amino-4-(3,5-dimethylphenyl)-1,3-thiazol-5-yl]-2-pyridyl]benzylamine dihydrochloride

The structures of the compounds obtained in Reference Examples D 1 to 6 are shown below: 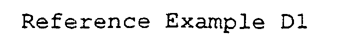 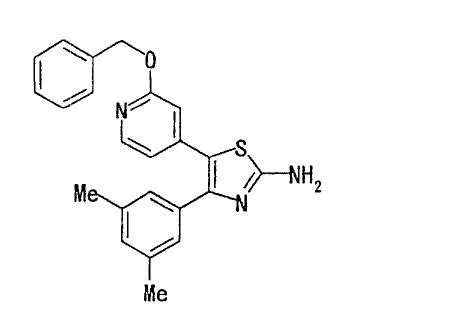 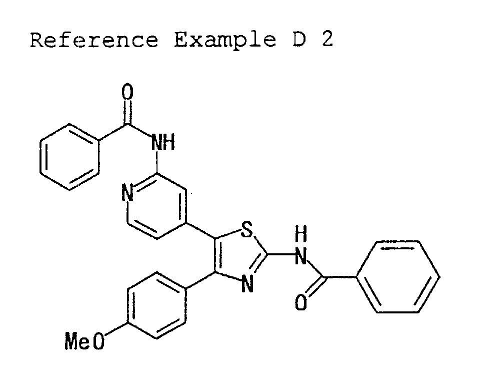 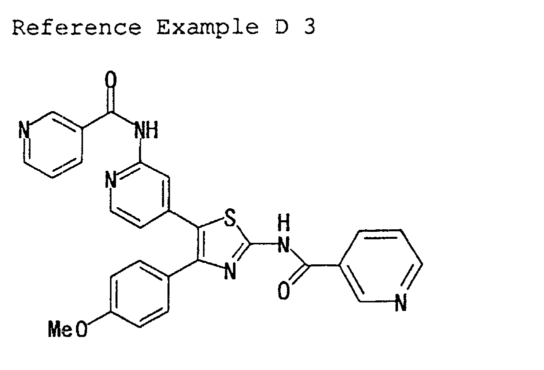 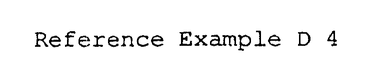 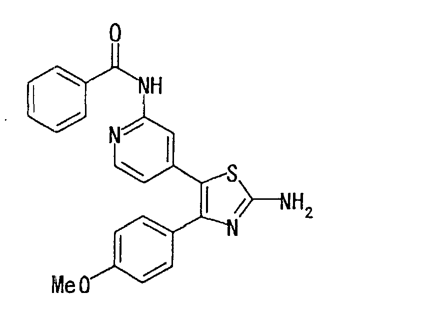 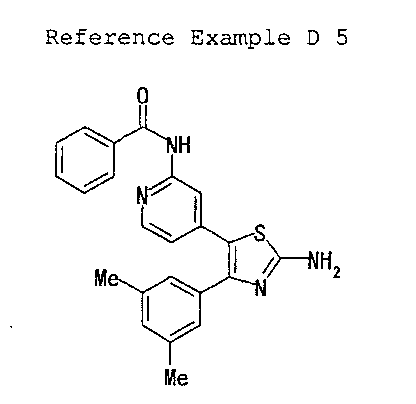 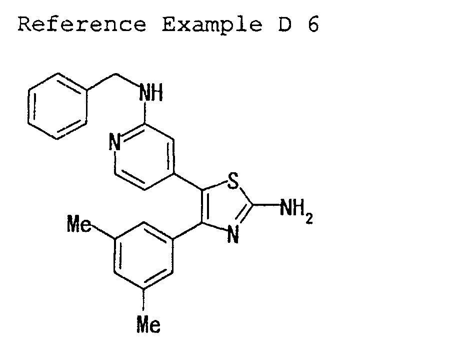
Reference Example D 9: N-[5-[2-benzoylamino-4-pyridyl)-4-(3,5-dimethylphenyl)-1,3-thiazol-2-yl]acetamide
Reference Example D 10: N-[5-(2-benzylamino-4-pyridyl)-4-(3,5-dimethylphenyl)-1,3-thiazol-2-yl]acetamide
Reference Example D 11: N-[4-[4-(4-methoxyphenyl)-2-methylamino-1,3-thiazol-5-yl]-2-pyridyl]benzamide
Reference Example D 12: N-[4-[2-amino-4-(3-methylphenyl)-1,3-thiazol-5-yl] -2-pyridyl]benzamide
Reference Example D 13: N-[4-[4-(4-methoxyphenyl)-2-methyl-1,3-thiazol-5-yl]-2-pyridyl]benzamide
Reference Example D 14: N-[4-[2-[(4-fluorophenyl)-4-(3-methylphenyl)-1,3-thiazol-5-yl]-2-pyridyl]phenylacetamide
Reference Example D 15:
   Reference Example D compound 15-1: N-[4-[4-(4-methoxyphenyl)-2-methyl-1,3-thiazol-5-yl]-2-pyridyl]phenylacetamide
   Reference Example D compound 15-2: N-[4-[2-ethyl-4-(3-methylphenyl)-1,3-thiazol-5-yl]-2-pyridyl]phenylacetamide
   Reference Example D compound 15-3: N-[4-[4-(3-methylphenyl)-2-propyl-1,3-thiazol-5-yl]-2-pyridyl]phenylacetamide
   Reference Example D compound 15-4: N-[4-[2-butyl-4-(3-methylphenyl)-1,3-thiazol-5-yl]-2-pyridyl]phenylacetamide
   Reference Example D compound 15-5: N-[4-[2-(2-chlorophenyl)-4-(3-methylphenyl)-1,3-thiazol-5-yl]-2-pyridyl]phenylacetamide
   Reference Example D compound 15-6: N-[4-[4-(3-methylphenyl)-2-(4-methylthiophenyl)-1,3-thiazol-5-yl]-2-pyridyl]phenylacetamide
Reference Example D 16:
   Reference Example D compound 16-1: N-[4-[2-ethyl-4-(3-methylphenyl)-1,3-thiazol-5-yl]-2-pyridyl]benzamide
   Reference Example D compound 16-2: N-[4-[2-ethyl-4-(3-methylphenyl)-1,3-thiazol-5-yl]-2-pyridyl]-3-phenylpropionamide
   Reference Example D compound 16-3: N-[4-[2-ethyl-4-(3-methylphenyl]-1,3-thiazol-5-yl]-2-pyridyl]-3-(4-methoxyphenyl)propionamide
   Reference Example D compound 16-4: N-[4-[2-ethyl-4-(3-methylphenyl)-1,3-thiazol-5-yl]-2-pyridyl]-3-(4-fluorophenyl)propionamide
   Reference Example D compound 16-5: N-[4-[2-ethyl-4-(3-methylphenyl)-1,3-thiazol-5-yl]-2-pyridyl]-4-phenylbutyramide
   Reference Example D compound 16-6: N-[4-[2-ethyl-4-(3-methylphenyl)-1,3-thiazol-5-yl]-2-pyridyl]-5-phenylvaleramide
   Reference Example D compound 16-7: N-[4-[4-(3-methylphenyl)-2-propyl-1,3-thiazol-5-yl]-2-pyridyl]benzamide
   Reference Example D compound 16-8: N-[4-[4-(3-methylphenyl)-2-propyl-1,3-thiazol-5-yl]-2-pyridyl]-3-phenylpropionamide
   Reference Example D compound 16-9: N-[4-[2-butyl-4-(3-methylphenyl)-1,3-thiazol-5-yl]-2-pyridyl]benzamide
   Reference Example D compound 16-10: N-[4-[2-butyl-4-(3-methylphenyl)-1,3-thiazol-5-yl]-2-pyridyl]-3-phenylpropionamide
   Reference Example D compound 16-11: N-[4-[2-(4-fluorophenyl)-4-(3-methylphenyl)-1,3-thiazol-5-yl]-2-pyridyl]benzamide
   Reference Example D compound 16-12: N-[4-[2-(4-fluorophenyl)-4-(3-methylphenyl)-1,3-thiazol-5-yl]-2-pyridyl]-3-phenylpropionamide
   Reference Example D compound 16-13: N-[4-[2-(2-chlorophenyl)-4-(3-methylphenyl)-1,3-thiazol-5-yl]-2-pyridyl]benzamide
   Reference Example D compound 16-14: N-[4-[2-(2-chlorophenyl)-4-(3-methylphenyl)-1,3-thiazol-5-yl]-2-pyridyl]-3-phenylpropionamide
   Reference Example D compound 16-15: N-[4-[4-(3-methylphenyl)-2-(4-methylthiophenyl)-1,3-thiazol-5-yl]-2-pyridyl]benzamide
   Reference Example D compound 16-16: N-[4-[4-(3-methylphenyl)-2-(4-methylthiophenyl)-1,3-thiazol-5-yl]-2-pyridyl]-3-phenylpropionamide
   Reference Example D compound 16-17: N-[4-[4-(3-methylphenyl)-2-(4-methylthiophenyl)-1,3-thiazol-5-yl]-2-pyridyl]-2-thiophenecarboxamide
   Reference Example D compound 16-18: N-[4-[4-(3-methylphenyl)-2-(4-methylthiophenyl)-1,3-thiazol-5-yl]-2-pyridyl]-2-naphthamide
Reference Example D 17: N-[4-[2-ethyl-4-(3-methylphenyl)-1,3-thiazol-5-yl]-2-pyridyl]-N-methylphenylacetamide
Reference Example D 18: N-[4-[2-ethyl-4-(3-methylphenyl)-1,3-thiazol-5-yl]-2-pyridyl]-N-methyl-3-phenylpropionamide
Reference Example D 19:
   Reference Example D compound 19-1: N-benzyl-N-[4-[4-(4-methoxyphenyl)-2-methyl-1,3-thiazol-5-yl]-2-pyridyl]amine
   Reference Example D compound 19-2: N-benzyl-N-[4-[2-ethyl-4-(3-methylphenyl)-1,3-thiazol-5-yl]-2-pyridyl]amine
   Reference Example D compound 19-3: N-[4-[2-ethyl-4-(3-methylphenyl)-1,3-thiazol-5-yl]-2-pyridyl]-N-(2-phenylethyl)amine
   Reference Example D compound 19-4: N-[4-[2-ethyl-4-(3-methylphenyl)-1,3-thiazol-5-yl]-2-pyridyl]-N-(3-phenylpropyl)amine
   Reference Example D compound 19-5: N-benzyl-N-[4-[4-(3-methylphenyl)-2-propyl-1,3-thiazol-5-yl]-2-pyridyl]amine
   Reference Example D compound 19-6: N-[4-[4-(3-methylphenyl)-2-propyl-1,3-thiazol-5-yl]-2-pyridyl]-N-(2-phenylethyl)amine
   Reference Example D compound 19-7: N-[4-[4-(3-methylphenyl)-2-propyl-1,3-thiazol-5-yl]-2-pyridyl]-N-(3-phenylpropyl)amine
   Reference Example D compound 19-8: N-benzyl-N-[4-[2-butyl-4-(3-methylphenyl)-1,3-thiazol-5-yl]-2-pyridyl]amine
   Reference Example D compound 19-9: N-[4-[2-butyl-4-(3-methylphenyl)-1,3-thiazol-5-yl]-2-pyridyl]-N-(2-phenylethyl)amine
   Reference Example D compound 19-10: N-[4-[2-butyl-4-(3-methylphenyl)-1,3-thiazol-5-yl]-2-pyridyl]-N-(3-phenylpropyl)amine
   Reference Example D compound 19-11: N-benzyl-N-[4-[2-(4-fluorophenyl)-4-(3-methylphenyl)-1,3-thiazol-5-yl]-2-pyridyl]amine
   Reference Example D compound 19-12: N-[4-[2-(4-fluorophenyl)-4-(3-methylphenyl)-1,3-thiazol-5-yl]-2-pyridyl]-N-(2-phenylethyl)amine
   Reference Example D compound 19-13: N-[4-[2-(4-fluorophenyl)-4-(3-methylphenyl)-1,3-thiazol-5-yl]-2-pyridyl]-N-(3-phenylpropyl)amine
   Reference Example D compound 19-14: N-benzyl-N-[4-[2-(2-chlorophenyl)-4-(3-methylphenyl)-1,3-thiazol-5-yl]-2-pyridyl]amine
   Reference Example D compound 19-15: N-[4-[2-(2-chlorophenyl)-4-(3-methylphenyl)-1,3-thiazol-5-yl]-2-pyridyl]-N-(2-phenylethyl)amine
   Reference Example D compound 19-16: N-[4-[2-(2-chlorophenyl)-4-(3-methylphenyl)-1,3-thiazol-5-yl]-2-pyridyl]-N-(3-phenylpropyl)amine
   Reference Example D compound 19-17: N-benzyl-N-[4-[4-(3-methylphenyl)-2-(4-methylthiophenyl)-1,3-thiazol-5-yl]-2-pyridyl]amine
   Reference Example D compound 19-18: N-[4-[4-(3-methylphenyl)-2-(4-methylthiophenyl)-1,3-thiazol-5-yl]-2-pyridyl]-N-(2-phenylethyl)amine
   Reference Example D compound 19-19: N-[4-[4-(3-methylphenyl)-2-(4-methylthiophenyl)-1,3-thiazol-5-yl]-2-pyridyl]-N-(3-phenylpropyl)amine
   Reference Example D compound 19-20: N-[4-[4-(3-methylphenyl)-2-(4-methylthiophenyl)-1,3-thiazol-5-yl]-2-pyridyl]-N-(2-naphthylmethyl) amine
Reference Example D 20: N-[4-[4-(3-methylphenyl)-2-(4-methylsulfonylphenyl)-1,3-thiazol-5-yl]-2-pyridyl]benzamide
Reference Example D 21:
   Reference Example D compound 21-1: N-[4-[4-(3-methylphenyl)-2-(4-methylsulfonylphenyl)-1,3-thiazol-5-yl]-2-pyridyl]phenylacetamide
   Reference Example D compound 21-2: N-[4-[4-(3-methylphenyl)-2-(4-methylsulfonylphenyl)-1,3-thiazol-5-yl]-2-pyridyl]-3-phenylpropionamide
   Reference Example D compound 21-3: N-[4-[4-(3-methylphenyl)-2-(4-methylsulfonylphenyl)-1,3-thiazol-5-yl]-2-pyridyl]-2-thiophenecarboxamide
   Reference Example D compound 21-4: N-[4-[4-(3-methylphenyl)-2-(4-methylsulfonylphenyl)-1,3-thiazol-5-yl]-2-pyridyl]-2-naphthamide
   Reference Example D compound 21-5: N-benzyl-N-[4-[4-(3-methylphenyl)-2-(4-methylsulfonylphenyl)-1,3-thiazol-5-yl]-2-pyridyl]amine
   Reference Example D compound 21-6: N-[4-[4-(3-methylphenyl)-2-(4-methylsulfonylphenyl)-1,3-thiazol-5-yl]-2-pyridyl]-N-(3-phenylpropyl)amine
   Reference Example D compound 21-7: N-[4-[4-(3-methylphenyl)-2-(4-methylsulfonylphenyl)-1,3-thiazol-5-yl]-2-pyridyl]-N-(2-naphthylmethyl)amine
Reference Example D 22: N-[4-[2-amino-4-(3-methylphenyl)-1,3-thiazol-5-yl]-2-pyridyl]-N-benzylamine
Reference Example D 23:
   Reference Example D compound 23-1: N-[4-[2-amino-4-(3-methylphenyl)-1,3-thiazol-5-yl]-2-pyridyl]-N-(4-methoxybenzyl)amine
   Reference Example D compound 23-2: N-[4-[2-amino-4-(3-methylphenyl)-1,3-thiazol-5-yl]-2-pyridyl]-N-(3-methoxybenzyl) amine
   Reference Example D compound 23-3: N-[4-[2-amino-4-(3-methylphenyl)-1,3-thiazol-5-yl]-2-pyridyl]-N-(2-methoxybenzyl) amine
   Reference Example D compound 23-4: N-[4-[2-amino-4-(3-methylphenyl)-1,3-thiazol-5-yl]-2-pyridyl]-N-(4-chlorobenzyl)amine
   Reference Example D compound 23-5: N-[4-[2-amino-4-(3-methylphenyl)-1,3-thiazol-5-yl]-2-pyridyl]-N-(3-chlorobenzyl)amine
   Reference Example D compound 23-6: (R)-N-[4-[2-amino-4-(3-methylphenyl)-1,3-thiazol-5-yl]-2-pyridyl]-N-(1-phenylethyl)amine
   Reference Example D compound 23-7: (S)-N-[4-[2-amino-4-(3-methylphenyl)-1,3-thiazol-5-yl]-2-pyridyl]-N-(1-phenylethyl)amine
   Reference Example D compound 23-8: N-[4-[2-amino-4-(3-methylphenyl)-1,3-thiazol-5-yl]-2-pyridyl]-N-benzyl-N-methylamine
Reference Example D 24: N-[4-[2-amino-4-(3-methoxyphenyl)-1,3-thiazol-5-yl]-2-pyridyl]-N-benzylamine
Example 25:
   Reference Example D compound 25-1: N-[4-[4-(3-methylphenyl)-2-(4-methylsulfonylphenyl)-1,3-thiazol-5-yl]-2-pyridyl]-N-(2-phenylethyl)amine
   Reference Example D compound 25-2: N-(4-fluorobenzyl)-N-[4-[4-(3-methylphenyl)-2-(4-methylsulfonylphenyl)-1,3-thiazol-5-yl]-2-pyridyl]amine
   Reference Example D compound 25-3: N-benzyl-N-methyl-N-[4-[4-(3-methylphenyl)-2-(4-methylsulfonylphenyl)-1,3-thiazol-5-yl]-2-pyridyl]amine
   Reference Example D compound 25-4: N-methyl-N-[4-[4-(3-methylphenyl)-2-(4-methylsulfonylphenyl)-1,3-thiazol-5-yl]-2-pyridyl]-N-(2-phenylethyl)amine
   Reference Example D compound 25-5: N-[4-[4-(3-methylphenyl)-2-(4-methylsulfonylphenyl)-1,3-thiazol-5-yl]-2-pyridyl]-N-(2-thienylmethyl)amine
Reference Example D 26: 4-(3-methylphenyl)-2-(4-methylsulfonylphenyl)-5-(2-phenylthio-4-pyridyl)-1,3-thiazole
Reference Example D 27: 5-(2-benzylthio-4-pyridyl)-4-(3-methylphenyl)-2-(4-methylsulfonylphenyl)-1,3-thiazole
Reference Example D 28: 4-(3-methylphenyl)-2-(4-methylsulfonylphenyl)-5-(2-phenylsulfonyl-4-pyridyl)-1,3-thiazole

Compounds prepared in the above Reference Examples D 9 to 28 are shown in Tables 47 to 52.

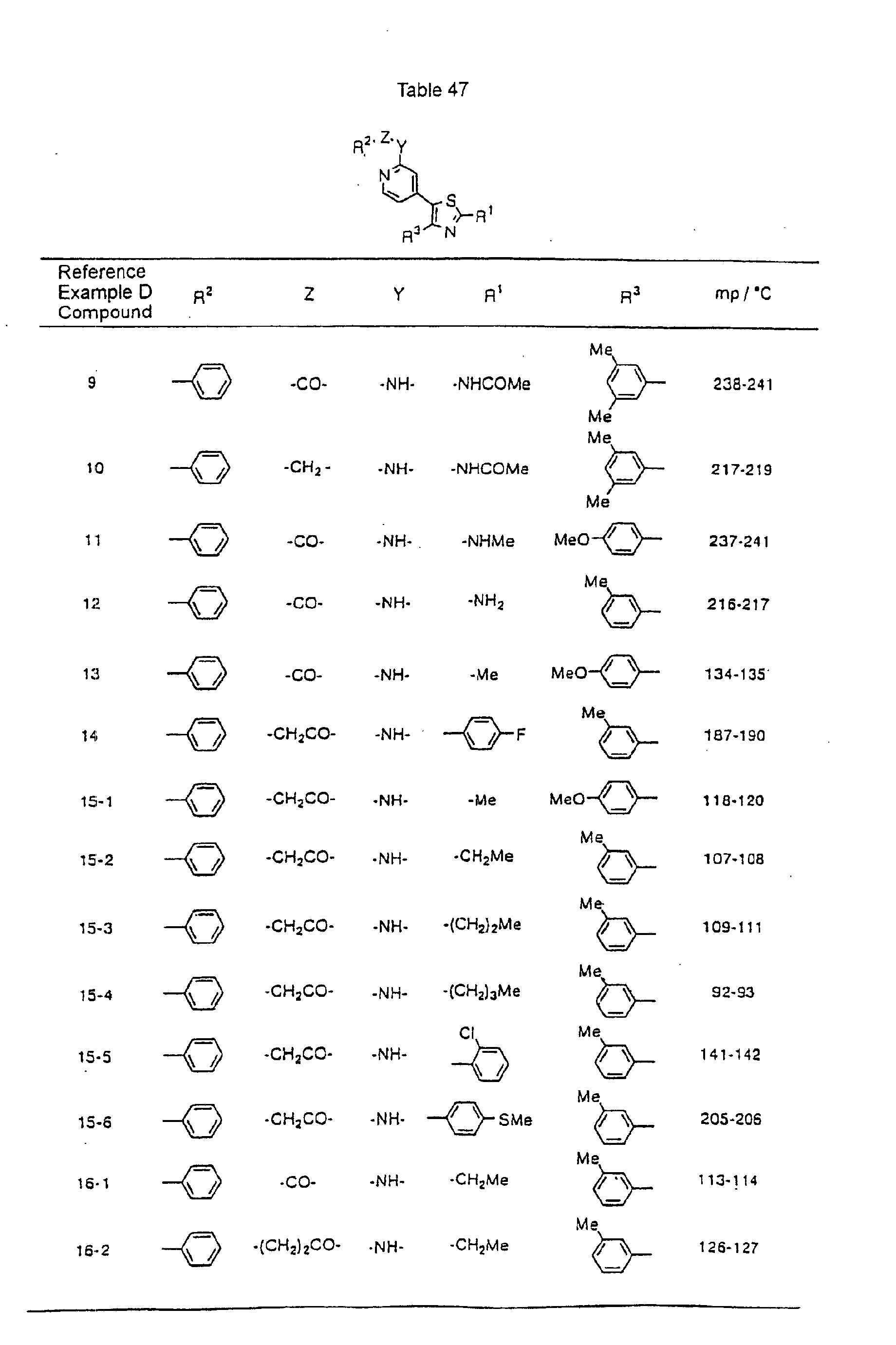

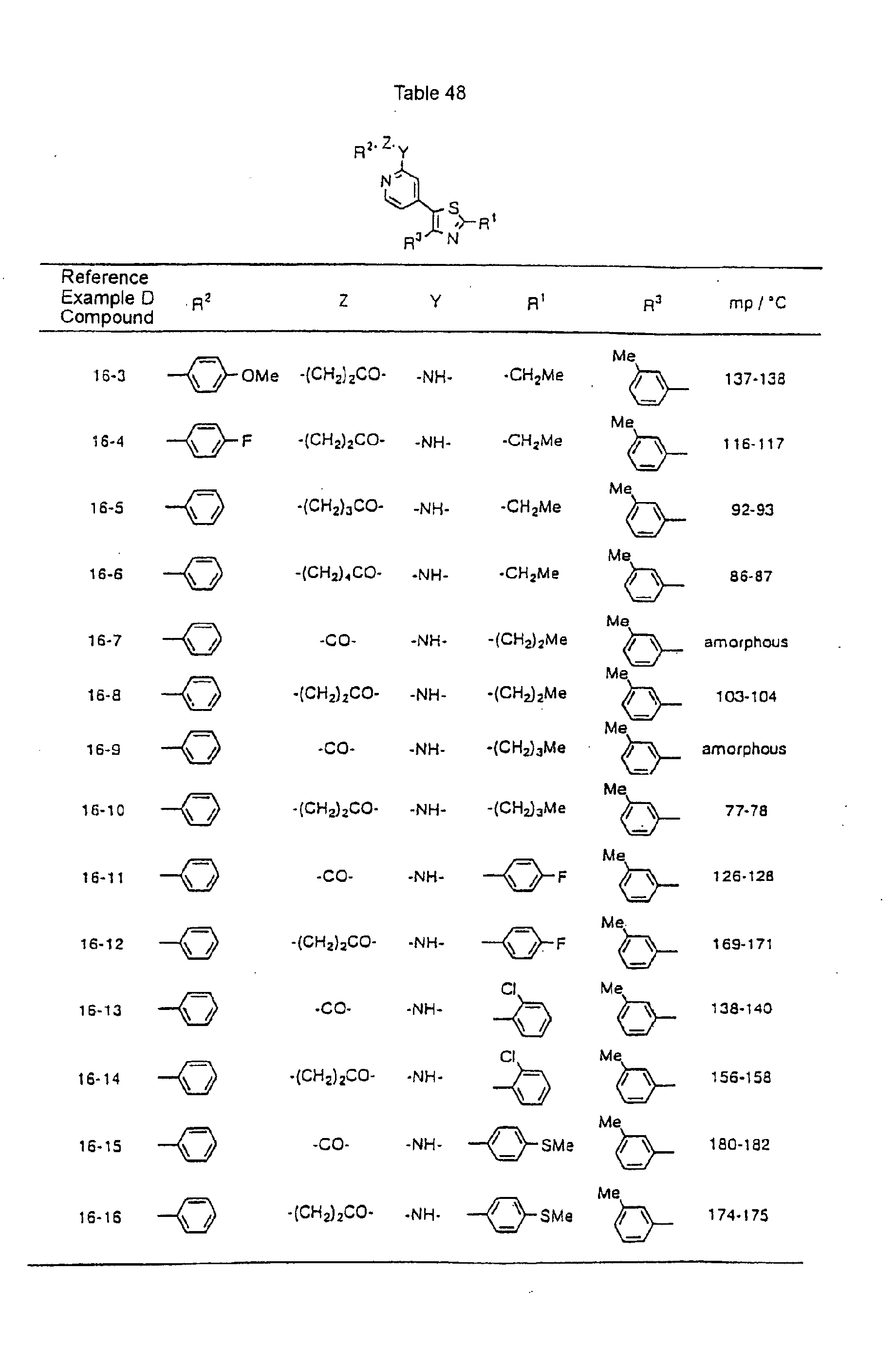

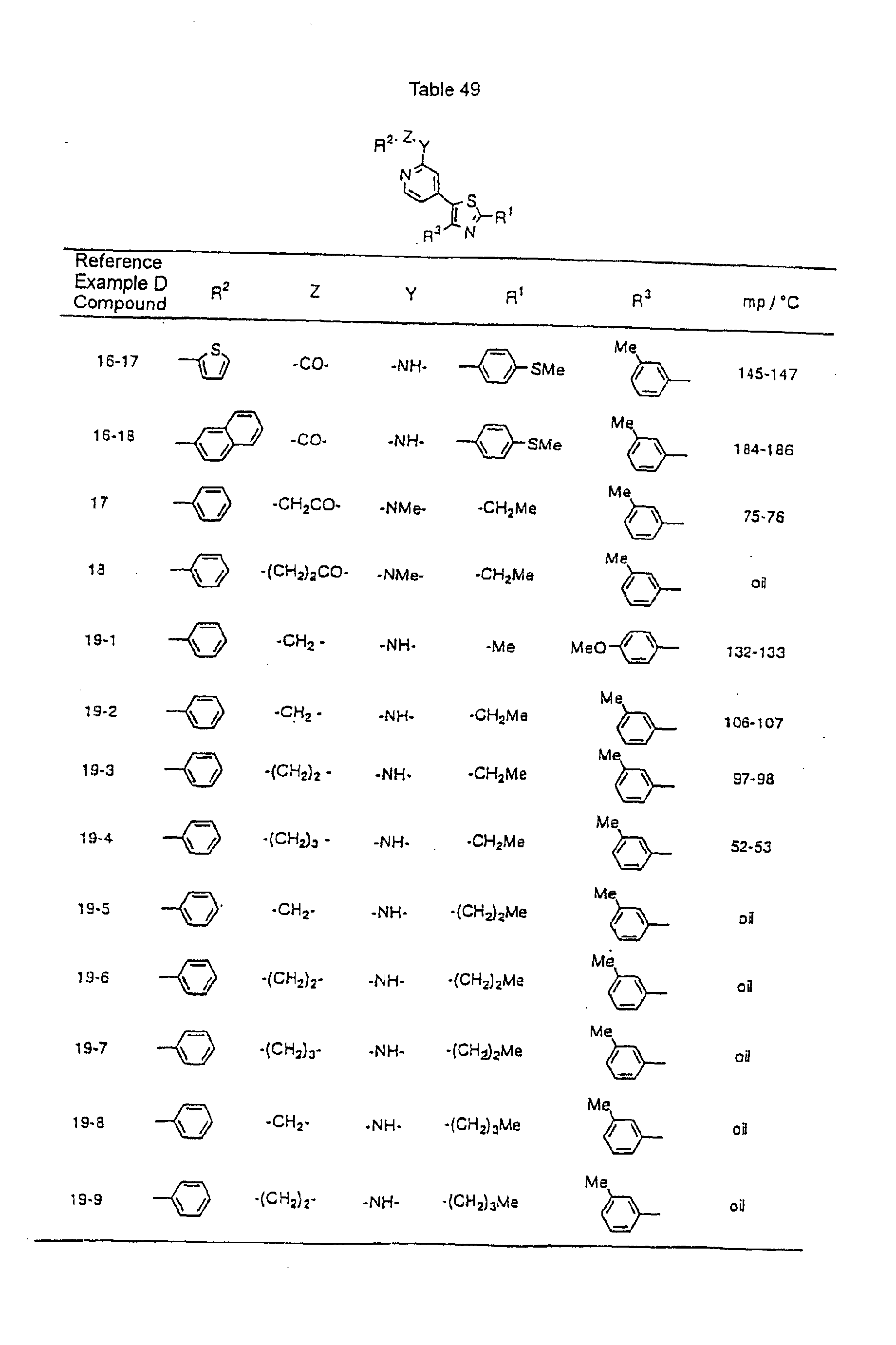

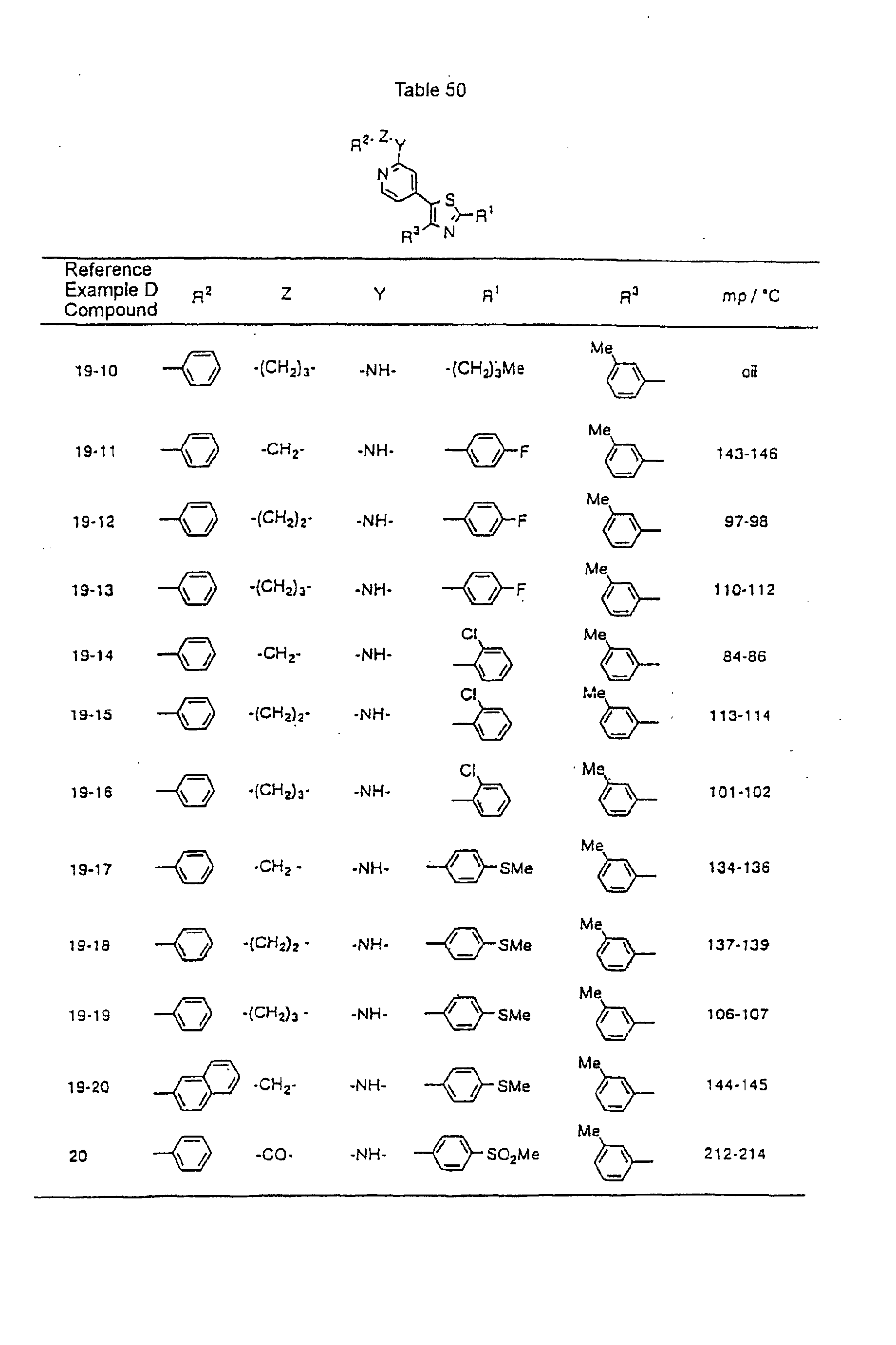

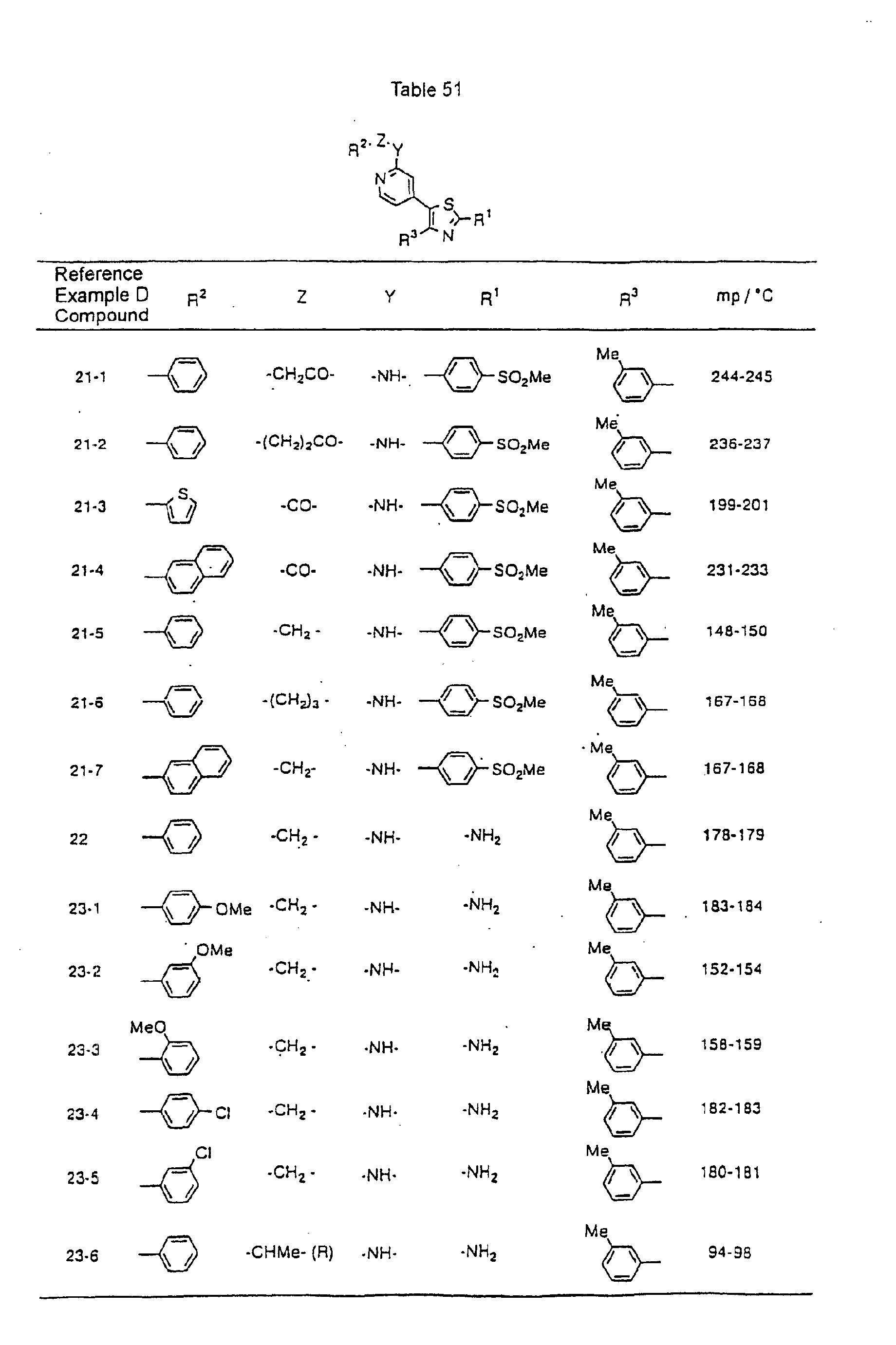

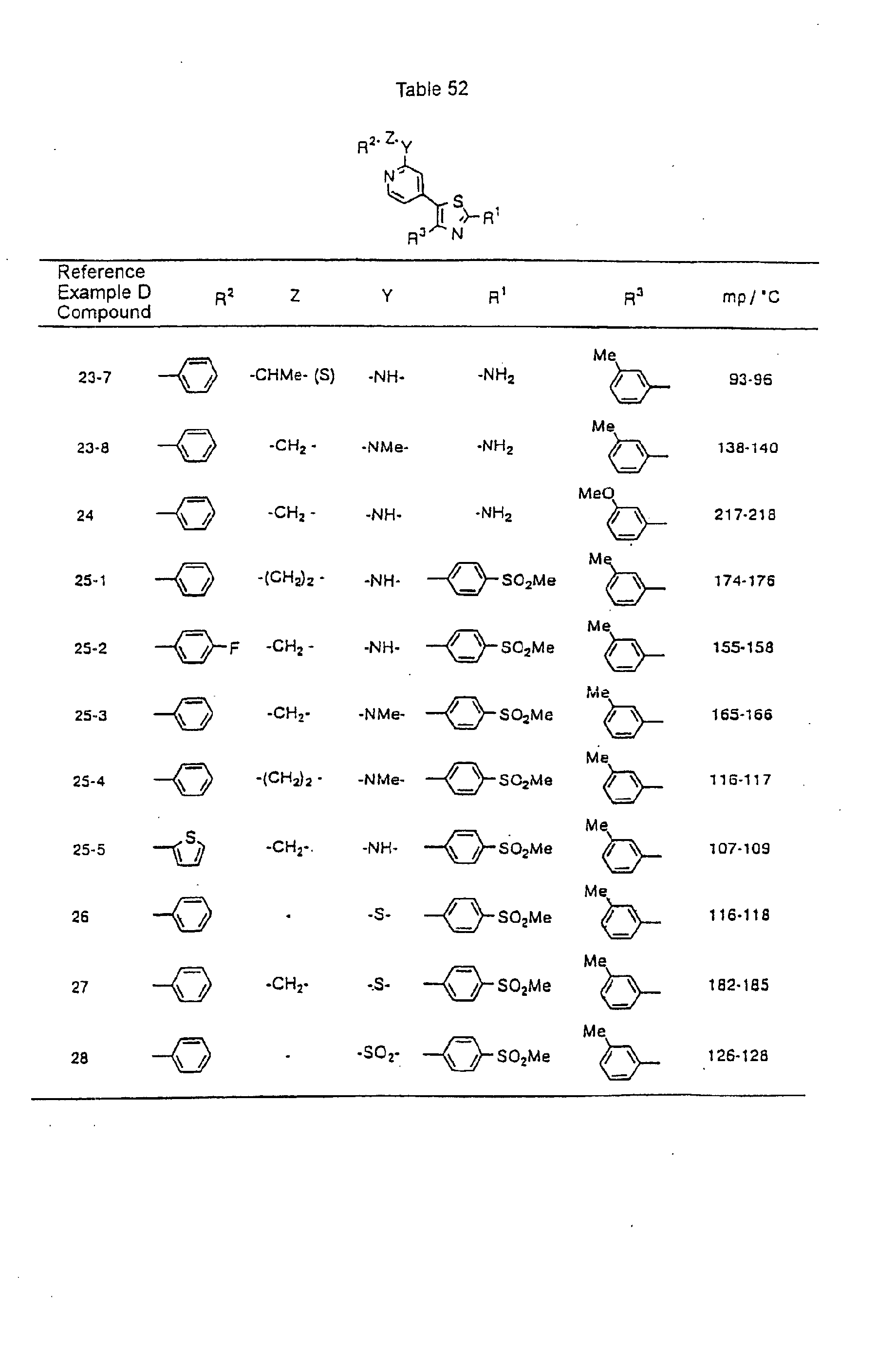

### Reference Example E1:

| | | |
|---|---|---|
| (1) | Compound of Reference Example D 1 | 50 mg |
| (2) | Lactose | 34 mg |
| (3) | Corn starch | 10.6 mg |
| (4) | Corn starch (pasty) | 5 mg |
| (5) | Magnesium stearate | 0.4 mg |
| (6) | Calcium carboxymethylcellulose | 20 mg |
| Total | | 120 mg |

According to conventional methods, the above (1) to (6) were mixed, compressed with a compressing machine to obtain tablets.

### Reference Example E2:

| | | |
|---|---|---|
| (1) | Reference Example D compound 16-1 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

10.0 mg of Reference Example D compound 16-1 and a mixture of 60.0 mg of lactose and 35.0 mg of corn starch were granulated by passing through a 1 mm mesh sieve using 0.03 ml of a 10% aqueous gelatin solution (3.0 mg as gelatin) and, thereafter, dried at 40°C and re-passed through a sieve. The granules thus obtained were mixed with 2.0 mg of magnesium stearate and compressed. The resulting core tablet is coated with a sugar coating of a suspension of sucrose, titanium dioxide, talc and arabic gum in water. The tablet coated with a coating is polished with beeswax to obtain a coated tablet.

### Reference Example E3:

| | | |
|---|---|---|
| (1) | Reference Example D compound 16-1 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

After 10.0 mg of Reference Example D compound 16-1 and 3.0 mg of magnesium stearate are granulated with 0.07 ml of an aqueous solution of soluble starch (7.0 mg as soluble starch), the granules are dried and mixed with 70.0 mg of lactose and 50.0 mg of corn starch. The mixture is compressed to obtain tablets.

### Reference Example E4:

| | | |
|---|---|---|
| (1) | Reference Example D compound 18 | 5.0 mg |
| (2) | Sodium chloride | 20.0 mg |
| (3) | Distilled water to total | 2.0 ml |

5.0 mg of Reference Example D compound 18 and 20.0 mg of sodium chloride are dissolved in distilled water and water is added to total 2.0 ml. The solution is filtered and filled into a 2 ml of ampoule under sterile conditions. After the ampoule is sterilized, it is sealed to obtain a solution for injection.

### Reference Example F1:

Genetic procedures were according to the methods described in Molecular Cloning, published by Cold Spring Harbor, Laboratory, 1989 or a method described in the attached protocol of the reagent.

### 1) Cloning of human adenosine A₃ receptor

Cloning of an adenosine A₃ receptor gene was performed from human brain cDNA by a PCR method. A PCR reaction was performed with a DNA thermal cycler 480 (Perkin Elmer) by using 1 ng of brain cDNA (Toyobo, QUICK-Clone cDNA) as a template, adding each 50 pmol of a primer set 5'-CGCCTCTAGACAAGATGCCCAACAACAGCACTGC-3' (SEQ ID NO:7) and 5'-CGGGGTCGACACTACTCAGAATTCTTCTCAATGC-3' (SEQ ID NO:8) made by reference to adenosine A₃ receptor gene base sequence reported by Salvatore et al. (Proc. Natl. Acad. Sci. U.S.A., 90:10365-10369, 1993) and employing Takara LA PCR Kit Ver.2 (Takara Shuzo) (reaction conditions: 35 cycles of 1 minute at 95°C, 1 minute at 66°C, 2 minutes at 75°C). The resulting PCR product was subjected to agarose gel electrophoresis and 1.0 kb of DNA fragment was recovered and, thereafter, an adenosine A₃ receptor gene was cloned using Original TA Cloning Kit (Funakoshi).

Next, the resulting plasmid was digested with a restriction enzyme XbaI (Takara Shuzo), treated with T4 DNA polymerase (Takara Shuzo) into end-blunted fragments and further digested with SalI (Takara Shuzo) to obtain adenosine A₃ receptor gene fragments.

### 2) Preparation of a plasmid for expressing of human adenosine A₃ receptor

A SRα promoter derived from pTB1411 described in JP-A 5-076385 was digested with BglII (Takara Shuzo), blunted, and ligated to EcoRI (Takara Shuzo)-digested pCI vector (Promega) with a DNA Ligation kit (Takara Shuzo) to make pCI-SRα. Next, this pCI-SRα was digested with ClaI (Takara Shuzo) and treated with T4 DNA polymerase (Takara Shuzo) to blunt-ended. On the other hand, after pGFP-C1 (Toyobo) was digested with Bsu36I (Daiichi Pure Chemicals), treated with T4 DNA polymerase (Takara Shuzo) to blunted end to obtain 1.63 kb of DNA fragment, and both were ligated with a DNA Ligation kit (Takara Shuzo) and competent cells of Escherichia coli JM109 were transformed to obtain the plasmid pMSRαneo.

Next, after pMSRαneo was digested with EcoRI (Takara Shuzo), treated with a T4 DNA polymerase (Takara Shuzo) to blunted end, and further digesting with SalI (Takara Shuzo) to obtain a 5.4 kb DNA fragment. The obtained DNA fragment and the fragments of adenosine A₃ receptor gene obtained in the above 1) were mixed, ligated with a DNA Ligation kit (Takara Shuzo) and competent cells of Escherichia coli JM109 (Takara Shuzo) were transformed to obtain the plasmid pA₃SR_{α}.

### 3) Introduction of a plasmid for expressing human adenosine A₃ receptor into CHO (dhfr-) cells and expression

CHO (dhfr-) cells obtained by culturing on Ham F12 medium (Nihonseiyaku) containing 10% bovine fetal serum (Lifetec Oriental) in a 750 ml tissue culture flask (Vecton Dickinson) were peeled with 0.5 g/L trypsin-0.2 g/L EDTA (Lifetec Oriental) and, thereafter, the cells were washed with PBS (Lifetec Oriental) and centrifuged (1000 rpm, 5 minutes), which was suspended in PBS.

Next, a DNA was introduced into cells using a gene pulser (BioRad) according to the following conditions. That is, 8×10⁶ cells and 10 µg of the plasmid pA₃SRα for expressing human adenosine A₃ receptor were added to 0.4 cm gapped cuvette and electroporation was performed with 0.8 ml volume, and under voltage 0.25 kV and capacitance 960 µF. Thereafter, the cells were transferred to Ham F12 medium containing 10% bovine fetal serum, cultured for 24 hours, the cells were peeled again and centrifuged, then, suspended in Ham F12 medium containing 10% bovine fetal serum to which Geneticin (Lifetec Oriental) had been added to 500 µg/ml, which was diluted to 10⁴ cells/ml to seed on a 96-well plate (Becton Dickinson) to obtain Geneticin-resistant strain.

Next, the resulting Geneticin-resistant strain was cultured on a 24 well-plate (Becton Dickinson) and, thereafter, an adenosine A₃ receptor expressing cell was selected among the resistant strains. That is, a reaction was conducted in an assay buffer I (HBSS (Wako Pure Chemicals) containing 0.1% BSA, 0.25 mM PMSF, 1µg/ml pepstatin and 20 µg/ml leupeptin) for 1 hour, washed with an assay buffer I, the radioactivity was measured with a γ-counter to select a cell to which a ligand is specifically bound, A₃AR/CHO strain.

### 4) Preparation of a cell membrane fraction of a cell for expressing adenosine A₃ receptor

After the A₃AR/CHO strain obtained in the above 3) was cultured in Ham F12 medium containing 10% bovine fetal serum for 2 days, the cells were peeled with 0.02% EDTA-containing PBS, the cells were recovered by centrifugation, suspended in an assay buffer II (50 mM Tris-hydrochloric acid (pH 7.5), 1mM EDTA, 10 mM magnesium chloride, 0.25 mM PMSF, 1 µg/mL pepstatin, 20 µg/ml leupeptin), and the cells were lysed by treating three times with a polytron homogenizer (Model PT-3000, KINEMATICA AG) at 20,000 rpm for 20 seconds. After the cells were ground, they were centrifuged at 20,000 rpm for 10 minutes to obtain the supernatant containing the membrane fraction. This supernatant was centrifuged with a supercentrifuge (Model L8-70M, rotor 70Ti, Beckmann) at 30,000 rpm for 1 hour to obtain the precipitates containing the membrane fraction.

Next, the precipitates were suspended in an assay buffer II containing 2 unit/ml adenosine deaminase (Boehringer Mannheim), treated at 30°C for 30 minutes and, thereafter, centrifuged again as described above to obtain the precipitates containing the membrane fraction.

### 5) Adenosine A₃ receptor binding test

On a 96 well-microplate, [³H]-NECA (Amersham) as a ligand was added to an assay buffer II containing the 100 µg/ml membrane fraction obtained in the above 4) and various concentrations of test compounds so that the concentration of the ligand was 10 nM, followed by reaction at room temperature for 1 hour. Then, the membrane fraction was transferred to unifilter GF/C (Packard) by filtering the reaction solution using Cell Harvester (Packard) and washed three times with 50 mM cooled Tris buffer (pH 7.5). After the filter was dried, Microscint 0 (Packard) was added to the filter, the radioactivity was measured with a TopCount (Packard) and the concentration (IC₅₀) of a test compound necessary for decreasing an amount of binding of [³H]-NECA to the membrane fraction by 50% was calculated with PRISM 2.01 (Graphpad Software).

As the result, the IC₅₀ value of the compound of Example 1 was 11.6 nM. It can be seen that Compound (I) is the excellent affinity for adenosine A₃ receptor.

### Reference Example F2:

The genetic manipulations described below were according to the methods described in the book (Maniatis et al., Molecular Cloning, Cold Spring Harbor Laboratory, 1989) or a method described in the protocol attached to the reagent.

### (1) Cloning of human p38 MAP kinase gene and preparation of recombinant Baculovirus

Cloning of human p38 MAP kinase gene was performed by a PCR method using a primer set P38-U:5'-ACCACTCGAGATGGACTACAAGGACGACGATGACAAGTCTCAGGAGAGGCCCACGTTCTACC -3' [SEQ ID NO:9] and PAG-L:5'-ACCCGGTACCACCAGGTGCTCAGGACTCCATCTCT-3' [SEQ ID NO:10] made by reference to the base sequence of p38 MAP kinase gene reported by Han et al. (Science 265 (5173), 808-811 (1994)) and employing kidney cDNA (Toyobo, QUICK-Clone cDNA) as a template.

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solution, and 10 µL sterile distilled water were mixed. As the upper mixed solution, 1 µL human cardiac cDNA (1 ng/mL) as a template, 3 µL 10×LA PCR Buffer, 1 µL 2.5 mM dNTP solution, 0.5 µL TaKaRa LA Taq DNA polymerase (Takara Shuzo), and 24.5 µL sterile distilled water were mixed. One AmpliWax PCR Gem 100 (Takara Shuzo) was added to the prepared lower mixed solution to treat at 70°C for 5 minutes and for 5 minutes in an ice and, thereafter, the upper mixed solution was added to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.1 kb DNA fragment containing p38 MAP kinase gene was recovered from the gel and, thereafter, which was inserted into pT7Blue-T vector (Takara Shuzo) to make the plasmid pHP38.

The 4.8 kb XhoI-KpnI fragment of the plasmid pFASTBAC1 (CIBCOBRL) and the 1.1 kb XhoI-Kpn fragment of the above plasmid pHP38 were ligated to make the plasmid pFBHP38.

The plasmid pFBHP38 and BAC-TO-BAC Baculovirus Expression System (GIBCOBRL) were used to prepare the recombinant Baculovirus virusstock BAC-HP38.

### (2) Cloning of human MKK3 gene and preparation of recombinant Baculovirus

Cloning of human MKK3 gene was performed by a PCR method using a primer set MKK-U:5'-ACAAGAATTCATAACATATGGCTCATCATCATCATCATCATTCCAAGCCACCCGCACCCAA-3' [SEQ ID NO:11] and MKK-L: 5'-TCCCGTCTAGACTATGAGTCTTCTCCCAGGAT-3' [SEQ ID NO:12] made by reference to the base sequence of MKK3 gene reported by Derijard, B. et al., Science 267 (5198), 682-685 (1995) and using kidney cDNA (Toyobo, QUICK-Clone cDNA).

A PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solution, and 10 µL sterile distilled water were mixed. As the upper mixed solution, 1 µL human kidney cDNA (1 ng/mL), 3 µL 10×LA PCR Buffer, 1 µL 2.5 mM dNTP solution, 0.5 µL TaKaRa LA taq DNA polymerase (Takara Shuzo) and 24.5 µL sterile distilled water were mixed. One AmpliWax PCR Gem 100 (Takara Shuzo) was added to the prepared lower mixed solution to treat at 70°C for 5 minutes and for 5 minutes in ice and, thereafter, the upper mixed solution was added to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.0 kb DNA fragment containing MKK3 gene was recovered from the gel and, thereafter, which was inserted into pT7Blue-T vector (Takara Shuzo) to make the plasmid pHMKK3.

In order to mutate MKK3 into a constitutive active form (from Ser to Glu at 189 position, from Thr to Glu at position 193), a primer set SER-U:5'-GGCTACTTGGTGGACGAGGTGGCCAAGGAGATGGATGCCGGCTGC-3' [SEQ ID NO:13] and SER-L:5'-GCAGCCGGCATCCATCTCCTTGGCCACCTCGTCCACCAAGTAGCC-3' [SEQ ID NO:14] was used to introduce a mutation by QuickChange Site-Directed Mutagenesis Kit (Stratagene), to obtain pcaMKK3.

4.8 kb EcoRI-XbaI fragment of the plasmid pFASTBAC1 (CIBCOBRL) and the 1.0 kb EcoRI-XbaI fragment of the above plasmid pcaMKK 3 were ligated to make the plasmid pFBcaMKK3.

The plasmid pFBcaMKK3 and BAC-TO-BAC Baculovirus Expression System (GIBCOBRL) were used to prepare the recombinant Baculovirus virusstock BAC-caMKK3.

### (3) Preparation of active form p38 MAP kinase

The Sf-21 cells were seeded on 100 ml Sf-900II SFM medium (GIBCOBRL) to 1×10⁶ cells/mL and cultured at 27°C for 24 hours. After each 0.2 mL of the virusstock BAC-HP38 of recombinant Baculovirus and BAC-caMKK3 were added, the culturing was further performed for 48 hours. After the cells were separated from the culturing solution by centrifugation (3000 rpm, 10 min), the cells were washed twice with PBS. After the cells were suspended in 10 ml Lysis buffer (25 mM HEPES (pH 7.5), 1% Triton X, 130 mM NaCl, 1 mM EDTA, 1 mM DTT, 25 mM β-glycerophosphate, 20 mM leupeptin, 1 mM APMSF, 1 mM Sodium orthovanadate), the cells were lysed by treating twice with a homogenizer (POLYTRON) at 20000 rpm for 2 minutes. By using Anti-FLAG M2 Affinity Gel (Eastman Chemical) from the supernatant obtained by centrifugation (40000 rpm, 45 minutes), active form p38 MAP kinase was purified.

### (4) Measurement of the p38 MAP kinase inhibitory activity

2.5 µL of a test compound dissolved in DMSO was added to 37.5 µL reaction solution (25 mM HEPES (pH 7.5), 10 mM Magnesium Acetate) containing 260 ng active form p38 MAP kinase and 1 µg Myelin Basic Protein, which was maintained at 30°C for 5 minutes. The reaction was initiated by adding 10 µL ATP solution (2.5 µM ATP, 0.1 µCi [g-³²P]ATP). After the reaction was performed at 30°C for 60 minutes, the reaction was stopped by adding 50 µL 20% TCA solution. After the reaction solution was allowed to stand at 0°C for 20 minutes, an acid insoluble fraction was transferred to GF/C filter (Packard Japan) using Cell Harvester (Packard Japan) and washed with 250 mM H₃PO₄. After drying at 45 °C for 60 minutes, 40 µM Microscint 0 (Packard Japan) was added and the radioactivity was measured with a TopCount (Packard Japan). The concentration (IC₅₀ value) necessary for inhibiting uptake of ³²P into an acid insoluble fraction by 50% was calculated with PRISM 2.01 (Graphpad Software).

The results are shown in Table 53.

**[Table 53]**

| Reference Example D No. | IC₅₀ (µM) |
|---|---|
| 1 | 0.43 |
| 2 | 0.063 |
| 3 | 0.023 |
| 4 | 0.020 |
| 5 | 0.029 |
| 6 | 0.023 |

From this, it can be seen that Compound (II) has the p38 MAP kinase inhibitory activity.

### Reference Example F3:

### Measurement of inhibiting activity of TNF-α production

After THP-1 cells which had been cultured in PRMI 1640 medium (manufactured by Life Technologies, Inc.) containing 1% non-activated bovine fetal serum (manufactured by Life Technologies, Inc., U.S.A.) and 10 mM HEPES (pH 7.5) seeded on a 96-well plate to 1×10⁵ cells/well, 1 µL test compound dissolved in DMSO was added to there. After incubation at 37°C for 1 hour in a CO₂ incubator, LPS (Wako Pure Chemicals) was added to the final concentration 5 µg/mL. After cultured at 37°C for 4 hours in a CO₂ incubator, the supernatant was obtained by centrifugation. The concentration of TNF-α in the supernatant was measured with ELISA (R&D System, Quantikine Kit). The concentration (IC₅₀ value) necessary for inhibiting TNF-α production by 50% was calculated by PRIMS 2.01 (Graphpad Software).

The results are shown in Table 54.

**[Table 54]**

| Reference Example D No. | IC₅₀ (µM) |
|---|---|
| 3 | 0.026 |
| 4 | 0.014 |
| 5 | 0.020 |
| 6 | 0.140 |

From this, it can be seen that Compound (II) has the excellent inhibitory activity of TNF-α production.

### Reference Example G1

| | | |
|---|---|---|
| (1) | Rofecoxib | 5.0 mg |
| (2) | Sodium chloride | 20.0 mg |
| (3) | Distilled water | to make the total amount 2.0 ml |

Rofecoxib (5.0 mg) and sodium chloride (20.0 mg) are dissolved in distilled water, to which water is added to make the total amount 2.0 ml. The solution is filtrated and filled in a 2 ml ampoule under aseptic conditions. The ampoule is sterilized and sealed to give a solution for injection.

### Reference Example G2

| | | |
|---|---|---|
| (1) | Rofecoxib | 50 mg |
| (2) | Lactose | 34 mg |
| (3) | Cornstarch | 10.6 mg |
| (4) | Cornstarch (paste) | 5 mg |
| (5) | Magnesium stearate | 0.4 mg |
| (6) | Calcium carboxymethyl cellulose | 20 mg |
| total | | 120 mg |

The above-mentioned (1)-(6) were mixed according to a conventional method and tableted with a tableting machine to give tablets.

### Example 1

Either the preparation produced in Reference Example B or that produced in Reference Example E is combined with the preparation of Reference Example G1.

### Industrial Applicability

The combination agent of the present invention is useful as a prophylactic or therapeutic agent of a disease such as rheumatism, arthritis etc. and other diseases.

This application is based on patent application Nos. 2000-396220 and 2001-27572 filed in Japan, the contents of which are hereby incorporated by reference.

### Sequence Listing Free Text

Sequence Listing SEQ ID NO:1 Oligonucleotide designed to act as a primer for PCR
Sequence Listing SEQ ID NO:2 Oligonucleotide designed to act as a primer for PCR
Sequence Listing SEQ ID NO:3 Oligonucleotide designed to act as a primer for PCR
Sequence Listing SEQ ID NO:4 Oligonucleotide designed to act as a primer for PCR
Sequence Listing SEQ ID NO:5 Oligonucleotide designed to act as a primer for PCR
Sequence Listing SEQ ID NO:6 Oligonucleotide designed to act as a primer for PCR
Sequence Listing SEQ ID NO:7 Oligonucleotide designed to act as a primer for PCR
Sequence Listing SEQ ID NO:8 Oligonucleotide designed to act as a primer for PCR
Sequence Listing SEQ ID NO:9 Oligonucleotide designed to act as a primer for PCR
Sequence Listing SEQ ID NO:10 Oligonucleotide designed to act as a primer for PCR
Sequence Listing SEQ ID NO:11 Oligonucleotide designed to act as a primer for PCR
Sequence Listing SEQ ID NO:12 Oligonucleotide designed to act as a primer for PCR
Sequence Listing SEQ ID NO:13 Oligonucleotide designed to act as a primer for PCR
Sequence Listing SEQ ID NO:14 Oligonucleotide designed to act as a primer for PCR

## Claims

1. A pharmaceutical agent comprising one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor in combination.

2. The pharmaceutical agent of claim 1, wherein the p38 MAP kinase inhibitor and/or the TNF-α production inhibitor are/is a 1,3-thiazole compound substituted at the 5-position by a pyridyl group optionally having substituents, or a salt thereof or a prodrug thereof.

3. The pharmaceutical agent of claim 1, wherein the p38 MAP kinase inhibitor and/or the TNF-α production inhibitor are/is a compound represented by the formula 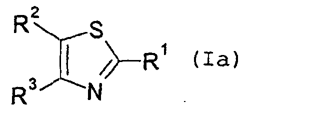 wherein
R¹ represents a hydrogen atom, a hydrocarbon group optionally having substituent(s), a heterocyclic group optionally having substituent(s), an amino group optionally having substituent(s) or an acyl group;
R² represents a pyridyl group optionally having substituent(s); and
R³ represents an aromatic group optionally having substituent(s), a salt thereof or a prodrug thereof.

4. The pharmaceutical agent of claim 1, wherein the p38 MAP kinase inhibitor and/or the TNF-α production inhibitor are/is an optionally N-oxidized compound represented by the formula: 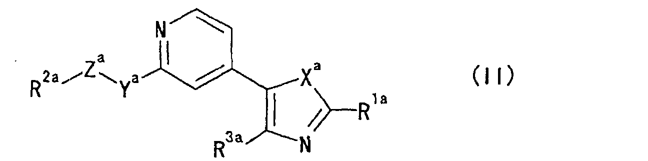 wherein R^{1a} represents a hydrogen atom, a hydrocarbon group optionally having substituents, a heterocyclic group optionally having substituents, an amino group optionally having substituents or an acyl group,
R^{2a} represents an aromatic group optionally having substituents,
R^{3a} represents a hydrogen atom, a pyridyl group optionally having substituents or an aromatic hydrocarbon group optionally having substituents,
X^{a} represents an oxygen atom or an optionally oxidized sulfur atom,
Y^{a} represents a bond, an oxygen atom, an optionally oxidized sulfur atom or a group represented by the formula: NR^{4a} (wherein R^{4a} represents a hydrogen atom, a hydrocarbon group optionally having substituents or an acyl group) and
Z^{a} represents a bond or a divalent acyclic hydrocarbon group optionally having substituents, or a salt thereof, or a prodrug thereof.

5. The pharmaceutical agent of claim 1, wherein the p38 MAP kinase inhibitor and/or the TNF-α production inhibitor are/is N-[5-(2-benzoylamino-4-pyridyl)-4-(3,5-dimethylphenyl)-1,3-thiazol-2-yl]acetamide,
N-[5-(2-benzylamino-4-pyridyl)-4-(3,5-dimethylphenyl)-1,3-thiazol-2-yl]acetamide,
N-[4-[4-(4-methoxyphenyl)-2-methyl-1,3-thiazol-5-yl]-2-pyridyl]benzamide,
N-[4-[2-(4-fluorophenyl)-4-(3-methylphenyl)-1,3-thiazol-5-yl]-2-pyridyl]phenylacetamide,
N-[4-[2-ethyl-4-(3-methylphenyl)-1,3-thiazol-5-yl]-2-pyridyl]phenylacetamide,
N-[4-[4-(3-methylphenyl)-2-propyl-1,3-thiazol-5-yl]-2-pyridyl]phenylacetamide,
N-[4-[2-butyl-4-(3-methylphenyl)-1,3-thiazol-5-yl]-2-pyridyl]phenylacetamide,
N-[4-[4-(3-methylphenyl)-2-(4-methylthiophenyl)-1,3-thiazol-5-yl]-2-pyridyl]phenylacetamide,
N-[4-[2-ethyl-4-(3-methylphenyl)-1,3-thiazol-5-yl]-2-pyridyl]benzamide,
N-[4-[2-ethyl-4-(3-methylphenyl)-1,3-thiazol-5-yl]-2-pyridyl]-3-phenylpropionamide,
N-[4-[2-ethyl-4-(3-methylphenyl)-1,3-thiazol-5-yl]-2-pyridyl]-3-(4-methoxyphenyl)propionamide,
N-[4-[2-ethyl-4-(3-methylphenyl)-1,3-thiazol-5-yl]-2-pyridyl]-4-phenylbutyramide,
N-[4-[4-(3-methylphenyl]-2-propyl-1,3-thiazol-5-yl]-2-pyridyl]benzamide,
N-[4-[4-(3-methylphenyl)-2-propyl-1,3-thiazol-5-yl]-2-pyridyl]-3-phenylpropionamide,
N-[4-[2-butyl-4-(3-methylphenyl)-1,3-thiazol-5-yl]-2-pyridyl]benzamide,
N-[4-[2-butyl-4-(3-methylphenyl)-1,3-thiazol-5-yl]-2-pyridyl]-3-phenylpropionamide,
N-[4-[2-(4-fluorophenyl)-4-(3-methylphenyl)-1,3-thiazol-5-yl]-2-pyridyl]benzamide,
N-[4-[2-(4-fluorophenyl)-4-(3-methylphenyl)-1,3-thiazol-5-yl]-2-pyridyl]-3-phenylpropionamide,
N-[4-[4-(3-methylphenyl)-2-(4-methylthiophenyl)-1,3-thiazol-5-yl]-2-pyridyl]benzamide,
N-[4-[4-(3-methylphenyl)-2-(4-methylthiophenyl)-1,3-thiazol-5-yl]-2-pyridyl]-3-phenylpropionamide,
N-benzyl-N-[4-[2-ethyl-4-(3-methylphenyl)-1,3-thiazol-5-yl]-2-pyridyl]amine,
N-[4-[2-ethyl-4-(3-methylphenyl)-1,3-thiazol-5-yl]-2-pyridyl]-N-(2-phenylethyl)amine,
N-[4-[2-ethyl-4-(3-methylphenyl)-1,3-thiazol-5-yl]-2-pyridyl]-N-(3-phenylpropyl)amine,
N-benzyl-N-[4-[4-(3-methylphenyl)-2-propyl-1,3-thiazol-5-yl]-2-pyridyl]amine,
N-[4-[4-(3-methylphenyl)-2-propyl-1,3-thiazol-5-yl]-2-pyridyl]-N-(2-phenylethyl)amine,
N-[4-[4-(3-methylphenyl)-2-propyl-1,3-thiazol-5-yl]-2-pyridyl]-N-(3-phenylpropyl)amine,
N-benzyl-N-[4-[2-butyl-4-(3-methylphenyl)-1,3-thiazol-5-yl]-2-pyridyl]amine,
N-[4-[2-butyl-4-(3-methylphenyl)-1,3-thiazol-5-yl]-2-pyridyl]-N-(2-phenylethyl)amine,
N-[4-[2-butyl-4-(3-methylphenyl)-1,3-thiazol-5-yl]-2-pyridyl]-N-(3-phenylpropyl)amine,
N-benzyl-N-[4-[4-(3-methylphenyl)-2-(4-methylthiophenyl)-1,3-thiazol-5-yl]-2-pyridyl]amine,
N-[4-[4-(3-methylphenyl)-2-(4-methylthiophenyl)-1,3-thiazol-5-yl]-2-pyridyl]-N-(2-phenylethyl)amine,
N-[4-[4-(3-methylphenyl)-2-(4-methylthiophenyl)-1,3-thiazol-5-yl]-2-pyridyl]-N-(3-phenylpropyl)amine,
N-[4-[4-(3-methylphenyl)-2-(4-methylsulfonylphenyl)-1,3-thiazol-5-yl]-2-pyridyl]benzamide,
N-[4-[4-(3-methylphenyl)-2-(4-methylsulfonylphenyl)-1,3-thiazol-5-yl]-2-pyridyl]phenylacetamide,
N-[4-[4-(3-methylphenyl)-2-(4-methylsulfonylphenyl)-1,3-thiazol-5-yl]-2-pyridyl]-3-phenylpropionamide,
N-benzyl-N-[4-[4-(3-methylphenyl)-2-(4-methylsulfonylphenyl)-1,3-thiazol-5-yl]-2-pyridyl]amine,
N-[4-[4-(3-methylphenyl)-2-(4-methylsulfonylphenyl)-1,3-thiazol-5-yl]-2-pyridyl]-N-(3-phenylpropyl)amine,
N-[4-[4-(3-methylphenyl)-2-(4-methylsulfonylphenyl)-1,3-thiazol-5-yl]-2-pyridyl]-N-(2-phenylethyl)amine,
N-(4-fluorobenzyl)-N-[4-[4-(3-methylphenyl)-2-(4-methylsulfonylphenyl)-1,3-thiazol-5-yl]-2-pyridyl]amine,
(S)-N-[4-(3-methylphenyl)-5-(2-(1-phenylethylamino)-4-pyridyl)-1,3-thiazol-2-yl]nicotinamide,
(R)-N-[4-(3-methylphenyl)-5-(2-(1-phenylethylamino)-4-pyridyl)-1,3-thiazol-2-yl]nicotinamide,
(S)-N-[4-(3-methylphenyl)-5-(2-(1-phenylethylamino)-4-pyridyl)-1,3-thiazol-2-yl]-2-methylnicotinamide,
(R)-N-[4-(3-methylphenyl)-5-(2-(1-phenylethylamino)-4-pyridyl)-1,3-thiazol-2-yl]-2-methylnicotinamide,
(S)-N-[4-(3-methylphenyl)-5-(2-(1-phenylethylamino)-4-pyridyl)-1,3-thiazol-2-yl]-2-chloronicotinamide,
(R)-N-[4-(3-methylphenyl)-5-(2-(1-phenylethylamino)-4-pyridyl)-1,3-thiazol-2-yl]-2-chloronicotinamide,
(S)-N-[4-(3-methylphenyl)-5-(2-(1-phenylethylamino)-4-pyridyl)-1,3-thiazol-2-yl]-2-methoxynicotinamide,
(R)-N-[4-(3-methylphenyl)-5-(2-(1-phenylethylamino)-4-pyridyl)-1,3-thiazol-2-yl]-2-methoxynicotinamide,
N-[5-(2-benzylamino-4-pyridyl)-4-(3-methylphenyl)-1,3-thiazol-2-yl]nicotinamide,
N-[5-(2-benzylamino-4-pyridyl)-4-(3-methylphenyl)-1,3-thiazol-2-yl]-2-methoxynicotinamide,
N-[5-(2-benzylamino-4-pyridyl)-4-(3-methylphenyl)-1,3-thiazol-2-yl]-2-chloronicotinamide,
N-[5-(2-benzylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]nicotinamide,
N-[5-(2-benzoylamino-4-pyridyl)-4-(3-methylphenyl)-1,3-thiazol-2-yl]-2-methylnicotinamide,
N-[5-(2-benzoylamino-4-pyridyl)-4-(3-methylphenyl)-1,3-thiazol-2-yl]-2-chloronicotinamide,
N-[5-(2-benzoylamino-4-pyridyl)-4-(3-methylphenyl)-1,3-thiazol-2-yl]-2-methoxynicotinamide,
(S)-N-(1-phenylethyl)-4-[2-ethyl-4-(3-methylphenyl)-1,3-thiazol-5-yl]-2-pyridylamine,
(R)-N-(1-phenylethyl)-4-[2-ethyl-4-(3-methylphenyl)-1,3-thiazol-5-yl]-2-pyridylamine,
(S)-N-(1-phenylethyl)-4-[4-(3-methylphenyl)-2-propyl-1,3-thiazol-5-yl]-2-pyridylamine,
(R)-N-(1-phenylethyl)-4-[4-(3-methylphenyl)-2-propyl-1,3-thiazol-5-yl]-2-pyridylamine,
(S)-N-(1-phenylethyl)-4-[2-butyl-4-(3-methylphenyl)-1,3-thiazol-5-yl]-2-pyridylamine,
(R)-N-(1-phenylethyl)-4-[2-butyl-4-(3-methylphenyl)-1,3-thiazol-5-yl]-2-pyridylamine,
(S)-N-(1-phenylethyl)-4-[4-(3-methylphenyl)-2-(4-methylthiophenyl)-1,3-thiazol-5-yl]-2-pyridylamine,
(R)-N-(1-phenylethyl)-4-[4-(3-methylphenyl)-2-(4-methylthiophenyl)-1,3-thiazol-5-yl]-2-pyridylamine,
(S)-N-(1-phenylethyl)-4-[4-(3-methylphenyl)-2-(4-methylsulfonylphenyl)-1,3-thiazol-5-yl]-2-pyridylamine,
(R)-N-(1-phenylethyl)-4-[4-(3-methylphenyl)-2-(4-methylsulfonylphenyl)-1,3-thiazol-5-yl]-2-pyridylamine,
(S)-N-(1-phenylethyl)-4-[2-(4-fluorophenyl)-4-(3-methylphenyl)-1,3-thiazol-5-yl]-2-pyridylamine,
(R)-N-(1-phenylethyl)-4-[2-(4-fluorophenyl)-4-(3-methylphenyl)-1,3-thiazol-5-yl]-2-pyridylamine, or a salt thereof.

6. The pharmaceutical agent of claim 1, wherein the p38 MAP kinase inhibitor and/or the TNF-α production inhibitor are/is a compound represented by the formula 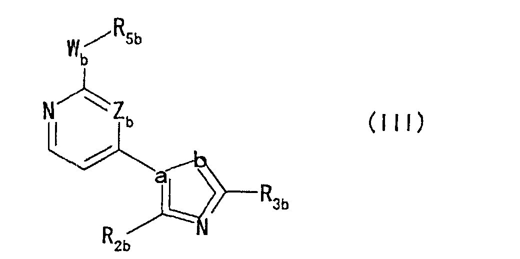 wherein
a is N or C;
b is CH when a is N, or O when a is C;
= denotes a single or a double bond dependent upon whether the azole ring is an imidazole or an oxazole ring;
Z_{b} is N or CH;
W_{b} is -NR_{6b}-Y_{b}-, -O- or -S-,
where R_{6b} is a hydrogen atom, C₁₋₄ alkyl group, C₃₋₈ cycloalkyl group, C₃₋₈ cycloalkyl-C₁₋₃ alkyl group, C₆₋₁₈ aryl group, C₃₋₁₈ heteroaryl group, C₇₋₁₉ aralkyl group or C₄₋₁₉ heteroaralkyl group, and -Y_{b}- is C₁₋₄ alkylene group or a bond;
R_{2b} is phenyl group, optionally substituted by one or more substituents selected from a halogen atom, trifluoromethyl, cyano, amido, thioamido, carboxylate, thiocarboxylate, C₁₋₄ alkoxy, C₁₋₄ alkyl, amino, and mono-or di-C₁₋₄ alkylamino;
R_{3b} is a hydrogen atom, a halogen atom, C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group or -CH=N-NH-C(NH)NH₂ (wherein C₁₋₁₀ alkyl group, C₂₋₄ alkenyl group, C₃₋₁₀ cycloalkyl group, C₃₋₁₈ heterocycloalkyl group, C₆₋₁₈ aryl group, C₃₋₁₈ heteroaryl group and -CH=N-NH-C(NH)NH₂ are each optionally substituted by 1 to 4 substituents selected from C₁₋₄ alkyl optionally substituted by hydroxy, halogen atom, halo-substituted-C₁₋₄ alkyl, hydroxy, C₁₋₄ alkoxy, C₁₋₄ alkylthio, carboxy, carbonyl optionally substituted by C₁₋₆ alkyl or C₁₋₆ alkoxy, amino, mono- or di-C₁₋₄ alkylamino and 5 to 7 membered N-heterocyclic group optionally further containing heteroatom(s));
R_{5b} is C₆₋₁₈ aryl group, C₃₋₁₈ heteroaryl group or C₃₋₁₂ cycloalkyl group each of which is optionally substituted by 1 to 4 substituents selected from C₁₋₄ alkyl, halogen, halo-substitued-C₁₋₄ alkyl, hydroxy, C₁₋₄ alkoxy, C₁₋₄ alkylthio, amino, mono- or di-C₁₋₄ alkylamino and 5 to 7 membered N-heterocyclic group optionally further containing heteroatom(s), or a salt thereof or a prodrug thereof.

7. The pharmaceutical agent of claim 1, which is a prophylactic or therapeutic agent of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation.

8. The pharmaceutical agent of claim 1, which is a prophylactic or therapeutic agent of chronic rheumatoid arthritis or osteoarthritis.

9. A method for the prophylaxis or treatment of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation, which comprises administration of an effective amount of one or more kinds of a p38 MAP kinase inhibitor and/or a TNF-α production inhibitor and an effective amount of one or more kinds of drugs selected from the group consisting of (1) a non-steroidal antiinflammatory drug, (2) a disease-modifying anti-rheumatic drug, (3) an anti-cytokine drug, (4) an immunomodulater, (5) a steroid and (6) a c-Jun N-terminal kinase inhibitor in combination to a mammal.

10. A method for the prophylaxis or treatment of chronic rheumatoid arthritis or osteoarthritis, which comprises administration of an effective amount of one or more kinds of a p38 MAP kinase inhibitor and/or a TNF-α production inhibitor and an effective amount of one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor in combination to a mammal.

11. Use of one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor for the production of a prophylactic or therapeutic agent of asthma, chronic obstructive pulmonary disease (COPD), allergic disease, inflammation, Addison's disease, autoimmune hemolytic anemia, systemic lupus erythematosus, Crohn's disease, psoriasis, rheumatism, cerebral hemorrhage, cerebral infarction, head trauma, spinal cord injury, brain edema, multiple sclerosis, Alzheimer's disease, Parkinson's disease, amyotrophic lateral sclerosis, diabetes, arthritis, osteoporosis, toxemia, Crohn's disease, ulcerative colitis, chronic pneumonia, pulmonary silicosis, pulmonary sarcoidosis, pulmonary tuberculosis, cachexia, arteriosclerosis, Creutzfeldt-Jakob disease, virus infection, atopic dermatitis, AIDS encephalopathy, meningitis, angina pectoris, cardiac infarction, congestive heart failure, chronic cardiac deficiency, acute myocardial infarction, prognosis of cardiac infarction, hypertension, acute cardiac deficiency, hepatitis, kidney failure, nephritis, malignant tumor, immunological rejection associated with transplantation, dialysis hypotension or disseminated intravascular coagulation.

12. Use of one or more kinds of a p38 MAP kinase inhibitor and/or a TNF-α production inhibitor and one or more kinds of drugs selected from the group consisting of (1) a non-steroidal antiinflammatory drug, (2) a disease-modifying anti-rheumatic drug, (3) an anti-cytokine drug, (4) an immunomodulator, (5) a steroid and (6) a c-Jun N-terminal kinase inhibitor for the production of a prophylactic or therapeutic agent of chronic rheumatoid arthritis or osteoarthritis.


Sequence Listing
<110> Takeda Chemical Industries, Ltd.

<120> Combined Composition

<130> 09433

<150> JP 2000-396220
<151> 2000-12-26

<150> JP 2001-27572
<151> 2001-02-02

<160> 14

<210> 1
<211> 62
<212> DNA
<213> Artificial Sequence

<220>
<223> Oligonucleotide designed to act as primer for PCR.

<400> 1
accactcgag atggactaca aggacgacga tgacaagtct caggagaggc ccacgttcta 60
cc 62

<210> 2
<211> 35
<212> DNA
<213> Artificial Sequence

<220>
<223> Oligonucleotide designed to act as primer for PCR.

<400> 2
acccggtacc accaggtgct caggactcca tctct 35

<210> 3
<211> 61
<212> DNA
<213> Artificial Sequence

<220>
<223> Oligonucleotide designed to act as primer for PCR.

<400> 3
acaagaattc ataacatatg gctcatcatc atcatcatca ttccaagcca cccgcaccca 60
a 61

<210> 4
<211> 32
<212> DNA
<213> Artificial Sequence

<220>
<223> Oligonucleotide designed to act as primer for PCR.

<400> 4
tcccgtctag actatgagtc ttctcccagg at 32

<210> 5
<211> 45
<212> DNA
<213> Artificial Sequence

<220>
<223> Oligonucleotide designed to act as primer for PCR.

<400> 5
ggctacttgg tggacgaggt ggccaaggag atggatgccg gctgc 45

<210> 6
<211> 45
<212> DNA
<213> Artificial Sequence

<220>
<223> Oligonucleotide designed to act as primer for PCR.

<400> 6
gcagccggca tccatctcct tggccacctc gtccaccaag tagcc 45

<210> 7
<211> 34
<212> DNA
<213> Artificial Sequence

<220>
<223> Oligonucleotide designed to act as primer for PCR.

<400> 7
cgcctctaga caagatgccc aacaacagca ctgc 34

<210> 8
<211> 34
<212> DNA
<213> Artificial Sequence

<220>
<223> Oligonucleotide designed to act as primer for PCR.

<400> 8
cggggtcgac actactcaga attcttctca atgc 34

<210> 9
<211> 62
<212> DNA
<213> Artificial Sequence

<220>
<223> Oligonucleotide designed to act as primer for PCR.

<400> 9

accactcgag atggactaca aggacgacga tgacaagtct caggagaggc ccacgttcta 60
cc 62

<210> 10
<211> 35
<212> DNA
<213> Artificial Sequence

<220>
<223> Oligonucleotide designed to act as primer for PCR.

<400> 10
acccggtacc accaggtgct caggactcca tctct 35

<210> 11
<211> 61
<212> DNA
<213> Artificial Sequence

<220>
<223> Oligonucleotide designed to act as primer for PCR.

<400> 11
acaagaattc ataacatatg gctcatcatc atcatcatca ttccaagcca cccgcaccca 60
a 61

<210> 12
<211> 32
<212> DNA
<213> Artificial Sequence

<220>
<223> Oligonucleotide designed to act as primer for PCR.

<400> 12
tcccgtctag actatgagtc ttctcccagg at 32

<210> 13

<211> 45
<212> DNA
<213> Artificial Sequence

<220>
<223> Oligonucleotide designed to act as primer for PCR.

<400> 13
ggctacttgg tggacgaggt ggccaaggag atggatgccg gctgc                45

<210> 14
<211> 45
<212> DNA
<213> Artificial Sequence

<220>
<223> Oligonucleotide designed to act as primer for PCR.

<400> 14
gcagccggca tccatctcct tggccacctc gtccaccaag tagcc                45